# EUROPÄISCHE PATENTSCHRIFT

(11) **EP 2 288 351 B1**
(45) Veröffentlichungstag und Bekanntmachung des Hinweises auf die Patenterteilung: **08.08.2018**
(21) Anmeldenummer: 09776608.3
(22) Anmeldetag: 13.05.2009
(51) Int. Cl.: A61K 31/33, A61P 35/00, C07D 471/04, C07D 473/34, C07D 487/04, C07D 513/04

(54) **NEUE PYRROLIDINDERIVATE ALS METAP-2 INHIBITOREN**
NOVEL PYRROLIDONE DERIVATIVES FOR USE AS METAP-2 INHIBITORS
NOUVEAUX DÉRIVÉS DE PYRROLIDINE UTILISÉS COMME INHIBITEURS DE LA METAP-2

(30) Priorität: 10.06.2008 DE 102008027574
(43) Veröffentlichungstag der Anmeldung: 02.03.2011
(73) Patentinhaber: Merck Patent GmbH, 64293 Darmstadt (DE)
(72) Erfinder: HEINRICH, Timo, 64823 Gross-Umstadt (DE); KRIER, Mireille, 64285 Darmstadt (DE); KNOECHEL, Thorsten, 64289 Darmstadt (DE); JONCZYK, Alfred, 64295 Darmstadt (DE); ZENKE, Frank, 64291 Darmstadt (DE); ENDERLE, Holger, 55437 Ockenheim (DE)
(86) Internationale Anmeldenummer: PCT/EP2009/003400
(87) Internationale Veröffentlichungsnummer: WO 2010/003475

(56) Entgegenhaltungen:
- WO-A1-2007/017069

## Beschreibung

Die Erfindung betrifft einzelne Verbindungen gemäß Anspruch 1 umfaßt von der Formel I 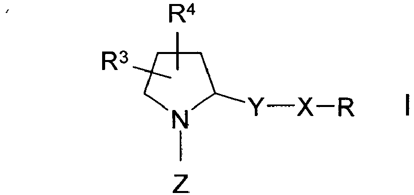 worin
- Z: 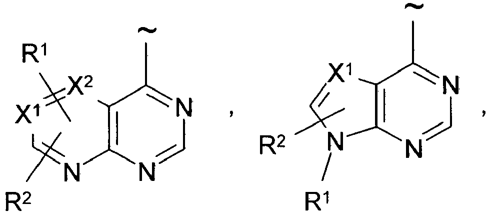 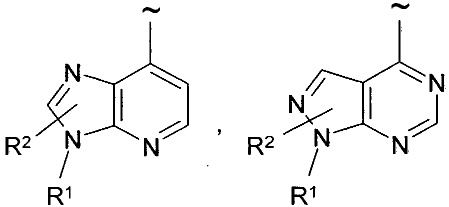 oder

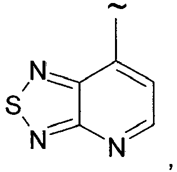

- R¹, R²: jeweils unabhängig voneinander H, A, Hal, NH₂, (CH₂)ₘHet², (CH₂)ₘCOOR⁶ oder (CH₂)ₘCONH₂,
- X¹: CH oder N,
- X²: CH oder N,
- R³, R⁴: jeweils unabhängig voneinander H, Hal, OH oder NH₂,
- R⁶: H oder Alkyl mit 1-6 C-Atomen,
- X: O, NH, NA, OC(=O) oder fehlt,
- Y: CH=CH oder (CH₂)ₙ,
- R: Ar, Het oder Carb¹,
- Ar: unsubstituiertes oder ein-, zwei-, drei-, vier- oder fünffach durch Hal, A, OR⁶, N(R⁶)₂, NO₂, CN, COOR⁶, CON(R⁶)₂, NR⁶COA, NR⁶SO₂A, COR⁶, SO₂N(R⁶)₂, S(O)_{q}A, SO₂OH, CH=CH-CONH(CH₂)ₚOH, NHCONH-Het, NHCONHA, (CH₂)ₘAr¹, O(CH₂)ₘAr¹, O(CH₂)ₘHet², O(CH₂)ₘCOOR⁶, NHCONH-(CH₂)ₘ 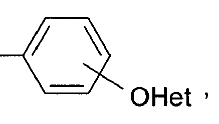 (CH₂)ₘHet, CH₂NH[(CH₂)₂O]_{q}[(CH₂)₂O]_{q}(CH₂)ₚNH₂, CH₂N(COA)CH₂CH(OH)CH₂OH, CH₂NH(CH₂)_{q}Het, CH₂N(COA)(CH₂)_{q}Het, CH₂N(CHO)(CH₂)_{q}Het, COHet, NHCOCH[(CH₂)ₘCOOA]NHCOO(CH₂)ₘAr¹, NHCOCH[(CH₂)ₘCONH₂]NHCOOA, NHCOCH[(CH₂)ₘCOOH]NHCOOH, NHCOCH[(CH₂)ₘHet²]NHCOOA, NHCOCH[(CH₂)ₘHet²]NH₂, NHCOCH[(CH₂)ₘCONH₂]NH₂, CH=CH-COOR⁶ und/oder CH=CH-CON(R⁶)₂ substituiertes Phenyl, Naphthyl oder Biphenyl,
- Het: einen unsubstituierten oder ein-, zwei- oder dreifach durch Hal, A, (CH₂)ₘOR⁶, N(R⁶)₂, NO₂, (CH₂)ₘCN, (CH₂)ₘCOOR⁶, CONH(CH₂)ₘCOOH, CONH(CH₂)ₘHet², CO(CH₂)ₘNH(CH₂)ᵣCOOA, COO(CH₂)ₘAr¹, (CH₂)ᵣCONH(CH₂)ₘAr¹, COCH[(CH₂)ₘCONH₂]NH₂, COCH[(CH₂)ₘCONH₂]NHCOOA, COCH[(CH₂)ₘHet²]NHCOOA, COCH[(CH₂)ₘHet²]NH₂, COCH[(CH₂)ₘNHCOOA]NHCOO(CH₂)ₘAr¹, COCH[(CH₂)ₘNH₂]NHCOO(CH₂)ₘAr¹, CO(CH₂)ₘN(R⁶)₂, NR⁶COA, NR⁶SO₂A, COR⁶, SO₂NR⁶, S(O)_{q}A, NHCONH-(CH₂)ₘ-Cyc-OR⁶, CONH(CH₂)ₚOR⁶, O(CH₂)ₚOR⁶, CHO, (CH₂)ₘHet², COHet², (CH₂)ᵣNH(CH₂)ₘHet², (CH₂)ₘNH(CH₂)ₘAr¹, NH(CH₂)ₚN(R⁶)₂, (CH₂)ₘAr¹, O(CH₂)ₘAr¹ und/oder =O (Carbonylsauerstoff) substituierten ein-, zwei- oder dreikernigen gesättigten, ungesättigten oder aromatischen Heterocyclus mit 1 bis 4 N-, und/oder O- und/oder S-Atomen, und worin ein N auch oxidiert sein kann,
- Cyc: Cycloalkylen mit 3-7 C-Atomen,
- Ar¹: unsubstituiertes oder ein-, zwei-, drei-, vier- oder fünffach durch Hal, A, OR⁶, COOR⁶, CON(R⁶)₂, NR⁶COA und/oder CONH(CH₂)ₚHet² substituiertes Phenyl,
- Carb¹: 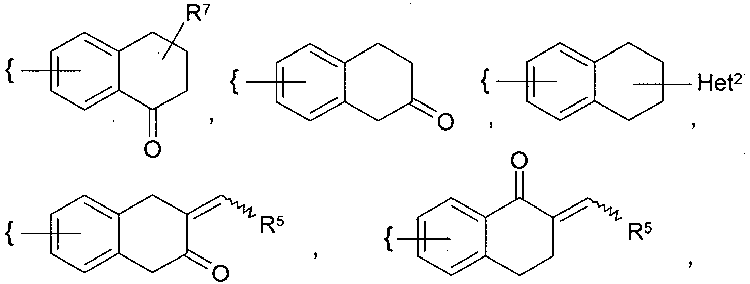 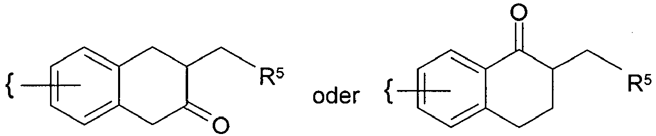
- R⁵: OR⁶, COOR⁶, CON(R⁶)₂ oder Het¹,
- R⁷: (CH₂)ᵣCON(R⁶)₂, (CH₂)ᵣCON[(CH₂CH₂)OH]₂ oder (CH₂)ᵣCONH(CH₂CH₂)OH,
- Het¹: Imidazolyl, Pyrazolyl oder 4-Chlor-2-methyl-pyrazolyl,
- Het²: einen unsubstituierten oder ein-, zwei- oder dreifach durch Hal, A, OR⁶, NHCOA, N(R⁶)₂ und/oder =O (Carbonylsauerstoff) substituierten einkernigen aromatischen oder gesättigten Heterocyclus mit 1 bis 2 N-, und/oder O- und/oder S-Atomen,
- A: unverzweigtes oder verzweigtes Alkyl mit 1-10 C-Atomen, worin 1-7 H-Atome durch F, Cl, Br und/oder OH ersetzt sein können,
oder
cyclisches Alkyl mit 3-7 C-Atomen,
- Hal: F, Cl, Br oder I,
- m: 0, 1, 2, 3, 4, 5 oder 6,
- n: 1 oder 2,
- p: 1, 2, 3 oder 4,
- q: 0, 1, 2, 3 oder 4,
- r: 0, 1 oder 2
bedeuten,
sowie ihre pharmazeutisch verwendbaren Salze, Tautomere und Stereoisomere, einschließlich deren Mischungen in allen Verhältnissen.

Der Anmeldungsgegenstand bezieht sich auf die Definition der Ansprüche; jede Offenbarung, die über den Umfang der Ansprüche hinausgeht, dient nur zu Informationszwecken.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die beanspruchten Verbindungen der Formel I und ihre Salze bei guter Verträglichkeit sehr wertvolle pharmakologische Eigenschaften besitzen.

Insbesondere zeigen sie eine regulatorische, modulatorische und/oder inhibierende Wirkung auf Metallproteasen, vorzugsweise auf die Methionin-Amino-Peptidase (MetAP), besonders auf den Subtyp MetAP-2.
Sie können als Arzneimittel gegen Krebs aber auch als Arzneimittel, die den Fett-Stoffwechsel positiv beeinflussen, aber auch als Arzneimittel gegen Entzündungen verwendet werden.

Andere Purinderivate zur Krebsbekämpfung sind in der WO 2007/017069 offenbart.

In der WO 01/79157 sind substituierte Hydrazide und N-Alkoxyamide beschrieben, die MetAP-2 inhibitorische Aktivität aufweisen und zur Inhibierung von Angiogenese verwendet werden können, insbesondere zur Behandlung von Krankheiten, wie z.B. Krebs, deren Entwicklung von Angiogenese abhängt.
In der WO 02/081415 sind MetAP-2 Inhibitoren beschrieben, die zur Behandlung von Krebs, Hämangiom, proliferativer Retinopathie, rheumatoider Arthritis, atherosklerotischer Neovaskularisation, Psoriasis, okularer Neovaskularisation und Fettleibigkeit verwendet werden können.
In der WO 2008/011114 sind Verbindungen als Angiogenese-Inhibitoren und MetAP-2-Inhibitoren beschrieben, die zur Behandlung von lymphoider Leukämie und Lymphom verwendet werden können.

Die Wirkung der erfindungsgemäßen Verbindungen gegen Krebs liegt besonders in ihrer Wirkung gegen Angiogenese. Angiogenese-Hemmung hat sich bei über 70 Krankheiten als hilfreich erwiesen, wie z. B. Eierstockkrebs (F. Spinella et al. J. Cardiovasc. Pharmacol. 2004, 44, S140), Brustkrebs (A. Morabito et al. Crit. Rev. Oncol./Hematol. 2004, 49, 91), Prostatakrebs (B. Nicholson et al. Cancer Metastas. Rev. 2001, 20, 297), diabetische Erblindung, Schuppenflechte und Makuladegeneration (E. Ng et al. Can. J. Ophthalmol. 2005, 23, 3706).

Proteasen regulieren viele unterschiedliche Zell-Prozesse, besonders die Modulation von Peptiden und Proteinen, besonders den Protein-Umsatz, die Protein-Reifung und Signalpeptid-Prozessierung, den Abbau von anormalen Proteinen sowie die In-/Aktivierung von regulatorischen Proteinen. Besonders die Amino-terminale Modifikation von naszierenden Polypeptiden stellt die häufigste Modulation dar. Aminoproteasen sind Metalloproteasen, die Aminosäuren vom ungeschützten N-Terminus von Peptiden oder Proteinen abspalten, was sowohl co- als auch posttranslatorisch erfolgen kann. Methionin Aminopeptidase (MetAP) spaltet terminales Methionin naszierender Peptide besonders, wenn die vorletzte Aminosäure klein und ungeladen ist (z. B. Gly, Ala, Ser, Thr, Val, Pro oder Cys).

Bei vielen Krankheitsprozessen steht die Angiogenese entweder ursächlich im Mittelpunkt der Erkrankung oder wirkt sich verschlimmernd auf die Progression der Erkrankung aus. Beispielsweise im Krebsgeschehen führt die Angiogenese dazu, dass der Tumor sich vergrößern und in andere Organe übertreten kann. Weitere Erkrankungen, bei denen Angiogenese eine wichtige Rolle spielt sind Psoriasis, Arthrose, Arteriosklerose sowie Augenerkrankungen wie diabetische Retinopathie, altersbedingte makulare Degeneration, Rubeosis iridis oder neovasculäres Glaukom, ferner bei Entzündungen. Die dieser Erfindung zugrunde liegenden Verbindungen der Formel I, Zusammensetzungen, die diese Verbindungen enthalten, sowie die beschriebenen Verfahren können somit zur Behandlung dieser Krankheiten eingesetzt werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome der Lungen, des Pankreas, der Schilddrüse, der Harnblase oder des Kolons, myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen anticancerogene Wirkung aufweisen. Die erfindungsgemäßen Verbindungen werden an einen Patienten mit einer Erkrankung verabreicht, z. B. zur Inhibierung des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es wurde gefunden, dass die erfindungsgemäßen Verbindungen eine spezifische Inhibierung der MetAP-2 bewirken. Die erfindungsgemäßen Verbindungen zeigen bevorzugt eine vorteilhafte biologische Aktivität, die in den, zum Beispiel hierin beschriebenen Tests nachweisbar ist. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Unter Verbindungen der Formel I versteht man auch die Hydrate und Solvate dieser Verbindungen.
Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I gemäß Anspruch 1, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I gemäß Anspruch 1 und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man
eine Verbindung der Formel II 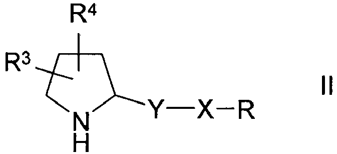 worin R³, R⁴, X, Y und R die in Anspruch 1 angegebenen Bedeutungen haben,
mit einer Verbindung der Formel III

Z-Cl III

worin Z die in Anspruch 1 angegebene Bedeutung hat,
umsetzt,
und/oder eine Base oder Säure der Formel I in eines ihrer Salze umwandelt.

Vor- und nachstehend haben die Reste R, X, Y, Z, R³ und R⁴ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1-, 1,2-, 1,3-, 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

R¹ bedeutet H, A, HaI, NH₂, (CH₂)ₘHet², (CH₂)ₘCOOR⁶ oder (CH₂)ₘCONH₂. R² bedeutet vorzugsweise H.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonyl-phenyl, o-, m- oder p-Ethoxycarbonylphenyl, o-, m- oder p-Acetylphenyl, o-, m- oder p-Formylphenyl, o-, m- oder p-Cyanphenyl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl; ferner Naphthyl oder Biphenyl.

Ar bedeutet weiterhin vorzugsweise unsubstituiertes oder ein-, zwei-, drei-, vier- oder fünffach durch HaI, A, OR⁶, N(R⁶)₂, NO₂, CN, COOR⁶, CON(R⁶)₂, NR⁶COA, NR⁶SO₂A, COR⁶, SO₂N(R⁶)₂, S(O)_{q}A, SO₂OH, CH=CH-CONH(CH₂)ₚOH, NHCONH-Het, NHCONHA, (CH₂)ₘAr¹, O(CH₂)ₘAr¹, O(CH₂)ₘHet², O(CH₂)ₘCOOR⁶,
NHCONH-(CH₂)ₘ 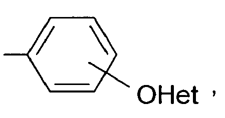 (CH₂)ₘHet, CH₂NH[(CH₂)₂O]_{q}[(CH₂)₂O]_{q}(CH₂)ₚNH₂,
CH₂N(COA)CH₂CH(OH)CH₂OH, CH₂NH(CH₂)_{q}Het, CH₂N(COA)(CH₂)_{q}Het, CH₂N(CHO)(CH₂)_{q}Het, COHet, NHCOCH[(CH₂)ₘCOOA]NHCOO(CH₂)ₘAr¹, NHCOCH[(CH₂)ₘCONH₂]NHCOOA, NHCOCH[(CH₂)ₘCOOH]NHCOOH, NHCOCH[(CH₂)ₘHet²]NHCOOA, NHCOCH[(CH₂)ₘHet²]NH₂, NHCOCH[(CH₂)ₘCONH₂]NH₂, CH=CH-COOR⁶ und/oder CH=CH-CON(R⁶)₂ substituiertes Phenyl, Naphthyl oder Biphenyl.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Unsubstituiertes Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl oder 2,3-Dihydro-2-oxo-furanyl.

Het bedeutet weiterhin vorzugsweise einen unsubstituierten oder ein-, zwei- oder dreifach durch HaI, A, OR⁶, N(R⁶)₂, NO₂, (CH₂)ₘCN, (CH₂)ₘCOOR⁶, CONH(CH₂)ₘCOOH, CONH(CH₂)ₘHet², CO(CH₂)ₘNH(CH₂)ᵣCOOA, COO(CH₂)ₘAr¹, (CH₂)ᵣCONH(CH₂)ₘAr¹, COCH[(CH₂)ₘCONH₂]NH₂, COCH[(CH₂)ₘCONH₂]NHCOOA, COCH[(CH₂)ₘHet²]NHCOOA, COCH[(CH₂)ₘHet²]NH₂, COCH[(CH₂)ₘNHCOOA]NHCOO(CH₂)ₘAr¹, COCH[(CH₂)ₘNH₂]NHCOO(CH₂)ₘAr¹, CO(CH₂)ₘN(R⁶)₂, NR⁶COA, NR⁶SO₂A, COR⁶, SO₂NR⁶, S(O)_{q}A, NHCONH-(CH₂)ₘ-Cyc-OR⁶, CONH(CH₂)ₚOR⁶, O(CH₂)ₚOR⁶, CHO, (CH₂)ₘHet², COHet², (CH₂)ᵣNH(CH₂)ₘHet², (CH₂)ₘNH(CH₂)ₘAr¹, NH(CH₂)ₚN(R⁶)₂, (CH₂)ₘAr¹, O(CH₂)ₘAr¹ und/oder =O (Carbonylsauerstoff) substituierten ein-, zwei- oder dreikernigen gesättigten, ungesättigten oder aromatischen Heterocyclus mit 1 bis 4 N-, und/oder O- und/oder S-Atomen,
und worin ein N auch oxidiert sein kann,
Het bedeutet besonders bevorzugt unsubstituiertes oder ein-, zwei- oder dreifach durch HaI, A, (CH₂)ₘOR⁶, N(R⁶)₂, NO₂, (CH₂)ₘCN, (CH₂)ₘCOOR⁶, CONH(CH₂)ₘCOOH, CONH(CH₂)ₘHet², CO(CH2)mNH(CH2)rCOOA, COO(CH₂)ₘAr¹, (CH₂)ᵣCONH(CH₂)ₘAr¹, COCH[(CH₂)ₘCONH₂]NH₂, COCH[(CH₂)ₘCONH₂]NHCOOA, COCH[(CH₂)ₘHet²]NHCOOA, COCH[(CH₂)ₘHet²]NH₂, COCH[(CH₂)ₘNHCOOA]NHCOO(CH₂)ₘAr¹, COCH[(CH₂)ₘNH₂]NHCOO(CH₂)ₘAr¹, CO(CH₂)ₘN(R⁶)₂, NR⁶COA, NR⁶SO₂A, COR⁶, SO₂NR⁶, S(O)_{q}A, NHCONH-(CH₂)ₘ-Cyc-OR⁶, CONH(CH₂)ₚOR⁶, O(CH₂)ₚOR⁶, CHO, (CH₂)ₘHet², COHet², (CH₂)ᵣNH(CH₂)ₘHet², (CH₂)ₘNH(CH₂)ₘAr¹, NH(CH₂)ₚN(R⁶)₂, (CH₂)ₘAr¹, O(CH₂)ₘAr¹ und/oder =O (Carbonylsauerstoff) substituiertes Pyridazinyl, Pyrazolyl, Benzimidazolyl, Pyridyl, Dibenzofuranyl, Carbazolyl, Indolyl, Dihydro-Indolyl, Benzofuranyl, Dihydro-benzofuranyl, Piperazinyl, Morpholinyl, Chinolinyl, Isochinolinyl, Dihydrochinolinyl, Dihydroisochinolinyl, Tetrahydrochinolinyl, Tetrahydroisochinolinyl, Chinazolinyl, Chinoxalinyl, Phthalazinyl, Purinyl, Naphthyridinyl, Pyrimidinyl, Indazolyl, Furyl, Thienyl, Pyrrolyl, Oxazolyl, Isoxazolyl, Thiazolyl, Triazolyl, Tetrazolyl, Thiadiazol, Benzothiazolyl,
Imidazo[1,2-a]pyridinyl, 1,3-Benzodioxolyl oder Benzoxazolyl,
und worin ein N auch oxidiert sein kann,

Cyc bedeutet vorzugsweise Cyclobutylen,Cyclopentylen oder Cyclohexylen.

Z bedeutet vorzugsweise 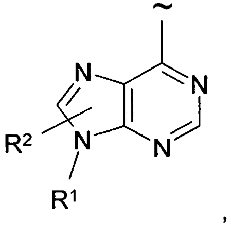 ferner 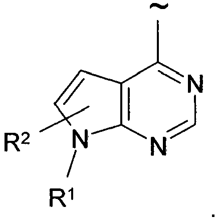 Z bedeutet weiterhin bevorzugt 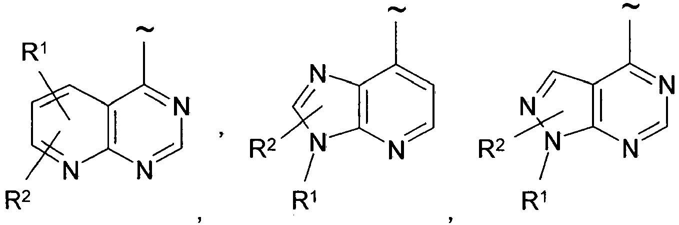 oder

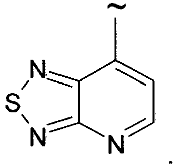

R bedeutet Ar, Het oder Carb¹, vorzugsweise Het oder Carb¹.

Het² bedeutet vorzugsweise unsubstituiertes oder ein- oder zweifach durch unsubstituiertes oder ein- oder zweifach durch A, OR⁶, NHCOA und/oder =O (Carbonylsauerstoff) substituiertes Furyl, Thienyl, Pyrrolyl, Oxazolyl, Isoxazolyl, Thiazolyl, Imidazolyl, Pyridyl, Pyrimidinyl, Piperazinyl, Morpholinyl, Piperidinyl, Pyrrolidinyl, Azetidinyl, Dihydrofuranyl oder Tetrahydrofuranyl.

HaI bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind. Die Verbindungen der Formel I gemäß Anspruch 1 können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II mit einer Verbindung der Formel III umsetzt. Die Verbindungen der Formel II und der Formel III sind in der Regel bekannt. Sind sie neu, so können sie aber nach an sich bekannten Methoden hergestellt werden.

Die Umsetzung erfolgt in einem inerten Lösungsmittel und erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -15° und 150°, normalerweise zwischen 40° und 130°, besonders bevorzugt zwischen 60° und 110°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt sind Glykolether, wie Ethylenglycolmonomethylether, THF, Dichlormethan und/oder DMF.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I gemäß Anspruch 1 die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium, sowie die Erdalkalimetalsalze Calcium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkyl-halogeniden, z.B. Decyl-, Dodecyl-, Lauryl-, Myristyl- und Stearylchlorid, -bromid und -iodid; sowie Aryl-(C₁-C₄)Alkylhatogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinyl-pyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Salze und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Salze und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung und Bekämpfung von Krankheiten. Zu diesen Krankheiten zählen die Proliferation von Tumorzellen, die pathologische Gefäßneubildung (oder Angiogenese), die das Wachstum fester Tumoren fördert, die Gefäß neubildung im Auge (diabetische Retinopathie, altersbedingte Makula-Degeneration und dergleichen) sowie Entzündung (Schuppenflechte, rheumatoide Arthritis und dergleichen), sowie proliferative Erkrankungen der Mesangiumzellen.

Die vorliegende Erfindung umfasst die Verwendung der Verbindungen der Formel I gemäß Anspruch 1 und/oder ihre physiologisch unbedenklichen Salze und Solvate zur Herstellung eines Arzneimittels zur Behandlung oder Vorbeugung von Tumoren, Tumorerkrankungen und/oder Tumormetastasen. Die Tumorerkrankung ist vorzugsweise ausgewählt aus der Gruppe
Tumor des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung von Osteoporose, Diabetes und Fettleibigkeit.

Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die angiogene Erkrankung ist vorzugsweise ausgewählt aus der Gruppe diabetische Retinopathie, Arthritis, Krebs, Psoriasis, Kaposi Sarkom, Hemangioma, myocardiale Angiogenesis, atherosklerotische Plaque-Neovaskularisation, angiogene Augenerkrankungen, choroidale Neovaskularisation, retrolentale Fibroplasie, makulare Degeneration, corneale Transplantatabstossung, Rubeosis iridis, neurosculares Glaukom, Oster Webber Syndrom.

Die proliferative Erkrankung der Mesangiumzellen ist vorzugsweise ausgewählt aus der Gruppe
Glomerulonephritis, diabetische Nephropathie, maligne Nephrosclerose, thrombotisches Mikroangiopathie-Syndrom, Transplantatabstossung, Glomerulopathie.

Die Verwendung von Verbindungen der Formel I gemäß Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Die inflammatorische Erkrankung ist vorzugsweise ausgewählt aus der Gruppe
entzündliche Darmerkrankung, Arthritis, Atherosclersose, Asthma, Allergien, entzündliche Nierenerkrankungen, multiple Sklerose, chronisch obstruktive Lungenerkrankung, entzündliche Hauterkrankungen, Pardontalerkrankungen, Psoriasis,
durch T-Zellen - vermittelte Immunerkrankung.

Die entzündliche Darmerkrankung ist vorzugsweise ausgewählt aus der Gruppe
ulcerative Colitis, Morbus Crohn, unbestimmte Colitis.

Die durch T-Zellen - vermittelte Immunerkrankung ist vorzugsweise ausgewählt aus der Gruppe
allergische Encephalomyelitis, allergische Neuritis, Transplantatabstossung, Graft-versus-Host-Reaktion, Myocarditis, Thyroiditis, Nephritis, systemischer Lupus erythematodes, insulinabhängiger Diabetes mellitus.

Die Arthritis-Erkrankung ist vorzugsweise ausgewählt iaus der Gruppe rheumatoide Arthritis, Osteoarthritis, Caplan Syndrom, Felty Syndrom, Sjogren Syndrom, Spondylitis ankylosans, Morbus Still, Chondrocalcinosis, metabolische Arthritis, rheumatisches Fieber, Morbus Reiter, Wissler Syndrom.

Die entzündliche Nierenerkrankung ist vorzugsweise ausgewählt aus der Gruppe
Glomerulonephritis, glomeruläre Verletzung, nephrotisches Syndrom, interstitielle Nephritis, Lupus nephritis, Goodpasture-Syndrom, Wegener-Granulomatose, Nierenvaskulitis, IgA-Nephropathie, idiopatische glomeruläre Erkrankung.

Die entzündliche Hauterkrankung ist vorzugsweise ausgewählt aus der Gruppe
Psoriasis, atopische Dermatitis, Kontaktempfindlichkeit, Akne.

Ebenfalls umfasst ist die Verwendung der Verbindungen der Formel I gemäß Anspruch 1 und/oder ihre physiologisch unbedenklichen Salze und Solvate zur Herstellung eines Arzneimittels zur Behandlung oder Vorbeugung einer Krankheit bzw. eines Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden. Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.

Ebenfalls umfasst ist die Verwendung der Verbindungen der Formel I gemäß Anspruch 1 und/oder ihre physiologisch unbedenklichen Salze zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die offenbarten Verbindungen der Formel I gemäß Anspruch 1 können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die Verbindungen der Formel I gemäß Anspruch 1 können auch gemeinsam mit anderen gut bekannten Therapeutika, die aufgrund ihrer jeweiligen Eignung für das behandelte Leiden ausgewählt werden, verabreicht werden. Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethyl-propanoat, 4,4'-Dihydroxybenzophenon-2,4-dinitrophenylhydrazon und SH646, was jedoch keine Einschränkung darstellen soll. "Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5a-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropyl-amino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### Wirkungsnachweis von pharmakologischen Inhibitoren auf die

### Proliferation/Vitalität von Tumorzellen in vitro

### 1.0 Hintergrund

In der vorliegenden Versuchsbeschreibung wird die Hemmung der Tumorzellproliferation/ Tumorzellvitalität durch Wirkstoffe beschrieben.

Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

### 2.0 Versuchsdurchführung

### 2.1 Zellkultur

Beispielsweise käuflich erhältliche Colon-Carcinom-Zelllinien, Zelllinien des Eierstocks, Zellinien der Prostata oder Zelllinien der Brust etc.

Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% CO₂ kultiviert.

### 2.2. Aussaat der Zellen

Eine definierte Zellzahl (z.B. 2000 Zellen) werden pro Kultur/ well in einem Volumen von 180µl Kulturmedium in Mikrotiterplatten (96 well Zellkulturplatten) mit einer Mehrkanalpipette ausgesät. Die Zellen werden anschließend in eienm CO2-Brutschrank (37°C und 10% CO2) kultiviert.

### 2.3. Zugabe der Testsubstanzen

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20µl Substanzlösung in die Kulturen/wells gegeben. Die Zellen werden für weitere 4 Tage bei 37°Celsius und 10% CO₂ kultiviert.

### 2.4. Messung der Farbreaktion

Pro well werden jeweils 20µl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

### 3. Auswertung

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt: $\begin{matrix}Rechnung: \\ \frac{100 * \left(Wert mit Zellen und Testsubstanz - Wert der Mediumkontrolle\right)}{\left(Wert mit Zellen - Wert der Mediumkontrolle\right)}\end{matrix}$

Die Bestimmung von IC₅₀ Werten (50%ige Hemmung) erfolgt mit Hilfe von Statistikprogrammen wie z.B. RS1.

IC₅₀-Daten erfindungsgemäßer Verbindungen sind in Tabelle 1 angegeben.

| Material | Best. Nr. | Hersteller | |
|---|---|---|---|
| Mikrotiterplatten für die Zellkultur (Nunclon Surface 96well Plate) | | 167008 | Nunc |
| DMEM | P04-03550 | Pan Biotech | |
| PBS (10x) Dulbecco | 14200-067 | Gibco | |
| 96well Platten (Polypropylen) | | 267334 | Nunc |
| AlamarBlue™ | BUF012B | Serotec | |
| FCS | 1302 | Pan Biotech GmbH | |
| Trypsin/EDTA Solution 10x | L 2153 | Biochrom AG | |
| 75cm² Kulturflaschen | 353136 | BD Falcon | |
| A2780 | 93112519 | ECACC | |
| Colo205 | CCL222 | ATCC | |
| MCF7 | HTB22 | ATCC | |
| PC3 | CRL-1435 | ATCC | |

### Bestimmung der Proliferationshemmung durch Inhibitoren der Methioninaminopeptidase 2 im BrdU Proliferationstest (zellulärer Assay)

Die Hemmung der Proliferation wird durch Inkorporation von Bromodesoxyuridin (BrdU) in humanen Endothelzellen aus der Nabelschnur (HUVEC, PromoCell, C-12200) bestimmt. Die HUVEC werden bei 37°C und 5% CO₂ in Basalmedium (PromoCell, C-22200) mit Supplementmix (PromoCell, C-39225) kultiviert. Nach Ablösung der Zellen mittels Trypsin/EDTA wird die Lebendzellzahl bestimmt und die Zellen in einer Dichte von 1000 Zellen pro Kavität in einem Gesamtvolumen von 175 µl ausgesät (Kavitäten werden zuvor entweder mit supplementiertem Kulturmedium für 1-2 Stunden bei 37°C oder mit 1,5% Gelatine für 0,5 - 2 Stunden bei 37°C beschichtet). Nach 24 stündiger Kultivierung werden die Testsubstanzen in verschiedenen Konzentrationen (z.B. finale Konzentrationen 30 µM bis 0,03 nM in 10-fach Verdünnungsschritten) und einem Volumen von 25 µl hinzugegeben. Die DMSO Konzentration wird mit 0,3% konstant gehalten. Nach insgesamt 48 oder 72 stündiger Kultivierung werden 20 µl Bromdesoxyuridin (Roche, # 11647229001 1:1000 verdünnt in Kulturmedium, Endkonzentration 10µM) hinzugegeben und für weitere 20 bis 24 Stunden kultiviert. Nach insgesamt 72 bzw. 96 Stunden Inkubation mit Testsubstanzen wird das Kulturmedium entfernt und ein immunhistochemischer Nachweis zur Detektion der BrdU-Inkorporation durchgeführt (BrdU ELISA, Roche, # 11647229001). Hierzu werden die Zellen für 30 min bei Raumtemperatur mit einem Fixativ behandelt und anschließend mit einem Peroxidase-markiertem anti-BrdU Antikörper (1:100 verdünnt in Antikörperverdünnungspuffer) für 60 min bei Raumtemperatur inkubiert. Nach dreimaligem Waschen mit 1-fach konzentriertem DPBS-Puffer (Gibco, # 14200) wird die enzymatische Umsetzung in TMB-Substratlösung initiiert. Die Farbentwicklung wird nach 15 min durch Zugabe von 25 µl einer 1M Schwefelsäurelösung abgestoppt. Eine Bestimmung der optischen Dichte erfolgt innerhalb von 5 min durch Messung bei einer Wellenlänge von 450 nM. Als Kontrollen dienen Kavitäten mit DMSO-behandelten Zellen (100% Kontrolle) oder leere Kavitäten (Leerwert). Die Sensitivität dieses Tests gegenüber Inhibitoren der Methioninaminopeptidase wird durch Verwendung des Inhibitors Fumagillin überprüft und bestätigt.

### MetAP-2 Aktivitätsmessung

Die MetAP-2 Aktivität wird durch eine Kopplung von enzymatischen Reaktionen nachgewiesen. Das Tripeptid Met-Arg-Ser (MAS) wird als Substrat eingesetzt. Das freigesetzte Methionin wird zunächst durch die L-Aminooxidase (AAO) zu Metox und H₂O₂ umgesetzt. Im zweiten Schritt katalysiert die Peroxidase (POD) mt Hilfe des H₂O₂ die Oxidation des Leukofarbstoffs Dianisidin zu Dianisidinₒₓ, dessen Zunahme photometrisch bei 450 nm detektiert wird.

MetAP-2 Aktivität kann als Kinetik kontinuierlich aufgezeichnet werden. Das Reaktionsschema verdeutlicht, dass pro mol Methionen ein mol Dianisidinₒₓ gebildet wird. Die MetAP-2 Enzymaktivität lässt sich deshalb direkt als Δ Absorption pro Zeiteinheit berechnen. Eine Qualifizierung der MetAP-2 Aktivität (mol Met/Zeiteinheit) ist mit Hilfe des Dianisidinₒₓ-Extinktionskoeffizienten möglich.

Die Extinktionsänderung pro Zeiteinheit wird graphisch dargestellt und eine Steigungsberechnung im visuell linearen Bereich der Reaktion durchgeführt.

### Löslichkeitsmessung

### Bestimmung nach "Shake flask solubility measurement"

### Eluentenherstellung:

| | |
|---|---|
| Eluent A: | 2 ml Diethylamin, zur Synthese + |
| | 1000 ml Methanol, LiChrosolv |
| Eluent B: | 5 g Ammoniumacetat, zur Analyse + |
| | 5 ml Methanol, LiChrosolv + |
| | 995 ml Reinstwasser |

### Probenlösungsmittel:

Puffer: 3,954 g Natriumdihydrogenphosphat-Monohydrat + 6,024 g Natriumchlorid + 950 ml Reinstwasser mit 0,1 M NaOH oder 0,1 M HCl wird der pH-Wert eingestellt.

### Probenvorbereitung:

Die Proben werden bei 37°C und 450 rpm 24 h lang geschüttelt.
Nach ca. 7h wird der pH Wert der Proben überprüft und gegebenenfalls nachgestellt.
Es wird auch kontrolliert, ob die Probe noch im Überschuss vorhanden ist.

Kurz vor Ende der 24h-Schüttelzeit werden die Proben nochmals auf pH-Wert und auf einen Niederschlag überprüft.
Reinstwasser Anlage: MilliQ Gradient, Millipore, Gerät: F3PN37462D
Schüttler: TiMix control, Bühler
Inkubationshaube: TH 15 Bühler
pH Meter: 766 Calimatic Knick Gerät: pH 1
pH Elektrode: InLab 423 Mettler

### Ergebnisse:

Die Verbindung (2-Morpholin-4-yl-ethyl)-{4-[(R)-1-(9H-purin-6-yl)-pyrrolidin-2-ylmethoxy]-naphthalin-1-ylmethyl}-amin zeigt eine gute Löslichkeit (283 g/ml) in Puffersystem bei pH 7, was ihre Anwendung in einer oral verabreichten Formulierung besonders positiv beeinflusst.

Weitere Ergebnisse:
Morpholin-4-yl-(4-{2-[(S)-1-(9H-purin-6-yl)-pyrrolidin-2-yl]-ethyl}-naphthalin-1-yl)-methanon: 108 µg/ml;
4-[(R)-1-(9H-Purin-6-yl)-pyrrolidin-2-ylmethoxy]-chinolin-2-carbonsäure-(2-hydroxy-propyl)-amid: 325 µg/ml;
N-Methyl-2-[1-oxo-5-[(R)-1-(9H-purin-6-yl)-pyrrolidin-2-ylmethoxy]-3,4-dihydro-1H-naphthalin-(2Z)-ylidene]-acetamid: 29 µg/ml;
[1-Oxo-5-[(R)-1-(9H-purin-6-yl)-pyrrolidin-2-ylmethoxy]-3,4-dihydro-1H-naphthalin-(2Z)-ylidene]-essigsäure: > 3.8 mg/ml.

Die in WO 2007/017069 offenbarte Verbindung 6-[(R)-2-(2,3-Dichlorphenoxymethyl)-pyrrolidin-1-yl]-9H-purin zeigt eine Löslichkeit von < 1 µg/ml.

APCI-MS (atmospheric pressure chemical ionization - mass spectrometry) (M+H)⁺.

### *Methode Info:

Säule: Chromolith SpeedROD RP-18e 50-4,6mm
Solvent A: Wasser + 0,1% TFA
Solvent B: Acetonitril + 0,1% TFA
Fluß : 2.4 mL/min
Gradient: 0,0min 4% B
2,6 min 100% B

### **HPLC: La Chrom Anlage

Chromolite Performance RP18-e 100-4,6mm
Gradient: ACN/H2O mit 0,01% Ameisensäure
Methode: Chromolith/Chromolith (extended)
Fluß: 3mL/min

### ^{$} Agilent Anlage

Chromolite Performance RP18-e 50-4,6mm
Gradient: ACN/H2O mit 0,04/0,05% Ameisensäure
Methode: Polar
Fluß: 2,4mL/min

Die Aufnahme der NMR-Spektren erfolgt in DMSO-d₆ und in DMSO-d₆ + TFA-d₁. Die angebenen Daten beziehen sich auf die DMSO-d₆ + TFA-d₁ - Spektren.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
F. : Schmelzpunkt
Massenspektrometrie (MS): EI (Elektronenstoß-Ionisation) M⁺
FAB (Fast Atom Bombardment) (M+H)⁺
ESI (Electrospray lonization) (M+H)⁺
APCI-MS (atmospheric pressure chemical ionization - mass spectrometry) (M+H)⁺.

### Beispiel 1

Die Herstellung von 6-[(R)-2-(Naphthalin-1-yloxymethyl)-pyrrolidin-1-yl]-9H-purin ("A1") erfolgt analog nachstehendem Schema 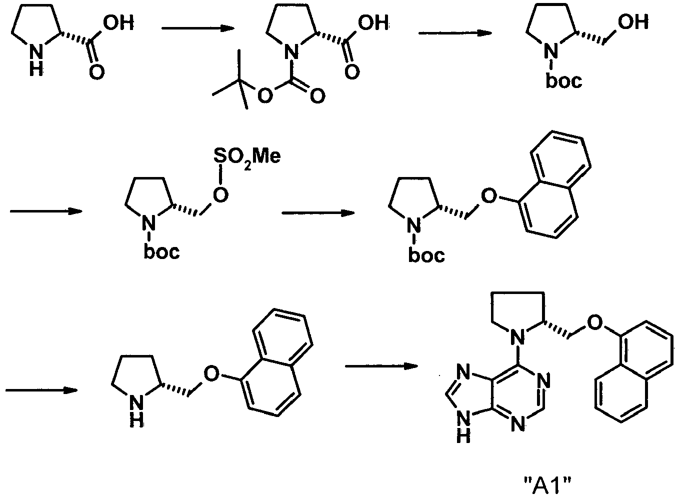
1.1 83.7 g D-Prolin werden in 900 mL tert.-Butanol gelöst und 151 mL Triethylamin zugesetzt. Di-tert.-butyldicarbonat wird in 300 ml tert.-Butanol gelöst und zu der ersten Lösung getropft. Nach 21 Stunden Rühren bei RT wird der Niederschlag abfiltriert und mit warmem tert.-Butanol gewaschen. Die vereinigten Filtrate werden in ca. 700 mL Diethylether aufgenommen und mit 500 mL 1N HCl Lösung, 500 mL gesättigter Natriumcarbonat-Lösung und 500 mL Natriumchlorid-Lösung gewaschen. Man arbeitet wie üblich weiter auf und erhält 86.7 g eines farblosen Öls (entspricht J. Org. Chem. 1988, 53 (3), 485).
1.2 12.3 g Lithiumchlorid werden in 140 ml Ethanol klar gelöst und auf -20°C abgekühlt. 11g NaBH₄ werden ebenfalls in 140 ml Ethanol suspendiert und zu der kalten Lithiumchlorid-Lösung gegeben. Nach 10 Minuten gibt man bei der angegebenen Temperatur eine Lösung von 29.7 g D-Pyrrolidin-1,2-dicarbonsäure-1-tert.-butylester in 140 ml THF hinzu und lässt für 19 Stunden auf RT erwärmen. Zur Aufarbeitung wird die Reaktionsmischung auf 0°C gekühlt und mit 300 ml gesättigter Zitronensäure vorsichtig versetzt. Die organische Phase wird abgetrennt, über Natriumsulfat getrocknet und zum Rückstand eingeengt. Man erhält 12.95 g eines hellgelben Öls (entspricht J. Org. Chem. 1993, 58 (5), 1213).
1.3 100 mg 2-Hydroxymethyl-pyrrolidin-1-carbonsäure-tert.-butylester werden in 2 mL Dichlormethan gelöst, mit 140 L Triethylamin versetzt und nachfolgend 45 L Methansulfonylchlorid zugetropft. Nach 45 Minuten Rühren bei RT wird die Reaktionslösung mit weiteren 2 mL Dichlormethan verdünnt und nacheinander mit 4 mL Wasser, 4 mL 10%iger Citronensäure und 4 mL gesättigter Natriumchlorid Lösung gewaschen. Die organische Phase wird über Natriumsulfat getrocknet, eingedampft und die erhaltenen 140 mg Rohprodukt, Rt.: 1.904 min, im nächsten Schritt gleich weiter umgesetzt.
1.4 2 g 2-Methansulfonyloxymethyl-pyrrolidin-1-carbonsäure-tert.-butylester werden zusammen mit 1.1 g 1-Naphthol und 3 g Cäsiumcarbonat in 50 mL DMF suspendiert und für 12 Stunden auf 80°C erwärmt. (Diese und alternative Bedingungen findet man in March, J. "Advanced Organic Chemistry: Reactions, Mechanisms, and Structure," 4th ed.; John Wiley & Sons: New York, 1992, pp 430-431, und den dort zitierten Referenzen.) Zur Aufarbeitung wird die Reaktionsmischung auf 50 mL Dichlormethan und 50 mL Wasser gegossen. Die organische Phase wird getrocknet und nach Entfernen des Lösungsmittels chromatographisch an Kieselgel gereinigt. Man erhält 1.25 g eines braunen Öls, welches direkt weiter umgesetzt wird; *Rt.: 2.96 min.
1.5 1.25 g 2(-Naphthalin-1-yloxymethyl)-pyrrolidin-1-carbonsäure-tert.-butylester werden in 10 mL THF gelöst und mit 5 ml ethanolischer Salzsäure versetzt. Die Reaktionsmischung wird 6 bei 80°C gerührt und der resultierende Niederschlag abgesaugt, mit THF gewaschen und in der nachfolgenden Reaktion direkt weiter umgesetzt; Rt.: 1.633 min; [M+H]⁺ 228.
1.6 235 mg 6-Chlorpurin, 400 mg 2(-Naphthalin-1-yloxymethyl)-pyrrolidin und 0.8 mL Triethylamin werden in 30 mL 1-Butanol gelöst und 6 h bei 120°C in der Mikrowelle bestrahlt. Nach Entfernen des Lösungsmittels im Vakuum wird der Rückstand in 10 mL Methanol aufgenommen, die dabei ausfallenden Kristalle werden abgesaugt und mit Methanol gewaschen. Man erhält 340 mg farbloser Kristalle 6-[(R)-2-(Naphthalin-1-yloxymethyl)-pyrrolidin-1-yl]-9H-purin ("A1"); Rt.: 1.806 min; [M+H]⁺ 346; F. 207-208°;
   ¹H-NMR (500 MHz, d₆-DMSO) δ [ppm] 12.97 (br. s, 1H), 8.25 (s, 1H), 8.12 (s, 2H), 7.87 (dd, 1H, J = 2.0Hz, J = 7.1 Hz), 7.54 - 7.49 (m, 2H), 7.46 (d, 1H, J = 8.2 Hz), 7.40 - 7.37 (m, 1H), 7.07 (m, 1H), 4.92 (dd, 1H, J = 3.4 Hz, J = 9.1 Hz), 4.27 (t, 1H, J = 8.1 Hz), 2.25 - 2.07 (m, 4H).

### Beispiel 2

### Die Herstellung von 4-[(R)-1-(9H-Purin-6-yl)-pyrrolidin-2-ylmethoxy]-naphthalin-1-carbaldehyd ("A2") erfolgt analog nachstehendem Schema

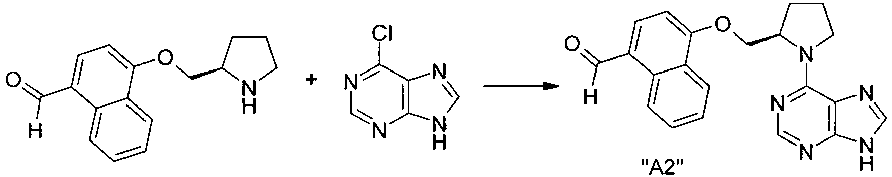

296 mg 6-Cl-Purin und 560 mg 4-((R)-1-Pyrrolidin-2-ylmethoxy)-naphthalene-1-carbaldehyd werden zusammen mit 1.0 mL N-Ethyldiisopropylamin in 30 mL1-Butanol für 6 h in der Mikrowelle auf 120°C erwärmt. Nach der üblichen wässrigen Aufarbeitung und Chromatographie über Kieselgel erhält man 100 mg 4-[(R)-1-(9H-Purin-6-yl)-pyrrolidin-2-ylmethoxy]-naphthalin-1-carbaldehyd ("A2") als farblose Kristalle; Rt: 1.729 Min; [M+H]⁺ 374.2; F.167-168°;
¹H-NMR (500MHz, d₆-DMSO) *δ* [ppm] 12.98 (br. s, 1H), 10.18 (s, 1H), 9.22 (d, 1H, J = 8.3 Hz), 8.25 (m, 2H), 8.13 (m, 2H), 7.76 (ddd, 1H, J = 1.3 HZ, J = 6.9 Hz, J = 8.3 HZ), 7.65 (t, 1H, J = 7.6 Hz), 7.37 (m, 1H), 4.68 (dd, 1H, J = 3.4 Hz, J = 9.3 Hz), 4.42 (t, 1H, J = 8.0 Hz), 4.00 (m, 3H), 2.25 (m, 3H), 2.08 (m, 1H).

### Beispiel 3

### Herstellung von 6-[(R)-2-(4-Morpholin-4-ylmethyl-naphthalin-1-yloxymethyl)-pyrrolidin-1-yl]-9H-purin ("A3")

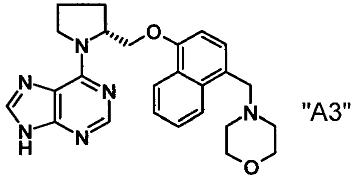

155 mg 6-CI-Purin und 320 mg 4-[4-((R))-1-Pyrrolidin-2-ylmethoxy)-naphthalin-1-ylmethyl]-morpholin werden zusammen mit 0.5 ml N-Ethyldiisopropylamin in 20 mL 1-Butanol für 6 h auf 120°C in der Mikrowelle erwärmt. Nach dem üblichen Aufarbeitungs- und Aufreinigungsprotokoll erhält man 4 mg 6-[(R)-2-(4-Morpholin-4-ylmethyl-naphthalin-1-yloxymethyl)-pyrrolidin-1-yl]-9H-purin als farblose Kristalle; * Rt.: 2.14 Min; F. 134 - 138°;
¹H-NMR (500 MHz, d₆-DMSO) *δ* [ppm] 12.97 (br. s, 1H), 8.23 (m, 1H), 8.20 (m, 2H), 8.11 (m, 2H), 7.53 (m, 2H), 7.28 (m, 1H), 6.98 (m, 1H), 4.90 (m, 1H), 4.48 (m, 1H), 4.25 (m, 2H), 3.75 (m, 3H), 3.51 (m, 3H), 2.24 (m, 2H), 2.06 (m, 1H), 1.09 (m, 1).

### Beispiel 4

### Die Herstellung von 6-[(R)-2-(4-Butoxymethyl-naphthalin-1-yloxymethyl)-pyrrolidin-1-yl]-9H-purin ("A4") erfolgt analog Beispiel 1

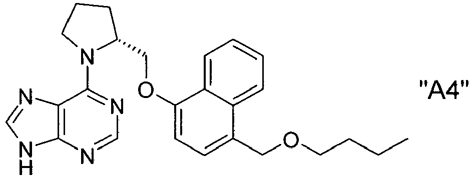

Rt.: 2.349 Min; [M+H]⁺ 432.2; F. 214-216°; ¹H-NMR (500 MHz, d₆-DMSO) *δ* [ppm] 12.95 (br. s, 1H), 8.24 (s, 1H), 8.16 (br. s, 1H), 8.11 (s, 1H), 8.04 (d, 1H, J = 8.3 Hz), 7.57 (dt, 1H, J = 1.5 Hz, J = 6.8 Hz), 7.52 (dt, 1H, J = 0.9 Hz, J= 8.2 Hz), 7.37 (d, 1H, J = 7.8 Hz), 5.46 (br. m, 1H), 4.77 (s, 2H), 4.51 (dd, 1H, J = 3.2 Hz, J = 9.2 Hz), 4.25 (t, 1H, J = 8.1 Hz), 3.84 (br. m, 2H), 3.44 (t, 2H), 2.23 (br. m, 3H), 2.06 (br. m, 1H), 1.49 (m, 2H), 1.29 (m, 2H), 0.83 (t, 3H, J = 7.4 Hz).

### Beispiel 5 (Vergleichsbeispiel)

### Herstellung von 6-{-2-[2-(2-Chlor-phenyl)-ethyl]-pyrrolidin-1-yl}-9H-purin ("A5")

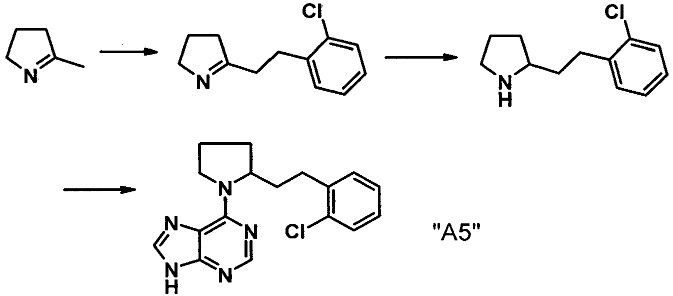

5.1 3 mL 1-Methyl-pyrrolin werden in 25 mL THF gelöst und bei -78°C mit 22.6 mL BuLi (1 M in Hexan) 30 Minuten deprotoniert. 6.5 g 2-Chlorbenzylbromid werden in 25 mL THF gelöst und bei der angegebenen Temperatur zugetropft. Nach 30 Minuten lässt man für 12 Stunden auf RT erwärmen. Zur Aufarbeitung gibt man 50 mL Wasser hinzu und extrahiert erschöpfend mit Dichlormethan. Die vereinigten organischen Phasen werden über Natriumsulfat getrocknet, eingedampft und an Kieselgel chromatographisch aufgereinigt. Man erhält 4,5 g 5-[2-(2-Chlor-phenyl)-ethyl]-3,4-dihydro-2H-pyrrol als ein farbloses Öl, das in der nächsten Reaktion eingesetzt wird; Rt.: 1.303 min; [M+H]⁺ 208.
5.2 4.5 g 5-[2-(2-Chlor-phenyl)-ethyl]-3,4-dihydro-2H-pyrrol werden in 150 mL Methanol gelöst und mit 1.5 g Natriumcyanoborhydrid versetzt. Bei 0°C gibt man solange ethanolische Salzsäure hinzu, bis ein pH Wert von 2 eingestellt ist. Nachfolgend lässt man die Reaktion 6 h bei RT laufen, bevor man das Reaktionsgemisch auf 10 mL konzentrierte Salzsäure gießt und mit Wasser verdünnt. Dann wird mit konzentrierter Natronlauge neutralisiert und die wässrige Phase mit Essigester extrahiert. Nach dem Trocknen über Natriumsulfat und Eindampfen erhält man 1 g farbloses Öl, welches gleich weiter umgesetzt wird; Rt.: 1.486 min; [M+H]⁺ 210.
5.3 500 mg 2-[2-(2-Chlor-phenyl)-ethyl]-pyrrolidin, 367 mg 6-Chlorpurin und 0.6 mL Triethylamin werden in 40 mL 1-Butanol gelöst und für 6 h bei 120°C in der Mikrowelle umgesetzt. Nach dem Entfernen des Lösungsmittels im Vakuum wird der Rückstand mit 50 mL Essigester und 50 mL Wasser aufgenommen, die organische Phase getrocknet, eingedampft und aus Ether umkristallisiert. Man erhält 500 mg beige Kristalle racemisches 6-{-2-[2-(2-Chlor-phenyl)-ethyl]-pyrrolidin-1-yl}-9H-purin ("A5"); Rt.: 1.793 min; [M+H]⁺ 328;
   ¹H-NMR (500 MHz, d₆-DMSO) *δ* [ppm] 12.91 (br. s, 1H), 8.18 (s, 1H), 8.09 (s, 2H), 7.40 - 7.35 (m, 2H), 7.28 - 7.19 (m, 2H), 2.79 (m, 2H), 2.12 (m, 6H), 1.70 (m, 1H).

110 mg des Racemats 6-{-2-[2-(2-Chlor-phenyl)-ethyl]-pyrrolidin-1-yl}-9H-purin werden in 7 mL Methanol und 2 mL Diethylamin gelöst und auf 9 Vials aufgeteilt. Die Lösungen werden mittels superkritischem CO₂ (SFC) über Chiralcel OD-H mit 5mL/min CO₂ + 40% MOH0, 5 DEA getrennt.
Man erhält 46,6 mg 6-{(R)-2-[2-(2-Chlor-phenyl)-ethyl]-pyrrolidin-1-yl}-9H-purin ("A5b") mit einem Enantiomerenverhältnis von 98,8% : 1,2%.
und 45,3 mg 6-{(S)-2-[2-(2-Chlor-phenyl)-ethyl]-pyrrolidin-1-yl}-9H-purin ("A5a") mit einem Enantiomerenverhältnis 0,4% : 99,6%.

### Beispiel 6

### Herstellung von Morpholin-4-yl-(4-{2-[-1-(9H-purin-6-yl)-pyrrolidin-2-yl]-ethyl}-naphthalin-1-yl)-methanon ("A6")

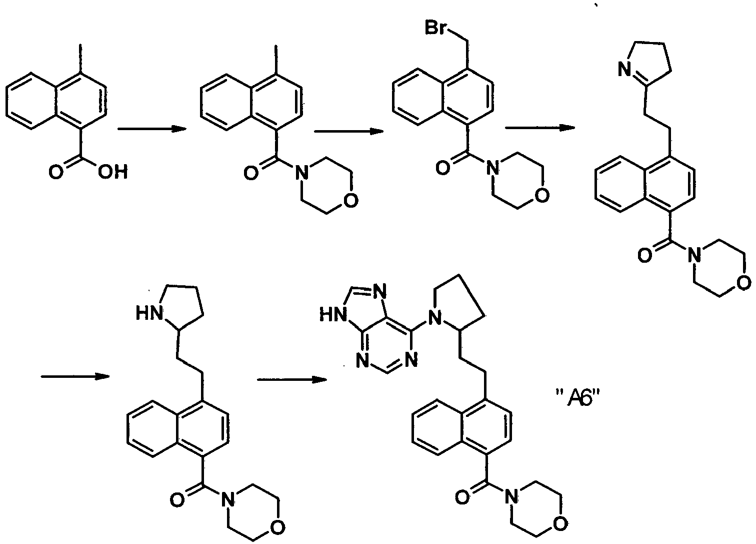

6.1 5 g kommerziell erhältliche 4-Methyl-1-naphthylsäure werden mit 4.7 mL Morpholin, 5.2 g N-(3-Dimethylaminopropyl)-N'-ethylcarbodiimidhydrochlorid und 3.6 g 1-Hydroxybenzotriazol für 12 h bei RT umgesetzt. Man erhält nach Aufarbeitung und Aufreinigung 5.4 g braunes Öl (4-Methyl-naphthalin-1-yl)-morphlin-4-yl-methanon; Rt.: 1.924 Min; [M+H]⁺ 256.2.
6.2 5.4 g (4-Methyl-naphthalin-1-yl)-morpohlin-4-yl-methanon werden mit 4.3 g N-Bromsuccinimid und 100 mg α,α-Azobisisobutyronitril in 100 mL Dichlorethan bei 80°C für 10 h umgesetzt. Nach wässriger Aufarbeitung und Chromatographie über Kieselgel erhält man 4.5 g eines farblosen Öls (4-Brommethyl-naphthalin-1-yl)-morpholin-4-yl-methanon; Rt.: 2.028 Min;
   [M+H]⁺ 334.0/336.0.
6.3 1.4 mL 2-Methyl-1-pyrrolin werden in 50 mL THF gelöst und auf -78°C abgekühlt. Dann werden 11.3 mL n-Butyllithium (15%ig in n-Hexan) zugetropft. Nach 30 Minuten Rühren gibt man die in 25 mL THF gelöstes (4-Brommethyl-naphthalin-1-yl)-morpholin-4-yl-methanon hinzu und lässt für 8 h auf RT erwärmen. Nach der üblichen Aufarbeitung und Aufreinigung erhält man 2 g gelbes Öl {4-[2-(4,5-Dihydro-3H-pyrrol-2-yl)-ethyl]-naphthalin-1-yl)-morpholin-4-yl-methanon; Rt: 1.407 Min; [M+H]⁺ 337.2.
6.4 1 g {4-[2-(4,5-Dihydro-3H-pyrrol-2-yl)-ethyl]-naphthalin-1-yl)-morpholin-4-yl-methanon wird in 50 mL Methanol gelöst und mit 0.26 g Natriumcyanborhydrid versetzt. Bei 0°C wird mit methanolischer HCl ein pH von 2 eingestellt. Anschließend lässt man 6 h bei RT nachrühren und arbeitet dann wässrig auf. Mit wässriger NaOH wird ein pH Wert von 9 eingestellt und mit Essigester extrahiert. Die getrocknete und eingeengte organische Phase wird chromatographisch aufgereinigt. Man erhält 460 mg Morpholin-4-yl-[4-(2-pyrrolidin-2-yl-ethyl)-naphthalin-1-yl]-methanon; Rt.: 1.420 Min; [M+H]⁺ 339.2.
6.5 231 mg 6-CI-Purin werde mit 460 mg Morpholin-4-yl-[4-(2-pyrrolidin-2-yl-ethyl)-naphthalin-1-yl]-methanon in N-ethyldiisopropylamin und 1-Butanol in der Mikrowelle wie beschrieben umgesetzt. Nach der üblichen Aufarbeitung und Aufreinigung erhält man 210 mg "A6" als farblose Kristalle; Rt.: 1.606 Min; [M+H]⁺ 457.2; F. 158 - 160°;
   ¹H-NMR (500 MHz, d₆-DMSO) *δ* [ppm] 12.89 (br. s, 1H), 8.12 (s, 2H), 7.78 (d, 1H, J = 8.2 Hz), 7.59 (m, 3H), 7.47 (d, 1H, J = 7.0 Hz), 7.35 (d, 1H, J = 7.1 Hz), 5.44 (m, 1H), 5.07 (br. m, 1H), 3.82 (m, 1H), 3.75 (m, 3H), 3.61 (m, 2H), 3.45 (m, 2H), 3.11 (m, 2H), 3.01 (m, 1H), 2.25 (m, 1H), 2.10 (m, 2H), 2.01 (m, 1H), 1.76 (m, 2H).
6.6 220 mg des racemischen Gemischs Morpholin-4-yl-(4-{2-[-1-(9H-purin-6-yl)-pyrrolidin-2-yl]-ethyl}-naphthalin-1-yl)-methanon ("A6") werden an der SFC über 1cm Chiralcel OD-H-Säule getrennt.

Die Trennung erfolgt mit einem Fluß von 5 mL/min. Die flüssige Phase besteht zu 60% aus flüssigem CO₂ und zu 40% aus einer Mischung von 99.5% Methanol mit 0.5% Diethylamin.
Fraktion 1: m=120 mg Enantiomerenverhältnis:
   Ena 1 90,9% : 9,1% Ena2;
Fraktion 2: m=123mg Enantiomerenverhältnis:
   Ena1 12% : 88% Ena2.

Beide Fraktionen wurden nachgetrennt; jeweils in Methanol gelöst und über Chiralcel OD-H 1cm Säule getrennt.
Die Trennung erfolgt mit einem Fluß von 5 mL/min. Die flüssige Phase besteht zu 60% aus flüssigem CO₂ und zu 40% aus einer Mischung von 99.5% Methanol mit 0.5% Diethylamin.
Fraktion 1: m= 64mg enantiomerenrein:
   Morpholin-4-yl-(4-{2-[(S)-1-(9H-purin-6-yl)-pyrrolidin-2-yl]-ethyl}-naphthalin-1-yl)-methanon ("A6a");
Fraktion 2: m= 73mg enantiomerenrein:
   Morpholin-4-yl-(4-{2-[(R)-1-(9H-purin-6-yl)-pyrrolidin-2-yl]-ethyl}-naphthalin-1-yl)-methanon ("A6b").

### Beispiel 7 (Vergleichsbeispiel)

### Herstellung von 6-[(2R,4S)-2-(2-Chlor-phenoxymethyl)-4-fluor-pyrrolidin-1-yl]-9H-purin ("A7")

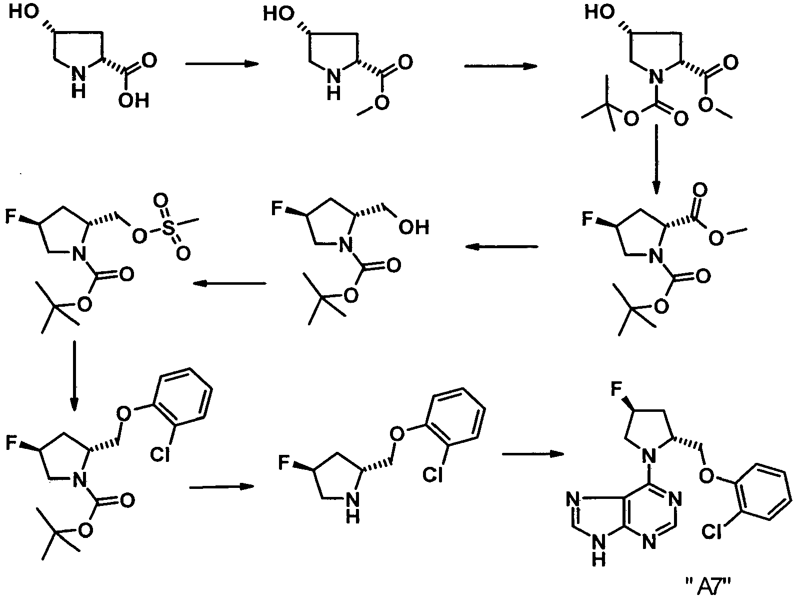

7.1 30 g (2R, 4R)-4-Hydroxy-pyrrolidin-2-carbonsäure werden in 200 mL Methanol suspendiert und auf 0°C abgekühlt. Anschließend wird über eine Stunde verteilt bei gleicher Temperatur das Thionylchlorid (18.1 mL) zugetropft. Das Reaktionsgemisch (RG) wird über 12 h auf Raumtemperatur (RT) erwärmt, wobei sich eine klare Lösung bildet. Das Lösungsmittel wird im Vakuum entfernt und der erhaltene Rückstand aus Ether umkristallisiert. Man erhält 50 g (2R, 4R)-4-Hydroxy-pyrrolidin-2-carbonsäuremethylester; Rt.: 0.386 min; [M+H]⁺ 146.2.
7.2 50 g (2R, 4R)-4-Hydroxy-pyrrolidin-2-carbonsäuremethylester, 64.2 mL Di-tert.-butyldicarbonat, 116.4 mL Triethylamin und 2.4 g 4-(Dimethylamino)-pyridin werden in 500 mL Dichlormethan gelöst und über 12 h bei RT gerührt. Das Reaktionsgemisch wird mit Wasser gewaschen und die organische Phase über Magnesiumsulfat getrocknet und eingeengt. Der Rückstand wird über eine Kieselgelsäule chromatographiert. Man erhält 37 g farblose Kristalle (2R,4R)-4-Hydroxy-pyrrolidin-1,2-carbonsäure-1-tert.-butylester-2-methylester.
7.3 5 g (2R, 4R)-4-Hydroxy-pyrrolidin-1,2-carbonsäure-1-tert.-butylester-2-methylester wird unter N₂-Atmosphäre in 100 mL CH₂Cl₂ gelöst und auf -78°C abgekühlt. Anschließend werden langsam 3 mL Diethylaminosulfurtrifluorid zugetropft. Das Reaktionsgemisch wird über 12 h auf RT erwärmt. Nach der wässrigen Aufarbeitung wird die organische Phase über Magnesiumsulfat getrocknet, im Vakuum eingeengt und ohne weitere Aufreinigung werden die erhaltenen 5.3 g öliges (2R, 4S)-4-Fluor-pyrrolidin-1,2-carbonsäure-1-tert.-butylester-2-methylester in der nächsten Reaktion weiter umgesetzt.
7.4 5.3 g (2R, 4S)-4-Fluor-pyrrolidin-1,2-carbonsäure-1-tert.-butylester-2-methylester werden in 100 mL THF gelöst und auf -20°C abgekühlt. Anschließend gibt man 1.2 g LiBH₄ hinzu. Das Reaktionsgemisch wird dann für 6 h bei RT nachgerührt. Das Gemisch wird wässrig aufgearbeitet und die erhaltenen 3.2 g (2R,4S)-4-Fluor-2-hydroxymethyl-pyrrolidin-1-carbonsäure-tert.-butylester werden direkt weiter umgesetzt.
7.5 3.2 g (2R,4S)-4-Fluor-2-hydroxymethyl-pyrrolidin-1-carbonsäure-tert.-butylester werden unter Rühren in 50 mL CH₂Cl₂ gelöst, mit 3.1 mL Triethylamin versetzt, auf 0-5°C abgekühlt und anschließend tropfenweise mit einer Lösung von 1.4 mL Methansulfonylchlorid in 10 mL CH₂Cl₂ versetzt. Das Gemisch wird anschließend für 4 h bei RT gerührt. Nach der wässrigen Aufarbeitung werden die erhaltenen 4.1 g (2R,4S)-4-Fluor-2-methansulfonyl-oxymethyl-pyrrolidin-1-carbonsäure-tert.-butylester) direkt weiter umgesetzt.
7.6 2 g (2R,4S)-4-Fluor-2-methansulfonyloxymethyl-pyrrolidin-1-carbonsäure-tert.-butylester, 0.9 mL 2-Chlorphenol und 3.6 g Cäsiumcarbonat werden in 60 mL DMF suspendiert und über 12 h bei 80°C gerührt. Nach der wässrigen Aufarbeitung werden die erhaltenen 2.5 g (2R,4S)-2-(2-Chlor-phenoxymethyl)-4-fluor-pyrrolidin-1-carbonsäure-tert.-butylester direkt weiter umgesetzt.
7.7 2.5 g (2R,4S)-2-(2-Chlor-phenoxymethyl)-4-fluor-pyrrolidin-1-carbonsäure-tert.-butylester werden in 20 mL THF gelöst und mit 5 mL ethanolischer Salzsäure für 2 h bei 80°C gerührt. Nach der basischen Aufarbeitung mit gesättigter Natriumhydrogencarbonat-Lösung wird die organische Phase wie üblich aufgearbeitet und der erhaltene Rückstand chromatographisch aufgereinigt. Man erhält 780 mg (2R,4S)-2-(2-Chlor-phenoxymethyl)-4-fluor-pyrrolidin als braunes Öl; Rt.: 0.700 min; [M+H]⁺ 230.2.
7.8 780 mg (2R,4S)-2-(2-Chlor-phenoxymethyl)-4-fluor-pyrrolidin und 525 mg 6-Chlorpurin werden zusammen mit 1.2 mL N-Ethyldiisoproylamin und 40 mL 1-Butanol für 6 h in der Mikrowelle bei 120°C bestrahlt, wobei ein Druck von 10 bar entsteht. Nach der üblichen wässrigen Aufarbeitung wird der erhaltene Rückstand aus Ether kristallisiert. Man erhält 350 mg 6-[(2R,4S)-2-(2-Chlor-phenoxymethyl)-4-fluor-pyrrolidin-1-yl]-9H-purin ("A7") als beige Kristalle; Rt.: 1.790 Min; [M+H]⁺ 348.2; F. 143 - 144°;
   ¹H-NMR (500 MHz, d₆-DMSO) *δ* [ppm] 13.07 (br. s, 1H), 8.26 (s, 1H), 8.17 (s, 1H), 7.40 (dd, 1H, *J* = 1.6 Hz, *J* = 7.9 Hz), 7.25 (dt, 1H, *J* = 1.2 Hz, *J* = 7.9 Hz), 7.14 (m, 1H), 6.93 (dt, 1H, *J* = 1.4 Hz, *J* = 7.7 Hz), 5.62 (dd, 1H, J = 3.16 Hz, J = 54.1 Hz), 4.99 (m, 1H), 4.49 (m, 1H), 4.36 (dd, 1H, *J* = 2.5 Hz, *J* = 9.5 Hz), 4.02 - 4.08 (m, 4H).

### Beispiel 8

### Herstellung von 6-[(R)-4,4-Difluor-2-(naphthalin-1-yloxymethyl)-pyrrolidin-1-yl]-9H-purin ("A8")

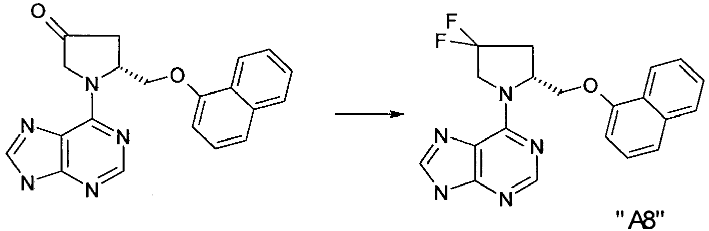

Man löst 200 mg (R)-5-(Naphthalin-1-yloxymethyl)-1-(9H-purin-6-yl)-pyrrolidin-3-on in 20 mL Dichlormethan und kühlt auf -78°C ab. Dann tropft man 0.18 mL Diethylaminosulfurtrifluorid zu und lässt für 8 h auf RT erwärmen. Nach der üblichen Aufarbeitung und Aufreinigung erhält man 3 mg "A8"; Rt.: 2.056;
-[M+H]⁺ 382.2.

### Beispiel 9 (Vergleichsbeispiel)

### Die Herstellung von (3-Fluor-phenyl)-[(R)-1-(9H-purin-6-yl)-pyrrolidin-2-ylmethyl]-amin ("A9"), (3-Fluor-phenyl)-methyl-[(R)-1-(9H-purin-6-yl)-pyrrolidin-2-ylmethyl]-amin ("A10") und 6-{(R)-2-[(E)-2-(3-Chlor-phenyl)-vinyl]-pyrrolidin-1-yl}-9H-purin ("A11") erfolgt analog nachstehendem Schema

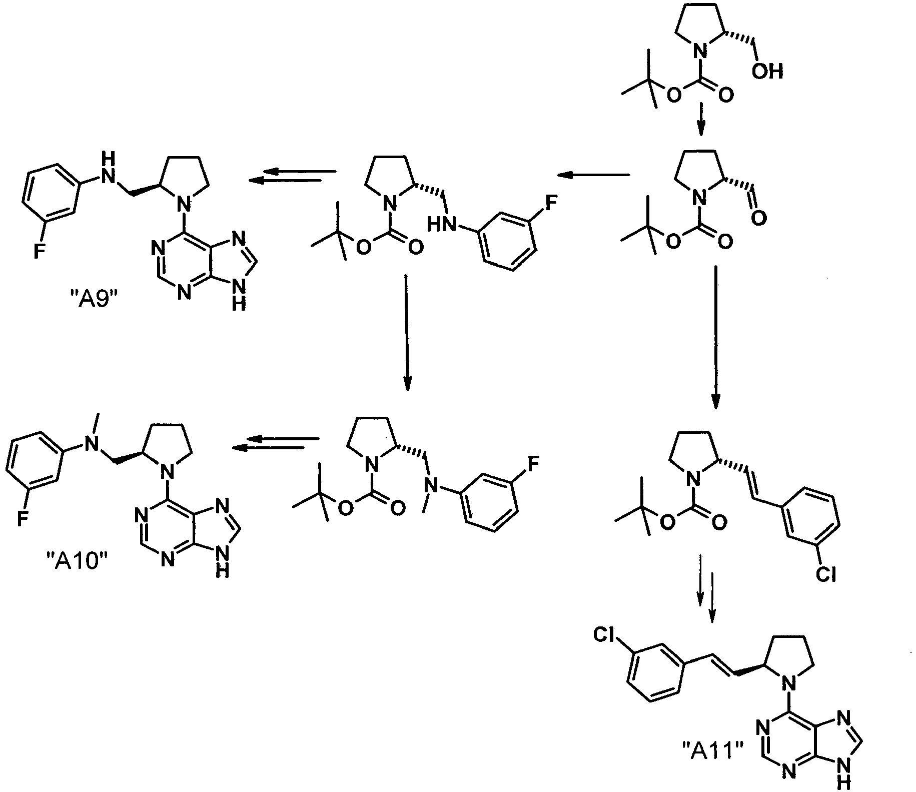

Ein Lösung von 3 mL DMSO in 100 mL Dichlormethan wird unter Schutzgas auf -78°C abgekühlt und mit 2.8 mL Oxalylchlorid tropfenweise versetzt. Nach 10 min werden 5 g 2-Hydroxymethyl-pyrrolidin-1-carbonsäure-tert.-butylester, in 5 mL Dichlormethan gelöst, zugetropft. Nach 2 h werden bei der angegebenen Temperatur 10 mL Triethylamin langsam zugegeben und der Ansatz nicht weiter gekühlt. Nach ca. 2 h hat die Reaktionsmischung Raumtemperatur erreicht und wird mit 10 mL Wasser versetzt. Nach 30 min werden die Phasen getrennt, die organische Phase erneut mit Wasser gewaschen und nach dem Trocknen über Magnesiumsulfat und dem Entfernen des Lösungsmittels im Vakuum über Kieselgel chromatographiert. Man erhält 3.8 g eines farblosen Öls (R)-2-Formyl-pyrrolidin-1-carbonsäure-tert.-butylester.

### Herstellung von "A9" :

2 g (R)-2-Formyl-pyrrolidin-1-carbonsäure-tert.-butylester und 1.1 mL 3-Fluoranilin werden in 50 mL 1,2-Dichlorethan gelöst und unter SchutzgasAtmosphäre auf 0°C abgekühlt. Dann werden portionsweise 3.3 g Natriumacetoxyborhydrid (95 %ig) eingetragen. Nach 12 h Reaktionszeit bei RT wird der Ansatz unter Standardbedingungen wässrig aufgearbeitet und 3.7 g eines farblosen Öls (R)-2-[(3-Fluor-phenylamino)-methyl]-pyrrolidin-1-carbonsäure-tert.-butylester im nächsten Schritt ohne weitere Aufreinigung eingesetzt.

1.7 g (R)-2-[(3-Fluor-phenylamino)-methyl]-pyrrolidin-1-carbonsäure-tert.-butylester werden in 10 mL THF gelöst und mit 5 mL ethanolischer Salzsäure versetzt. Die Mischung wird für 4 h bei 80°C gerührt und zur Aufarbeitung bei Raumtemperatur zwischen 50 mL Wasser und 50 mL Essigester verteilt. Nach der üblichen Aufarbeitung und Aufreinigung durch Chromatographie an Kieselgel erhält man 400 mg eines hellbraunen Öls (3-Fluor-phenyl)-(R)-1-pyrrolidin-2-ylmethyl-amin; Rt.: 1.291 Min; [M+H]⁺ 195.2.

155 mg 6-Chlorpurin und 200 mg (3-Fluor-phenyl)-(R)-1-pyrrolidin-2-ylmethyl-amin werden in 40 mL 1-Butanol gelöst und mit 0.3 mL N-Ethyldiisopropylamin versetzt. Die Reaktionsmischung wird für 6 h in der Mikrowelle bei 120°C bestrahlt, wobei ein Druckanstieg (∼4 bar) zu verzeichnen ist. Nach beendeter Reaktion wird der Ansatz von flüchtigen Bestandteilen im Vakuum befreit und über Kieselgel chromatographiert. Man erhält 120 mg "A9"; Rt.: 1.825 Min; [M+H]⁺ 420.2;
¹H-NMR (500 MHz, d₆-DMSO) *δ* [ppm] 12.94 (br. s, 1H), 8.24 (s, 1H), 8.10 (s, 1H), 7.05 (q, 1H, J = 7.7 Hz), 6.76 (d, 1H, J = 12.4 Hz), 6.59 (dd, 1H, J = 1.2 Hz, J = 8.0 Hz), 6.29 (dt, 1H, J = 2.0 Hz, J = 8.2 Hz), 4.57 (m, 1H), 4.22 (m, 1H), 3.94 (m, 1H), 3.49 (m, 1H), 3.00 (m, 1H), 2.06 (m ,2H), 1.96 (m, 2H).

### Herstellung von "A10" :

2 g (R)-2-[(3-Fluor-phenylamino)-methyl]-pyrrolidin-1-carbonsäure-tert.-butylester werden mit 360 mg Natriumhydrid (60%ig in Mineralöl) in 50 mL THF für 30 min deprotoniert. Anschließend werden 0.9 mL lodmethan zugesetzt und 12 h bei RT gerührt. Es werden nochmals 0.9 g Kaliumcarbonat und 7.9 mL lodmethan zugeführt. Nach 12 h bei 50°C wird der Ansatz wässrig aufgearbeitet und 920 mg (R)-2-{[(3-Fluor-phenyl)-methyl-amino]-methyl}-pyrrolidin-1-carbonsäure-tert.-butylester erhalten, die ohne weitere Aufreinigung direkt umgesetzt werden; Rt.: 2.499 Min; [M+H]⁺ 309.2.

920 mg (R)-2-{[(3-Fluor-phenyl)-methyl-amino]-methyl}-pyrrolidin-1-carbon-säure-tert.-butylester werden in 10 mL THF gelöst und mit 5 mL ethanolischer Salzsäure versetzt. Die Mischung wird für 4 h bei 80°C gerührt und zur Aufarbeitung bei Raumtemperatur zwischen 50 mL Wasser und 50 mL Essigester verteilt. Nach der üblichen Aufarbeitung und Aufreinigung durch Chromatographie an Kieselgel erhält man 300 mg (3-Fluoro-phenyl)-methyl-(R)-1-pyrrolidin-2-ylmethyl-amin; Rt.: 1.377 Min; [M+H]⁺ 209.2.

232 mg 6-Chlorpurin und 300 mg (3-Fluor-phenyl)-methyl-(R)-1-pyrrolidin-2-ylmethyl-amin werden in 40 mL 1-Butanol gelöst und mit 0.3 mL N-Ethyldiisopropylamin versetzt. Die Reaktionsmischung wird für 6 h in der Mikrowelle bei 120°C bestrahlt, wobei ein Druckanstieg (∼4 bar) zu verzeichnen ist. Nach beendeter Reaktion wird der Ansatz von flüchtigen Bestandteilen im Vakuum befreit und über Kieselgel chromatographiert. Man erhält 250 mg "A10" (gelbe Kristalle);
¹H-NMR (500 MHz, d₆-DMSO) *δ* [ppm] 12.96 (br. s, 1H), 8.26 (s, 1H), 8.12 (s, 1H), 7.19 (q, 1H, J = 8.0 Hz), 7.05 (m, 1H), 6.82 (d, 1H, J = 6.5 Hz), 6.38 (dt, 1H, J = 2.0 Hz, J = 8.2 Hz), 5.31 (m, 1H), 4.65 (m, 1H), 4.21 (m, 1H), 4.00 (m, 1H), 3.84 (m, 1H), 3.06 (s, 3H), 2.17 (m, 1H), 2.00 (m, 1H), 1.91 (m, 2H).

### Herstellung von "A11" :

775 mg kommerziell erhältliches Diethyl-3-chlor-benzylphosphonat werden in 20 mL THF gelöst und unter Schutzgas bei -78°C mit 3 mL Lithium- Hexamethyldisilazan tropfenweise versetzt. Nach 1 h wird bei der angegebenen Temperatur eine Lösung von 500 mg (R)-2-Formyl-pyrrolidin-1-carbonsäure-tert.-butylester in 5 mL THF zugetropft. Der Ansatz wird für 3 h bei RT gerührt und anschließend wässrig aufgearbeitet. Man erhält 1 g (R)-2-[(E)-2-(3-Chlorphenyl)-vinyl]-pyrrolidin-1-carbonsäure-tert.-butylester als farbloses Öl, welches gleich weiter umgesetzt wird.

1 g (R)-2-[(E)-2-(3-Chlor-phenyl)-vinyl]-pyrrolidin-1-carbonsäure-tert.-butylester werden in 10 mL THF gelöst und mit 5 mL ethanolischer Salzsäure versetzt. Die Mischung wird für 4 h bei 80°C gerührt und zur Aufarbeitung bei Raumtemperatur zwischen 50 mL Wasser und 50 mL Essigester verteilt. Nach der üblichen Aufarbeitung und Aufreinigung durch Chromatographie an Kieselgel erhält man 750 mg (R)-2-[(E)-2-(3-Chlor-phenyl)-vinyl]-pyrrolidin; Rt.: 1.554 Min; [M+H]⁺ 208.2.

695 mg 6-Chlor-purin und 850 mg (R)-2-[(E)-2-(3-Chlor-phenyl)-vinyl]-pyrrolidin werden in 20 ml 1-Butanol gelöst und mit 3.1 ml Triethylamin für 6 h in der Mikrowelle auf 130°C erwärmt. Zur Aufarbeitung wird der Ansatz zwischen Essigester und Wasser verteilt, die organische Phase über Natriumsulfat getrocknet, abfiltriert und im Vakuum eingeengt. Der Rückstand wird chromatographisch aufgereinigt. Man erhält 220 mg 6-{(R)-2-[(E)-2-(3-Chlorphenyl)-vinyl]-pyrrolidin-1-yl}-9H-purin ("A11"); Rt: 1.778 Min; [M+H]⁺ 326.2; F. 194 - 196°;
¹H-NMR (500 MHz, d₆-DMSO) *δ* [ppm] 12.89 (br. S, 1H), 8.19 (s, 1H), 8.07 (s, 1H), 7.46 (s, 1H), 7.34 - 7.22 (m, 3H), 6.54 (dd, 1H, J = 4.9 Hz, J = 15.9 Hz), 6.37 (d, 1H, J = 15.9 Hz), 5.49 (m, 1H), 4.02 (m, 2H), 2.17 - 1.95, (m 4H).

### Beispiel 10

### Herstellung von 6-[(R)-2-((E)-2-Naphthalin-1-yl-vinyl)-pyrrolidin-1-yl]-9H-purin ("A12")

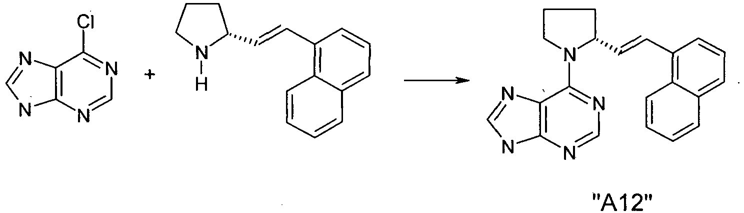

3.1 g 6-CI-Purin und 3.6 g (R)-2-((E)-2-Naphthalin-1-yl-vinyl)-pyrrolidin werden mit 6.8 mL N-Ethyldiisopropylamin in 50 mL 1-Butanol für 6h in der Mikrowelle auf 120°C erwärmt. Nach der üblichen wässrigen Aufarbeitung und chromatographischen Aufreinigung erhält man 1.8 g 6-[(R)-2-((E)-2-Naphthalin-1-yl-vinyl)-pyrrolidin-1-yl]-9H-purin ("A12") als farblose Kristalle; Rt.: 1.848 Min; [M+H]⁺ 342.2.

### Beispiel 11 (Vergleichsbeispiel)

### Herstellung von 7-[(R)-2-(2-Chlor-phenoxymethyl)-pyrrolidin-1-yl]-[1,2,5]thiadiazolo[3,4-b]pyridin ("A13")

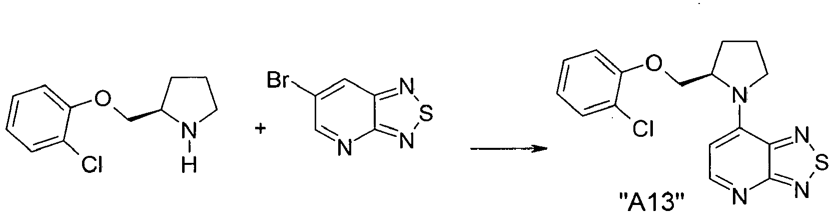

100 mg 6-Brom-[1,2,5]thiadiazolo[3,4-b]pyrimidin werden zusammen mit 402 mg (R)-2-(2-Chlor-phenoxymethyl)-pyrrolidin für 12h auf 100 °C erwärmt. Nach beendeter Reaktion wird die Mischung in Methanol aufgenommen und chromatographisch gereinigt. Man erhält 20 mg 7-[(R)-2-(2-Chlor-phenoxymethyl)-pyrrolidin-1-yl]-[1,2,5]thiadiazolo[3,4-b]pyridin (gelbe Kristalle); Rt: 1.830 Min; [M+H]⁺ 347.0;
¹H-NMR (500 MHz, d₆-DMSO) *δ* [ppm] 8.51 (d, 1H, J = 5.4 Hz), 7.37 (d, 1H, J = Hz); 7.22 (t, 1H, J = 7.9 Hz), 7.10 (d, 1H, J = 7.9 Hz), 6.92 (d, 1H, J = 7.9 Hz), 6.47 (d, 1H, J = 5.4 Hz), 5.41 (m, 1H), 4.30 (dd, 1H, J = 3.9 Hz, J = 9.6 Hz), 4.18 (dd, 1H, J = 6.0 Hz, J = 9.6 Hz), 3.90 (m, 1H), 3.69 (m, 1H), 2.37 (m, 1H), 2.22 (m, 2H), 2.10 (m, 1H).

### Beispiel 12

### Die Herstellung von 4-[(R)-1-(9H-Purin-6-yl)-pyrrolidin-2-ylmethoxy]-chinolin-2-carbonsäure-cyclopropylamid ("A14") erfolgt analog nachstehendem Schema

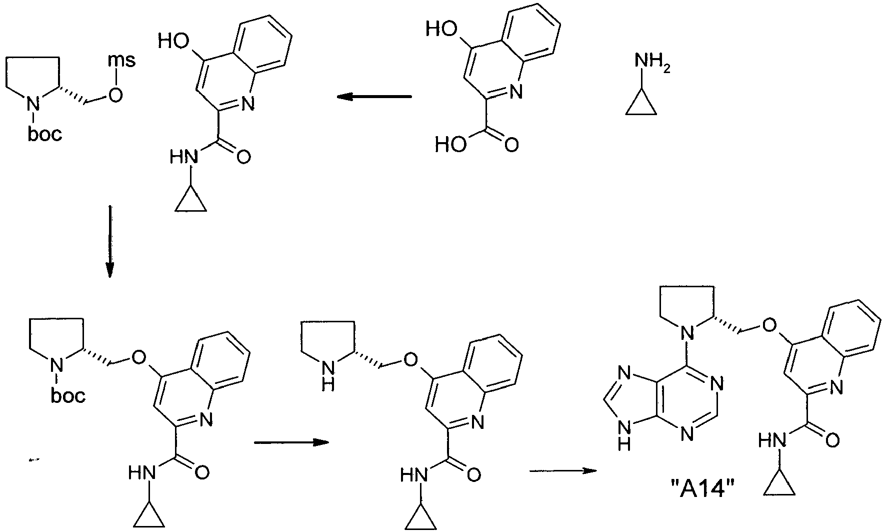

12.1 1 g kommerziell erhältlicher 4-Hydroxychinolin-2-carbonsäure und 0.37 mL kommerziell erhältliches Cyclopropylamin werden in 50 mL DMF zusammen mit 1.7 mL N-Methylmorpholin, 1 g N-(3-Dimethylaminopropyl)-N'-ethylcarbodiimidhydrochlorid und 0.7 g 1-Hydroxybenzotriazol für 12 h bei RT umgesetzt. Zur Aufarbeitung wird der Ansatz auf 50 mL Essigester und 50 mL Wasser gegossen. Die organische Phase wird mit gesättigter KHSO₄-Lösung extrahiert, über Magnesiumsulfat getrocknet und nach Entfernung des Lösungsmittels an Kieselgel chromatographiert. Man erhält 440 mg farbloser Kristalle; Rt.: 1.324 Min; [M+H]⁺ 229.2.
12.2 440 mg 4-Hydroxy-chinolin-2-carbonsäurecyclopropylamid und 539 mg (R)-2-Methansulfonyloxymethyl-pyrrolidin-1-carbonsäure-tert.-butylester werden mit Cäsiumcarbonat in DMF wie beschrieben umgesetzt und aufgereinigt. Man erhält 500 mg eines leicht gelben Öls (R)-2-(2-Cyclopropyl-carbamoyl-chinolin-4-yloxymethyl)-pyrrolidin-1-carbonsäure-tert.-butylester; Rt.: 2.274 Min; [M+H]⁺ 412.2.
12.3 500 mg (R)-2-(2-Cyclopropylcarbamoyl-chinolin-4-yloxymethyl)-pyrrolidin-1-carbonsäure-tert.-butylester werden mit Trifluoressigsäure in Dichlormethan umgesetzt und aufgearbeitet. Man erhält 80 mg eines braunen Öls 4-((R)-1-Pyrrolidin-2-ylmethoxy)-chinolin-2-carbonsäurecyclopropylamid, welches ohne weitere Aufreinigung direkt weiter umgesetzt wird.
12.4 77 mg 6-CI-Purin und 70 mg 4-((R)-1-Pyrrolidin-2-ylmethoxy)-chinolin-2-carbonsäurecyclopropylamid werden in 1-Butanol und Diisopropylethylamin wie beschrieben umgesetzt. Nach der üblichen Aufarbeitung erhält man 25 mg 4-[(R)-1-(9H-Purin-6-yl)-pyrrolidin-2-ylmethoxy]-chinolin-2-carbonsäurecyclo-propylamid Hydrochlorid ("A14"); Rt.:1.919 Min; [M+H]⁺ 458.2; F. 80-82°C.

### Beispiel 13

Die Herstellung von 2-[1-(1H-Imidazol-4-yl)-meth-(Z)-yliden]-5-[(R)-1-(9H-purin-6-yl)-pyrrolidin-2-ylmethoxy]-3,4-dihydro-2H-naphthalin-1-on ("A15") und 2-(1H-Imidazol-4-ylmethyl)-5-[(R)-1-(9H-purin-6-yl)-pyrrolidin-2-ylmethoxy]-3,4-dihydro-2H-naphthalin-1-on ("A15.1") erfolgt analog nachstehendem Schema 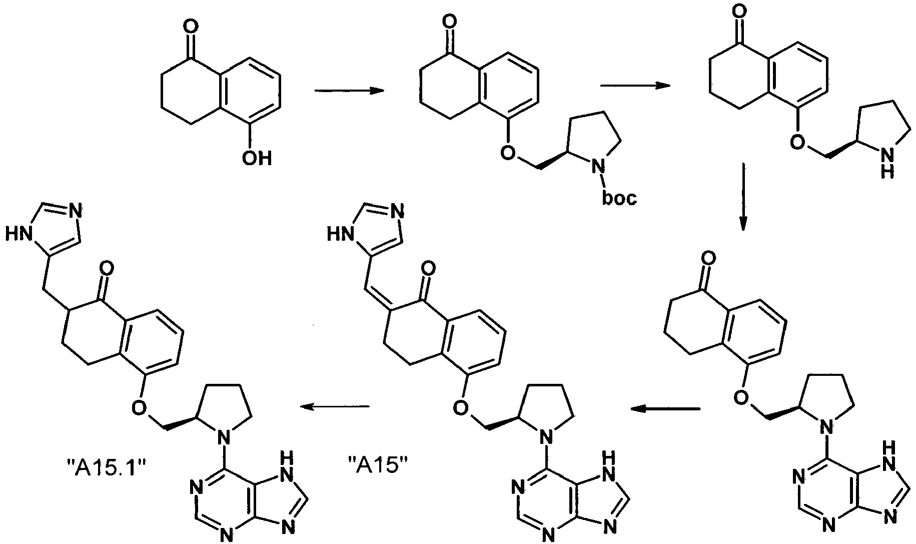
13.1 3.75 g kommerziell erhältliches 5-Hydroxy-3,4-dihydro-2H-naphthalin-1-on werden mit 10 g (R)-2-Methansulfonyloxymethyl-pyrrolidin-1-carbonsäure-tert.-butylester wie beschrieben in DMF mit Cäsiumcarbonat umgesetzt. Man isoliert 6.5 g (R)-2-(5-Oxo-5,6,7,8-tetrahydro-naphthalin-1-yloxymethyl)-pyrrolidin-1-carbonsäure-tert.-butylester; Rt.: 2.472 Min; [M+H]⁺ 346.2 (wird nur in Spuren detektiert, besonders ausgeprägt sind die Peaks der Zerfallsprodukte mit [M+H]⁺ = 290.2 und 246.2).
13.2 6.5 g (R)-2-(5-Oxo-5,6,7,8-tetrahydro-naphthalin-1-yloxymethyl)-pyrrolidin-1-carbonsäure-tert.-butylester werden in Dichlorethan mit Trifluoressigsäure wie beschrieben umgesetzt und aufgearbeitet. Man erhält 4.1 g 5-((R)-1-Pyrrolidin-2-ylmethoxy)-3,4-dihydro-2H-naphthalin-1-on; Rt.: 1.325 Min; [M+H]⁺ 246.2.
13.3 5.2 g 6-CI-Purin und 4.1 g 5-((R)-1-Pyrrolidin-2-ylmethoxy)-3,4-dihydro-2H-naphthalin-1-on werden wie beschrieben in 1-Butanol in der Mikrowelle umgesetzt und entsprechend aufgearbeitet und nach chromatographischer Aufreinigung werden 4.7 g 5-[(R)-1-(9H-Purin-6-yl)-pyrrolidin-2-ylmethoxy]-3,4-dihydro-2H-naphthalin-1-on isoliert; F. 205 - 206°; Rt.: 1.593 Min; [M+H]⁺ 364.2;
   ¹H-NMR (500 MHz, d₆-DMSO) *δ* [ppm] 12.96 (br. s, 1H), 8.23 (s, 1H), 8.11 (s, 1H), 7.45 (d, 1H, J = 7.4 Hz), 7.30 (m, 1H), 7.26 (m, 1H), 5.38 (m, 1H), 4.80 (m, 1H), 4.37 (dd, 1H, J = 3.1 Hz, J = 9.4 Hz), 4.13 (dd, 1H, J = 7.3 Hz, J = 8.5 Hz), 3.78 (m, 1H), 2.82 (m, 2H), 2.56 (dd, 2H, J = 5.5 Hz, J = 7.8 Hz), 2.17 (m, 3H), 2.02 (m, 3H).
13.4 500 mg 5-[(R)-1-(7H-Purin-6-yl)-pyrrolidin-2-ylmethoxy]-3,4-dihydro-2H-naphthalin-1-on und 520 mg 3H-Imidazol-4-carbaldehyd werden mit 270 mg Natriumhydroxid in 10 mL Wasser und 3 mL Ethanol für 3 h zum Rückfluß erhitzt. Nach dem Abkühlen wird mit konz. wässriger HCl der pH Wert auf 3 eingestellt und 30 Minuten gerührt. Nach dem Entfernen des Alkohols im Vakuum wird mit wässriger NaOH neutralisiert und bis zum Rückstand eingedampft. Der Rückstand wird an Kieselgel chromatographiert. Man erhält 180 mg farblose Kristalle 2-[1-(1H-Imidazol-4-yl)-meth-(Z)-yliden]-5-[(R)-1-(9H-purin-6-yl)-pyrrolidin-2-ylmethoxy]-3,4-dihydro-2H-naphthalin-1-on ("A15"); F. 195 - 196.5°; Rt.: 1.403 Min; [M+H]⁺442.2;
   ¹H-NMR (500 MHz, d₆-DMSO) *δ* [ppm] 13.00 (br. s, 1H), 12.54 (br. s, 1H), 8.25 (s, 1H), 8.13 (s, 1H), 7.66 (s, 1H), 7.84 (s, 1H), 7.54 (m, 2H), 7.31 (m, 2H), 5.38 (br. m, 1H), 4.83 (br. m, 1H), 4.39 (dd, 1H J = 2.7 Hz, J = 9.2 Hz), 4.15 (t, 1H, J = 8.1 Hz), 3.79 (br. m, 1H), 3.46 (m, 2H), 2.88 (m, 2H), 2.18 (m, 2H), 2.04 (m ,2H).
13.5 100 mg 2-[1-(1H-Imidazol-4-yl)-meth-(Z)-yliden]-5-[(R)-1-(9H-purin-6-yl)-pyrrolidin-2-ylmethoxy]-3,4-dihydro-2H-naphthalin-1-on werden zusammen mit 50 mg Zinkpulver in 10 ml konz. Essigsäure und 5 ml Wasser für 30 Min. auf 80°C erhitzt. Hierbei ändert sich die Farbe von grünlich nach gelblich. Der Ansatz wird mit konz. NaOH Lösung auf pH 7 eingestellt und mit Ethylacetat erschöpfend extrahiert. Die organische Phase wird über Magnesiumsulfat getrocknet und im Vakuum vom Lösungsmittel befreit. Man erhält 50 mg 2-(1H-Imidazol-4-ylmethyl)-5-[(R)-1-(9H-purin-6-yl)-pyrrolidin-2-ylmethoxy]-3,4-dihydro-2H-naphthalin-1-on ("A15.1") als farbloses Öl. Rt.: 1.405 Min; [M+H]⁺ 444.2.

### Analog den Beispielen 1-13 werden die nachstehenden Verbindungen erhalten

c

| Verbindung Nr. | Struktur und/oder Name | F. [°C] | HPLC-MS; rt; [M+H⁺]* |
|---|---|---|---|
| "A18" | 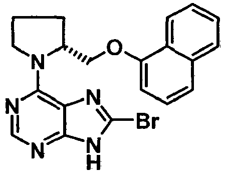 | | 2.025 min |
| | | | [424.0; 426.0] |
| | 8-Brom-6-[(R)-2-(naphthalin-1-yloxymethyl)-pyrrolidin-1-yl]-9H-purin | | |
| "A19" | 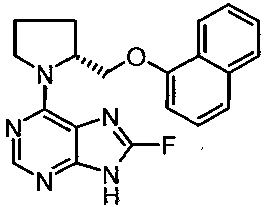 | | |
| "A20" | 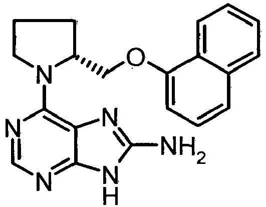 | | |
| "A23" | 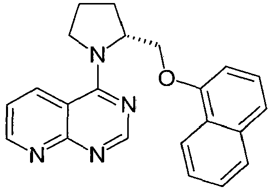 | | |
| | 4-[(R)-2-(Naphthalin-1-yloxymethyl)-pyrrolidin-1-yl]-pyrido[2,3-d]pyrimidin | | |
| ¹H NMR (500 MHz, d₆-DMSO) δ [ppm] 8.96 (dd, 1H, J = 1.4 Hz, J = 4.1 Hz), 8.70 (dd, 1H, J = 1.4 Hz, J = 8.4 Hz), 8.66 (s, 1H), 8.14 (m, 1H), 7.84 (m, 1H), 7.49 (m, 4H), 7.37 (m, 1H), 7.05 (d, 1H, J = 7.6 Hz), 5.14 (m, 1H), 4.56 (dd, 1H, J = 3.3 Hz, J = 9.6 Hz), 4.37 (dd, 1H, J = 6.5 Hz, J = 9.6 Hz), 4.24 (m, 1H), 4.02 (m, 1H), 2.25 (m, 3H), 2.01 (m, 1H) | | | |
| "A25" | 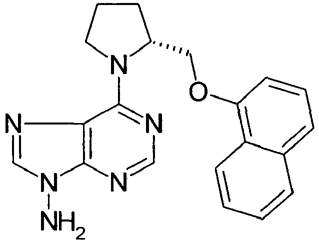 | | 3.95** Min |
| | 6-[(R)-2-(Naphthalin-1-yloxymethyl)-pyrrolidin-1-yl]-purin-9-ylamin | | |
| "A26" | 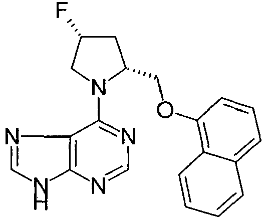 | 166-169 | 1.983 Min |
| | | | [364.2] |
| | 6-[(2R,4R)-4-Fluor-2-(naphthalin-1-yloxymethyl)-pyrrolidin-1-yl]-9H-purin | | |
| ¹H NMR (500 MHz, d₆-DMSO) δ [ppm] 13.08 (br. s, 1H), 8.31 (s, 1H), 8.17 (s, 1H), 7.87 (m, 2H), 7.56 - 7.37 (m, 5H), 7.11 (m, 1H), 5.59 (d, 1H, J = 54.2 Hz), 4.85 - 4.73 (m, 1H), 4.10 (t, 1H, J = 9.4 Hz) | | | |
| "A28" | 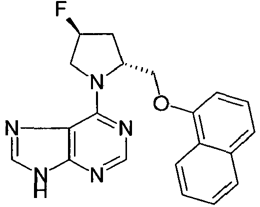 | 168-172 | 1.949 Min |
| | | | [364.2] |
| | 6-[(2R,4S)-4-Fluor-2-(naphthalin-1-yloxymethyl)-pyrrolidin-1-yl]-9H-purin | | |
| ¹H NMR (500 MHz, d₆-DMSO) δ [ppm] 8.26 (s, 1H), 8.16 (s, 1H), 8.09 (br. s, 1H), 7.84 (dd, 1H, *J* = 2.0 Hz, *J* = 6.4 Hz), 7.50 (m, 5H), 7.36 (t, 1H, *J*= 8.0Hz), 5.63 (d, 1H, J = 54.0 Hz), 4.50 - 4.55 (m, 7H) | | | |
| "A30" | 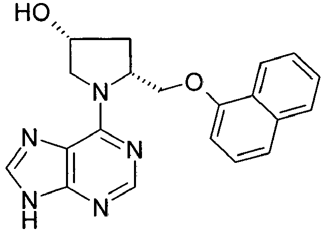 | 132-134 | 1.640 Min |
| | | | [362.2] |
| | (3R,5R)-5-(Naphthalin-1-yloxymethyl)-1-(9H-purin-6-yl)-pyrrolidin-3-ol | | |
| "A31" | 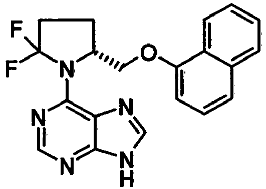 | | |
| | 6-[(R)-2,2-Difluor-5-(naphthalin-1-yloxymethyl)-pyrrolidin-1-yl]-9H-purin | | |
| "A33" | 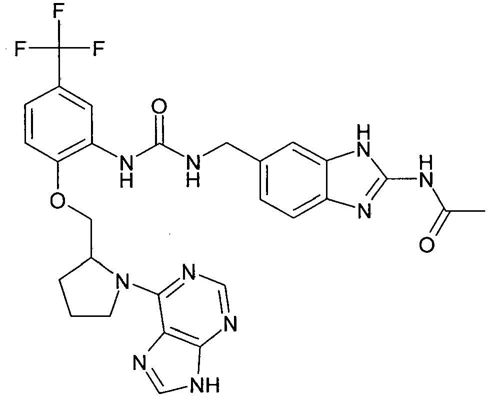 | 190-192 | 1.733 Min |
| | | | [609.2] |
| | N-[6-(3-{2-[1-(9H-Purin-6-yl)-pyrrolid in-2-ylmethoxy]-5-trifluormethyl-phenyl}-ureidomethyl)-1H-benzimidazol-2-yl]-acetamid | | |
| ¹H NMR (500 MHz, d₆-DMSO) δ [ppm] 12.97 (br. s, 1H), 11.95 (d, 1H, J = 9.8 Hz), 11.46 (m, 1H), 8.57 (m, 1H), 8.25 (m, 1H), 8.11 (m, 1H), 7.59 (br. s, 1H), 7.56 (m, 1H), 7.38 (m, 1H), 7.23 (m, 1H), 7.07 (m, 1H), 5.36 (m, 1H), 4.77 (m, 1H), 4.52 (dd, 1H, J = 3.6 Hz, J = 9.4 Hz), 4.38 (m, 2H), 3.99 (t, 1H, 9.8 Hz), 3.74 (m, 1H), 2.15 (s, 3H), 2.04 (m, 4) | | | |
| "A34" | 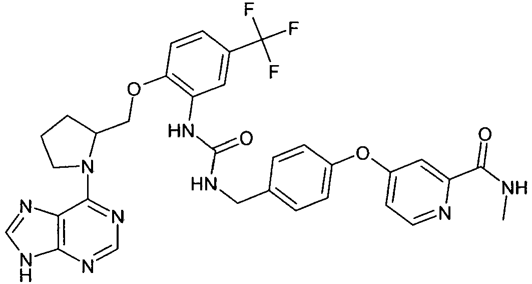 | 180-183 | 1.975 Min |
| | | | [662.2] |
| | 4-[4-(3-{2-[1-(9H-Purin-6-yl)-pyrrolidin-2-ylmethoxy]-5-trifluormethyl-phenyl}-ureidomethyl)-phenoxy]-pyridin-2-carbonsäure-methylamid | | |
| ¹H NMR (500 MHz, d₆-DMSO) δ [ppm] 12.99 (br. s, 1H), 8.74 (br. q, 1H, J = 4.9 Hz), 8.55 (d, 1H, J = 2.0 Hz), 8.51 (d, 1H, J = 5.7 Hz), 8.27 (br. s, 1H), 8.14 (s, 2H), 7.69 (br. s, 1H), 7.48 (d, 3H, J = 8.4 Hz), 7.38 (d, 1H, J = 2.6 Hz), 7.26 (br. s, 1H), 7.23 (d, 2H, J = 8.4 Hz), 7.17 (dd, 1H, J = 2.5 Hz, J = 5.5 Hz), 5.36 (m, 1H), 4.81 (m, 1H), 4.57 (dd, 1H, J = 3.5 Hz, J = 9.6 Hz), 4.39 (m, 2H), 4.01 (t, 1H, J = 9.7 Hz), 3.73 (m, 1H), 2.78 (d, 3H, J = 4.9 Hz), 2.07 (m, 4H) | | | |
| "A35" | 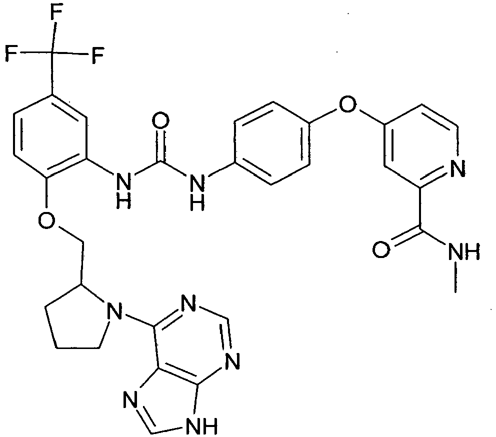 | | |
| | 4-[4-(3-{2-[1-(9H-Purin-6-yl)-pyrrolid in-2-ylmethoxy]-5-trifluormethyl-phenyl}-ureido)-phenoxy]-pyridin-2-carbonsäure-methylamid | | |
| "A36" | 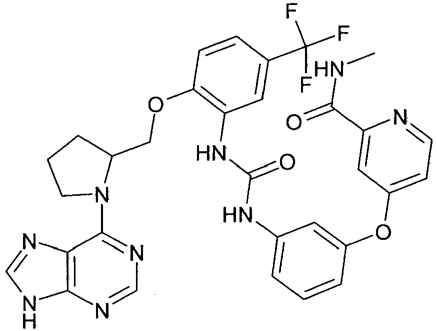 | | |
| | 4-[3-(3-{2-[1-(9H-Purin-6-yl)-pyrrolid in-2-ylmethoxy]-5-trifluormethyl-phenyl}-ureido)-phenoxy]-pyridin-2-carbonsäure-methylamid | | |
| "A37" | 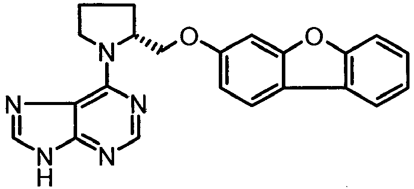 | 237-238 | 2.069 Min |
| | | | [386.2] |
| | 6-[(R)-2-(Dibenzofuran-3-yloxymethyl)-pyrrolidin-1-yl]-9H-purin | | |
| ¹H NMR (500 MHz, d₆-DMSO) δ [ppm] 12.98 (br. s, 1H), 8.27 (s, 1H), 8.13 (s, 1H), 8.08 (d, 1H, J = 7.6 Hz), 7.80 (br. s, 1H), 7.65 (d, 1H, J= 8.4 Hz), 7.59 (d, 1H, J = 8.9 Hz), 7.50 (dd, 1H, J = 1.2 Hz, J = 7.3 Hz), 7.36 (t, 1H, J = 7.3 Hz), 7.17 (br. dd, J = 1.4 Hz, J = 8.8 Hz), 5.36 (br. m, 1H), 4.82 (br. m, 1H), 4.46 (dd, 1H, J = 3.1 Hz, J = 9.1 Hz), 4.12 (t, 1H, J = 8.6 Hz), 3.80 (br. m, 1H), 2.20 (br. m, 1H), 2.15 (br. m, 2H), 2.04 (br. m, 1H) | | | |
| "A38" | 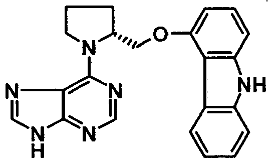 | 165-167 | 1.862 Min |
| | | | [385.2] |
| | 4-[(R)-1-(9H-Purin-6-yl)-pyrrolidin-2-ylmethoxy]-9H-carbazol | | |
| ¹H NMR (500 MHz, d₆-DMSO) δ [ppm] 12.99 (br. s, 1H), 11.26 (br. s, 1H), 8.26 (s, 1H), 8.14 (br. m, 1H), 8.12 (s, 1H), 7.45 (d, 1H, J= 8.2 Hz), 7.34 (dd, 1H, J= 0.8 Hz, J= 7.1 Hz), 7.26 (br. t, 1H, J = 8.0 hz), 7.14 (br. t, 1H, J = 7.4 Hz), 7.06 (d, 1H, J = 8.0 Hz), 6.79 (br. m, 1H), 5.52 (br. m, 1H), 4.97 (br. m, 1H), 4.61 (dd, 1H, J = 3.6 Hz, J = 8.9 Hz), 4.22 (m, 1H), 3.83 (br. m, 1H), 2.24 (m, 3H), 2.06 (m, 1H) | | | |
| "A39" | 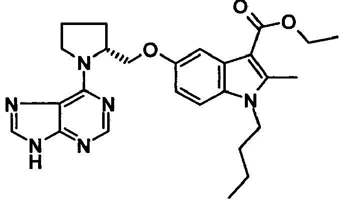 | 110-111 | 2.107 Min |
| | | | [477.2] |
| | 1-Butyl-2-methyl-5-[(R)-1-(9H-purin-6-yl)-pyrrolidin-2-ylmethoxy]-1 H-indol-3-carbonsäure-ethylester | | |
| ¹H NMR (500 MHz, d₆-DMSO) δ [ppm] 12.94 (br. s, 1H), 8.23 (s, 1H), 8.09 (s, 1H), 7.48 (s, 1H), 7.40 (d, 1H, J = 8.7 Hz), 6.88 (br. d, 1H, J = 8.7 Hz), 5.28 (br. m, 1H), 4.78 (br. m, 1H), 4.34 (dd, 1H, J = 3.0 Hz, J = 8.9 Hz), 4.24 (q, 2H, J = 7.0 Hz), 4.14 (t, 2H, J = 7.0 Hz), 4.06 (br. t, 1H, J = 8.1 Hz), 3.80 (br. m, 1H), 2.68 (s, 3H), 2.19 (m, 1H), 2.12 (m, 2H), 2.01 (1H), 1.62 (quint., 2H, J = 7.3 Hz), 1.29 (m, 7H), 0.88 (t, 3H, J = 7.0 Hz) | | | |
| "A40" | 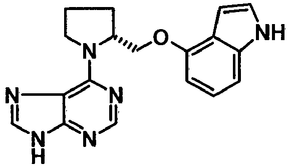 | 214-216 | 1.554 Min |
| | | | [335.2] |
| | 6-[(R)-2-(1H-Indol-4-yloxymethyl)-pyrrolidin-1-yl]-9H-purin | | |
| ¹H NMR (500 MHz, d₆-DMSO) δ [ppm] 12.94 (br. s, 1H), 11.03 (br. s, 1H), 8.23 (s, 1H), 8.09 (s, 1H), 7.19 (m, 1H), 6.95 (m, 2H), 5.53 (br. m, 1H), 6.38 (m, 1H), 5.36 (br. m, 1H), 4.84 (br. m, 1H), 4.39 (dd, 1H, J = 3.2 Hz, J = 9.0 Hz), 4.19 (dd, 1H, J = 7.0 Hz, J= 9.0 Hz), 4.04 (br. m, 1H), 2.25 (m, 1H), 2.1 5 (m, 2H), 2.04 (m, 1H). | | | |
| "A41" | 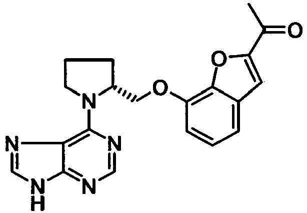 | 210-212 | 1.599 Min |
| | | | [378.2] |
| | 1-{7-[(R)-1-(9H-Purin-6-yl)-pyrrolidin-2-y)methoxy]-benzofuran-2-y)}-ethanon | | |
| ¹H NMR (500 MHz, d₆-DMSO) δ [ppm] 12.96 (br. s, 1H), 8.24 (s, 1H), 8.11 (s, 1H), 7.87 (s, 1H), 7.36 (d, 1H, J = 8.0 Hz), 7.26 (br. m, 2H), 5.38 (br. m, 1H), 4.84 (br. m, 1H), 4.53 (dd, 1H, J = 3.1 Hz, J = 9.2 Hz), 4.27 (dd, 1H, J = 8.2 Hz, J = 9.2 Hz), 3.79 (br. m, 1H), 2.56 (s, 3H), 2.24 (m, 1H), 2.16 (m, 1H), 2.04 (m, 2H) | | | |
| "A42" | 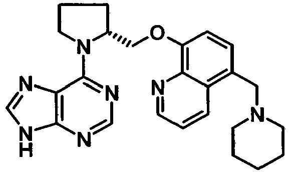 | | 1.226 Min. |
| | | | [444.2] |
| | 5-Piperidin-1-ylmethyl-8-[(R)-1-(9H-purin-6-yl)-pyrrolidin-2-ylmethoxy]-chinolin Formiat | | |
| "A43" | 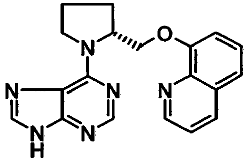 | 222-223 | 1.229 Min |
| | | | [347.2] |
| | 8-[(R)-1-(9H-Purin-6-yl)-pyrrolidin-2-ylmethoxy]-chinolin | | |
| "A44" | 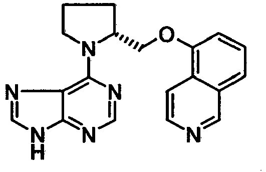 | 154-155 | 1.390 Min |
| | | | [347.2] |
| | 5-[(R)-1-(9H-Purin-6-yl)-pyrrolidin-2-ylmethoxy]-isochinolin | | |
| ¹H NMR (500 MHz, d₆-DMSO) δ [ppm] 13.04 (br. s, 1H) 9.26 (s, 1H), 8.51 (d, 1H, J = 5.8 Hz), 8.25 (s, 1H), 8.13 (s, 1H), 7.91 (br. s, 1H), 7.65 (d, 1H, J = 8.2 Hz), 7.56 (t, 1H, J = 7.9 Hz), 7.36 (br. s, 1H), 5.34 (br. m, 1H), 4.93 (br. m, 1H), 4.54 (dd, 1H, J = 3.2 Hz, J = 9.2 Hz), 4.29 (br. t, 1H, J = 8.2 Hz), 3.84 (br. m, 1H), 2.23 (br. m, 3H), 2.07 (br. m, 1H). | | | |
| "A45" | 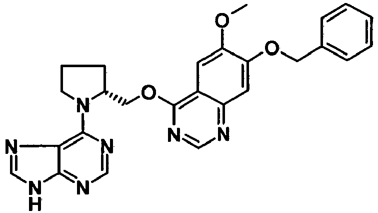 | 161-163 | 1.951** |
| | 7-Benzyloxy-6-methoxy-4-[(R)-1-(9H-purin-6-yl)-pyrrolidin-2-ylmethoxy]-chinazolin | | |
| "A46" | 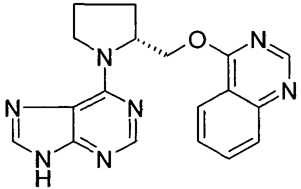 | | 1.456 Min |
| | | | [348.2] |
| | 4-[(R)-1-(9H-Purin-6-yl)-pyrrolidin-2-ylmethoxy]-chinazolin | | |
| "A47" | 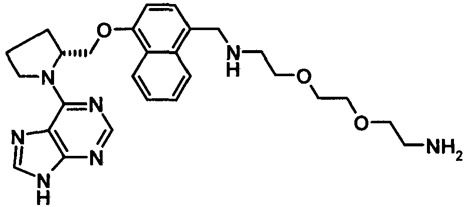 | | 1.403 Min |
| | | | [506.2] |
| | 2-{2-[2-({4-[(R)-1-(9H-Purin-6-yl)-pyrrolidin-2-ylmethoxy]-naphthalin-1-ylmethyl}-amino)-ethoxy]-ethoxy}-ethylamin | | |
| "A48" | 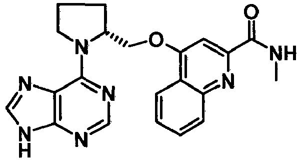 | | 1.555 Min |
| | | | [404.2] |
| | 4-[(R)-1-(9H-Purin-6-yl)-pyrrolidin-2-ylmethoxy]-chinolin-2-carbonsäure-methylamid Formiat | | |
| "A49" | 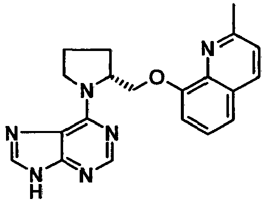 | 130-131 | 1.214** |
| | 2-Methyl-8-[(R)-1-(9H-purin-6-yl)-pyrrolidin-2-ylmethoxy]-chinolin | | |
| "A50" | 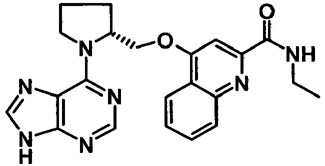 | | 1.626 Min |
| | | | [418.2] |
| | 4-[(R)-1-(9H-Purin-6-yl)-pyrrolidin-2-ylmethoxy]-chinolin-2-carbonsäure-ethylamid Formiat | | |
| ¹H NMR (500 MHz, d₆-DMSO) δ [ppm] 12.99 (br. s, 1H), 8.85 (t, 1H, J = 5.9 Hz), 8.34 (br. s, 2H), 8.14 (br. m, 1H), 8.06 (d, 1H, J = 8.8 Hz), 7.84 (ddd, 1H, J = 1,1 Hz, J = 7.0 Hz, J = 8.3 Hz), 7.66 (t, 1H, J = 7.6 Hz), 5.44 (br. m, 1H), 4.88 (br. m, 1H), 4.74 (dd, 1H, J = 3.3 Hz, J = 9,6 Hz), 4.46 (br. t, 1H), 3.98 (br. m, 1H), 3.38 (q, 2H, J = 6.7 Hz), 2.25 (br. m, 2H), 2.19 (br. m, 1H), 2.07 (br. m, 1H), 1.17 (t, 3H, J = 7.2 Hz) | | | |
| "A51" | 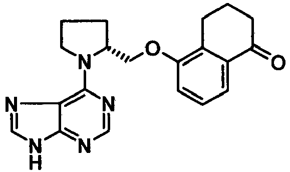 | 205-206 | 1.593 Min |
| | | | [364.2] |
| | 5-[(R)-1-(9H-Purin-6-yl)-pyrrolidin-2-ylmethoxy]-3,4-dihydro-2H-naphthalin-1-on | | |
| ¹H NMR (500 MHz, d₆-DMSO) δ [ppm] 12.96 (br. s, 1H), 8.23 (s, 1H), 8.11 (s, 1H), 7.45 (d, 1H, J = 7.4 Hz), 7.30 (m, 1H), 7.26 (m, 1H), 5.38 (m, 1H), 4.80 (m, 1H), 4.37 (dd, 1H, J = 3.1 Hz, J = 9.4 Hz), 4.13 (dd, 1H, J = 7.3 Hz, J = 8.5 Hz), 3.78 (m, 1H), 2.82 (m, 2H), 2.56 (dd, 2H, J = 5.5 Hz, J = 7.8 Hz), 2.17 (m, 3H), 2.02 (m, 3H) | | | |
| "A52" | 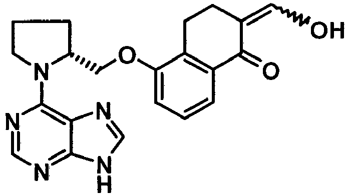 | | |
| | 2-Hydroxymethylen-5-[(R)-1-(9H-purin-6-yl)-pyrrolidin-2-ylmethoxy]-3,4-dihydro-2H-naphthalin-1-on | | |
| "A53" | 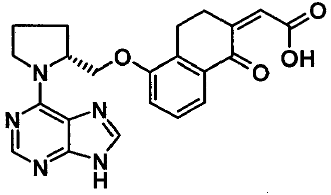 | 256-257 | 1.582 Min |
| | | | [420.2] |
| | [1-Oxo-5-[(R)-1-(9H-purin-6-yl)-pyrrolidin-2-ylmethoxy]-3,4-dihydro-1H-naphthalin-(2Z)-yliden]-essigsäure | | |
| ¹H NMR (500 MHz, d₆-DMSO) δ [ppm] 8.23 (s, 1H), 8.11 (s, 1H), 7.50 (d, 1H), 7.28 (m, 2H), 6.70 (s, 1H), 5.33 (br. m, 1H), 4.85 (br. m, 1H), 4.37 (dd, 1H, J = 3.5 Hz, J = 9.3 Hz), 4.12 (t, 1H, J = 8.1 Hz), 3.84 (br. m, 1H), 3.00 (m, 2H), 2.77 (m, 2H), 2.16 (m, 3H), 2.02 (m, 1H) | | | |
| "A54" | 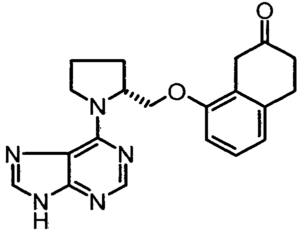 | | |
| | 8-[(R)-1-(9H-Purin-6-yl)-pyrrolidin-2-ylmethoxy]-3,4-dihydro-1H-naphthalin-2-on | | |
| "A55" | 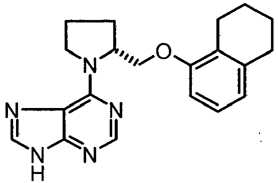 | 138-140 | 1.902 Min |
| | | | [350.2] |
| | 6-[(R)-2-(5,6,7,8-Tetrahydro-naphthalin-1-yloxymethyl)-pyrrolidin-1-yl]-9H-purin | | |
| ¹H NMR (500 MHz, d₆-DMSO) δ [ppm] 8.27 (s, 1H), 8.17 (s, 1H), 6.98 (t, 1H, J = 7.8 Hz), 6.75 (m, 1H), 6.62 (d, 1H, J = 7.8 Hz), 5.36 (m, 1H), 4.79 (m, 1H), 4.25 (dd, 1H, J = 3.5 Hz, J = 9.3 Hz), 4.07 (dd, 1H, J = 6.9 Hz, J = 9.3 Hz), 3.79 (m, 1H), 2.65 (m, 4H), 2.20 - 2.00 (m, 4H), 1.68 (m, 4H) | | | |
| "A56" | 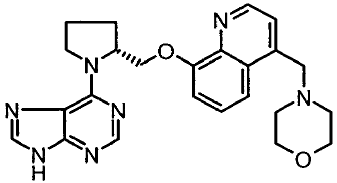 | 112-114 | 1.090** |
| | 4-Morpholin-4-ylmethyl-8-[(R)-1-(9H-purin-6-yl)-pyrrolidin-2-ylmethoxy]-chinolin | | |
| "A57" | 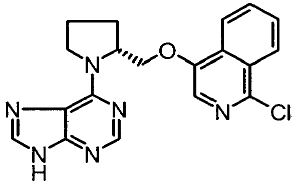 | 102-104 | 1.761 Min |
| | | | [381.2] |
| | 1-Chloro-4-[(R)-1-(9H-purin-6-yl)-pyrrolidin-2-ylmethoxy]-isochinolin | | |
| ¹H NMR (500 MHz, d₆-DMSO) δ [ppm] 12.99 (br. s, 1H), 8.73 (s, 1H), 8.65 (s, 1H), 8.25 (s, 1H), 8.22 (m, 1H), 8.12 (m, 1H), 8.09 (m, 1H), 7.92 (m, 1H), 4.62 (dd, 1H, J = 3.5 Hz, J = 9.2 Hz), 4.45 (dd, 1H, J = 7.9 Hz, J = 8.8 Hz), 4.38 (dd, 1H, J= 7.9 Hz, J = 8.8 Hz), 4.16 (dd, J = 3.5 Hz, J = 8.8 Hz), 3.88 (m, 1H), 2.23 (m, 4H) | | | |
| "A58" | 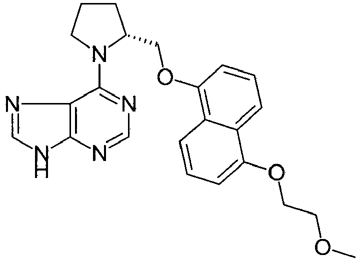 | 206-207 | 1.589 Min |
| | | | [313.2] |
| | 6-{(R)-2-[5-(2-Methoxy-ethoxy)-naphthalin-1-yloxymethyl]-pyrrolidin-1-yl}-9H-purin | | |
| ¹H NMR (500 MHz, d₆-DMSO) δ [ppm] 12.95 (br. s, 1H), 8.24 (s, 1H), 8.12 (s, 1H), 7.71 (m, 2H), 7.38 (m, 2H), 7.08 (m, 1H), 7.00 (d, 1H, J = 7.7 Hz), 5.43 (m, 1H), 4.92 (m, 1H), 4.50 (dd, 1H, J = 3.2 Hz, J = 9.1 Hz), 4.27 (m, 4H), 3.81 (m, 2H), 3.38 (s ,3H), 2.25 (m, 3H), 2.07 (m, 1H) | | | |
| "A59" | 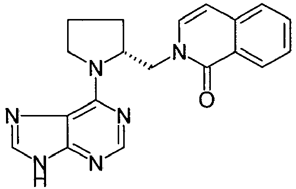 | 134-136 | 1.615 Min |
| | | | [347.2] |
| | 2-[(R)-1-(9H-Purin-6-yl)-pyrrolidin-2-ylmethyl]-2H-isochinolin-1-on | | |
| ¹H NMR (500 MHz, d₆-DMSO) δ [ppm] | | | |
| "A60" | 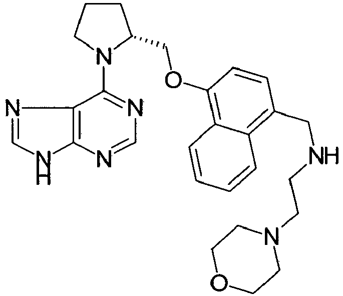 | 106-108 | 1.331 min |
| | | | [488.2] |
| | (2-Morpholin-4-yl-ethyl)-{4-[(R)-1-(9H-purin-6-yl)-pyrrolidin-2-ylmethoxy]-naphthalin-1-ylmethyl}-amin | | |
| ¹H NMR (500 MHz, d₆-DMSO) δ [ppm] 12.93 (br. s, 1H), 8.23 (s, 1H), 8.18 (s, 1H), 8.14 (d, 1H, J = 8.4 Hz), 8.10 (s, 1H), 7.57 (m, 1H), 7.52 (m, 1H), 7.37 (d, 1H, J = 7.6 Hz), 7.00 (m 1H), 5.47 (m, 1H), 4.90 (m, 1H), 4.50 (dd, 1H, J = 3.4 Hz, J = 9.2 Hz), 4.25 (m, 1H), 4.12 (m, 2H), 3.51 (m, 4H), 3.31 (m, 4H), 2.73, m, 2H), 2.42 (m, 1H), 2.31 (m, 4H), 2.23 (m 1H), 2.07 (m, 1H) | | | |
| "A61" | 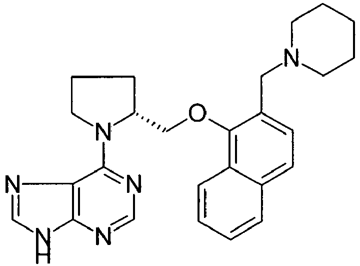 | | 1.544 Min |
| | | | [443.2] |
| | 6-[(R)-2-(2-Piperidin-1-ylmethyl-naphthalin-1-yloxymethyl)-pyrrolidin-1-yl]-9H-purin | | |
| "A62" | 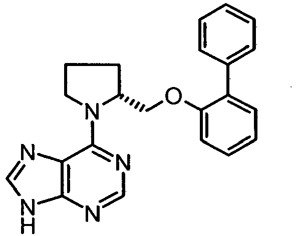 | 154-156 | 1.849 Min |
| | | | [372.2] |
| | 6-[(R)-2-(Biphenyl-2-yloxymethyl)-pyrrolidin-1-yl]-9H-purin | | |
| ¹H NMR (500 MHz, d₆-DMSO) δ [ppm] 12.95 (br. S, 1H), 8.22 (s, 1H), 8.10 (s, 1H), 7.47 (m, 2H), 7.41 (t, 2H, J = 7.6 Hz), 7.34 - 7.26 (m, 4H), 7.01 (t, 2H, J= 7.6 Hz), 5.20 (m, 1H), 4.65 (m, 1H), 4.31 (m, 1H), 4.17 (m, 1H), 3.61 (m, 1H), 2.03 - 1.82 (m, 4H) | | | |
| "A63" | 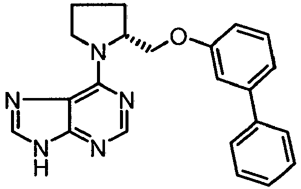 | 152-153 | 1.930 Min |
| | | | [372.2] |
| | 6-[(R)-2-(Biphenyl-3-yloxymethyl)-pyrrolidin-1-yl]-9H-purin | | |
| ¹H NMR (500 MHz, d₆-DMSO) δ [ppm] 12.96 (br. S, 1H), 8.22 (s, 1H), 8.09 (s, 1H), 7.63 (d, 2H, J = 7.7 Hz), 7.44 (t, 2H, J = 7.7.Hz), 7.35 (t, 2H, J = 7.7 Hz), 7.21 (d, 1H, J = 7.7 Hz), 7.00 (d, 1H = J = 7.7 Hz), 5.30 (m, 1H), 4.79 (m, 1H), 4.43 (dd, 1H, J = 3.3 Hz, J = 9.6 Hz), 4.11 (dd, 1H, J= 8.6 Hz, J = 9.6 Hz), 3.78 (m, 1H), 2.20 - 2.00 (m, 4H) | | | |
| "A64" | 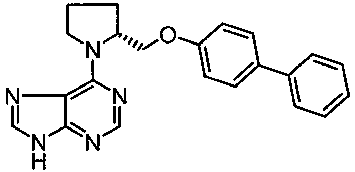 | 255-256 | 1.970 Min |
| | 6-[(R)-2-(Biphenyl-4-yloxymethyl)-pyrrolidin-1-yl]-9H-purin | | [372.2] |
| ¹H NMR (500 MHz, d₆-DMSO) δ [ppm] 8.24 (s, 1H), 8.10 (s, 1H), 7.59 (m, 4H), 7.42 (m, 2H), 7.29 (m, 1H), 7.11 (d, 2H, J = 8.5 Hz), 5.00 (m, 1H), 4.39 (dd, 1H, J = 3.0 Hz, J = 9.1 Hz), 4.06 (dd, 1H, J =8.2 Hz, J = 9.1 Hz), 3.90 (m, 1H), 3.34 (m, 1H), 2.20 - 2.00 (m, 4H) | | | |
| "A65" | 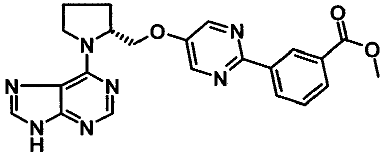 | 210-211 | 1.764 Min |
| | | | [432.2] |
| | 3-{5-[(R)-1-(9H-Purin-6-yl)-pyrrolidin-2-ylmethoxy]-pyrimidin-2-yl}-benzoesäure-methylester | | |
| ¹H NMR (500 MHz, d₆-DMSO) δ [ppm] 12.99 (br. s, 1H), 8.90 (s, 1H), 8.75 (s, 2H), 8.54 (d, 1H, J = 7.7 Hz), 8.25 (s, 1H), 8.12 (s, 1H), 8.04 (d, 1H, J = 7.7 Hz), 7.65 (t, 1H, J = 7.7 Hz), 5.33 (br. m, 1H), 4.80 (br. m, 1H), 4.56 (d, 1H, J = 7.7 Hz), 4.27 (t, 1H, J = 8.8 Hz), 4.06 (br. m, 1H), 3.90 (s, 3H), 2.15 (br. m, 3H), 2.03 (br. m, 1H) | | | |
| "A66" | 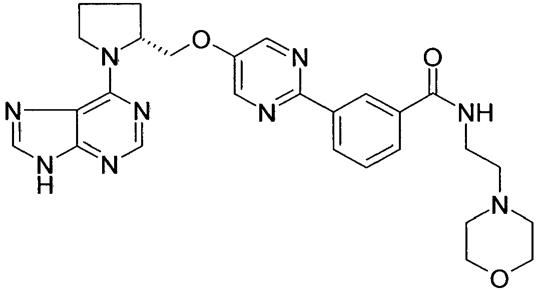 | 144-145 | 1.410 Min |
| | | | [530.2] |
| | N-(2-Morpholin-4-yl-ethyl)-3-{5-[(R)-1-(9H-purin-6-yl)-pyrrolidin-2-ylmethoxy]-pyrimidin-2-yl}-benzamid | | |
| ¹H NMR (500 MHz, d₆-DMSO) δ [ppm] 8.75 (s, 3H), 8.54 (t, 1H, J = 5.5 Hz), 8.41 (d, 1H, J = 7.8 Hz), 8.22 (s, 1H), 8.02 (s, 1H), 7.90 (d, 1H, J = 7.8 Hz), 7.57 (t, 1H, J = 7.8 Hz), 5.04 (br. m, 1H), 4.57 (dd, 1H, J = 3.3 Hz, J = 9.1 Hz), 4.26 (t, 1H, J = 8.7 Hz),3.90 (br. m, 2H), 3.58 (m, 4H), 3.41 (m, 2), 3.29 (m, 2H), 2.89 (s, 1H), 2.73 (s, 1H), 2.43 (m, 4H). | | | |
| "A67" | 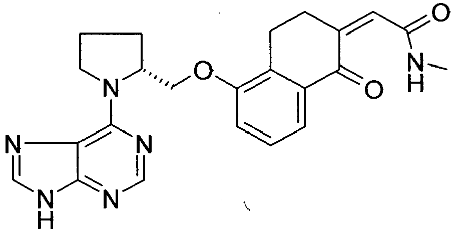 | 138-140 | 1.559 Min |
| | | | [433.2] |
| | N-Methyl-2-[1-oxo-5-[(R)-1-(9H-purin-6-yl)-pyrrolidin-2-ylmethoxy]-3,4-dihydro-1H-naphthalin-(2Z)-yliden]-acetamid | | |
| ¹H NMR (500 MHz, d₆-DMSO) δ [ppm] 12.97 (br. s, 1H), 8.42 (m, 1H), 8.24 (s, 1H), 8.12 (s, 1H), 7.55 (d, 1H, J = 8.24 Hz), 7.35 (m, 2H), 6.79 (s, 1H), 5.36 (m, 1H), 4.83 (m, 1H), 4.40 (dd, 1H, J = 3.1 Hz, J = 9.3 Hz), 4.15 (t, 1H, J = 8.1 Hz), 3.81 (m, 1H), 3.38 (m, 1H), 2.87 (m, 1H), 2.70 (d, 3H, 4.7 Hz), 2.18 (m, 3H), 2.04 (m, 1H). | | | |
| "A68" | 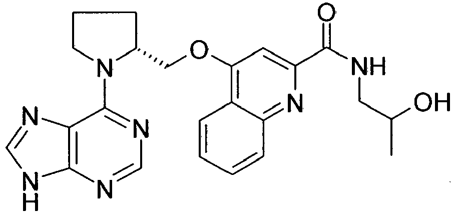 | 125-127 | 1.546 Min |
| | | | [448.2] |
| | 4-[(R)-1-(9H-Purin-6-yl)-pyrrolidin-2-ylmethoxy]-chinolin-2-carbonsäure-(2-hydroxypropyl)-amid Hydrochlorid | | |
| ¹H NMR (500 MHz, d₆-DMSO) δ [ppm] | | | |
| "A69" | 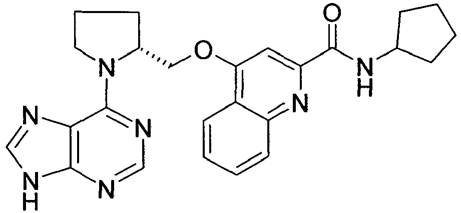 | | 1.724 Min |
| | | | [430.2] |
| | 4-[(R)-1-(9H-Purin-6-yl)-pyrrolidin-2-ylmethoxy]-chinolin-2-carbonsäure-cyclopentylamid | | |
| "A70" | 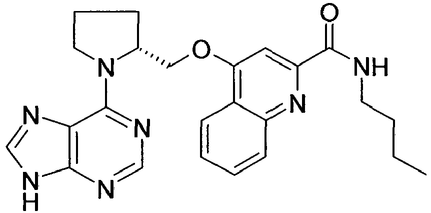 | | 1.920 Min |
| | | | [446.2] |
| | 4-[(R)-1-(9H-Purin-6-yl)-pyrrolidin-2-ylmethoxy]-chinolin-2-carbonsäure-butylamid | | |
| "A71" | 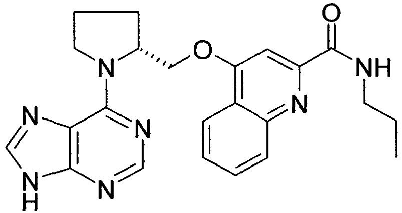 | | 1.793 Min |
| | | | [432.2] |
| | 4-[(R)-1-(9H-Purin-6-yl)-pyrrolidin-2-ylmethoxy]-chinolin-2-carbonsäure-propylamid | | |
| "A72" | 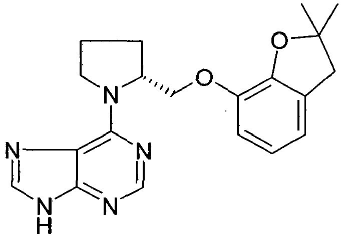 | 184-185 | 1.873 Min |
| | | | [366.2] |
| | 6-[(R)-2-(2,2-Dimethyl-2,3-dihydro-benzofuran-7-yloxymethyl)-pyrrolidin-1-yl]-9H-purin | | |
| ¹H NMR (500 MHz, d₆-DMSO) δ [ppm] 12.96 (br. s, 1H), 8.23 (s, 1H), 8.11 (s, 1H), 6.89 (br. s, 1H), 6.75 (d, 1H, J = 7.2 Hz), 6.68 (t, 1H, J = 7.6 Hz), 5.24 (br. m, 1H), 4.70 (br. m, 1H), 4.30 (dd, 1H, J = 2.7 Hz, J = 8.9 Hz), 3.98 (t, 1H, J = 8.7 Hz), 3.71 (br. m 1H), 3.33 (s, 2H), 2.14 - 2.00 (m, 4H), 1.40 (s, 3H), 1.39 (s, 3H). | | | |
| "A73" | 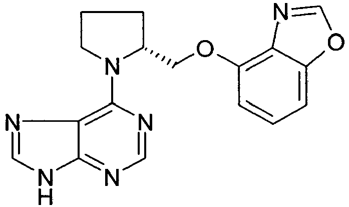 | 215-216 | 1.597 Min |
| | | | [337.2] |
| | 6-[(R)-2-(Benzoxazol-4-yloxymethyl)-pyrrolidin-1-yl]-9H-purin | | |
| ¹H NMR (500 MHz, d₆-DMSO) δ [ppm] 12.95 (br. s, 1H), 8.61 (s, 1H), 8.24 (s, 1H), 8.11 (s, 1H), 7.31 (m, 2H), 7.06 (br. m, 1H), 5.29 - 4.81 (br. m, 1H), 4.57 (dd, 1H, J = 1.7 Hz, J = 8.7 Hz), 4.35 (dd, 1H, J = 7.7 Hz, J = 9.3 Hz), 3.76 (m, 2H), 2.26 (m, 1H), 2.14 (m, 2H), 2.02 (m, 1H). | | | |
| "A75" | 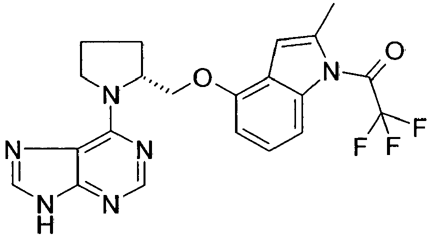 | 142-144 | 1.869 Min |
| | | | [445.2] |
| | 2,2,2-Trifluor-1-{2-methyl-4-[(R)-1-(9H-purin-6-yl)-pyrrolidin-2-ylmethoxy]-indol-1-yl}-ethanon | | |
| ¹H NMR (500 MHz, d₆-DMSO) δ [ppm] 12.96 (br. s, 1H), 12.31 (s, 1H), 8.25 (s, 1H), 8.10 (s, 1H), 7.13 (m, 1H), 7.02 (m, 2H), 5.33 (br. m, 1H), 4.79 (br. m, 1H), 4.48 (dd, 1H, J = 3.3 Hz, J = 9.3 Hz), 4.26 (br. m, 1H), 3.95 (m, 1H), 2.49 (s, 3H), 2.14 - 2.10 (m, 4H). | | | |
| "A77" | 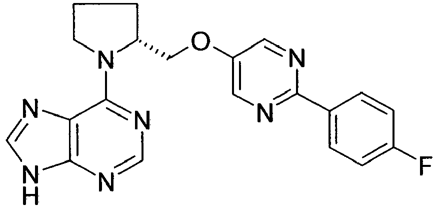 | 194-196 | 1.815 Min |
| | | | [392.2] |
| | 6-{(R)-2-[2-(4-Fluor-phenyl)-pyrimidin-5-yloxymethyl]-pyrrolidin-1-yl}-9H-purin | | |
| ¹H NMR (500 MHz, d₆-DMSO) δ [ppm] 13.00 (br. s, 1H), 8.71 (s, 2H), 8.33 (d, 1H, J = 5.7 Hz), 8.31d, 1H, J = 5.7 Hz), 8.24 (s, 1H), 8.13 (s, 1H), 7.31 (t, 2H, J = 8.8 Hz), 5.29 (br. m, 1H), 4.77 (br. m, 1H), 4.53 (dd, 1H, J = 2.6 Hz, J = 9.4 Hz), 4.24 (t, 1H, J = 8.6 Hz), 3.76 (m, 1H), 2.13 (m, 3H), 2.01 (m, 1H). | | | |
| "A80" | 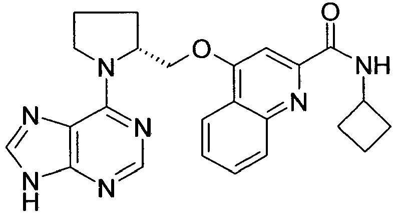 | 160-161 | |
| | 4-[(R)-1-(9H-Purin-6-yl)-pyrrolidin-2-ylmethoxy]-chinolin-2-carbonsäure-cyclobutylamid | | |
| ¹H NMR (500 MHz, d₆-DMSO) δ [ppm] 12.99 (br. s, 1H), 8.95 (d, 1H), J = 8.5 Hz), 8.39 (br. m, 1H), 8.14 (br. m, 2H), 8.09 (d, 1H, J = 8.4 Hz), 7.84 (ddd, 1H, J = 1.3 Hz, J = 6.9 Hz, J = 8.3 Hz), 7.66 (m, 1H), 5.46 - 4.91 (m, 1H), 4.73 (dd, 1H, J = 3.4 Hz, J = 9.8 Hz), 4.52 - 4.42 (m, 2H), 4.07 (m, 1H), 3.83 (m, 1H), 3.45 (m, 1H), 2.22 (m, 7H), 2.07 (m, 1H), 1.70 (m, 2H). | | | |
| "A81" | 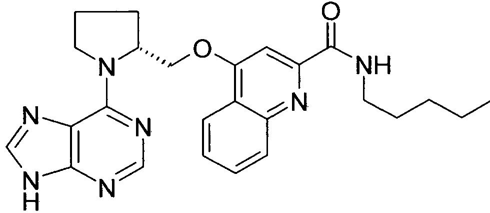 | 150-152 | |
| | 4-[(R)-1-(9H-Purin-6-yl)-pyrrolidin-2-ylmethoxy]-chinolin-2-carbonsäure-pentylamid | | |
| ¹H NMR (500 MHz, d₆-DMSO) δ [ppm] 13.00 (br. s, 1H), 8.84 (t, 1H, J = 6.1 Hz), 8.39 (br. m, 1H), 8.14 (br. s, 2H), 8.06 (d, 1H, J = 8.3 Hz), 7.84 (ddd, 1H, J = 1.3 Hz, J = 6.9 Hz, J = 8.3 Hz), 7.66 (m, 1H), 5.47 - 4,90 (m, 1H), 4.74 (dd, 1H, J= 3.3 Hz, J = 9.6 Hz), 4.46 (m, 1H), 4.33 (m, 1H), 4.09 (m, 1H), 3.89 (m, 1H), 3.33 (m, 1H), 2.25 (m, 3H), 2.07 (m, 1H), 1.57 (m, 2H), 1.31 (m, 4H), 0.88 (m, 3H). | | | |
| "A82" | 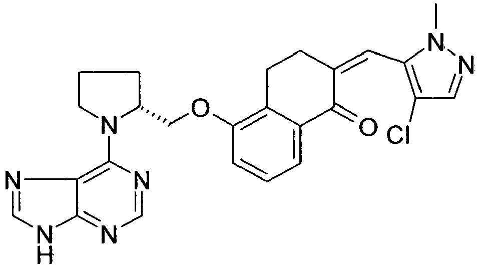 | 150.5-152 | 1.865 Min |
| | | | [490.2] |
| | 2-[1-(4-Chlor-2-methyl-2H-pyrazol-3-yl)-meth-(Z)-yliden]-5-[(R)-1-(9H-purin-6-yl)-pyrrolidin-2-ylmethoxy]-3,4-dihydro-2H-naphthalin-1-on | | |
| ¹H NMR (500 MHz, d₆-DMSO) δ [ppm] 12.96 (br. s, 1H), 8.24 (s, 1H), 8.11 (s, 1H), 7.66 (s, 1H), 7.62 (d, 1H, J = 7.7 Hz), 7.37 (m, 2H), 7.30 (s, 1H), 5.35 (m, 1H), 4.80 (m, 1H), 4.40 (dd, 1H, J = 3.0 Hz, J = 9.4 Hz), 4.16 (t, 1H, J = 8.3 Hz), 3.8 (s, 3H), 2.90 (m, 2H), 2.78 (m, 2H), 2.16 (m, 3H), 2.02 (m, 2H). | | | |
| "A83" | 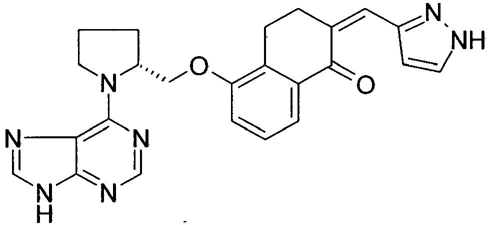 | 174-176 | 1.658 Min |
| | | | [442.2] |
| | 5-[(R)-1-(9H-Purin-6-yl)-pyrrolidin-2-ylmethoxy]-2-[1-(1H-pyrazol-3-yl)-meth-(Z)-yliden]-3,4-dihydro-2H-naphthalin-1-on | | |
| ¹H NMR (500 MHz, d₆-DMSO) δ [ppm] 13.29 (br. s, 1H), 12.97 (br. s, 1H), 8.25 (s, 1H), 8.13 (s, 1H), 7.86 (br. s, 1H), 7.65 (br. m, 1H), 7.56 (m, 2H), 7.34 (m, 2H), 4.84 (br. m, 1H), 3.81 (br. m, 2H), 4.40 (dd, 1H, J = 2.6 Hz, J = 8.8 Hz), 4.15 (t, 1H, J = 8.0 Hz), 3.08 (br. m, 1H), 2.91 (br. m, 3H), 2.18 (m, 3H), 2.04 (m, 1H). | | | |
| "A84" | 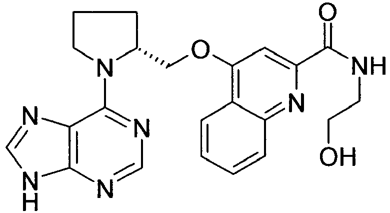 | >299 | 1.177 Min |
| | 4-[(R)-1-(9H-Purin-6-yl)-pyrrolidin-2-ylmethoxy]-chinolin-2-carbonsäure-(2-hydroxy-ethyl)-amid | | |
| ¹H NMR (500 MHz, d₆-DMSO) δ [ppm] 13.06 (br. s, 1H), 8.77 (t, 1H, J = 5.7 Hz), 8.41 (br. m, 1H), 8.16 (br. s, 2H), 8.06 (d, 1H, J = 8.4 Hz), 7.84 (ddd, 1H, J = 1.2 Hz, J = 7.0 Hz, J = 8.2 Hz), 7.66 (t, 1H, J = 7.5 Hz), 5.48 - 4.90 (m, 1H), 4.75 (dd, 1H, J = 3.5 Hz, J = 9.7 Hz), 4.30 - 4.08 (m, 1H), 3.84 (m, 1H), 3.57 (t, 2H, J = 5.9 Hz), 3.44 (q, 2H, J = 5.9 Hz), 2.26 - 2.19 (m, 3H), 2.08 (m, 1H). | | | |
| "A85" | 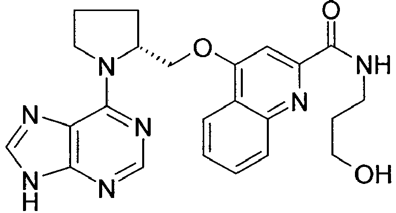 | >299 | 1.510 Min |
| | | | [448.2] |
| | 4-[(R)-1-(9H-Purin-6-yl)-pyrrolidin-2-ylmethoxy]-chinolin-2-carbonsäure-(3-hydroxypropyl)-amid | | |
| ¹H NMR (500 MHz, d₆-DMSO) δ [ppm] 13.08 (br. s, 1H), 8.89 (t, 1H, J = 5.9 Hz), 8.39 (br. m, 1H), 8.16 (br. s, 2H), 8.04 (d, 1H, J = 8.4 Hz), 7.84 (ddd, 1H, J = 1.2 Hz, J = 7.0 Hz, J = 8.3 Hz), 7.66 (t, 1H, J = 7.5 Hz), 5.47 - 4.90 (m, 1H), 4.74 (dd, 1H, J = 3.5 Hz, J = 9.7 Hz), 4.58 - 4.29 (m, 1H), 4.07 - 3.89 (m, 1H), 3.49 (t, 2H, J = 6.2 Hz), 3.42 (q, 3H, J = 6.5 Hz), 2.26 - 2.20 (m, 3H), 2.08 (m, 1H), 1.72 (quint., 2H, J = 6.4 Hz). | | | |
| "A86" | 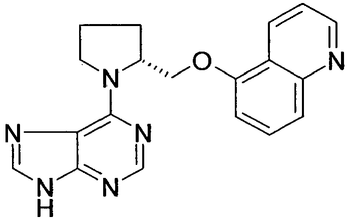 | 93-94 | 1.216 Min |
| | | | [347.2] |
| | 5-[(R)-1-(9H-Purin-6-yl)-pyrrolidin-2-ylmethoxy]-chinolin | | |
| ¹H NMR (500 MHz, d₆-DMSO) δ [ppm] 8.89 (dd, 1H, J = 1.5 Hz, J = 4.1 Hz), 8.48 (br. d, 1H, J = 6.5 Hz), 8.24 (s, 1H), 8.10 (s, 1H), 7.64 (t, 1H, J = 8.2 Hz), 7.58 (d, 1H, J = 8.4 Hz), 7.52 (dd, 1H, J = 4.2 Hz, J = 8.4 Hz), 7.17 (br. d, J = 6.5 Hz), 5.22 (br. m, 1H), 4.54 (dd, 1H, J = 3.5 Hz, J = 9.3 Hz), 4.29 (dd, 1H, J = 7.7 Hz, J = 9.3 Hz), 4.00 (br. m, 2H), 2.23 (m, 3H), 2.06 (m, 1H). | | | |
| "A87" | 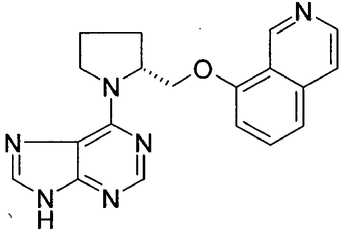 | 134-136 | 1.222 Min |
| | | | [347.2] |
| | 8-[(R)-1-(9H-Purin-6-yl)-pyrrolidin-2-ylmethoxy]-isochinolin | | |
| ¹H NMR (500 MHz, d₆-DMSO) δ [ppm] 12.98 (br. s, 1H), 9.45 (br. s, 1H), 8.51 (d, 1H, J = 5.8 Hz), 8.26 (s, 1H), 8.12 (s, 1H), 7.76 (d, 1H, J = 5.8 Hz), 7.66 (t, 1H, J = 8.1 Hz), 7.49 (d, 1H, J = 8.1 Hz), 7.24 (br. s, 1H), 5.42 - 4.95 (m, 1H), 4.57 (dd, 1H, J = 3.3 Hz, J = 9.2 Hz), 4.33 (t, 1H, J = 8.0 Hz), 3.87 (m, 2H), 2.25 (m, 3H), 2.08 (m, 1H). | | | |
| "A88" | 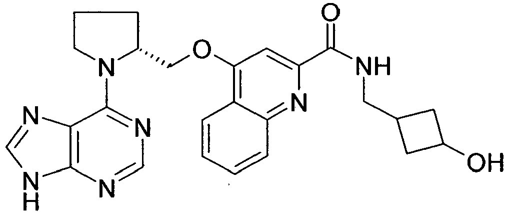 | 116-117 | 1.557 Min |
| | | | [474.2] |
| | 4-[(R)-1-(9H-Purin-6-yl)-pyrrolidin-2-ylmethoxy]-chinolin-2-carbonsäure-(3-hydroxy-cyclobutylmethyl)-amid | | |
| ¹H NMR (500 MHz, d₆-DMSO) δ [ppm] 13.00 (br. s, 1H), 8.82 (t, 1H, J = 6.0 Hz), 8.39 (br. m, 1H), 8.15 (br. s, 2H), 8.07 (d, 1H, J = 8.4 Hz), 7.85 (t, 1H, J = 7.4 Hz), 7.67 (t, 1H, J = 7.4 Hz), 5.47 (m, 1H), 4.94 (m, 1H), 4.76 (dd, 1H, J = 3.3 Hz, J = 9.8 Hz), 4.47 (m, 1H), 3.89 (quint. 1H, J = 7.4 Hz), 3.37 (m, 3H), 2.26 (m, 4H), 2.08 (m, 1H), 1.99 (m, 2H), 1.58 (m, 2H). | | | |
| "A89" | 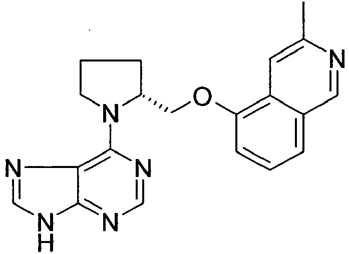 | 137-138 | 1.258 Min |
| | | | [361.2] |
| | 3-Methyl-5-[(R)-1-(9H-purin-6-yl)-pyrrolidin-2-ylmethoxy]-isochinolin | | |
| ¹H NMR (500 MHz, d₆-DMSO) δ [ppm] 12.99 (br. s, 1H), 9.15 (s, 1H), 8.26 (s, 1H), 8.12 (s, 1H), 7.59 (d, 1H, J = 8.12 Hz), 7. 46 (t, 1H, J = )7.7 Hz, 7.29 (br. s, 1H), 5.53 (br. m, 1H), 4.95 (br. m, 1H), 4.50 (dd, 1H, J = 4.0 Hz, J = 9.1 Hz), 4.25 (t, 1H, J = 8.4 Hz), 3.84 (br. m, 1H), 2.61 (s, 3H), 2.21 (m, 3H), 2.07 (m, 1H). | | | |
| "A92" | | 150-151 | 1.441 Min |
| | | | [530.2] |
| | 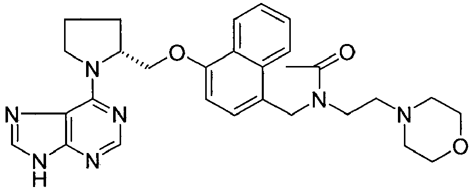 | | |
| | N-(2-Morpholin-4-yl-ethyl)-N-{4-[(R)-1-(9H-purin-6-yl)-pyrrolidin-2-ylmethoxy]-naphthalin-1-ylmethyl}-acetamid | | |
| "A93" | 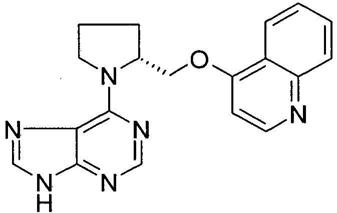 | 130-131,5 | 1.240 Min |
| | | | [347.2] |
| | 4-[(R)-1-(9H-Purin-6-yl)-pyrrolidin-2-ylmethoxy]-chinolin | | |
| ¹H NMR (500 MHz, d₆-DMSO) δ [ppm] 8.71 (d, 1H, J = 5.1 Hz), 8.27 (s, 1H), 8.13 (s, 1H), 8.11 (br. s, 1H), 7.95 (d, 1H, J = 8.4 Hz); 7.75 (ddd, 1H, J = 1.3 Hz, J = 6.9 Hz, J = 8.3 Hz), 7.58 (t, 1H, J = 7.5 Hz), 7.17 (br. s, 1H), 5.47 - 4.98 4.98 (br. m, 1H), 4.61 (dd, 1H, J = 3.4 Hz, J = 9.5 Hz), 4.38 (t, 1H, J = 8.3 Hz), 4.11 (m, 1H), 3.86 (m, 1H), 2.23 (m, 3H), 2.11 (m, 1H). | | | |
| "A94" | 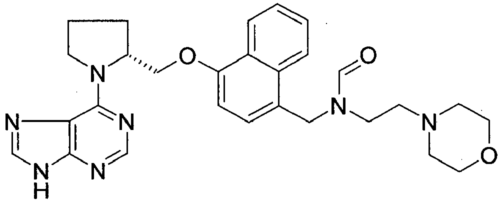 | 184-187 | 1.444 Min |
| | | | [516.3] |
| | N-(2-Morpholin-4-yl-ethyl)-N-{4-[(R)-1-(9H-purin-6-yl)-pyrrolidin-2-ylmethoxy]-naphthalin-1-ylmethyl}-formamid | | |
| "A95" | 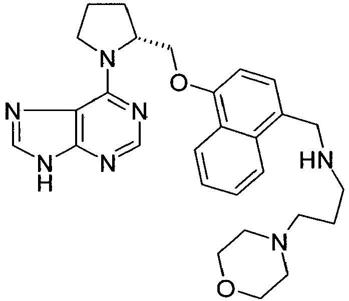 | 148-150 | |
| | (3-Morpholin-4-yl-propyl)-{4-[(R)-1-(9H-purin-6-yl)-pyrrolidin-2-ylmethoxy]-naphthalin-1-ylmethyl}-amin | | |
| "A96" | 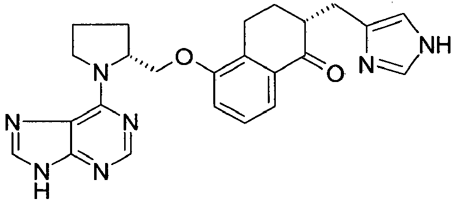 | | |
| | (R)-2-(1H-Imidazol-4-ylmethyl)-5-[(R)-1-(9H-purin-6-yl)-pyrrolidin-2-ylmethoxy]-3,4-dihydro-2H-naphthalin-1-on | | |
| "A97" | 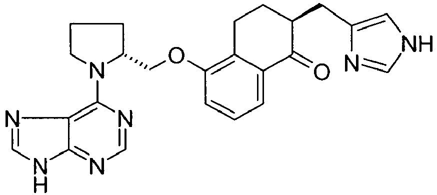 | | |
| | (S)-2-(1H-Imidazol-4-ylmethyl)-5-[(R)-1-(9H-purin-6-yl)-pyrrolidin-2-ylmethoxy]-3,4-dihydro-2H-naphthalin-1-on | | |
| "A98" | 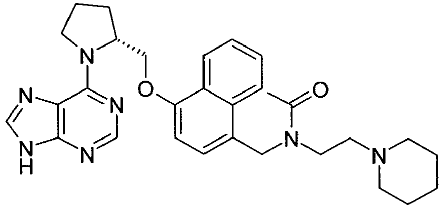 | | |
| | N-(2-Piperidin-1-yl-ethyl)-N-{4-[(R)-1-(9H-purin-6-yl)-pyrrolidin-2-ylmethoxy]-naphthalin-1-ylmethyl}-acetamid | | |
| ¹H NMR (500 MHz, d₆-DMSO) δ [ppm] 8.23 (s, 1H), 8.18 (br. m, 1H), 8.10 (s, 1H), 8.00 (m, 1H), 7.64 - 7.51 (m, 2H), 7.28 (d, 1H, J= 8.0 Hz), 7.03 (m, 1H), 4.99 (s, 1H), 4.92 (s, 1H), 4.52 (dd, 1H, J = 3.3 Hz, J = 9.1 Hz), 3.20 (m, 3H), 2.24 (m, 9H), 2.12 (s, 3H), 2.06 (m 1H), 1.41 (m, 4H), 1.32 (m, 2H). | | | |
| "A99" | 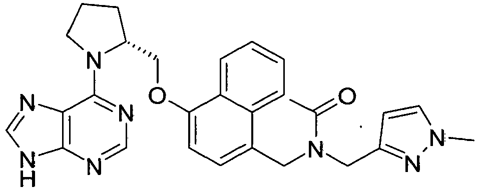 | 117-119 | 1.466 Min |
| | | | [511.2] |
| | N-(1-Methyl-1H-pyrazol-3-ylmethyl)-N-{4-[(R)-1-(9H-purin-6-yl)-pyrrolidin-2-ylmethoxy]-naphthalin-1-ylmethyl}-acetamid | | |
| ¹H NMR (500 MHz, d₆-DMSO) δ [ppm] 12.97 (br. s. 1H), 8.25 (s, 1H), 8.19 (br. m, 1H), 8.12 (s, 1H), 8.06 (m, 1H), 7.60 - 7.51 (m, 4H), 7.28 (br. d, 1H, J = 7.7 Hz), 7.07 (br. m, 1H), 7.03 (br. m 1H), 6.04 (d, 1H, J = 1.9 Hz), 5.48 (br. m, 1H), 4.87 (s, 2H), 4.53 (dd, 1H, J = 3.1 Hz, J = 9.1 Hz), 4.27 (m, 1H), 4.24 (s, 3H), 2.24 (m 3H), 2.19 (s, 3H), 2.07 (m 1H). | | | |
| "A100" | 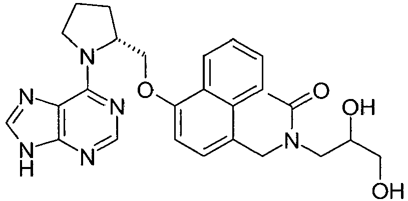 | Zersetzung ab 145° | 1.652 Min |
| | N-(2,3-Dihydroxy-propyl)-N-{4-[(R)-1-(9H-purin-6-yl)-pyrrolidin-2-ylmethoxy]-naphthalin-1-ylmethyl}-acetamid | | [491.2] |
| "A101" | 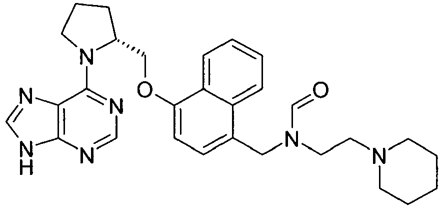 | Zersetzung ab 190° | 1.511 Min |
| | | | [514.2] |
| | N-(2-Piperidin-1-yl-ethyl)-N-{4-[(R)-1-(9H-purin-6-yl)-pyrrolidin-2-ylmethoxy]-naphthalin-1-ylmethyl}-formamid | | |
| "A102" | 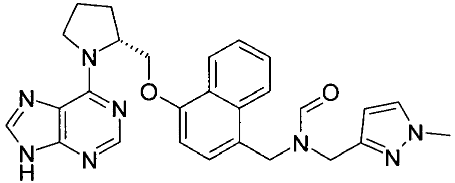 | 206-207 | 1.568 Min |
| | | | [497.2] |
| | N-(1-Methyl-1H-pyrazol-3-ylmethyl)-N-{4-[(R)-1-(9H-purin-6-yl)-pyrrolidin-2-ylmethoxy]-naphthalin-1-ylmethyl}-formamid | | |
| ¹H NMR (500 MHz, d₆-DMSO) δ [ppm] 13.00 (br. s, 1H), 8.29 (s, 1H), 8.24 (s, 1H), 8.20 (br. s, 1H), 8.11 (s, 1H), 8.05 (br. d, 1H, J = 7.6 Hz), 7.54 - 7.49 (m, 3H), 7.23 (br. s, 1H), 6.99 (br. s, 1H), 6.03 (br. s, 1H), 5.36 (m, 2H), 4.90 (m, 1H), 4.49 (dd, 1H, J = 2.9 Hz, J = 9.0 Hz), 4.24 (m, 1H), 3.78 (s, 3H), 2.24 (m, 3H), 2.07 (m, 1H). | | | |
| "A103" | 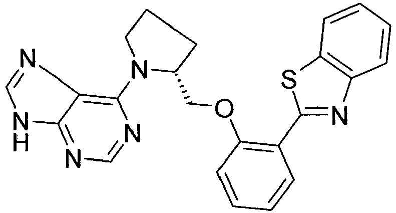 | 165-166 | 1.903 Min |
| | | | |
| | 6-[(R)-2-(2-Benzothiazol-2-yl-phenoxymethyl)-pyrrolidin-1-yl]-9H-purin | | [429.2] |
| ¹H NMR (500 MHz, d₆-DMSO) δ [ppm] 12.98 (br. s, 1H), 8.44 (dd, 1H, J = 1.5 Hz, J = 7.8 Hz), 8.27 (s, 1H), 8.13 (m, 2H), 8.07 (d, 1H, J = 8.1 Hz), 7.55 (ddd, 1H, J = 1.1 Hz, J = 7.3 Hz, J = 9.3 Hz), 7.52 (m 2H), 7.45 (ddd, 1H, J = 1.0 Hz, J = 7.3 Hz, J = (8.0 Hz), 7.17 (ddd, 1H, J = 1.3 Hz, J = 6.6 Hz, J = 7.9 Hz), 5.51 (br. m, 1H), 4.95 (br. m, 1H), 4.68 (dd, 1H, J = 3.4 Hz, J = 9.1 Hz), 4.25 (t, 1H, J = 9.1 Hz), 3.84 (br. m 1H), 2.21 (m, 3H), 2.05 (m, 1H). | | | |
| "A104" | 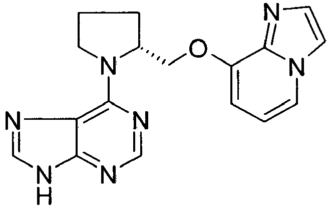 | 115-117 | 1.105 Min |
| | | | [336.2] |
| | 6-[(R)-2-(Imidazo[1,2-a]pyridin-8-yloxymethyl)-pyrrolidin-1-yl]-9H-purin | | |
| ¹H NMR (500 MHz, d₆-DMSO) δ [ppm] 12.97 (br. s, 1H), 8.24 (s, 1H), 8.12 (m, 2H), 7.91 (s, 1H), 7.48 (s, 1H), 6.73 (m, 2H), 5.36 (br. m, 1H), 4.82 (br. m, 1H), 4.47 (br. d, 1H, J = 8.5 Hz), 4.26 (dd, 1H, J = 7.8 Hz, J = 9.1 Hz), 3.76 (br. m, 1H), 2.31 (m 1H), 2.14 (m, 2H), 2.02 (m, 1H). | | | |
| "A105" | 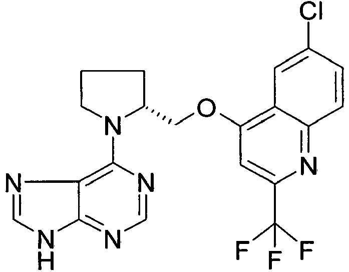 | 230-231 | 1.955 Min |
| | | | [449.2] |
| | 6-Chlor-4-[(R)-1-(9H-purin-6-yl)-pyrrolidin-2-ylmethoxy]-2-trifluormethyl-chinolin | | |
| ¹H NMR (500 MHz, d₆-DMSO) δ [ppm] 13.02 (br. s, 1H), 8.25 (s, 1H), 8.11 (m, 3H), 7.98 (dd, 1H, J = 2.0 Hz, J = 9.0 Hz), 7.81 (m, 1H), 5.53 (br. m, 1H), 4.94 (br. m, 1H), 4.75 (dd, 1H, J = 4.4 Hz, J = 10.1 Hz), 4.47 (dd. 1H, J = 7.9 Hz, J = 10.1 Hz), 3.99 (m, 1H), 2.19 (m 3H), 2.07 (m, 1H). | | | |
| "A106" | 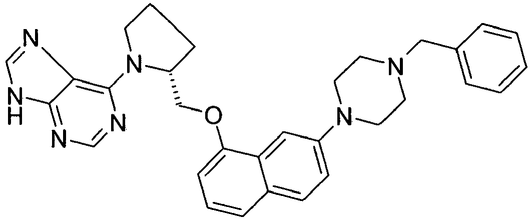 | 78-80 | 1.687 Min |
| | | | [520.2] |
| | 6-{(R)-2-[7-(4-Benzyl-piperazin-1-yl)-naphthalin-1-yloxymethyl]-pyrrolidin-1-yl}-9H-purin | | |
| ¹H NMR (500 MHz, d₆-DMSO) δ [ppm] 12.94 (br. s, 1H), 8.22 (s, 1H), 8.08 (br. s, 1H), 7.69 (d, 1H, J = 9.1 Hz), 7.37 - 7.29 (m, 8H), 7.15 (t, 1H, J = 7.6 Hz), 6.95 (br. s, 1H), 5.47 (br. m, 1H), 4.89 (m 1H), 4.43 (br. dd, 1H, J = 2.2 Hz, J = 9.3 Hz), 4.20 (br. m, 1H), 3.84 (br. m, 1H), 3.21 (m, 4H), 2.58 (m, 4H), 2.25 (br. m, 3H), 2.06 (br. m, 1H). | | | |
| "A107" | 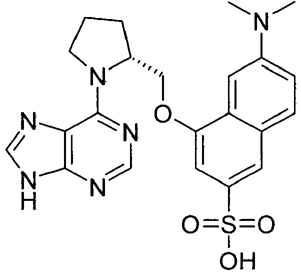 | | 1.173 Min |
| | | | [469.2] |
| | 6-Dimethylamino-4-[(R)-1-(9H-purin-6-yl)-pyrrolidin-2-ylmethoxy]-naphthalin-2-sulfonsäure | | |
| ¹H NMR (500 MHz, d₆-DMSO) δ [ppm] 8.93 (br. s, 1H), 8.63 (br. s, 1H), 8.58 (s, 1H), 8.19 (d, 1H, J = 9.0 Hz), 7.90 (d, 1H, J = 6.0 Hz), 7.87 (d, 1H, J = 6.0 Hz), 7.47 (s, 1H), 5.69 -5.03 (br. m, 1H), 4.66 (m, 1H), 4.38 (m, 1H), 4.13 - 4.03 (m, 1H), 3.85 (br. m, 1H), 3.32 (s, 6H), 2.6 (br. m, 4H). | | | |
| "A108" | 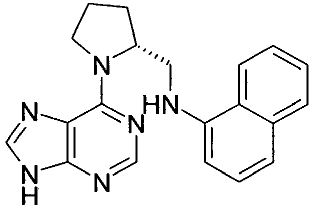 | | 1.721 Min |
| | | | [345.2] |
| | Naphthalin-1-yl-[(R)-1-(9H-purin-6-yl)-pyrrolidin-2-ylmethyl]-amin | | |
| "A109" | 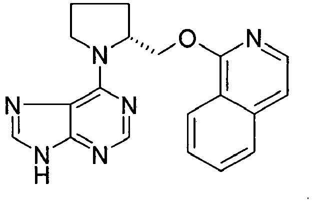 | 201-202 | 1.656 Min |
| | | | [347.2] |
| | 1-[(R)-1-(9H-Purin-6-yl)-pyrrolidin-2-ylmethoxy]-isochinolin | | |
| ¹H NMR (500 MHz, d₆-DMSO) δ [ppm] 12.94 (br. s, 1H), 8.20 (s, 1H), 8.09 (s, 2H), 7.92 (d, 1H, J = 5.6 Hz), 7.88 (d, 1H, 8.1 Hz), 7.76 (ddd, 1H, J = 1.1 Hz, J = 7.1 Hz, J = 8.1 Hz), 7.62 (t, 1H, J = 7.5 Hz), 7.35 (d, 1H, J = 5.8 Hz), 5.21 (br. m, 1H), 4.72 (dd, 1H, J = 3.8 Hz, J = 10.5 Hz), 4.67 (dd, 1H, J = 6.3 Hz, J = 10.5 Hz), 3.97 (br. m, 2H), 2.22 (br. m, 3H), 2.05 (br. m, 1H). | | | |
| "A110" | 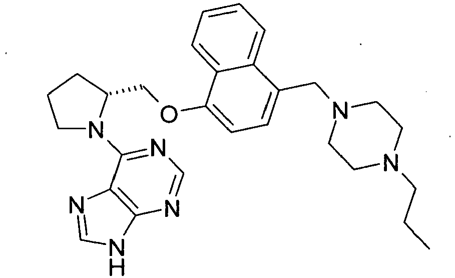 | 145-147 | 1.370 Min |
| | | | [486.4] |
| | 6-{(R)-2-[4-(4-Propyl-piperazin-1-ylmethyl)-naphthalin-1-yloxymethyl]-pyrrolidin-1-yl}-9H-purin | | |
| ¹H NMR (500 MHz, d₆-DMSO) δ [ppm] 8.24 (s, 1H), 8.20 (d, 1H, J = 8.5 Hz), 8.15 (d, 1H, J = 6.7 Hz), 8.10 (s, 1H), 7.55 (t, 1H, J = 7.1 Hz), 7.50 (t, 1H, J = 7.1 Hz), 7.24 (d, 1H, J = 7.8 Hz), 5.13 (br. m, 1H), 4.49 (dd, 1H, J = 2.9 Hz, J = 8.9 Hz), 4.25 (t, 1H, J = 8.0 Hz), 3.98 (br. m, 2H), 3.74 (s, 2H), 3.38 (m, H), 2.39 (br. m, 4H), 2.26 (br. m, 6H), 2.17 (t, 3H, J = 7.2 Hz), 2.07 (br. m, 1H), 1.39 (hex, 2H, J = 7.3 Hz), 1.10 (t, 2H, J = 6.9 Hz), 0.83 (t, 3H, J = 7.3 Hz). | | | |
| "A111" | 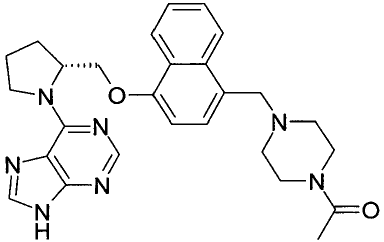 | 150-152 | 1.370 Min |
| | | | [486.2] |
| | 1-(4-{4-[(R)-1-(9H-Purin-6-yl)-pyrrolidin-2-ylmethoxy]-naphthalin-1-ylmethyl}-piperazin-1-yl)-ethanon | | |
| ¹H NMR (500 MHz, d₆-DMSO) δ [ppm] 12.97 (br. s, 1H), 8.25 (s, 1H), 8.23 (d, 1H, J = 8.5 Hz), 8.16 (br. s, 1H), 8.12 (s, 1H), 7.56 (ddd, 1H, J = 1.2 Hz, J = 7.0 Hz, J = 8.0 Hz), 7.51 (t, 1H, J = 7.0 Hz), 7.29 (d, 1H, J = 7.7 Hz), 6.99 (br. s, 1H), 5.19 (br. m, 1H), 4.50 (dd, 1H, J = 3.2 Hz, J = 9.3 Hz), 4.26 (t, 1H, J = 8.1 Hz), 4.16 (br. m, 1H), 3.78 (d, 2H, J = 2.1 Hz), 3.61 (m, 3H), 3.36 (m, 5H), 2.40 (m, 2H), 2.33 (m, 2H), 2.24 (br. m, 3H), 2.08 (br. m, 1H), 1.97 (s, 3H), 1.77 (m, 3H). | | | |
| "A112" | 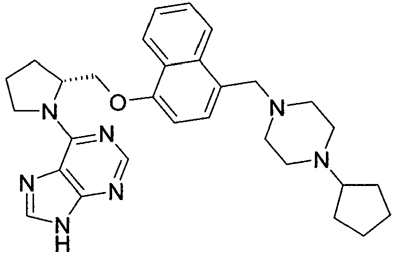 | 170-172 | 1.381 Min |
| | | | [512.4] |
| | 6-{(R)-2-[4-(4-Cyclopentyl-piperazin-1-ylmethyl)-naphthalin-1-yloxymethyl]-pyrrolidin-1-yl}-9H-purin | | |
| ¹H NMR (500 MHz, d₆-DMSO) δ [ppm] 12.98 (br. s, 1H), 8.24 (s, 1H), 8.20 (d, 1H, J = 8.5 Hz), 8.14 (br. s, 1H), 8.11 (s, 1H), 7.55 (m, 1H), 7.50 (m, 1H), 7.27 (d, 1H, J = 7.6 Hz), 6.97 (br. s, 1H), 4.91 (br. m, 1H), 4.49 (dd, 1H, J = 3.2 Hz, J = 9.2 Hz), 4.25 (t, 1H, J = 8.2 Hz), 3.99 (br. m, 2H), 3.73 (s, 2H), 3.61 (m, 2H), 2.39 (m, 8H), 2.24 (m, 4H), 2.07 (m, 1H), 1.77 (m, 2H), 1.72 (m, 2H), 1.58 (m, 2H), 1.46 (m, 2H), 1.28 (m, 2H). | | | |
| "A113" | 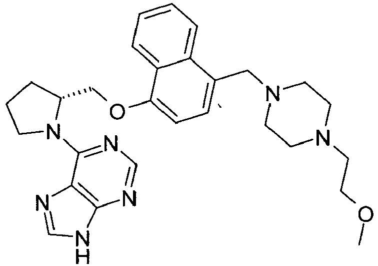 | 210-212 | 1.363 Min |
| | | | [502.2] |
| | 6-((R)-2-{4-[4-(2-Methoxy-ethyl)-piperazin-1-ylmethyl]-naphthalin-1-yloxymethyl}-pyrrolidin-1-yl)-9H-purin | | |
| "A114" | 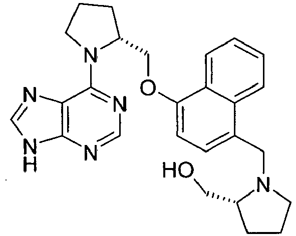 | 137-139 | 1.378 Min |
| | | | [459.2] |
| | ((R)-1-{4-[(R)-1-(9H-Purin-6-yl)-pyrrolidin-2-ylmethoxy]-naphthalin-1-ylmethyl}-pyrrolidin-2-yl)-methanol | | |
| ¹H NMR (500 MHz, d₆-DMSO) δ [ppm] 12.97 (br. s, 1H), 8.30 (dd, 1H, J = 0.9 Hz, J = 8.3 Hz), 8.25 (s, 1H), 8.14 (br. m, 1H), 8.12 (s, 1H), 7.52 (m, 2H), 7.31 (d, 1H, 8.3 Hz), 6.96 (br. m, 1H), 5.19 (br. m, 1H), 4.48 (m, 3H), 4.24 (m, 1H), 4.08 (m, 1H), 3.83 (br. m, 1H), 3.54 (d, 1H, J = 12.5 hz), 3.49 (dd, 1H, J = 4.3 Hz, J = 9.9 Hz), 3.18 (s, 1H), 2.62 (m, 2H), 2.22 (m, 4H), 2.07 (m, 1H), 1.87 (m, 1H), 1.56 (m, 3H). | | | |
| "A115" | 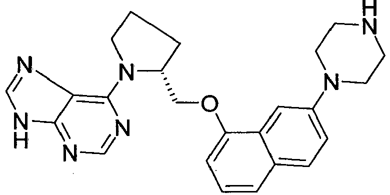 | 100-101 | 1.485 Min |
| | | | [430.2] |
| | 6-[(R)-2-(7-Piperazin-1-yl-naphthalin-1-yloxymethyl)-pyrrolidin-1-yl]-9H-purin | | |
| ¹H NMR (500 MHz, d₆-DMSO) δ [ppm] 8.23 (s, 1H), 8.11 (s, 1H), 7.69 (d, 1H, J =9.1 Hz), 7.33 (dd, 1H, J = 2.4 Hz, J = 9.0 Hz), 7.30 (m. 2H), 7.13 (t, 1H, J = 7.8 Hz), 6.94 (br. s, 1H), 5.21 (br. m, 1H), 4.43 (dd, 1H, J = 3.3 Hz, J = 9.3 Hz), 4.33 (dd, 1H, J = 6.4 Hz, J = 9.3 Hz), 4.06 (m, 2H), 3.09 (m, 4H), 2.89 (m, 4H), 2.31 (m, 1H), 2.23 (m, 2H), 2.08 (m, 1H). | | | |
| "A116" | 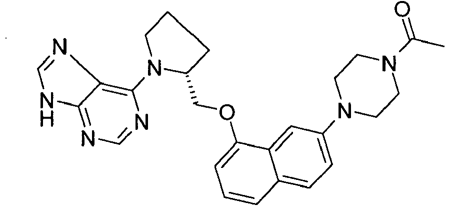 | 131.5-133 | 1.685 Min |
| | | | [472.2] |
| | 1-(4-{8-[(R)-1-(9H-Purin-6-yl)-pyrrolidin-2-ylmethoxy]-naphthalin-2-yl}-piperazin-1-yl)-ethanon | | |
| ¹H NMR (500 MHz, d₆-DMSO) δ [ppm] 12.96 (br. s, 1H), 8.23 (s, 1H), 8.10 (s, 1H), 7.72 (d, 1H, J = 9.1 Hz), 7.38 (m, 1H), 7.33 (m, 1H), 7.31 (m, 1H), 7.16 (m, 1H), 6.98 (m, 1H), 5.21 (br. m, 1H), 4.44 (dd, 1H, J = 3.0 Hz, J = 9.1 Hz), 4.31 (m, 1H), 3.87 (br. m, 2H), 3.64 (m, 3H), 3.59 (m, 2H), 3.18 (m, 4H), 2.29 (m, 2H), 2.22 (m, 2H), 2.08 (s, 3H). | | | |
| "A117" | 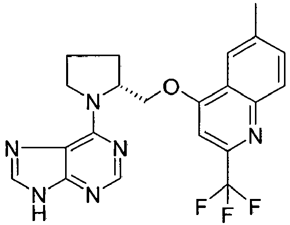 | 245-246 | 1.869 Min |
| | | | |
| | 6-Methyl-4-[(R)-1-(9H-purin-6-yl)-pyrrolidin-2-ylmethoxy]-2-trifluormethyl-chinolin | | [429.2] |
| ¹H NMR (500 MHz, d₆-DMSO) δ [ppm] 13.02 (br. s, 1H), 8.26 (s, 1H), 8.12 (s, 1H), 7.97 (d, 1H, J = 8.6 Hz), 7.87 (br. s, 1H), 7.72 (dd, 2H, J = 1.5 Hz, J = 8.5 Hz), 5.26 (br. m, 1H), 4.72 (dd, 1H, J = 4.1 Hz, J = 10.0 Hz), 4.45 (dd, 1H, J = 8.1 Hz, J = 10.0 Hz), 4.26 (br. m, 1H), 3.82 (br. m, 1H), 2.53 (s, 3H), 2.20 (br. m, 3H), 2.07 (br. m, 1 H). | | | |
| "A118" | 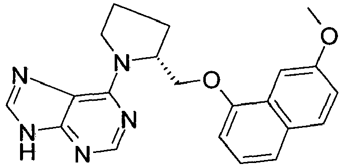 | 140-141.5 | 1.830 Min |
| | | | [376.2] |
| | 6-[(R)-2-(7-Methoxy-naphthalin-1-yloxymethyl)-pyrrolidin-1-yl]-9H-purin | | |
| ¹H NMR (500 MHz, d₆-DMSO) δ [ppm] 12.96 (br. s, 1H), 8.25 (s, 1H), 8.12 (s, 1H), 7.78 (d, 1H, J = 8.9 Hz), 7.42 (br. s, 1H), 7.39 (d, 1H, J= 8.1 Hz), 7.23 (br. t, 1H, J = 7.8 Hz), 7.17 (dd, 1H, J = 2.6 Hz, J= 8.9 Hz), 7.04 (br. s, 1H), 5.21 (br. m, 1H), 4.49 (dd, 1H, J= 2.9 Hz, J = 9.2 Hz), 4.32 (dd, 1H, J = 6.9 Hz, J = 9.2 Hz), 4.19 (br. m, 1H), 3.85 (s, 3H), 2.26 (br. m, 3H), 2.08 (br. m, 1H). | | | |
| "A119" | 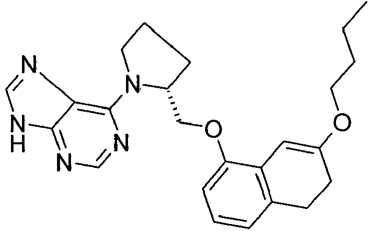 | 92-94 | 2.207 Min |
| | | | [420.2] |
| | 6-[(R)-2-(7-Butoxy-5,6-dihydro-naphthalin-1-yloxymethyl)-pyrrolidin-1-yl]-9H-purin | | |
| ¹H NMR (500 MHz, d₆-DMSO) δ [ppm] 12.94 (br. s, 1H), 8.21 (s, 1H), 8.09 (s, 1H), 6.87 (m, 1H), 6.80 (br. m, 1H), 6.66 (d, 1H, J = 7.3 Hz), 5.74 (s, 1H), 5.03 (br. m, 1H), 4.24 (m, 1H), 4.14 (m, 1H), 3.76 (m, 2H), 3.42 (m, 1H), 2.74 (m, 2H), 2.69 (m, 1H), 2.26 (m, 2H), 2.16 (br. m, 3H), 2.02 (br. m, 1H), 1.67 (m, 2H), 1.37 (m, 2H), 0.82 (t, 3H, J = 7.3 Hz). | | | |
| "A120" | 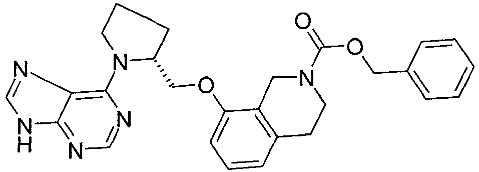 | 88-90 | 1.957 Min |
| | | | [485.2] |
| | 8-[(R)-1-(9H-Purin-6-yl)-pyrrolidin-2-ylmethoxy]-3,4-dihydro-1H-isochinolin-2-carbonsäure-benzylester | | |
| ¹H NMR (500 MHz, d₆-DMSO) δ [ppm] 12.59 (br. s, 1H), 8.23 (s, 1H), 8.10 (s, 1H), 7.40 (m, 4H), 7.34 (m, 1H), 7.11 (t, 1H, J= 7.5 Hz), 6.91 (br. m, 1H), 6.74 (d, 1H, J = 7.5 Hz), 5.14 (s, 2H), 4.74 (br. m, 1H), 4.50 (m, 2H), 4.32 (m, 1H), 4.15 (m, 1H), 3.74 (m, 1H), 3.61 (m, 2H), 2.76 (m, 2H), 2.11 (br. m, 3H), 1.91 (br. m, 1H). | | | |
| "A121" | 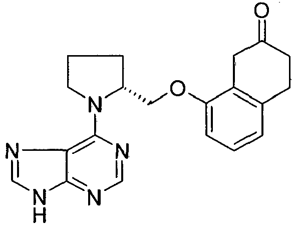 | 140.5-142 | 1.590 Min |
| | | | [364.2] |
| | 8-[(R)-1-(9H-Purin-6-yl)-pyrrolidin-2-ylmethoxy]-3,4-dihydro-1H-naphthalin-2-on | | |
| ¹H NMR (500 MHz, d₆-DMSO) δ [ppm] 12.97 (br. s, 1H), 8.22 (s, 1H), 8.10 (s, 1H), 7.12 (t, 1H, J = 7.6 Hz), 6.93 (br. m, 1H), 6.84 (d, 1H, J = 7.6 Hz), 5.05 (br. m, 1H), 4.30 (dd, 1H, J = 3.3 Hz, J= 9.3 Hz), 4.08 (dd, 1H, J = 6.6 Hz, J = 9.3 Hz), 3.76 (br. m, 4H), 2.99 (m, 2H), 2.46 (dd, 2H, J = 5.8 Hz, J= 7.7 Hz), 2.15 (br. m, 3H), 2.03 (br. m, 1H). | | | |
| "A122" | 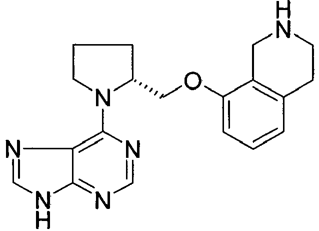 | 85-87 | 1.231 Min |
| | | | [351.2] |
| | 8-[(R)-1-(9H-Purin-6-yl)-pyrrolidin-2-ylmethoxy]-1,2,3,4-tetrahydro-isochinolin | | |
| ¹H NMR (500 MHz, d₆-DMSO) δ [ppm] 8.23 (s, 1H), 8.11 (s, 1H), 7.01 (br. t, 1H, J= 7.7 Hz), 6.78 (br. m, 1H), 6.64 (d, 1H, J = 7.7 Hz), 5.02 (br. m, 1H), 4.27 (dd, 1H, J= 2.8, J = 9.1 Hz), 4.09 (dd, 1H, J = 6.9 Hz, J = 9.1 Hz), 3.67 (m, 2H), 3.38 (m, 2H), 2.88 (m, 2H), 2.63 (m, 2H), 2.15 (br. m, 3H), 2.02 (br. m 1H). | | | |
| "A123" | 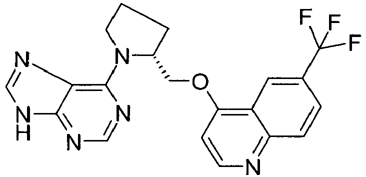 | 139-140 | 1.380 Min |
| | | | [415.2] |
| | 4-[(R)-1-(9H-Purin-6-yl)-pyrrolidin-2-ylmethoxy]-6-trifluormethyl-chinolin | | |
| ¹H NMR (500 MHz, d₆-DMSO) δ [ppm] 12.98 (br. s, 1H), 8.88 (d, 1H, J = 5.2 Hz), 8.36 (br. s, 1H), 8.26 (s, 1H), 8.15 (d, 1H, J = 8.8 Hz), 8.13 (s, 1H), 7.99 (dd, 1H, J = 2.0 Hz, J = 8.8 Hz), 7.39 (br. s, 1H), 5.26 (br. m, 1H), 4.65 (dd, 1H, J = 4.2 Hz, J = 9.7 Hz), 4.42 (dd, 1H, J = 7.3 Hz, J = 9.7 Hz), 3.93 (br. m, 2H), 2.21 (br. m, 3H), 2.08 (br. m, 1H). | | | |
| "A124" | 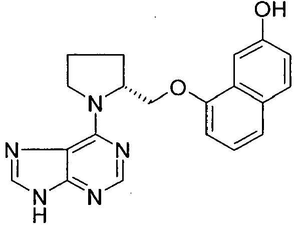 | 146.5-148 | 1.648 Min |
| | | | [362.2] |
| | 8-[(R)-1-(9H-Purin-6-yl)-pyrrolidin-2-ylmethoxy]-naphthalin-2-ol | | |
| ¹H NMR (500 MHz, d₆-DMSO) δ [ppm] 12.96 (br. s, 1H), 9.74 (br. s, 1H), 8.24 (s, 1H), 8.12 (s, 1H), 7.70 (d, 1H, J = 8.8 Hz), 7.43 (d, 1H, J = 2.3 Hz), 7.33 (d, 1H, J = 8.2 Hz), 7.12 (br. t, 1H, J =7.7 Hz), 7.07 (dd, 1H, J = 2.4 Hz, J = 8.8 Hz), 6.93 (br. s, 1H), 5.15 (br. m, 1H), 4.46 (dd, 1H, J = 1.9 Hz, J = 9.1 Hz), 4.24 (dd, 1H, J = 7.6Hz, J = 9.1 Hz), 3.85 (br. m, 2H), 2.25 (br. m, 3H), 2.08 (br. m, 1H). | | | |
| "A125" | 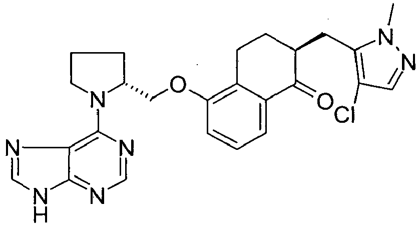 | | |
| | (S)-2-(4-Chlor-2-methyl-2H-pyrazol-3-ylmethyl)-5-[(R)-1-(9H-purin-6-yl)-pyrrolidin-2-ylmethoxy]-3,4-dihydro-2H-naphthalin-1-on | | |
| "A126" | 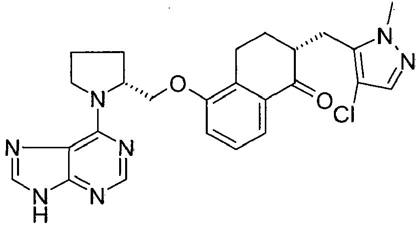 | | |
| | (R)-2-(4-Chlor-2-methyl-2H-pyrazol-3-ylmethyl)-5-[(R)-1-(9H-purin-6-yl)-pyrrolidin-2-ylmethoxy]-3,4-dihydro-2H-naphthalin-1-on | | |
| "A127" | 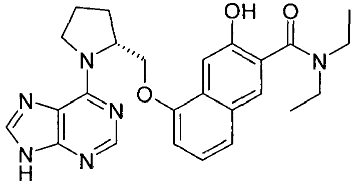 | | 1.643 Min |
| | | | [461.2] |
| | 3-Hydroxy-5-[(R)-1-(9H-purin-6-yl)-pyrrolidin-2-ylmethoxy]-naphthalin-2-carbonsäure-diethylamid | | |
| ¹H NMR (500 MHz, d₆-DMSO) δ [ppm] 12.96 (br. s, 1H), 10.04 (br. s, 1H), 8.25 (s, 1H), 8.12 (s, 1H), 7.61 (s, 1H), 7.54 (s, 1H), 7.30 (d, 1H, J = 8.1 Hz), 7.17 (t, 1H, J = 7.7 Hz), 6.85 (d, 1H, J = 7.5 Hz), 5.03 (br. m, 1H), 4.46 (m, 1H), 4.28 (m, 2H), 3.91 (m, 1H), 3.61 (m, 1H), 3.48 (m, 1H), 3.14 (m, 1H), 3.00 (m, 1H), 2.13 (m, 3H), 1.98 (m, 1H), 1.20 (m, 4), 1.00 (m, 2H). | | | |
| "A128" | 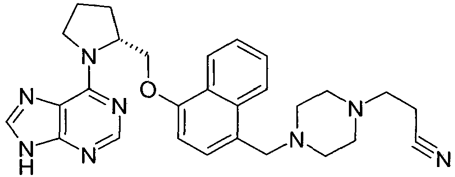 | | 1.399 Min |
| | | | [497.2] |
| | 3-(4-{4-[(R)-1-(9H-Purin-6-yl)-pyrrolidin-2-ylmethoxy]-naphthalin-1-ylmethyl}-piperazin-1-yl)-propionitril | | |
| "A129" | 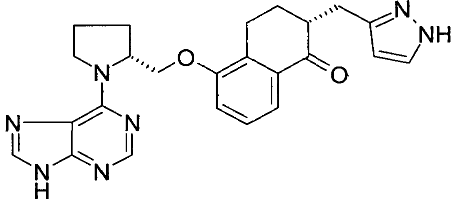 | | |
| | (R)-5-[(R)-1-(9H-Purin-6-yl)-pyrrolidin-2-ylmethoxy]-2-(1H-pyrazol-3-ylmethyl)-3,4-dihydro-2H-naphthalin-1-on | | |
| "A130" | 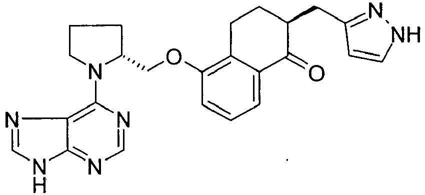 | | |
| | (S)-5-[(R)-1-(9H-Purin-6-yl)-pyrrolidin-2-ylmethoxy]-2-(1H-pyrazol-3-ylmethyl)-3,4-dihydro-2H-naphthalin-1-on | | |
| "A131" | 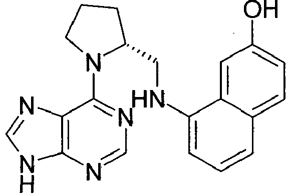 | | |
| | 8-{[(R)-1-(9H-Purin-6-yl)-pyrrolidin-2-ylmethyl]-amino}-naphthalin-2-ol | | |
| ¹H NMR (500 MHz, d₆-DMSO) δ [ppm] 12.97 (br. s, 1H), 9.41 (s, 1H), 8.37 (br, s. 1H), 8.13 (br. s, 1H), 7.59 (d, 1H, J = 8.7 Hz), 7.29 (br. s, 1H), 7.08 (dd, 1H, J = 7.5 Hz, J = 7.9 Hz), 7.02 (dd, 1H, J = 2.2 Hz, J = 8.7 Hz), 6.99 (d, 1H, J = 7.9 Hz), 6.92 (d, 1H, J = 7.5 Hz), 6.33 (br, m, 1H), 4.86 (br, m, 1H), 4.18 (br. m, 1H), 3.61 (m, 1H), 3.18 (m, 1H), 2.13 (m, 2H), 1.99 (m, 2H). | | | |
| "A132" | 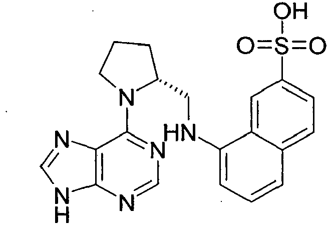 | | |
| | 8-{[(R)-1-(9H-Purin-6-yl)-pyrrolidin-2-ylmethyl]-amino}-naphthalin-2-sulfonsäure | | |
| ¹H NMR (500 MHz, d₆-DMSO) δ [ppm] 8.58 (br. s, 1H), 8.43 (s, 1H), 8.09 (br. s, 1H), 7.67 (d, 1H, J = 8.5 Hz), 7.63 (dd, 1H, J = 1.2 Hz, J = 8.5Hz), 7.30 (t, 1H, J = 7.8 Hz), 7.15 (br. s, 1H), 7.05 (d, 1H, J = 8.1 Hz), 6.99 (d, 1H, J = 7.7 Hz), 4.86 (br. m, 1H), 4.19 (br. m, 1H), 3.77 (br. m, 1H), 3.62 (m, 1H), 2.97 (m, 1H), 2.14 (m, 2H), 2.01 (m, 2H). | | | |
| "A133" | 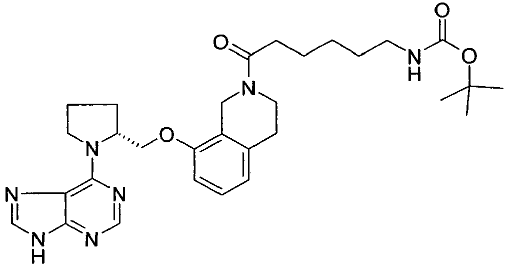 | 82-83.5 | 2.039 Min |
| | (6-Oxo-6-{8-[(R)-1-(9H-purin-6-yl)-pyrrolidin-2-ylmethoxy]-3,4-dihydro-1H-isochinolin-2-yl}-hexyl)-carbaminsäure-tert-butylester | | [564.3]^{§} |
| "A134" | 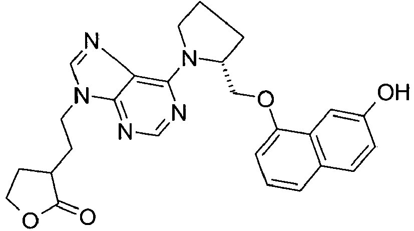 | | 2.012 Min |
| | | | [474.2]^{§} |
| | 3-(2-{6-[(R)-2-(7-Hydroxy-naphthalin-1-yloxymethyl)-pyrrolidin-1-yl]-purin-9-yl}-ethyl)-dihydro-furan-2-on | | |
| "A135" | 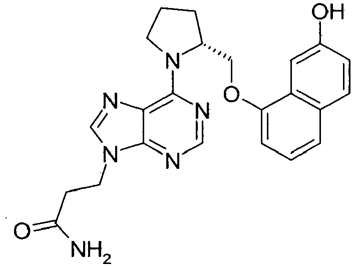 | | 1.737 Min |
| | | | [433.2]^{§} |
| | 3-{6-[(R)-2-(7-Hydroxy-naphthalin-1-yloxymethyl)-pyrrolidin-1-yl]-purin-9-yl}-propionamid | | |
| "A136" | 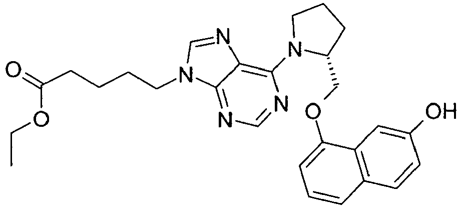 | | 2.190 Min |
| | | | 490.2]^{§} |
| | 5-{6-[(R)-2-(7-Hydroxy-naphthalin-1-yloxymethyl)-pyrrolidin-1-yl]-purin-9-yl}-pentansäure-ethylester | | |
| "A137" | 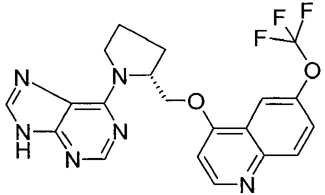 | 111-112 | 1.443 Min |
| | | | [431.2] |
| | 4-[(R)-1-(9H-Purin-6-yl)-pyrrolidin-2-ylmethoxy]-6-trifluormethoxy-chinolin | | |
| ¹H NMR (500 MHz, d₆-DMSO) δ [ppm] 12.98 (br. s, 1H), 8.77 (d, 1H, J= 5.1 Hz), 8.25 (s, 1H), 8.12 (s, 1H), 8.08 (d, 1H, J = 9.1 Hz), 7.89 (br. m, 1H), 7.73 (dd, 1H, J = 2.5 Hz, J = 9.1 Hz), 7.29 (br. m, 1H), 5.16 (br. m, 1H), 4.62 (dd, 1H, J = 3.8 Hz, J = 9.7 Hz), 4.41 (dd, 1H, J = 7.4 Hz, J = 9.7 Hz), 3.93 (br. m, 2H), 2.20 (br. m, 3H), 2.07 (br. m, 1H). | | | |
| "A138" | 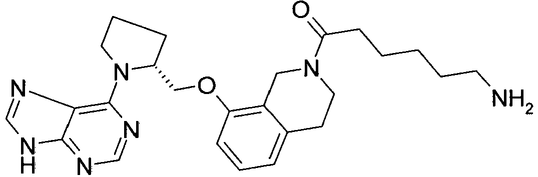 | 76-78 | 1.372 Min |
| | | | [464.3]^{§} |
| | 6-Amino-1-{8-[(R)-1-(9H-purin-6-yl)-pyrrolidin-2-ylmethoxy]-3,4-dihydro-1H-isochinolin-2-yl}-hexan-1-on | | |
| "A139" | 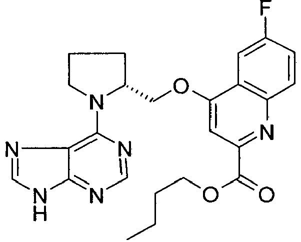 | 108-109 | 2.154 Min |
| | | | [465.2]^{§} |
| | 6-Fluor-4-[(R)-1-(9H-purin-6-yl)-pyrrolidin-2-ylmethoxy]-chinolin-2-carbonsäure-butylester | | |
| "A140" | 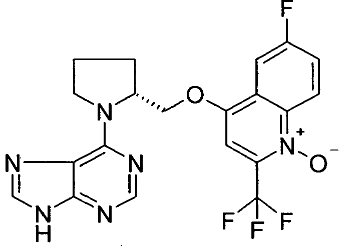 | 232-233 | 2.303 Min |
| | | | [449.1]^{§} |
| | 6-Fluor-4-[(R)-1-(9H-purin-6-yl)-pyrrolidin-2-ylmethoxy]-2-trifluormethyl-chinolin-1-oxid | | |
| "A141" | 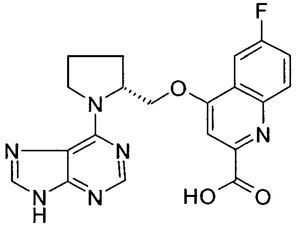 | 197-198 | 1.382 Min |
| | | | [409.1]^{§} |
| | 6-Fluor-4-[(R)-1-(9H-purin-6-yl)-pyrrolidin-2-ylmethoxy]-chinolin-2-carbonsäure | | |
| "A142" | 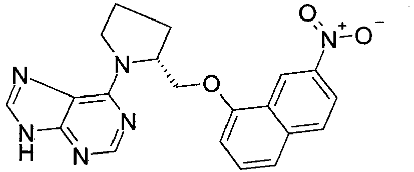 | | 2.020 Min |
| | | | [391.1]^{§} |
| | 6-[(R)-2-(7-Nitro-naphthalin-1-yloxymethyl)-pyrrolidin-1-yl]-9H-purin | | |
| "A144" | 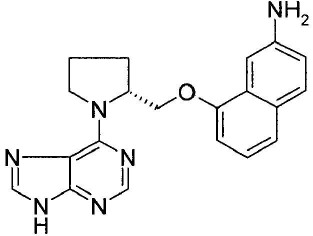 | | |
| | 8-[(R)-1-(9H-Purin-6-yl)-pyrrolidin-2-ylmethoxy]-naphthalin-2-ylamin | | |
| "A145" | 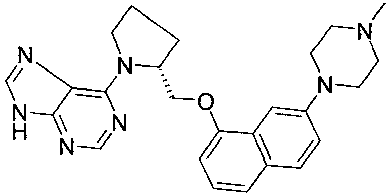 | 108-110 | 1.439 Min^{§} |
| | | | [444.2] |
| | 6-{(R)-2-[7-(4-Methyl-piperazin-1-yl)-naphthalin-1-yloxymethyl]-pyrrolidin-1-yl}-9H-purin | | |
| "A146" | 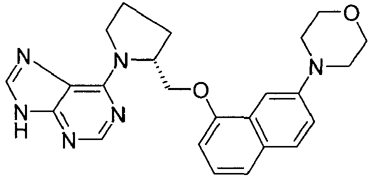 | 210-211 | 1.917 Min^{$} |
| | | | [431.2] |
| | 6-[(R)-2-(7-Morpholin-4-yl-naphthalin-1-yloxymethyl)-pyrrolidin-1-yl]-9H-purin | | |
| "A147" | 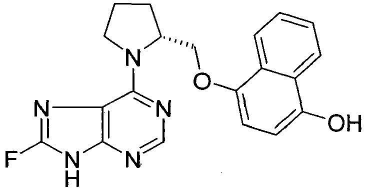 | 110-112 | 2.434 Min^{$} |
| | | | [380.1] |
| | 4-[(R)-1-(8-Fluor-9H-purin-6-yl)-pyrrolidin-2-ylmethoxy]-naphthalin-1-ol | | |
| "A148" | 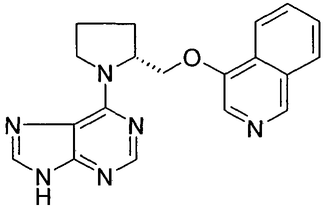 | 157-158.5 | 1.318 Min^{$} |
| | | | [347.1] |
| | 4-[(R)-1-(9H-Purin-6-yl)-pyrrolidin-2-ylmethoxy]-isochinolin | | |
| ¹H NMR (500 MHz, d₆-DMSO) δ [ppm] 12.86 (br. s, 1H), 8.92 (s, 1H), 8.23 (s, 2H), 8.09 (m, 3H), 7.78 (ddd, 1H, J = 0.9 Hz, J = 7.0 Hz, J = 8.0 Hz), 5.12 (br. m, 1H), 4.60 (dd, 1H, J = 3.4 Hz, J = 9.3 Hz), 2.55 (br. m, 2H), 2.23 (m, 3H), 2.06 (m, 1H). | | | |
| "A149" | 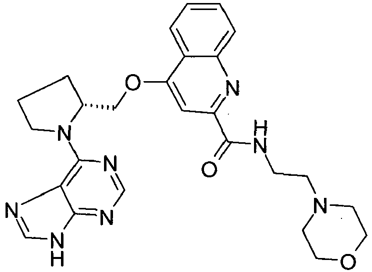 | 136-138 | 2.32** 1.299 min^{$} |
| | | | [503.3] |
| | 4-[(R)-1-(9H-Purin-6-yl)-pyrrolidin-2-ylmethoxy]-chinolin-2-carbonsäure-(2-morpholin-4-yl-ethyl)-amid | | |
| "A150" | 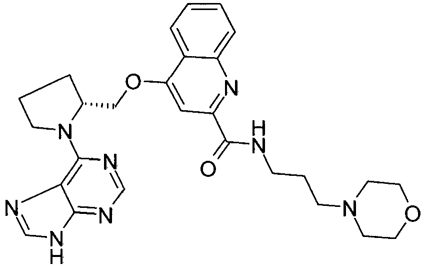 | 173-174 | 2.37** 1.356 min^{$} |
| | | | [517.3] |
| | 4-[(R)-1-(9H-Purin-6-yl)-pyrrolidin-2-ylmethoxy]-chinolin-2-carbonsäure-(3-morpholin-4-yl-propyl)-amid | | |
| "A151" | 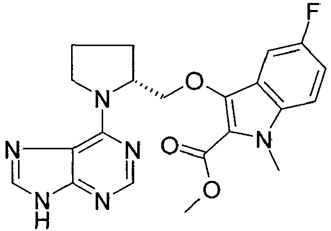 | 170-171 | 1.975 Min^{$} |
| | | | [425.1] |
| | 5-Fluor-1-methyl-3-[(R)-1-(9H-purin-6-yl)-pyrrolidin-2-ylmethoxy]-1H-indol-2-carbonsäure-methylester | | |
| ¹H NMR (500 MHz, d₆-DMSO) δ [ppm] 12.94 (br. s, 1H), 8.20 (s, 1H), 8.09 (s, 1H), 7.59 (dd, 1H, J = 4.3 Hz, J = 9.2 Hz),7.49 (dd, 1H, J = 2.5 Hz, J = 9.2 Hz), 7.22 (dt, 1H, J = 2.5 Hz, J = 9.2 Hz), 5.13 (br. m, 1H), 4.56 (m, 2H), 4.20 (br. m, 2H), 3.91 (s, 3H), 3.87 (s, 3H), 2.16 (m, 3H), 1.97 (br. m, 1H). | | | |
| "A152" | 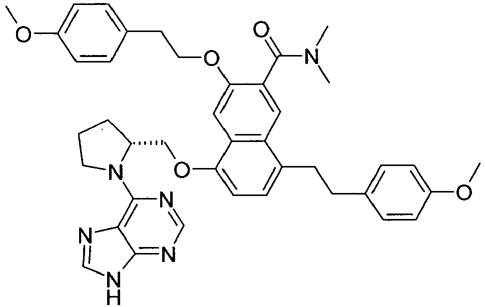 | | |
| | 3-[2-(4-Methoxy-phenyl)-ethoxy]-8-[2-(4-methoxy-phenyl)-ethyl]-5-[(R)-1-(9H-purin-6-yl)-pyrrolidin-2-ylmethoxy]-naphthalin-2-carbonsäure-dimethylamid | | |
| "A153" | 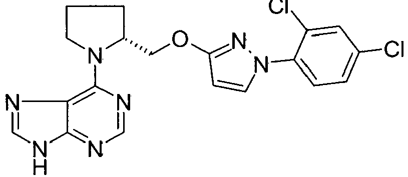 | 147-149 | 1.988 min^{$} |
| | | | [430.1] |
| | 6-{(R)-2-[1-(2,4-Dichlor-phenyl)-1H-pyrazol-3-yloxymethyl]-pyrrolidin-1-yl}-9H-purin | | |
| ¹H NMR (500 MHz, d₆-DMSO) δ [ppm] 12.95 (br. s, 1H), 8.21 (s, 1H), 8.10 (s, 1H), 8.00 (br. s, 1H), 7.82 (d, 1H, J = 1.2 Hz), 7.63 (s, 1H), 7.54 (m, 2H), 4.73 (br. m, 1H), 4.26 (br. m, 1H), 3.99 (t, 1H, J = 8.8 Hz), 3.67 (br. m, 2H), 2.10 (br. m, 3H), 1.99 (br. m, 1H). | | | |
| "A154" | 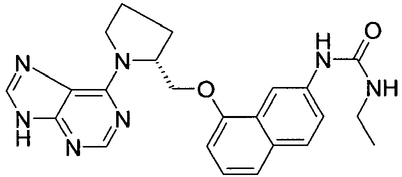 | 160-162 | 1.702 min^{$} |
| | | | [432.2] |
| | 1-Ethyl-3-{8-[(R)-1-(9H-purin-6-yl)-pyrrolidin-2-ylmethoxy]-naphthalin-2-yl}-harnstoff | | |
| "A155" | 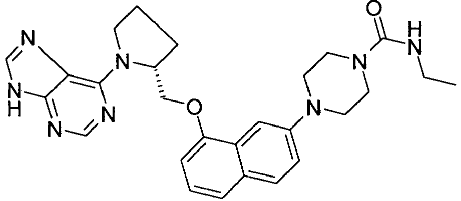 | 141.5-143 | 1.840 min^{$} |
| | | | [501.3] |
| | 4-{8-[(R)-1-(9H-Purin-6-yl)-pyrrolidin-2-ylmethoxy]-naphthalin-2-yl}-piperazin-1-carbonsäure-ethylamid | | |
| "A156" | 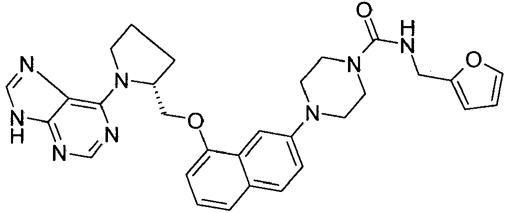 | 136-137 | 1.974 min^{$} |
| | | | [553.3] |
| | 4-{8-[(R)-1-(9H-Purin-6-yl)-pyrrolidin-2-ylmethoxy]-naphthalin-2-yl}-piperazin-1-carbonsäure-(furan-2-ylmethyl)-amid | | |
| "A157" | 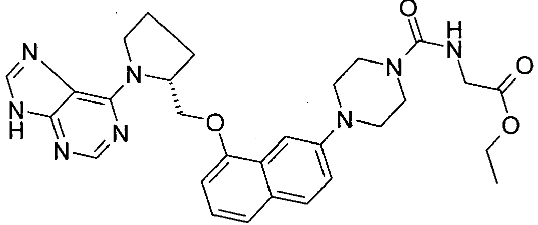 | 130-131 | 1.881 min^{$} |
| | | | [559.3] |
| | [(4-{8-[(R)-1-(9H-Purin-6-yl)-pyrrolidin-2-ylmethoxy]-naphthalin-2-yl}-piperazin-1-carbonyl)-amino]-essigsäure-ethylester | | |
| "A158" | 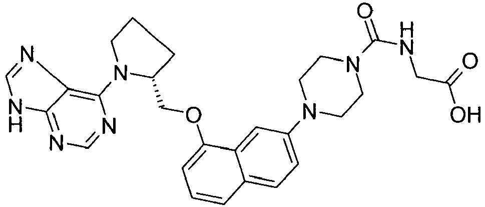 | 210 | 1.748 min^{$} |
| | | | |
| | [(4-{8-[(R)-1-(9H-Purin-6-yl)-pyrrolidin-2-ylmethoxy]-naphthalin-2-yl}-piperazin-1-carbonyl)-amino]-essigsäure | | [531.3] |
| "A159" | 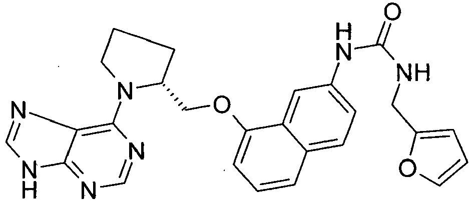 | 141-143 | 1.981 min^{$} |
| | | | [484.2] |
| | 1-Furan-2-ylmethyl-3-{8-[(R)-1-(9H-purin-6-yl)-pyrrolidin-2-ylmethoxy]-naphthalin-2-yl}-harnstoff | | |
| "A160" | 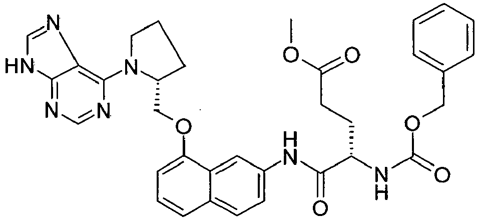 | 122-124 | |
| | (S)-4-Benzyloxycarbonylamino-4-{8-[(R)-1-(9H-purin-6-yl)-pyrrolidin-2-ylmethoxy]-naphthalin-2-ylcarbamoyl}-buttersäure-methylester | | |
| "A161" | 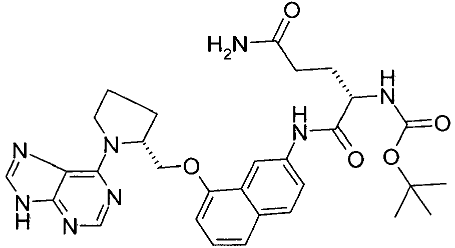 | 146-148 | 1.867 min^{$} |
| | | | [589.3] |
| | ((S)-3-Carbamoyl-1-{8-[(R)-1-(9H-purin-6-yl)-pyrrolidin-2-ylmethoxy]-naphthalin-2-ylcarbamoyl}-propyl)-carbaminsäure-tert.-butylester | | |
| "A162" | 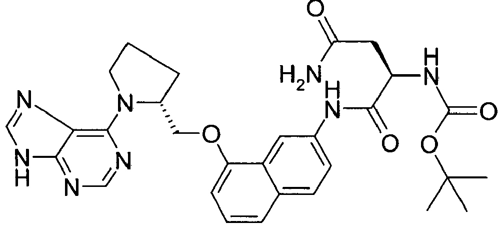 | 157-160 | 1.864 min^{$} |
| | | | [575.3] |
| | ((R)-2-Carbamoyl-1-{8-[(R)-1-(9H-purin-6-yl)-pyrrolidin-2-ylmethoxy]-naphthalin-2-ylcarbamoyl}-ethyl)-carbaminsäure-tert.-butylester | | |
| "A163" | 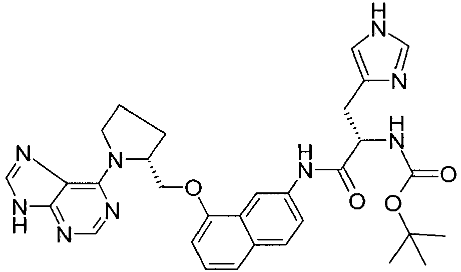 | 181-182 | 1.793 min^{$} |
| | | | [598.3] |
| | ((S)-2-(1H-Imidazol-4-yl)-1-{8-[(R)-1-(9H-purin-6-yl)-pyrrolidin-2-ylmethoxy]-naphthalin-2-ylcarbamoyl}-ethyl)-carbaminsäure-tert.-butyl ester | | |
| "A164" | 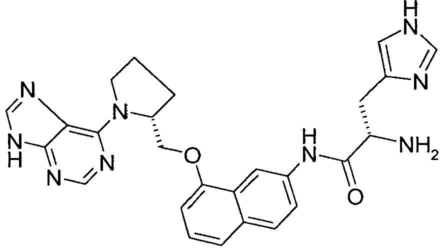 | 210 (Zers.) | 1.333 min [498.2] |
| | (S)-2-Amino-3-(1H-imidazol-4-yl)-N-{8-[(R)-1-(9H-purin-6-yl)-pyrrolidin-2-ylmethoxy]-naphthalin-2-yl}-propionamid * HCl | | |
| "A165" | 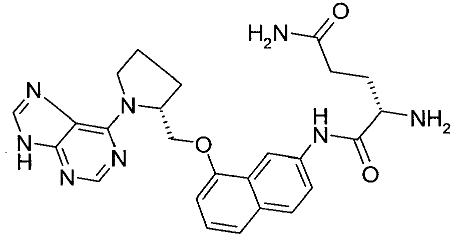 | 210-212 | 1.441 min^{$} |
| | | | [489.2] |
| | (S)-2-Amino-pentansäure-5-amid-1-({8-[(R)-1-(9H-purin-6-yl)-pyrrolidin-2-ylmethoxy]-naphthalin-2-yl}-amid) * HCl | | |
| "A166" | 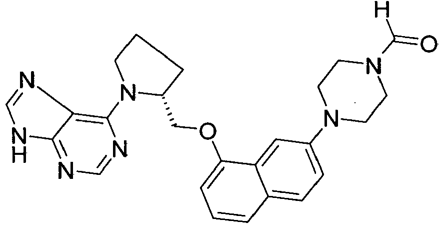 | 134-135 | 1.829 min^{$} |
| | | | [458.2] |
| | 4-{8-[(R)-1-(9H-Purin-6-yl)-pyrrolidin-2-ylmethoxy]-naphthalin-2-yl}-piperazin-1-carbaldehyd | | |
| "A167" | 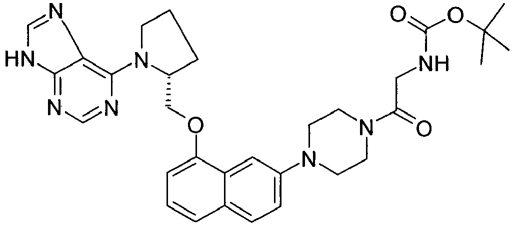 | 140-141 | 2.150 min^{$} |
| | | | [587.3] |
| | [2-Oxo-2-(4-{8-[(R)-1-(9H-purin-6-yl)-pyrrolidin-2-ylmethoxy]-naphthalin-2-yl}-piperazin-1-yl)-ethyl]-carbaminsäure-tert.-butylester | | |
| "A168" | 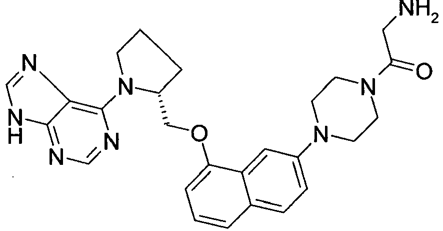 | 247-249 | 1.546 Min^{$} |
| | | | [487.3] |
| | 2-Amino-1-( 4-{8-[(R)-1-(9H-purin-6-yl)-pyrrolidin-2-ylmethoxy]-naphthalin-2-yl}-piperazin-1-yl)-ethanon | | |
| "A169" | 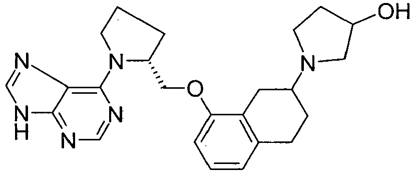 | 73-75 | 1.370 min^{$} |
| | | | [435.3] |
| | 1-{8-[(R)-1-(9H-Purin-6-yl)-pyrrolidin-2-ylmethoxy]-1,2,3,4-tetrahydro-naphthalin-2-yl}-pyrrolidin-3-ol | | |
| "A170" | 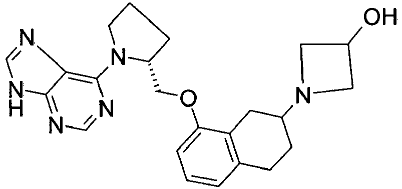 | 295-297 | 1.359 min^{$} |
| | | | [421.2] |
| | 1-{8-[(R)-1-(9H-Purin-6-yl)-pyrrolidin-2-ylmethoxy]-1,2,3,4-tetrahydro-naphthalin-2-yl}-azetidin-3-ol | | |
| "A171" | 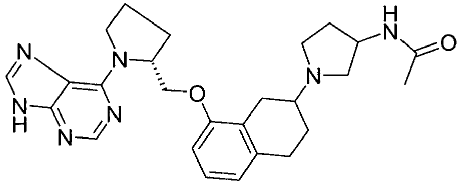 | 163-164.5 | 1.398 min^{$} |
| | | | [476.3] |
| | N-( 1-{8-[(R)-1-(9H-Purin-6-yl)-pyrrolidin-2-ylmethoxy]-1,2,3,4-tetrahydro-naphthalin-2-yl}-pyrrolidin-3-yl)-acetamid | | |
| "A172" | 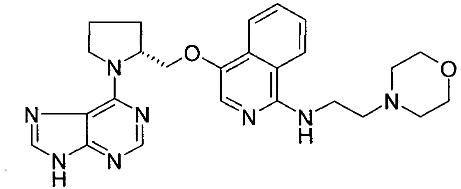 | 87-88 | 1.295 min^{$} |
| | | | [475.2] |
| | (2-Morpholin-4-yl-ethyl)-{4-[(R)-1-(9H-purin-6-yl)-pyrrolidin-2-ylmethoxy]-isochinolin-1-yl}-amin | | |
| "A173" | 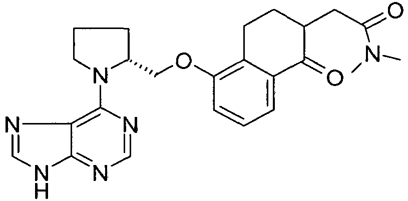 | 79-81.5 | 1.657 min^{$} |
| | | | [449.2] |
| | N,N-Dimethyl-2-{1-oxo-5-[(R)-1-(9H-purin-6-yl)-pyrrolidin-2-ylmethoxy]-1,2,3,4-tetrahydro-naphthalin-2-yl}-acetamid | | |
| "A174" | 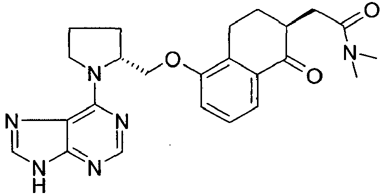 | | |
| | N,N-Dimethyl-2-{(S)-1-oxo-5-[(R)-1-(9H-purin-6-yl)-pyrrolidin-2-ylmethoxy]-1,2,3,4-tetrahydro-naphthalin-2-yl}-acetamid | | |
| "A175" | 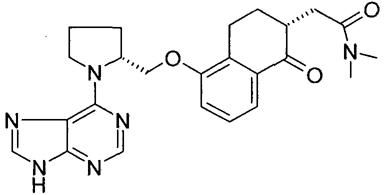 | | |
| | N,N-Dimethyl-2-{(R)-1-oxo-5-[(R)-1-(9H-purin-6-yl)-pyrrolidin-2-ylmethoxy]-1,2,3,4-tetrahydro-naphthalin-2-yl}-acetamid | | |
| "A176" | 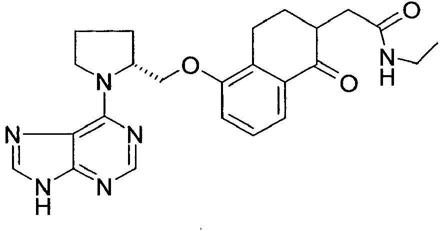 | 93-95 | 1.613 min^{$} |
| | | | [449.2] |
| | N-Ethyl-2-{1-oxo-5-[(R)-1-(9H-purin-6-yl)-pyrrolidin-2-ylmethoxy]-1,2,3,4-tetrahydronaphthalin-2-yl}-acetamid | | |
| "A177" | 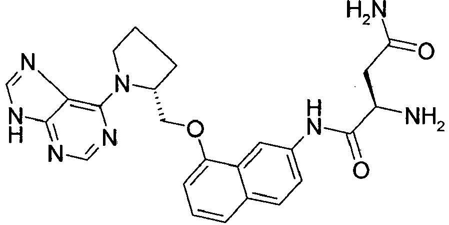 | | |
| | (R)-2-Amino-N1-{8-[(R)-1-(9H-purin-6-yl)-pyrrolidin-2-ylmethoxy]-naphthalin-2-yl}-succinamid | | |
| "A178" | 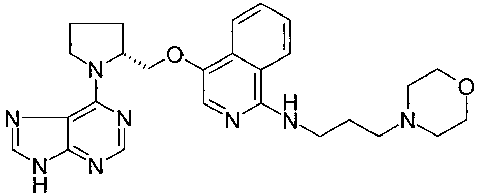 | 73-75 | 1.222 min^{$} |
| | | | [489.3] |
| | (3-Morpholin-4-yl-propyl)-{4-[(R)-1-(9H-purin-6-yl)-pyrrolidin-2-ylmethoxy]-isochinolin-1-yl}-amin | | |
| "A179" | 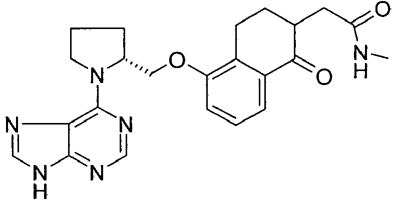 | 97-98 | 1.551 min^{$} |
| | | | [435.2] |
| | N-Methyl-2-{1-oxo-5-[(R)-1-(9H-purin-6-yl)-pyrrolidin-2-ylmethoxy]-1,2,3,4-tetrahydro-naphthalin-2-yl}-acetamid | | |
| "A180" | 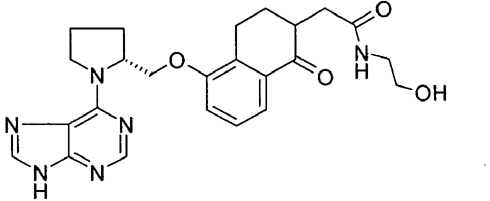 | 101-103 | 1.474 min^{$} |
| | | | [465.2] |
| | N-(2-Hydroxy-ethyl)-2-{1-oxo-5-[(R)-1-(9H-purin-6-yl)-pyrrolidin-2-ylmethoxy]-1,2,3,4-tetrahydro-naphthalin-2-yl}-acetamid | | |
| "A181" | 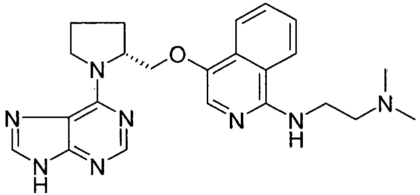 | 63-65 | 1.261 min^{$} |
| | | | [433.2] |
| | N,N-Dimethyl-N'-{4-[(R)-1-(9H-purin-6-yl)-pyrrolidin-2-ylmethoxy]-isochinolin-1-yl}-ethan-1,2-diamin | | |
| "A182" | 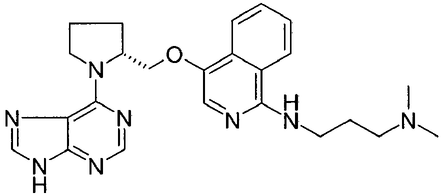 | 74-75 | 1.255 min^{$} |
| | | | [447.2] |
| | N,N-Dimethyl-N'-{4-[(R)-1-(9H-purin-6-yl)-pyrrolidin-2-ylmethoxy]-isochinolin-1-yl}-propan-1,3-diamin | | |
| "A183" | 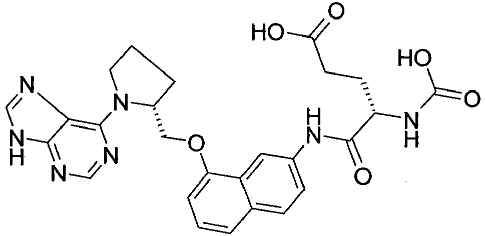 | 176-178 | 1.653 min^{$} |
| | | | [534.2] |
| | (S)-4-Carboxyamino-4-{8-[(R)-1-(9H-purin-6-yl)-pyrrolidin-2-ylmethoxy]-naphthalin-2-ylcarbamoyl}-buttersäure | | |
| "A184" | 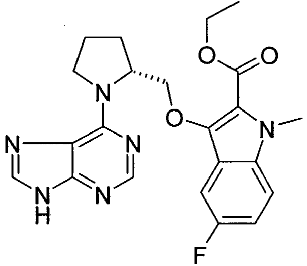 | 132-134 | 2.095 min^{$} |
| | | | [439.2] |
| | 5-Fluor-1-methyl-3-[(R)-1-(9H-purin-6-yl)-pyrrolidin-2-ylmethoxy]-1H-indol-2-carbonsäure-ethylester | | |
| "A185" | 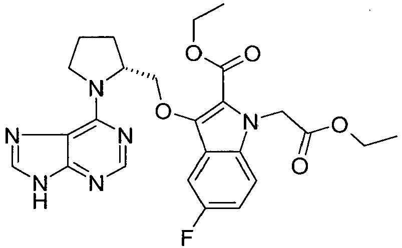 | 178-180 | 2.110 min^{$} |
| | | | [511.2] |
| | 1-Ethoxycarbonylmethyl-5-fluor-3-[(R)-1-(9H-purin-6-yl)-pyrrolidin-2-ylmethoxy]-1H-indol-2-carbonsäure-ethylester | | |
| "A186" | 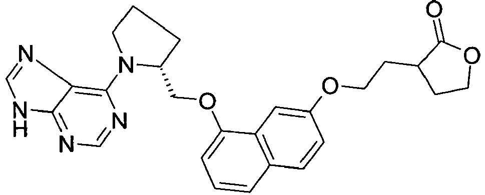 | | 2.075 Min^{$} |
| | | | [474.2] |
| | 3-(2-{8-[(R)-1-(9H-Purin-6-yl)-pyrrolidin-2-ylmethoxy]-naphthalin-2-yloxy}-ethyl)-dihydro-furan-2-on | | |
| "A187" | 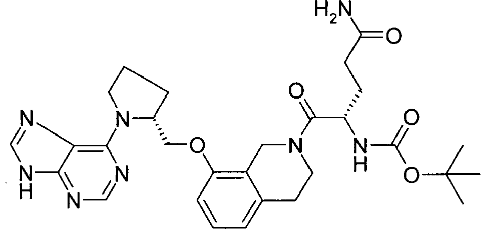 | 78-80 | 1.737 min^{$} |
| | | | [579.3] |
| | ((S)-3-Carbamoyl-1-{8-[(R)-1-(9H-purin-6-yl)-pyrrolidin-2-ylmethoxy]-3,4-dihydro-1H-isochinolin-2-carbonyl}-propyl)-carbaminsäure-tert. -butylester | | |
| "A188" | 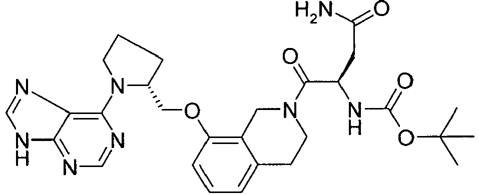 | 82-83.5 | 1.738 min^{$} |
| | | | [565.3] |
| | ((R)-1-Carbamoylmethyl-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butylester | | |
| "A189" | 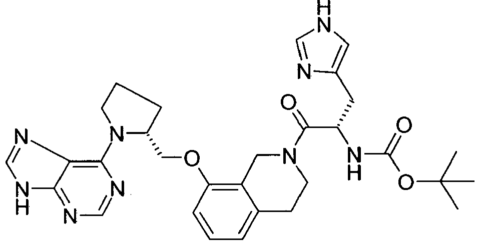 | 93-95 | 1.587 min^{$} |
| | | | [588.3] |
| | ((S)-1-(1H-Imidazo)-4-ylmethyl)-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butylester | | |
| "A190" | 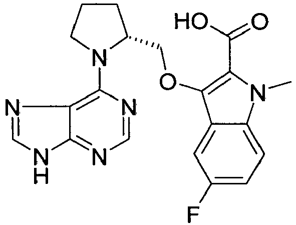 | | 1.750 min^{$} |
| | | | [411.1] |
| | 5-Fluor-1-methyl-3-[(R)-1-(9H-purin-6-yl)-pyrrolidin-2-ylmethoxy]-1H-indol-2-carbonsäure | | |
| "A191" | 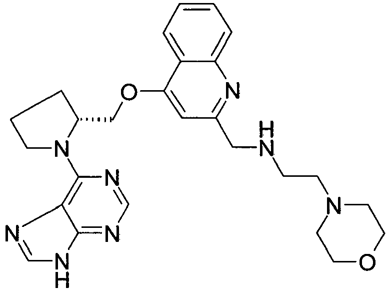 | | 1.339 min^{$} |
| | | | [489.2] |
| | (2-Morpholin-4-yl-ethyl)-{4-[(R)-1-(9H-purin-6-yl)-pyrrolidin-2-ylmethoxy]-chinolin-2-ylmethyl}-amin | | |
| "A192" | 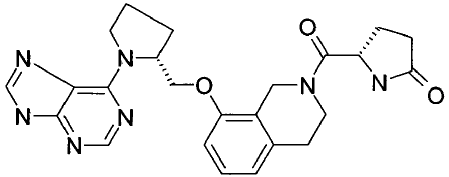 | 112-113 | 1.565 min^{$} |
| | | | [462.2] |
| | (S)-5-{8-[(R)-1-(9H-purin-6-yl)-pyrrolidin-2-ylmethoxy]-3,4-dihydro-1H-isochinolin-2-carbonyl}-pyrrolidin-2-on | | |
| "A193" | 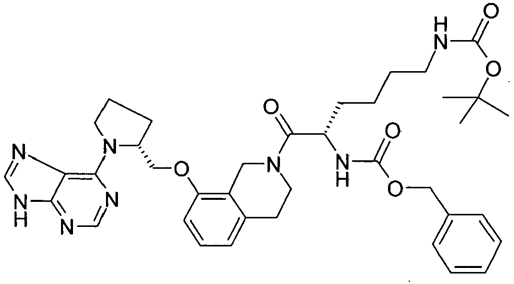 | 94-96 | 2.305 min^{$} |
| | | | [713.4] |
| | ((S)-5-Benzyloxycarbonylamino-6-oxo-6-{8-[(R)-1-(9H-purin-6-yl)-pyrrolidin-2-ylmethoxy]-3,4-dihydro-1H-isochinolin-2-yl}-hexyl)-carbaminsäure-tert.-butylester | | |
| "A194" | 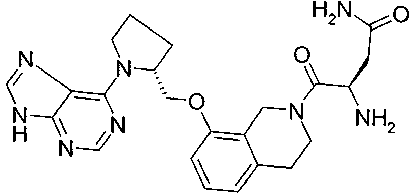 | 123-124 | 1.295 min^{$} |
| | | | [465.3] |
| | (R)-3-Amino-4-oxo-4-{8-[(R)-1-(9H-purin-6-yl)-pyrrolidin-2-ylmethoxy]-3,4-dihydro-1H-isochinolin-2-yl}-butyramid | | |
| "A195" | 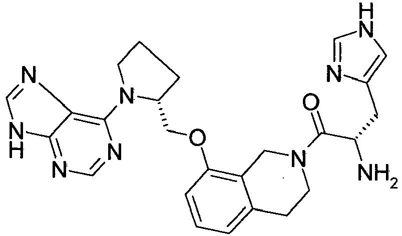 | 93-94 | 1.194 min^{$} |
| | | | [488.2] |
| | (S)-2-Amino-3-(1H-imidazol-4-yl)-1-{8-[(R)-1-(9H-purin-6-yl)-pyrrolidin-2-ylmethoxy]-3,4-dihydro-1H-isochinolin-2-yl}-propan-1-on | | |
| "A196" | 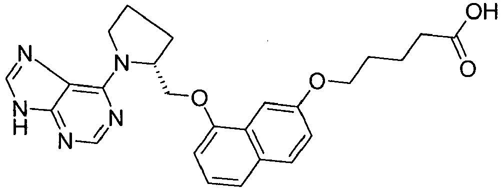 | >299 | 2.024 min^{$} |
| | | | [462.2] |
| | 5-{8-[(R)-1-(9H-Purin-6-yl)-pyrrolidin-2-ylmethoxy]-naphthalin-2-yloxy}-pentansäure | | |
| "A197" | 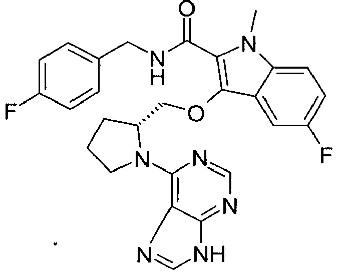 | 135-137 | 2.274 min [518.2] |
| | 5-Fluor-1-methyl-3-[(R)-1-(9H-purin-6-yl)-pyrrolidin-2-ylmethoxy]-1H-indol-2-carbonsäure-4-fluor-benzylamid | | |
| "A198" | 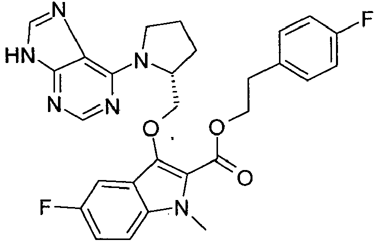 | 197-199 | 2.339 min^{$} |
| | | | [532.2] |
| | 5-Fluor-1-methyl-3-[(R)-1-(9H-purin-6-yl)-pyrrolidin-2-ylmethoxy]-1H-indol-2-carbonsäure-2-(4-fluor-phenyl)-ethylester | | |
| "A199" | 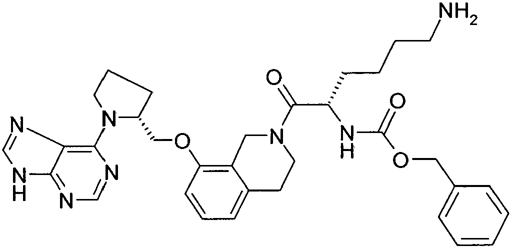 | 104-106 | 1.667 min^{$} |
| | | | [613.3] |
| | ((S)-5-Amino-1-{8-[(R)-1-(9H-purin-6-yl)-pyrrolidin-2-ylmethoxy]-3,4-dihydro-1H-isochinolin-2-carbonyl}-pentyl)-carbaminsäure-benzylester | | |
| "A200" | 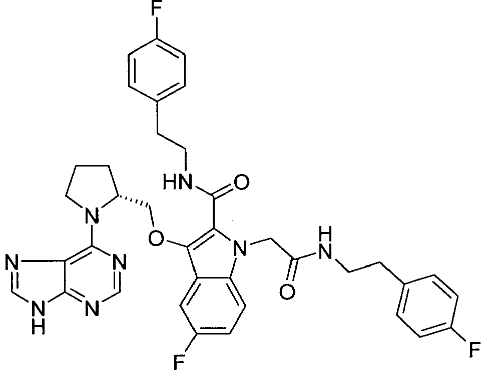 | | 2.403 min^{$} |
| | | | [697.3] |
| | 5-Fluor-1-{[2-(4-fluor-phenyl)-ethylcarbamoyl]-methyl}-3-[(R)-1-(9H-purin-6-yl)-pyrrolidin-2-ylmethoxy]-1H-indol-2-carbonsäure-[2-(4-fluor-phenyl)-ethyl]-amid | | |
| "A201" | 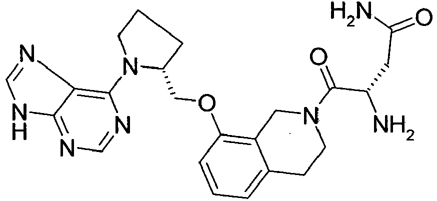 | 103-104.5 | 1.290 min^{$} |
| | | | [465.2] |
| | (S)-3-Amino-4-oxo-4-{8-[(R)-1-(9H-purin-6-yl)-pyrrolidin-2-ylmethoxy]-3,4-dihydro-1H-isochinolin-2-yl}-butyramid | | |
| "A202" | 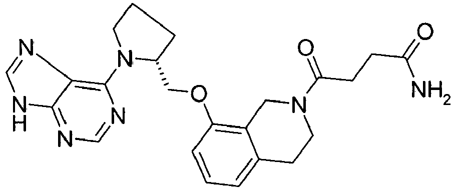 | 102-104 | 1.527 min^{$} [450.2] |
| | 4-Oxo-4-{8-[(R)-1-(9H-purin-6-yl)-pyrrolidin-2-ylmethoxy]-3,4-dihydro-1H-isochinolin-2-yl}-butyramid | | |
| "A203" | 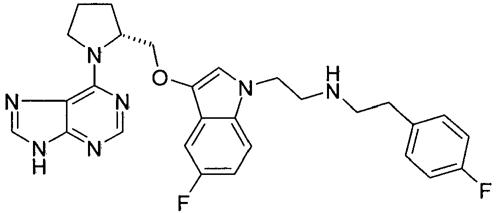 | 89-91 | 2.155 min^{$} [518.2] |
| | [2-(4-Fluor-phenyl)-ethyl]-(2-{5-fluor-3-[(R)-1-(9H-purin-6-yl)-pyrrolidin-2-ylmethoxy]-indol-1-yl}-ethyl)-amin | | |

**Tabelle 1**

| Inhibierung der MetAP-2 | | | |
|---|---|---|---|
| IC₅₀ von erfindungsgemäßen Verbindungen | | | |
| Verbindung Nr. | IC₅₀ Enzym | Verbindung Nr. | IC₅₀ Enzym |
| "A1" | A | "A31" | |
| "A2" | A | | |
| "A3" | A | "A33" | C |
| "A4" | A | "A34" | C |
| | | "A35" | |
| "A6" | | "A36" | |
| | | "A37" | B |
| "A8" | B | "A38" | A |
| | | "A39" | B |
| | | "A40" | B |
| | | "A41" | A |
| "A12" | B | "A42" | B |
| | | "A43" | B |
| "A14" | B | "A44" | A |
| "A15" | A | "A45" | C |
| | | "A46" | C |
| | | "A47" | B |
| "A18" | C | "A48" | A |
| "A19" | | "A49" | B |
| "A20" | | "A50" | A |
| | | "A51" | A |
| | | "A52" | |
| "A23" | C | "A53" | A |
| | | "A54" | |
| "A25" | B | "A55" | A |
| "A26" | C | "A56" | B |
| | | "A57" | A |
| "A28" | B | "A58" | A |
| | | "A59" | C |
| "A30" | C | "A60" | A |
| | | | |
| | | | |

| Verbindung Nr. | IC₅₀ Enzym | Verbindung Nr. | IC₅₀ Enzym |
|---|---|---|---|
| "A61" | C | "A81" | A |
| "A62" | B | "A82" | A |
| "A63" | B | "A83" | A |
| "A64" | C | "A84" | A |
| "A65" | A | "A85" | A |
| "A66" | A | "A86" | A |
| "A67" | A | "A87" | A |
| "A68" | A | "A88" | A |
| "A69" | B | "A89" | A |
| "A70" | A | | |
| "A71" | A | | |
| "A72" | B | "A92" | A |
| "A73" | B | "A93" | A |
| | | "A94" | A |
| "A75" | B | "A95" | A |
| | | "A96" | A |
| "A77" | B | "A97" | A |
| | | "A98" | A |
| | | "A99" | A |
| "A80" | A | "A100" | A |
| "A101" | A | "A121" | B |
| "A102" | A | "A122" | A |
| "A103" | B | "A123" | A |
| "A104" | B | "A124" | B |
| "A105" | A | "A125" | A |
| "A106" | C | "A126" | A |
| "A107" | B | "A127" | A |
| "A108" | C | "A128" | A |
| "A109" | A | "A129" | A |
| "A110" | A | "A130" | A |
| "A111" | A | "A131" | C |
| "A112" | A | "A132" | C |
| "A113" | A | "A133" | B |
| "A114" | A | "A134" | B |
| "A115" | A | "A135" | C |
| "A116" | A | "A136" | C |
| "A117" | A | "A137" | A |
| "A118" | A | "A138" | A |
| "A119" | B | "A139" | A |
| "A120" | B | "A140" | A |
| | | | |

| Verbindung Nr. | IC₅₀ Enzym | Verbindung Nr. | IC₅₀ Enzym |
|---|---|---|---|
| "A141" | A | "A161" | B |
| "A142" | A | "A162" | B |
| | | "A163" | B |
| "A144" | A | "A164" | B |
| "A145" | B | "A165" | B |
| "A146" | A | "A166" | A |
| "A147" | B | "A167" | B |
| "A148" | A | "A168" | B |
| "A149" | C | "A169" | B |
| "A150" | A | "A170" | A |
| "A151" | A | "A171" | B |
| "A152" | C | "A172" | B |
| "A153" | A | "A173" | A |
| "A154" | B | "A174" | A |
| "A155" | B | "A175" | A |
| "A156" | B | "A176" | A |
| "A157" | B | "A177" | B |
| "A158" | C | "A178" | B |
| "A159" | B | "A179" | A |
| "A160" | C | "A180" | A |
| | | | |

| Verbindung Nr. | IC₅₀ Enzym | Verbindung Nr. | IC₅₀ Enzym |
|---|---|---|---|
| "A181" | A | "A191" | A |
| "A182" | A | "A192" | A |
| "A183" | C | "A193" | C |
| "A184" | C | "A194" | A |
| "A185" | C | "A195" | A |
| "A186" | B | "A196" | C |
| "A187" | B | "A197" | C |
| "A188" | B | "A198" | C |
| "A189" | B | "A199" | B |
| "A190" | C | "A200" | C |
| | | "A201" | B |
| | | "A202" | B |
| | | "A203" | C |

| | | | |
|---|---|---|---|
| IC₅₀: 10 nM - 1 µM = A 1 µM -10 µM = B > 10 µM = C | | | |

Die nachfolgenden Beispiele betreffen Arzneimittel:

### Beispiel A: Injektionsgläser

Eine Lösung von 100 g eines Wirkstoffes der Formel I gemäß Anspruch 1 und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Verbindung Nr. | Name und/oder Struktur |
|---|---|
| "A1" | 6-[(R)-2-(Naphthalin-1-yloxymethyl)-pyrrolidin-1-yl]-9H-purin |
| "A2" | 4-[(R)-1-(9H-Purin-6-yl)-pyrrolidin-2-ylmethoxy]-naphthalin-1-carbaldehyd |
| "A3" | 6-[(R)-2-(4-Morpholin-4-ylmethyl-naphthalin-1-yloxymethyl)-pyrrolidin-1-yl]-9H-purin |
| "A4" | 6-[(R)-2-(4-Butoxymethyl-naphthalin-1-yloxymethyl)-pyrrolidin-1-yl]-9H-purin |
| "A6" | Morpholin-4-yl-(4-{2-[-1-(9H-purin-6-yl)-pyrrolidin-2-yl]-ethyl}-naphthalin-1-yl)-methanon |
| "A8" | 6-[(R)-4,4-Difluor-2-(naphthalin-1-yloxymethyl)-pyrrolidin-1-yl]-9H-purin |
| "A12" | 6-[(R)-2-((E)-2-Naphthalin-1-yl-vinyl)-pyrrolidin-1-yl]-9H-purin |
| "A14" | 4-[(R)-1-(9H-Purin-6-yl)-pyrrolidin-2-ylmethoxy]-chinolin-2-carbonsäure-cyclopropylamid |
| "A15" | 2-[1-(1H-Imidazol-4-yl)-meth-(Z)-yliden]-5-[(R)-1-(9H-purin-6-yl)-pyrrolidin-2-ylmethoxy]-3,4-dihydro-2H-naphthalin-1-on |
| "A15.1" | 2-(1H-Imidazol-4-ylmethyl)-5-[(R)-1-(9H-purin-6-yl)-pyrrolidin-2-ylmethoxy]-3,4-dihydro-2H-naphthalin-1-on |
| "A18" | 8-Brom-6-[(R)-2-(naphthalin-1-yloxymethyl)-pyrrolidin-1-yl]-9H-purin |
| "A19" | 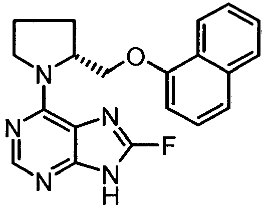 |
| "A20" | 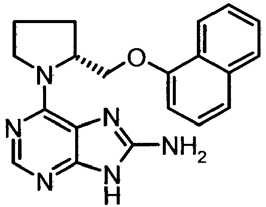 |
| "A23" | 4-[(R)-2-(Naphthalin-1-yloxymethyl)-pyrrolidin-1-yl]-pyrido[2,3-d]pyrimidin |
| "A25" | 6-[(R)-2-(Naphthalin-1-yloxymethyl)-pyrrolidin-1-yl]-purin-9-ylamin |
| "A26" | 6-[(2R,4R)-4-Fluor-2-(naphthalin-1-yloxymethyl)-pyrrolidin-1-yl]-9H-purin |
| "A28" | 6-[(2R,4S)-4-Fluor-2-(naphthalin-1-yloxymethyl)-pyrrolidin-1-yl]-9H-purin |
| "A30" | (3R,5R)-5-(Naphthalin-1-yloxymethyl)-1-(9H-purin-6-yl)-pyrrolidin-3-ol |
| "A31" | 6-[(R)-2,2-Difluor-5-(naphthalin-1-yloxymethyl)-pyrrolidin-1-yl]-9H-purin |
| "A33" | N-[6-(3-{2-[1-(9H-Purin-6-yl)-pyrrolidin-2-ylmethoxy]-5-trifluormethyl-phenyl}-ureidomethyl)-1H-benzimidazol-2-yl]-acetamid |
| "A34" | 4-[4-(3-{2-[1-(9H-Purin-6-yl)-pyrrolidin-2-ylmethoxy]-5-trifluormethyl-phenyl}-ureidomethyl)-phenoxy]-pyridin-2-carbonsäure-methylamid |
| "A35" | 4-[4-(3-{2-[1-(9H-Purin-6-yl)-pyrrolidin-2-ylmethoxy]-5-trifluormethyl-phenyl}-ureido)-phenoxy]-pyridin-2-carbonsäure-methylamid |
| "A36" | 4-[3-(3-{2-[1-(9H-Purin-6-yl)-pyrrolidin-2-ylmethoxy]-5-trifluormethyl-phenyl}-ureido)-phenoxy]-pyridin-2-carbonsäure-methylamid |
| "A37" | 6-[(R)-2-(Dibenzofuran-3-yloxymethyl)-pyrrolidin-1-yl]-9H-purin |
| "A38" | 4-[(R)-1-(9H-Purin-6-yl)-pyrrolidin-2-ylmethoxy]-9H-carbazol |
| "A39" | 1-Butyl-2-methyl-5-[(R)-1-(9H-purin-6-yl)-pyrrolidin-2-ylmethoxy]-1H-indol-3-carbonsäure-ethylester |
| "A40" | 6-[(R)-2-(1H-Indol-4-yloxymethyl)-pyrrolidin-1-yl]-9H-purin |
| "A41" | 1-{7-[(R)-1-(9H-Purin-6-yl)-pyrrolidin-2-ylmethoxy]-benzofuran-2-yl}-ethanon |
| "A42" | 5-Piperidin-1-ylmethyl-8-[(R)-1-(9H-purin-6-yl)-pyrrolidin-2-ylmethoxy]-chinolin |
| "A43" | 8-[(R)-1-(9H-Purin-6-yl)-pyrrolidin-2-ylmethoxy]-chinolin |
| "A44" | 5-[(R)-1-(9H-Purin-6-yl)-pyrrolidin-2-ylmethoxy]-isochinolin |
| "A45" | 7-Benzyloxy-6-methoxy-4-[(R)-1-(9H-purin-6-yl)-pyrrolidin-2-ylmethoxy]-chinazolin |
| "A46" | 4-[(R)-1-(9H-Purin-6-yl)-pyrrolidin-2-ylmethoxy]-chinazolin |
| "A47" | 2-{2-[2-({4-[(R)-1-(9H-Purin-6-yl)-pyrrolidin-2-ylmethoxy]-naphthalin-1-ylmethyl}-amino)-ethoxy]-ethoxy}-ethylamin |
| "A48" | 4-[(R)-1-(9H-Purin-6-yl)-pyrrolidin-2-ylmethoxy]-chinolin-2-carbonsäure-methylamid |
| "A49" | 2-Methyl-8-[(R)-1-(9H-purin-6-yl)-pyrrolidin-2-ylmethoxy]-chinolin |
| "A50" | 4-[(R)-1-(9H-Purin-6-yl)-pyrrolidin-2-ylmethoxy]-chinolin-2-carbonsäure-ethylamid |
| "A51" | 5-[(R)-1-(9H-Purin-6-yl)-pyrrolidin-2-ylmethoxy]-3,4-dihydro-2H-naphthalin-1-on |
| "A52" | 2-Hydroxymethylen-5-[(R)-1-(9H-purin-6-yl)-pyrrolidin-2-ylmethoxy]-3,4-dihydro-2H-naphthalin-1-on |
| "A53" | [1-Oxo-5-[(R)-1-(9H-purin-6-yl)-pyrrolidin-2-ylmethoxy]-3,4-dihydro-1H-naphthalin-(2Z)-yliden]-essigsäure |
| "A54" | 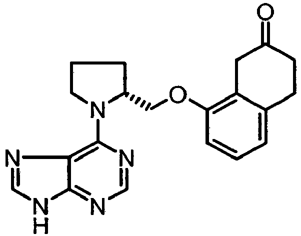 |
| | 8-[(R)-1-(9H-Purin-6-yl)-pyrrolidin-2-ylmethoxy]-3,4-dihydro-1H-naphthalin-2-on |
| "A55" | 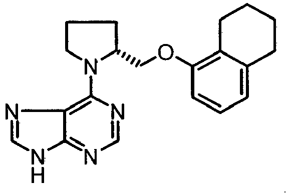 |
| | 6-[(R)-2-(5,6,7,8-Tetrahydro-naphthalin-1-yloxymethyl)-pyrrolidin-1-yl]-9H-purin |
| "A56" | 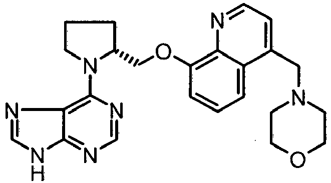 |
| | 4-Morpholin-4-ylmethyl-8-[(R)-1-(9H-purin-6-yl)-pyrrolidin-2-ylmethoxy]-chinolin |
| "A57" | 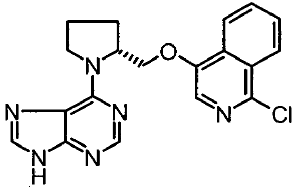 |
| | 1-Chloro-4-[(R)-1-(9H-purin-6-yl)-pyrrolidin-2-ylmethoxy]-isochinolin |
| "A58" | 6-{(R)-2-[5-(2-Methoxy-ethoxy)-naphthalin-1-yloxymethyl]-pyrrolidin-1-yl}-9H-purin |
| "A59" | 2-[(R)-1-(9H-Purin-6-yl)-pyrrolidin-2-ylmethyl]-2H-isochinolin-1-on |
| "A60" | (2-Morpholin-4-yl-ethyl)-{4-[(R)-1-(9H-purin-6-yl)-pyrrolidin-2-ylmethoxy]-naphthalin-1-ylmethyl}-amin |
| "A61" | 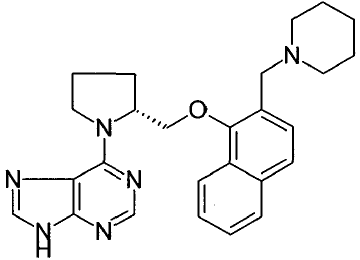 |
| | 6-[(R)-2-(2-Piperidin-1-ylmethyl-naphthalin-1-yloxymethyl)-pyrrolidin-1-yl]-9H-purin |
| "A62" | 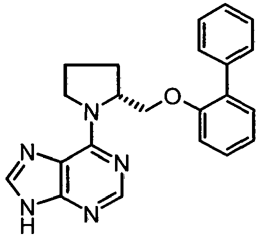 |
| | 6-[(R)-2-(Biphenyl-2-yloxymethyl)-pyrrolidin-1-yl]-9H-purin |
| "A63" | 6-[(R)-2-(Biphenyl-3-yloxymethyl)-pyrrolidin-1-yl]-9H-purin |
| "A64" | 6-[(R)-2-(Biphenyl-4-yloxymethyl)-pyrrolidin-1-yl]-9H-purin |
| "A65" | 3-{5-[(R)-1-(9H-Purin-6-yl)-pyrrolidin-2-ylmethoxy]-pyrimidin-2-yl}-benzoesäure-methylester |
| "A66" | N-(2-Morpholin-4-yl-ethyl)-3-{5-[(R)-1-(9H-purin-6-yl)-pyrrolidin-2-ylmethoxy]-pyrimidin-2-yl}-benzamid |
| "A67" | N-Methyl-2-[1-oxo-5-[(R)-1-(9H-purin-6-yl)-pyrrolidin-2-ylmethoxy]-3,4-dihydro-1H-naphthalin-(2Z)-yliden]-acetamid |
| "A68" | 4-[(R)-1-(9H-urin-6-yl)-pyrrolidin-2-ylmethoxy]-chinolin-2-carbonsäure-(2-hydroxy-propyl)-amid |
| "A69" | 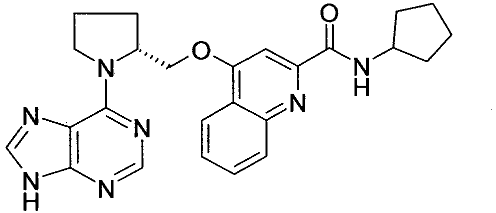 |
| | 4-[(R)-1-(9H-Purin-6-yl)-pyrrolidin-2-ylmethoxy]-chinolin-2-carbonsäure-cyclopentylamid |
| "A70" | 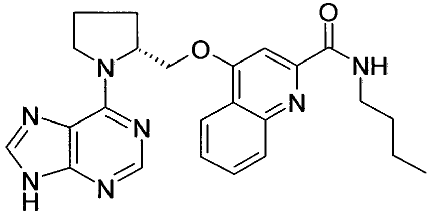 |
| | 4-[(R)-1-(9H-Purin-6-yl)-pyrrolidin-2-ylmethoxy]-chinolin-2-carbonsäure-butylamid |
| "A71" | 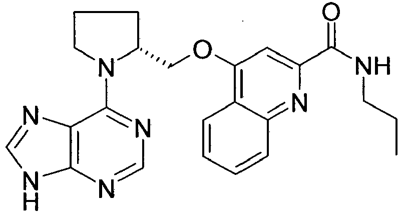 |
| | 4-[(R)-1-(9H-Purin-6-yl)-pyrrolidin-2-ylmethoxy]-chinolin-2-carbonsäure-propylamid |
| "A72" | 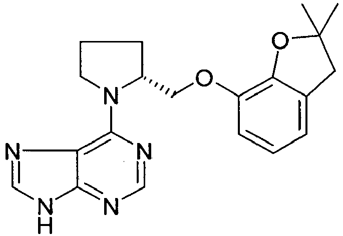 |
| | 6-[(R)-2-(2,2-Dimethyl-2,3-dihydro-benzofuran-7-yloxymethyl)-pyrrolidin-1-yl]-9H-purin |
| "A73" | 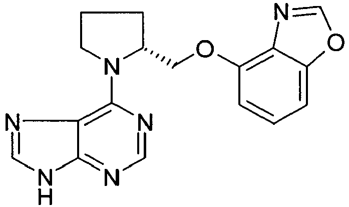 |
| | 6-[(R)-2-(Benzoxazol-4-yloxymethyl)-pyrrolidin-1-yl]-9H-purin |
| "A75" | 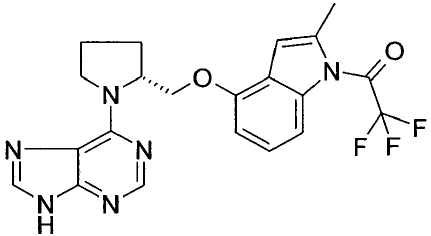 |
| | 2,2,2-Trifluor-1-{2-methyl-4-[(R)-1-(9H-purin-6-yl)-pyrrolidin-2-ylmethoxy]-indol-1-yl}-ethanon |
| "A77" | 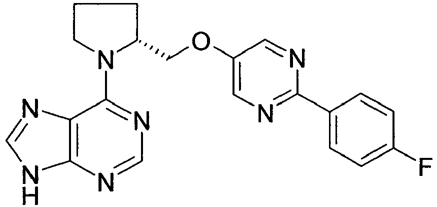 |
| | 6-{(R)-2-[2-(4-Fluor-phenyl)-pyrimidin-5-yloxymethyl]-pyrrolidin-1-yl}-9H-purin |
| "A80" | 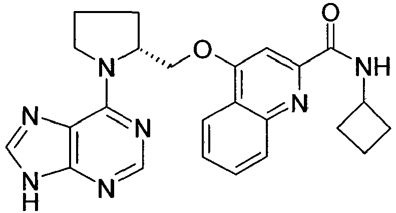 |
| | 4-[(R)-1-(9H-Purin-6-yl)-pyrrolidin-2-ylmethoxy]-chinolin-2-carbonsäure-cyclobutylamid |
| "A81" | 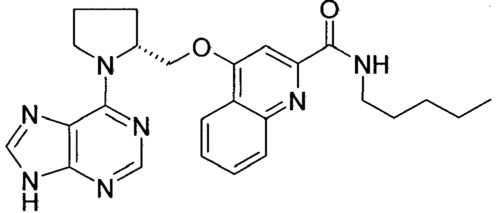 |
| | 4-[(R)-1-(9H-Purin-6-yl)-pyrrolidin-2-ylmethoxy]-chinolin-2-carbonsäure-pentylamid |
| "A82" | 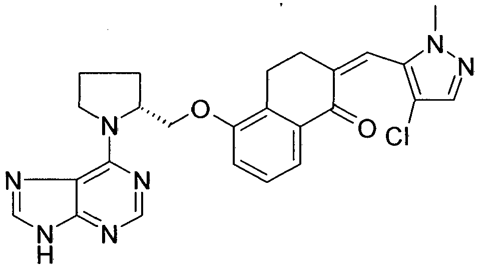 |
| | 2-[1-(4-Chlor-2-methyl-2H-pyrazol-3-yl)-meth-(Z)-yliden]-5-[(R)-1-(9H-purin-6-yl)-pyrrolidin-2-ylmethoxy]-3,4-dihydro-2H-naphthalin-1-on |
| "A83" | 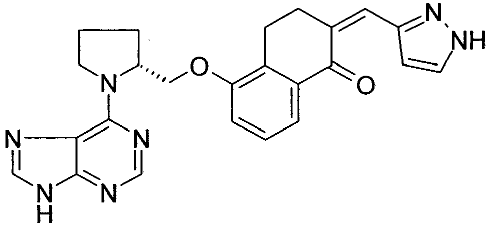 |
| | 5-[(R)-1-(9H-Purin-6-yl)-pyrrolidin-2-ylmethoxy]-2-[1-(1H-pyrazol-3-yl)-meth-(Z)-yliden]-3,4-dihydro-2H-naphthalin-1-on |
| "A84" | 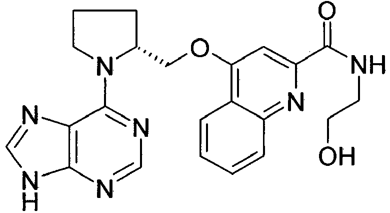 |
| | 4-[(R)-1-(9H-Purin-6-yl)-pyrrolidin-2-ylmethoxy]-chinolin-2-carbonsäure-(2-hydroxy-ethyl)-amid |
| "A85" | 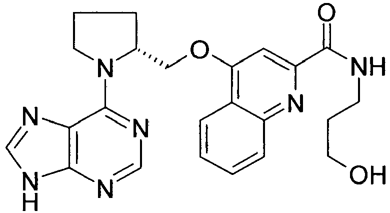 |
| | 4-[(R)-1-(9H-Purin-6-yl)-pyrrolidin-2-ylmethoxy]-chinolin-2-carbonsäure-(3-hydroxy-propyl)-amid |
| "A86" | 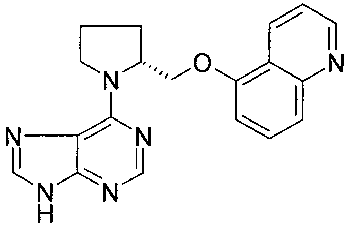 |
| | 5-[(R)-1-(9H-Purin-6-yl)-pyrrolidin-2-ylmethoxy]-chinolin |
| "A87" | 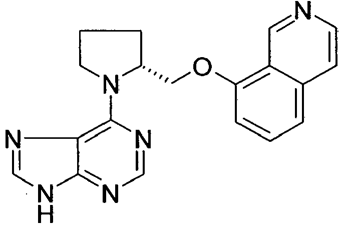 |
| | 8-[(R)-1-(9H-Purin-6-yl)-pyrrolidin-2-ylmethoxy]-isochinolin |
| "A88" | 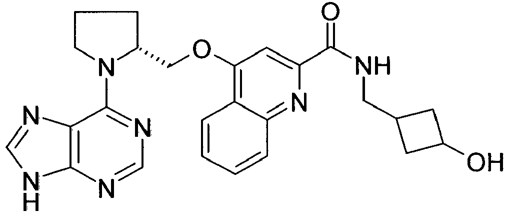 |
| | 4-[(R)-1-(9H-Purin-6-yl)-pyrrolidin-2-ylmethoxy]-chinolin-2-carbonsäure-(3-hydroxy-cyclobutylmethyl)-amid |
| "A89" | 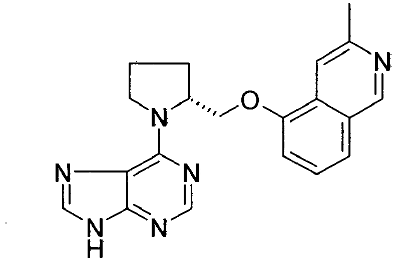 |
| | 3-Methyl-5-[(R)-1-(9H-purin-6-yl)-pyrrolidin-2-ylmethoxy]-isochinolin |
| "A92" | N-(2-Morpholin-4-yl-ethyl)-N-{4-[(R)-1-(9H-purin-6-yl)-pyrrolidin-2-ylmethoxy]-naphthalin-1-ylmethyl}-acetamid |
| "A93" | 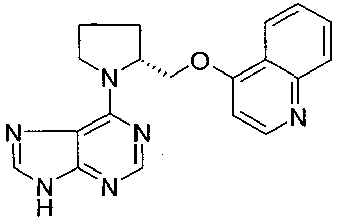 |
| | 4-[(R)-1-(9H-Purin-6-yl)-pyrrolidin-2-ylmethoxy]-chinolin |
| "A94" | 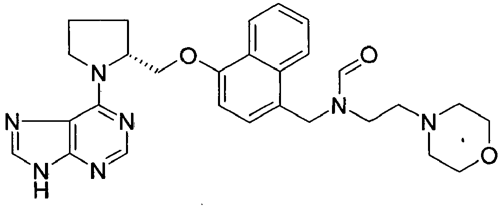 |
| | N-(2-Morpholin-4-yl-ethyl)-N-{4-[(R)-1-(9H-purin-6-yl)-pyrrolidin-2-ylmethoxy]-naphthalin-1-ylmethyl}-formamid |
| "A95" | 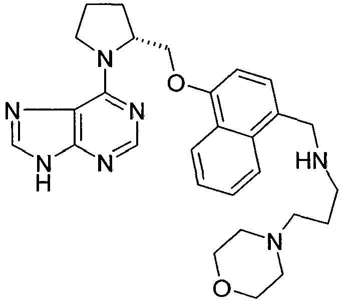 |
| | (3-Morpholin-4-yl-propyl)-{4-[(R)-1-(9H-purin-6-yl)-pyrrolidin-2-ylmethoxy]-naphthalin-1-ylmethyl}-amin |
| "A96" | 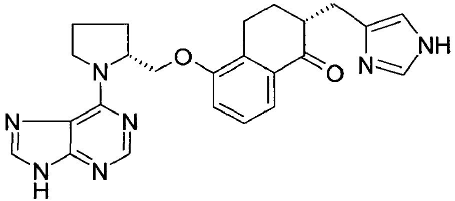 |
| | (R)-2-(1H-Imidazol-4-ylmethyl)-5-[(R)-1-(9H-purin-6-yl)-pyrrolidin-2-ylmethoxy]-3,4-dihydro-2H-naphthalin-1-on |
| "A97" | 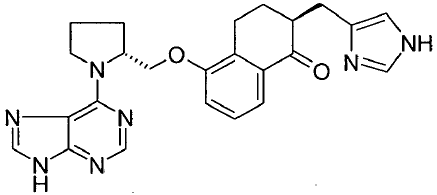 |
| | (S)-2-(1H-Imidazol-4-ylmethyl)-5-[(R)-1-(9H-purin-6-yl)-pyrrolidin-2-ylmethoxy]-3,4-dihydro-2H-naphthalin-1-on |
| "A98" | 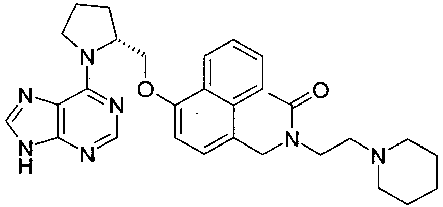 |
| | N-(2-Piperidin-1-yl-ethyl)-N-{4-[(R)-1-(9H-purin-6-yl)-pyrrolidin-2-ylmethoxy]-naphthalin-1-ylmethyl}-acetamid |
| "A99" | 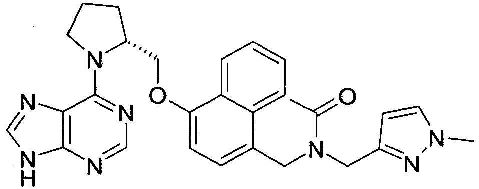 |
| | N-(1-Methyl-1H-pyrazol-3-ylmethyl)-N-{4-[(R)-1-(9H-purin-6-yl)-pyrrolidin-2-ylmethoxy]-naphthalin-1-ylmethyl}-acetamid |
| "A100" | 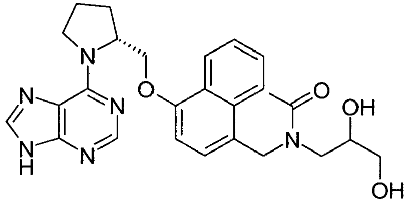 |
| | N-(2,3-Dihydroxy-propyl)-N-{4-[(R)-1-(9H-purin-6-yl)-pyrrolidin-2-ylmethoxy]-naphthalin-1-ylmethyl}-acetamid |
| "A101" | 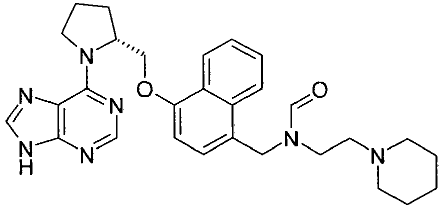 |
| | N-(2-Piperidin-1-yl-ethyl)-N-{4-[(R)-1-(9H-purin-6-yl)-pyrrolidin-2-ylmethoxy]-naphthalin-1-ylmethyl}-formamid |
| "A102" | 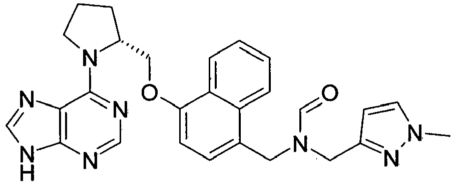 |
| | N-(1-Methyl-1H-pyrazol-3-ylmethyl)-N-{4-[(R)-1-(9H-purin-6-yl)-pyrrolidin-2-ylmethoxy]-naphthalin-1-ylmethyl}-formamid |
| "A103" | 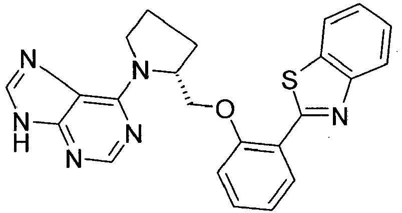 |
| | 6-[(R)-2-(2-Benzothiazol-2-yl-phenoxymethyl)-pyrrolidin-1-yl]-9H-purin |
| "A104" | 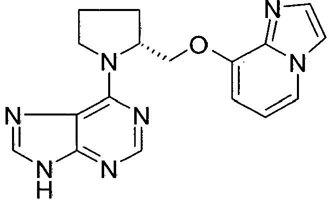 |
| | 6-[(R)-2-(Imidazo[1,2-a]pyridin-8-yloxymethyl)-pyrrolidin-1-yl]-9H-purin |
| "A105" | 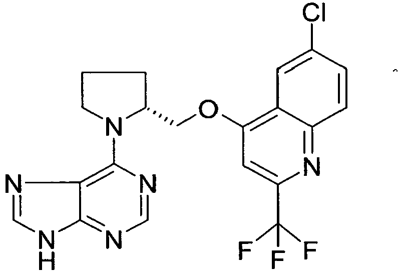 |
| | 6-Chlor-4-[(R)-1-(9H-purin-6-yl)-pyrrolidin-2-ylmethoxy]-2-trifluormethyl-chinolin |
| "A106" | 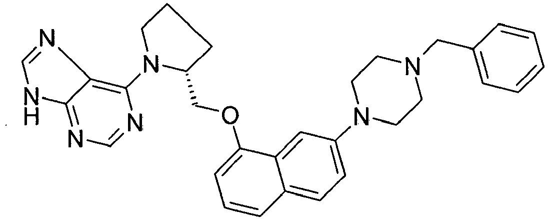 |
| | 6-{(R)-2-[7-(4-Benzyl-piperazin-1-yl)-naphthalin-1-yloxymethyl]-pyrrolidin-1-yl}-9H-purin |
| "A107" | 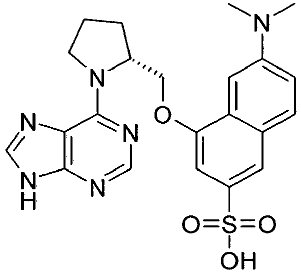 |
| | 6-Dimethylamino-4-[(R)-1-(9H-purin-6-yl)-pyrrolidin-2-ylmethoxy]-naphthalin-2-sulfonsäure |
| "A108" | 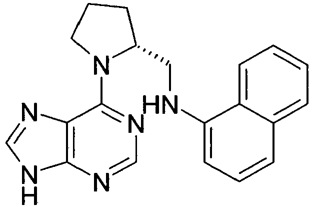 |
| | Naphthalin-1-yl-[(R)-1-(9H-purin-6-yl)-pyrrolidin-2-ylmethyl]-amin |
| "A109" | 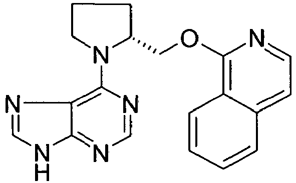 |
| | 1-[(R)-1-(9H-Purin-6-yl)-pyrrolidin-2-ylmethoxy]-isochinolin |
| "A110" | 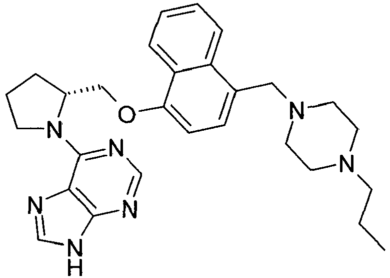 |
| | 6-{(R)-2-[4-(4-Propyl-piperazin-1-ylmethyl)-naphthalin-1-yloxymethyl]-pyrrolidin-1-yl}-9H-purin |
| "A111" | 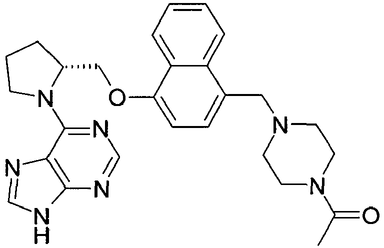 |
| | 1-(4-{4-[(R)-1-(9H-Purin-6-yl)-pyrrolidin-2-ylmethoxy]-naphthalin-1-ylmethyl}-piperazin-1-yl)-ethanon |
| "A112" | 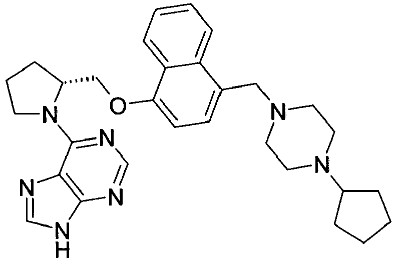 |
| | 6-{(R)-2-[4-(4-Cyclopentyl-piperazin-1-ylmethyl)-naphthalin-1-yloxymethyl]-pyrrolidin-1-yl}-9H-purin |
| "A113" | 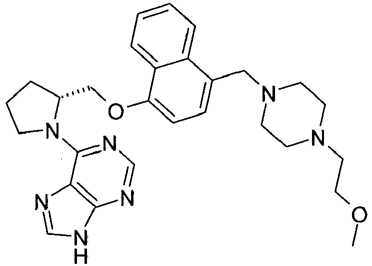 |
| | 6-((R)-2-{4-[4-(2-Methoxy-ethyl)-piperazin-1-ylmethyl]-naphthalin-1-yloxymethyl}-pyrrolidin-1-yl)-9H-purin |
| "A114" | 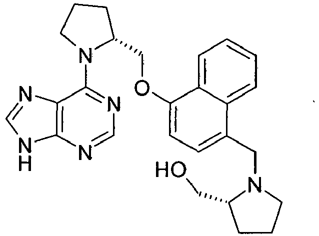 |
| | ((R)-1-{4-[(R)-1-(9H-Purin-6-yl)-pyrrolidin-2-ylmethoxy]-naphthalin-1-ylmethyl}-pyrrolidin-2-yl)-methanol |
| "A115" | 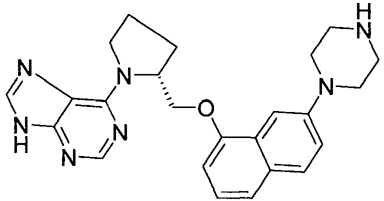 |
| | 6-[(R)-2-(7-Piperazin-1-yl-naphthalin-1-yloxymethyl)-pyrrolidin-1-yl]-9H-purin |
| "A116" | 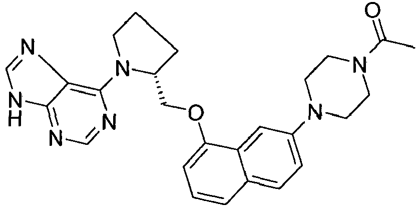 |
| | 1-(4-{8-[(R)-1-(9H-Purin-6-yl)-pyrrolidin-2-ylmethoxy]-naphthalin-2-yl}-piperazin-1-yl)-ethanon |
| "A117" | 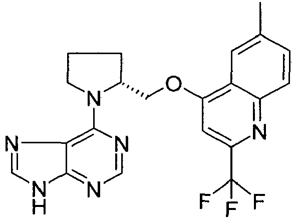 |
| | 6-Methyl-4-[(R)-1-(9H-purin-6-yl)-pyrrolidin-2-ylmethoxy]-2-trifluormethyl-chinolin |
| "A118" | 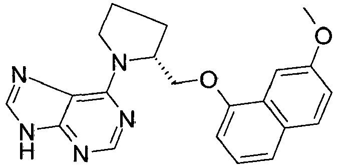 |
| | 6-[(R)-2-(7-Methoxy-naphthalin-1-yloxymethyl)-pyrrolidin-1-yl]-9H-purin |
| "a119" | 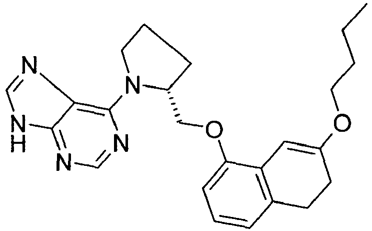 |
| | 6-[(R)-2-(7-Butoxy-5,6-dihydro-naphthalin-1-yloxymethyl)-pyrrolidin-1-yl]-9H-purin |
| "A120" | 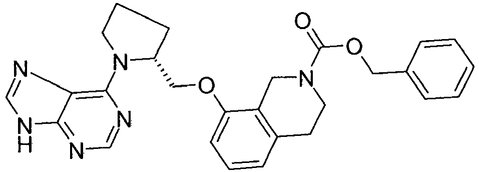 |
| | 8-[(R)-1-(9H-Purin-6-yl)-pyrrolidin-2-ylmethoxy]-3,4-dihydro-1H-isochinolin-2-carbonsäure-benzylester |
| "A121" | 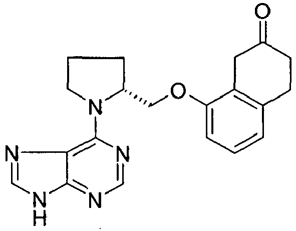 |
| | 8-[(R)-1-(9H-Purin-6-yl)-pyrrolidin-2-ylmethoxy]-3,4-dihydro-1H-naphthalin-2-on |
| "A122" | 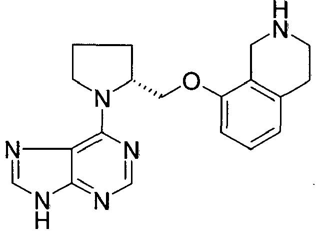 |
| | 8-[(R)-1-(9H-Purin-6-yl)-pyrrolidin-2-ylmethoxy]-1,2,3,4-tetrahydro-isochinolin |
| "A123" | 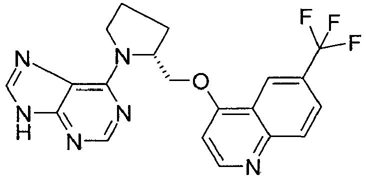 |
| | 4-[(R)-1-(9H-Purin-6-yl)-pyrrolidin-2-ylmethoxy]-6-trifluormethyl-chinolin |
| "A124" | 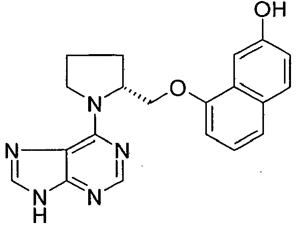 |
| | 8-[(R)-1-(9H-Purin-6-yl)-pyrrolidin-2-ylmethoxy]-naphthalin-2-ol |
| "A125" | 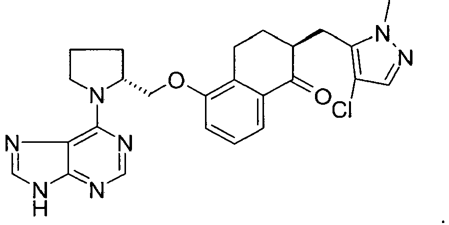 |
| | (S)-2-(4-Chlor-2-methyl-2H-pyrazol-3-ylmethyl)-5-[(R)-1-(9H-purin-6-yl)-pyrrolidin-2-ylmethoxy]-3,4-dihydro-2H-naphthalin-1-on |
| "A126" | 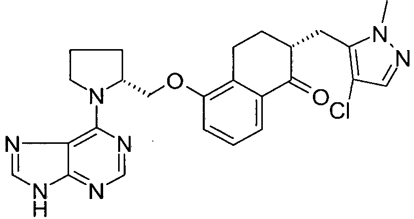 |
| | (R)-2-(4-Chlor-2-methyl-2H-pyrazol-3-ylmethyl)-5-[(R)-1-(9H-purin-6-yl)-pyrrolidin-2-ylmethoxy]-3,4-dihydro-2H-naphthalin-1-on |
| "A127" | 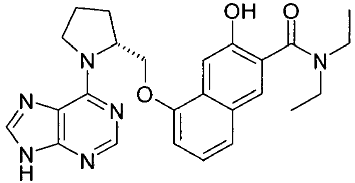 |
| | 3-Hydroxy-5-[(R)-1-(9H-purin-6-yl)-pyrrolidin-2-ylmethoxy]-naphthalin-2-carbonsäure-diethylamid |
| "A128" | 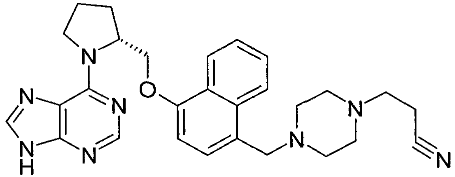 |
| | 3-(4-{4-[(R)-1-(9H-Purin-6-yl)-pyrrolidin-2-ylmethoxy]-naphthalin-1-ylmethyl}-piperazin-1-yl)-propionitril |
| "A129" | 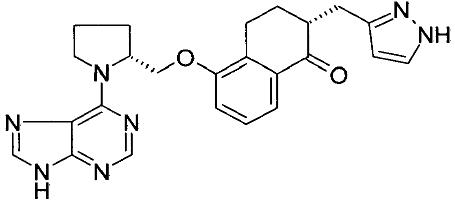 |
| | (R)-5-[(R)-1-(9H-Purin-6-yl)-pyrrolidin-2-ylmethoxy]-2-(1H-pyrazol-3-ylmethyl)-3,4-dihydro-2H-naphthalin-1-on |
| "A130" | 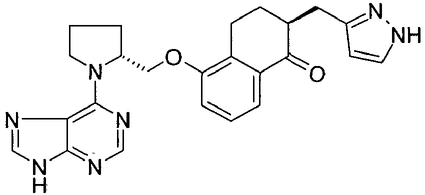 |
| | (S)-5-[(R)-1-(9H-Purin-6-yl)-pyrrolidin-2-ylmethoxy]-2-(1H-pyrazol-3-ylmethyl)-3,4-dihydro-2H-naphthalin-1-on |
| "A131" | 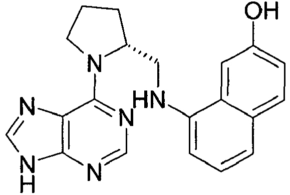 |
| | 8-{[(R)-1-(9H-Purin-6-yl)-pyrrolidin-2-ylmethyl]-amino}-naphthalin-2-ol |
| "A132" | 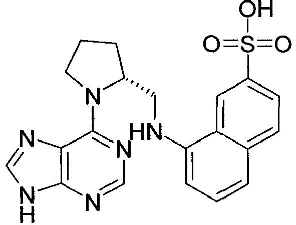 |
| | 8-{[(R)-1-(9H-Purin-6-yl)-pyrrolidin-2-ylmethyl]-amino}-naphthalin-2-sulfonsäure |
| "A133" | 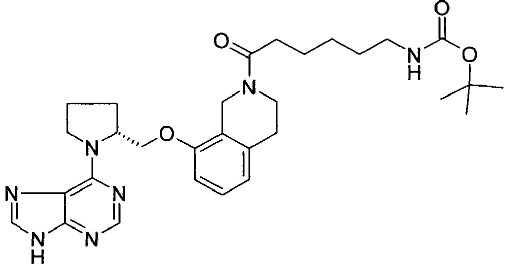 |
| | (6-Oxo-6-{8-[(R)-1-(9H-purin-6-yl)-pyrrolidin-2-ylmethoxy]-3,4-dihydro-1H-isochinolin-2-yl}-hexyl)-carbaminsäure-tert.-butylester |
| "A134" | 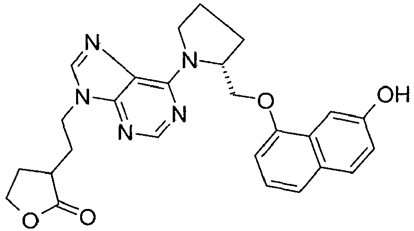 |
| | 3-(2-{6-[(R)-2-(7-Hydroxy-naphthalin-1-yloxymethyl)-pyrrolidin-1-yl]-purin-9-yl}-ethyl)-dihydro-furan-2-on |
| "A135" | 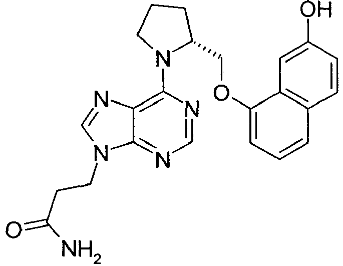 |
| | 3-{6-[(R)-2-(7-Hydroxy-naphthalin-1-yloxymethyl)-pyrrolidin-1-yl]-purin-9-yl}-propionamid |
| "A136" | 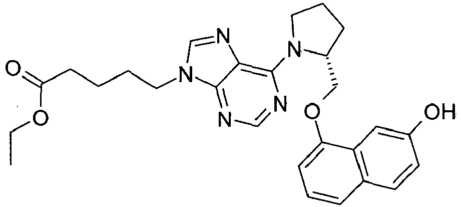 |
| | 5-{6-[(R)-2-(7-Hydroxy-naphthalin-1-yloxymethyl)-pyrrolidin-1-yl]-purin-9-yl}-pentansäure-ethylester |
| "A137" | 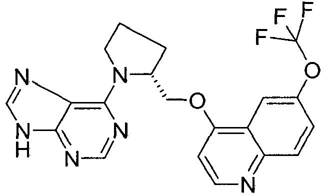 |
| | 4-[(R)-1-(9H-Purin-6-yl)-pyrrolidin-2-ylmethoxy]-6-trifluormethoxy-chinolin |
| "A138" | 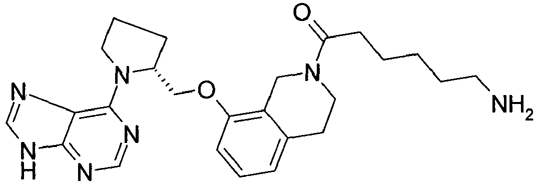 |
| | 6-Amino-1-{8-[(R)-1-(9H-purin-6-yl)-pyrrolidin-2-ylmethoxy]-3,4-dihydro-1H-isochinolin-2-yl}-hexan-1-on |
| "A139" | 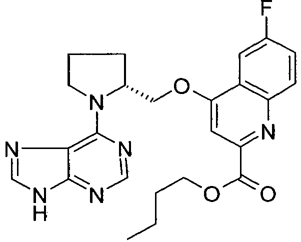 |
| | 6-Fluor-4-[(R)-1-(9H-purin-6-yl)-pyrrolidin-2-ylmethoxy]-chinolin-2-carbonsäure-butylester |
| "A140" | 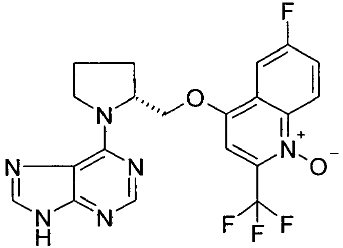 |
| | 6-Fluor-4-[(R)-1-(9H-purin-6-yl)-pyrrolidin-2-ylmethoxy]-2-trifluormethyl-chinolin-1-oxid |
| "A141" | 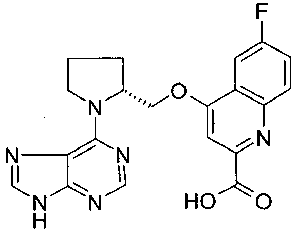 |
| | 6-Fluor-4-[(R)-1-(9H-purin-6-yl)-pyrrolidin-2-ylmethoxy]-chinolin-2-carbonsäure |
| "A142" | 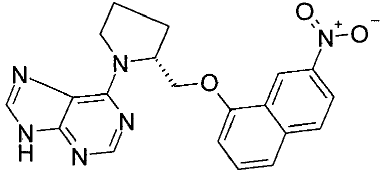 |
| | 6-[(R)-2-(7-Nitro-naphthalin-1-yloxymethyl)-pyrrolidin-1-yl]-9H-purin |
| "A144" | 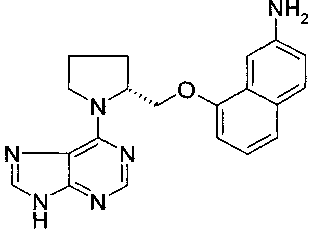 |
| | 8-[(R)-1-(9H-Purin-6-yl)-pyrrolidin-2-ylmethoxy]-naphthalin-2-ylamin |
| "A145" | 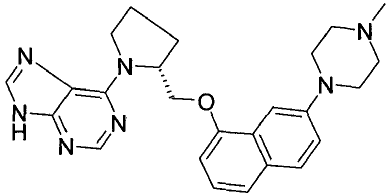 |
| | 6-{(R)-2-[7-(4-Methyl-piperazin-1-yl)-naphthalin-1-yloxymethyl]-pyrrolidin-1-yl}-9H-purin |
| "A146" | 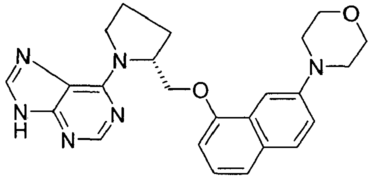 |
| | 6-[(R)-2-(7-Morpholin-4-yl-naphthalin-1-yloxymethyl)-pyrrolidin-1-yl]-9H-purin |
| "A147" | 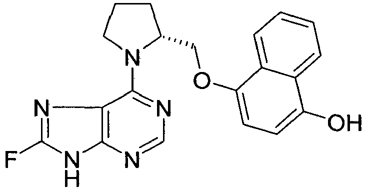 |
| | 4-[(R)-1-(8-Fluor-9H-purin-6-yl)-pyrrolidin-2-ylmethoxy]-naphthalin-1-ol |
| "A148" | 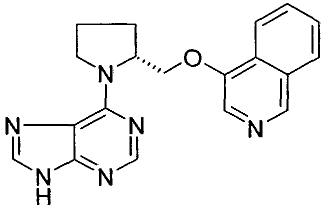 |
| | 4-[(R)-1-(9H-Purin-6-yl)-pyrrolidin-2-ylmethoxy]-isochinolin |
| "A149" | 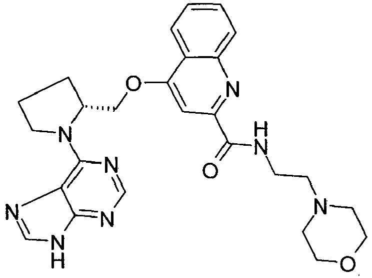 |
| | 4-[(R)-1-(9H-Purin-6-yl)-pyrrolidin-2-ylmethoxy]-chinolin-2-carbonsäure-(2-morpholin-4-yl-ethyl)-amid |
| "A150" | 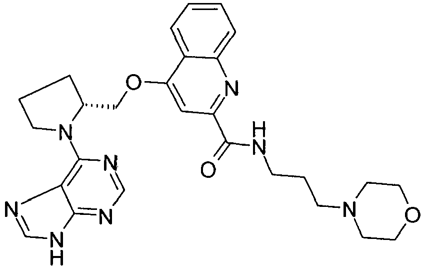 |
| | 4-[(R)-1-(9H-Purin-6-yl)-pyrrolidin-2-ylmethoxy]-chinolin-2-carbonsäure-(3-morpholin-4-yl-propyl)-amid |
| "A151" | 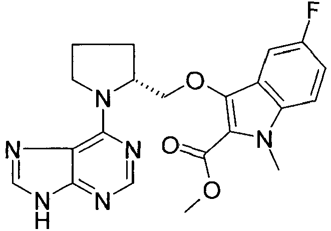 |
| | 5-Fluor-1-methyl-3-[(R)-1-(9H-purin-6-yl)-pyrrolidin-2-ylmethoxy]-1H-indol-2-carbonsäure-methylester |
| "A152" | 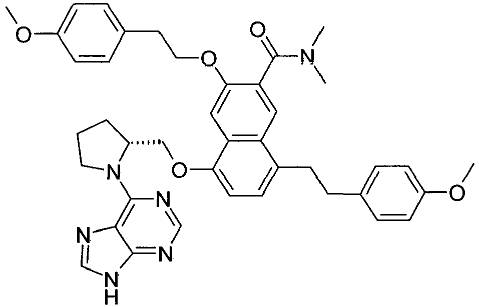 |
| | 3-[2-(4-Methoxy-phenyl)-ethoxy]-8-[2-(4-methoxy-phenyl)-ethyl]-5-[(R)-1-(9H-purin-6-yl)-pyrrolidin-2-ylmethoxy]-naphthalin-2-carbonsäure-dimethylamid |
| "A153" | 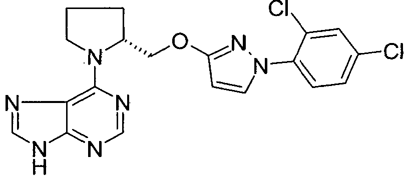 |
| | 6-{(R)-2-[1-(2,4-Dichlor-phenyl)-1H-pyrazol-3-yloxymethyl]-pyrrolidin-1 -yl}-9H-purin |
| "A154" | 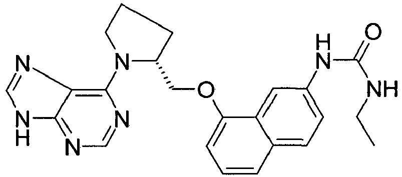 |
| | 1-Ethyl-3-{8-[(R)-1-(9H-purin-6-yl)-pyrrolidin-2-ylmethoxy]-naphthalin-2-yl}-harnstoff |
| "A155" | 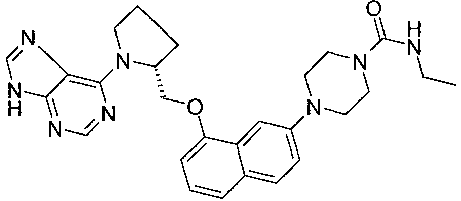 |
| | 4-{8-[(R)-1-(9H-Purin-6-yl)-pyrrolidin-2-ylmethoxy]-naphthalin-2-yl}-piperazin-1-carbonsäure-ethylamid |
| "A156" | 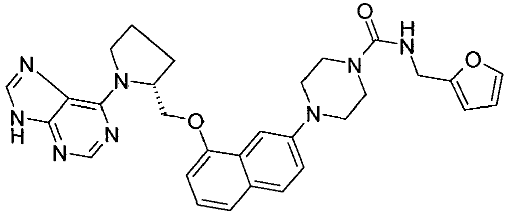 |
| | 4-{8-[(R)-1-(9H-Purin-6-yl)-pyrrolidin-2-ylmethoxy]-naphthalin-2-yl}-piperazin-1-carbonsäure-(furan-2-ylmethyl)-amid |
| "A157" | 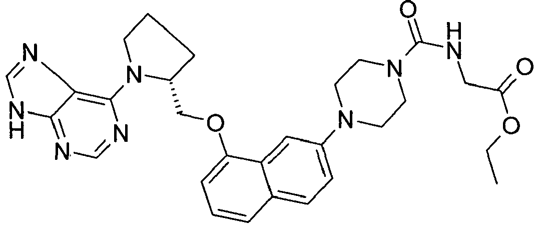 |
| | [(4-{8-[(R)-1-(9H-Purin-6-yl)-pyrrolidin-2-ylmethoxy]-naphthalin-2-yl}-piperazin-1-carbonyl)-amino]-essigsäure-ethylester |
| "A158" | 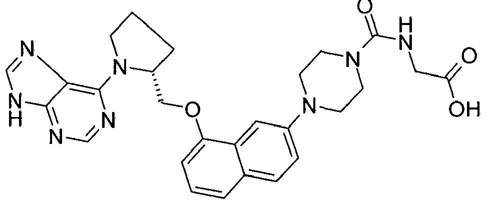 |
| | [(4-{8-[(R)-1-(9H-Purin-6-yl)-pyrrolidin-2-ylmethoxy]-naphthalin-2-yl}-piperazin-1-carbonyl)-amino]-essigsäure |
| "A159" | 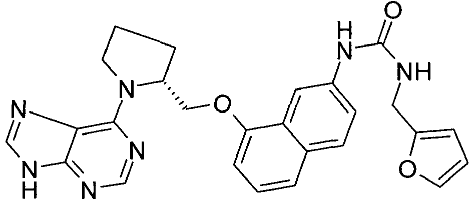 |
| | 1-Furan-2-ylmethyl-3-{8-[(R)-1-(9H-purin-6-yl)-pyrrolidin-2-ylmethoxy]-naphthalin-2-yl}-harnstoff |
| "A160" | 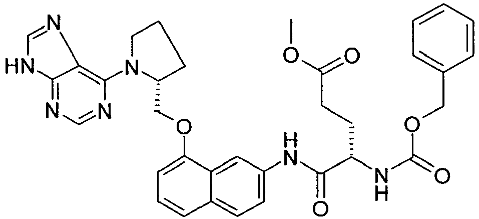 |
| | (S)-4-Benzyloxycarbonylamino-4-{8-[(R)-1-(9H-purin-6-yl)-pyrrolidin-2-ylmethoxy]-naphthalin-2-ylcarbamoyl}-buttersäure-methylester |
| "A161" | 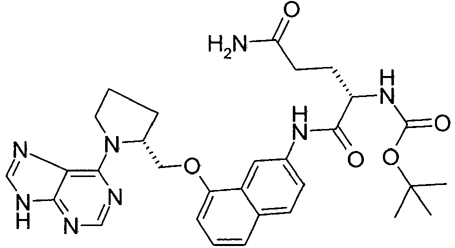 |
| | ((S)-3-Carbamoyl-1-{8-[(R)-1-(9H-purin-6-yl)-pyrrolidin-2-ylmethoxy]-naphthalin-2-ylcarbamoyl}-propyl)-carbaminsäure-tert.-butylester |
| "A162" | 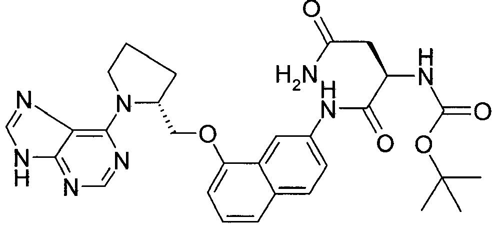 |
| | ((R)-2-Carbamoyl-1-{8-[(R)-1-(9H-purin-6-yl)-pyrrolidin-2-ylmethoxy]-naphthalin-2-ylcarbamoyl}-ethyl)-carbaminsäure-tert.-butylester |
| "A163" | 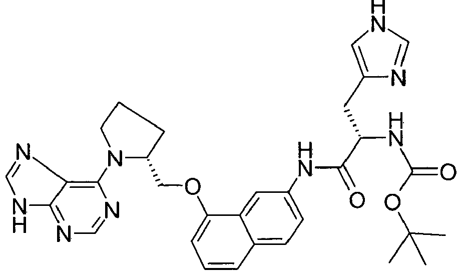 |
| | ((S)-2-(1H-Imidazol-4-yl)-1-{8-[(R)-1-(9H-purin-6-yl)-pyrrolidin-2-ylmethoxy]-naphthalin-2-ylcarbamoyl}-ethyl)-carbaminsäure-tert.-butyl ester |
| "A164" | 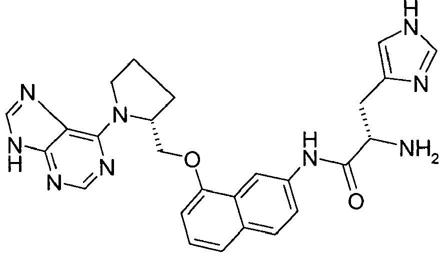 |
| | (S)-2-Amino-3-(1H-imidazol-4-yl)-N-{8-[(R)-1-(9H-purin-6-yl)-pyrrolidin-2-ylmethoxy]-naphthalin-2-yl}-propionamid |
| "A165" | 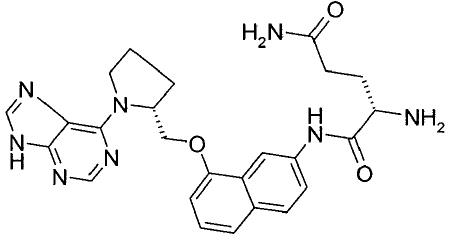 |
| | (S)-2-Amino-pentansäure-5-amid-1-({8-[(R)-1-(9H-purin-6-yl)-pyrrolidin-2-ylmethoxy]-naphthalin-2-yl}-amid) |
| "A166" | 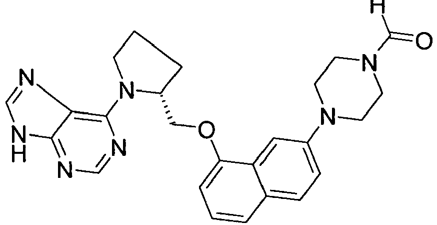 |
| | 4-{8-[(R)-1-(9H-Purin-6-yl)-pyrrolidin-2-ylmethoxy]-naphthalin-2-yl}-piperazin-1-carbaldehyd |
| "A167" | 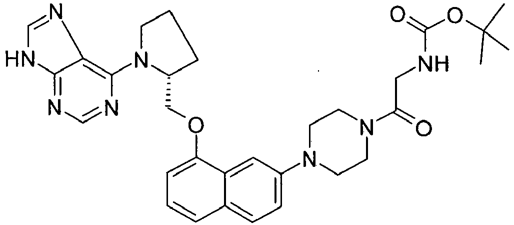 |
| | [2-Oxo-2-(4-{8-[(R)-1-(9H-purin-6-yl)-pyrrolidin-2-ylmethoxy]-naphthalin-2-yl}-piperazin-1-yl)-ethyl]-carbaminsäure-tert.-butylester |
| "A168" | 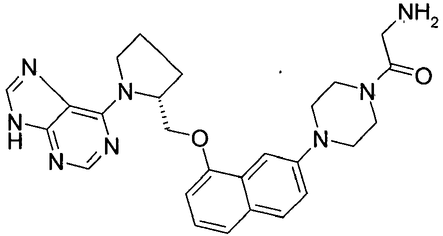 |
| | 2-Amino-1-( 4-{8-[(R)-1-(9H-purin-6-yl)-pyrrolidin-2-ylmethoxy]-naphthalin-2-yl}-piperazin-1-yl)-ethanon |
| "A169" | 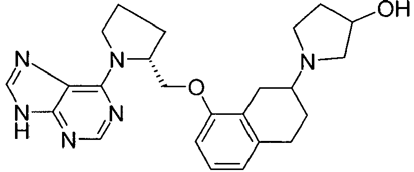 |
| | 1-{8-[(R)-1-(9H-Purin-6-yl)-pyrrolidin-2-ylmethoxy]-1,2,3,4-tetrahydro-naphthalin-2-yl}-pyrrolidin-3-ol |
| "A170" | 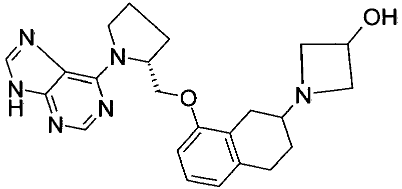 |
| | 1-{8-[(R)-1-(9H-Purin-6-yl)-pyrrolidin-2-ylmethoxy]-1,2,3,4-tetrahydro-naphthalin-2-yl}-azetidin-3-ol |
| "A171 " | 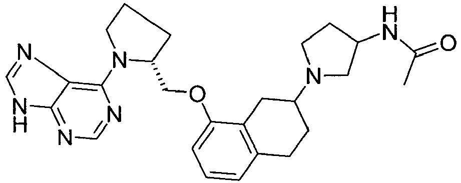 |
| | N-( 1-{8-[(R)-1-(9H-Purin-6-yl)-pyrrolid in-2-ylmethoxy]-1,2,3,4-tetrahydro-naphthalin-2-yl}-pyrrolidin-3-yl)-acetamid |
| "A172" | 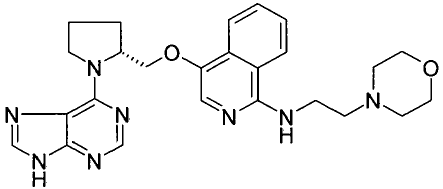 |
| | (2-Morpholin-4-yl-ethyl)-{4-[(R)-1-(9H-purin-6-yl)-pyrrolidin-2-ylmethoxy]-isochinolin-1-yl}-amin |
| "A173" | 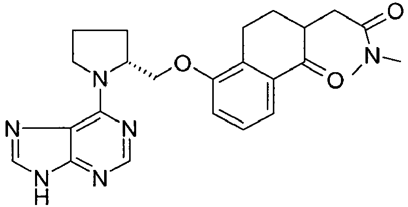 |
| | N, N-Dimethyl-2-{1-oxo-5-[(R)-1-(9H-purin-6-yl)-pyrrolidin-2-ylmethoxy]-1,2,3,4-tetrahydro-naphthalin-2-yl}-acetamid |
| "A174" | 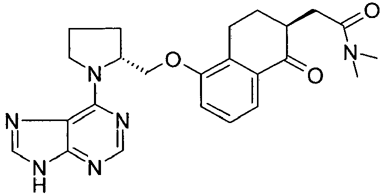 |
| | N, N-Dimethyl-2-{(S)-1-oxo-5-[(R)-1-(9H-purin-6-yl)-pyrrolidin-2-ylmethoxy]-1,2,3,4-tetrahydro-naphthalin-2-yl}-acetamid |
| "A175" | 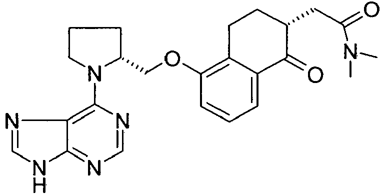 |
| | N,N-Dimethyl-2-{(R)-1-oxo-5-[(R)-1-(9H-purin-6-yl)-pyrrolidin-2-ylmethoxy]-1,2,3,4-tetrahydro-naphthalin-2-yl}-acetamid |
| "A176" | 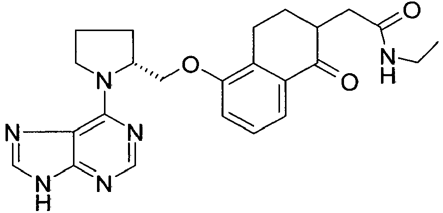 |
| | N-Ethyl-2-{1-oxo-5-[(R)-1-(9H-purin-6-yl)-pyrrolidin-2-ylmethoxy]-1,2,3,4-tetrahydro-naphthalin-2-yl}-acetamid |
| "A177" | 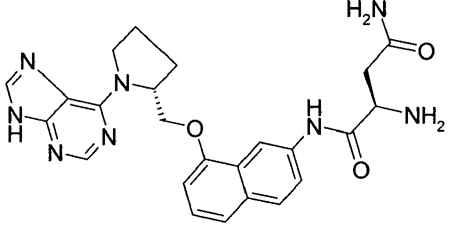 |
| | (R)-2-Amino-N1-{8-[(R)-1-(9H-purin-6-yl)-pyrrolidin-2-ylmethoxy]-naphthalin-2-yl}-succinamid |
| "A178" | 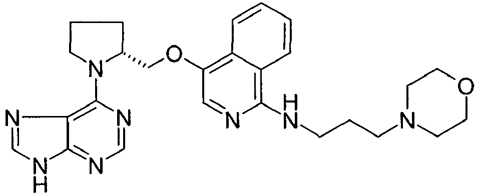 |
| | (3-Morpholin-4-yl-propyl)-{4-[(R)-1-(9H-purin-6-yl)-pyrrolidin-2-ylmethoxy]-isochinolin-1-yl}-amin |
| "A179" | 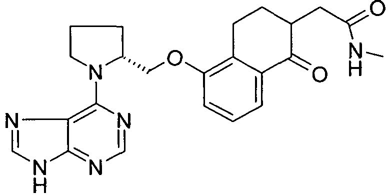 |
| | N-Methyl-2-{1-oxo-5-[(R)-1 -(9H-purin-6-yl)-pyrrolidin-2-ylmethoxy]-1,2,3,4-tetrahydro-naphthalin-2-yl}-acetamid |
| "A180" | 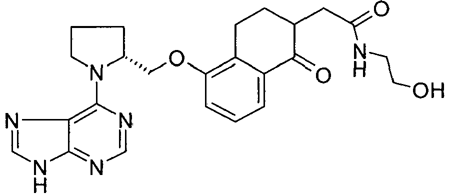 |
| | N-(2-Hydroxy-ethyt)-2-{1-oxo-5-[(R)-1-(9H-purin-6-yl)-pyrrolidin-2-ylmethoxy]-1,2,3,4-tetrahydro-naphthalin-2-yl}-acetamid |
| "A181" | 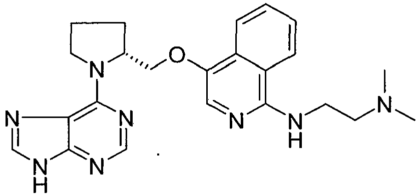 |
| | N,N-Dimethyl-N'-{4-[(R)-1-(9H-purin-6-yl)-pyrrolidin-2-ylmethoxy]-isochinolin-1-yl}-ethan-1,2-diamin |
| "A182" | 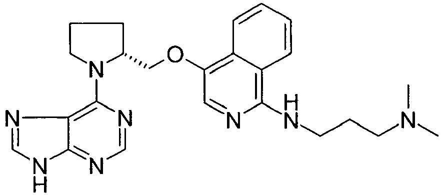 |
| | N,N-Dimethyl-N'-{4-[(R)-1-(9H-purin-6-yl)-pyrrolidin-2-ylmethoxy]-isochinolin-1-yl}-propan-1,3-diamin |
| "A183" | 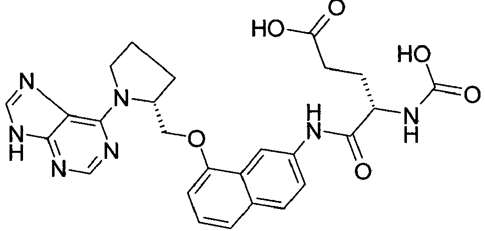 |
| | (S)-4-Carboxyamino-4-{8-[(R)-1-(9H-purin-6-yl)-pyrrolidin-2-ytmethoxy]-naphthalin-2-ylcarbamoyl}-buttersäure |
| "A184" | 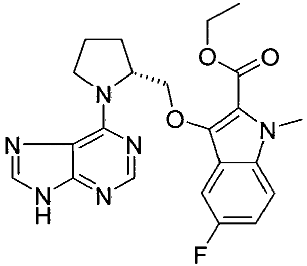 |
| | 5-Fluor-1-methyl-3-[(R)-1-(9H-purin-6-yl)-pyrrolidin-2-ylmethoxy]-1H-indol-2-carbonsäure-ethylester |
| "A185" | 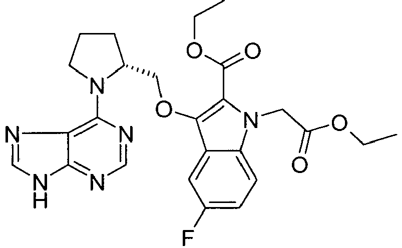 |
| | 1-Ethoxycarbonylmethyl-5-fluor-3-[(R)-1 -(9H-purin-6-yl)-pyrrolidin-2-ylmethoxy]-1H-indol-2-carbonsäure-ethylester |
| "A186" | 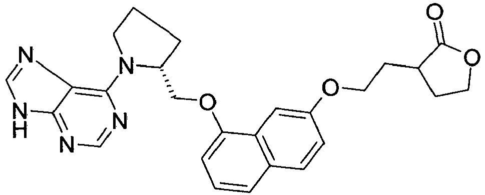 |
| | 3-(2-{8-[(R)-1-(9H-Purin-6-yl)-pyrrolidin-2-ylmethoxy]-naphthalin-2-yloxy}-ethyl)-dihydro-furan-2-on |
| "A187" | 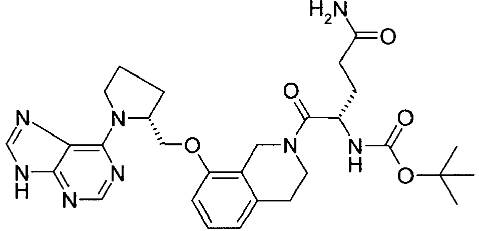 |
| | ((S)-3-Carbamoyl-1-{8-[(R)-1-(9H-purin-6-yl)-pyrrolidin-2-ylmethoxy]-3,4-dihydro-1H-isochinolin-2-carbonyl}-propyl)-carbaminsäure-tert.-butylester |
| "A188" | 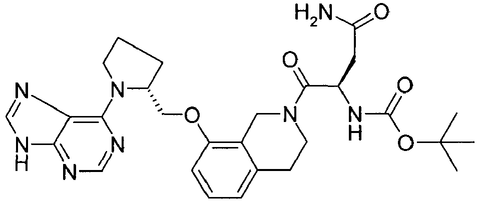 |
| | ((R)-1-Carbamoylmethyl-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butylester |
| "A189" | 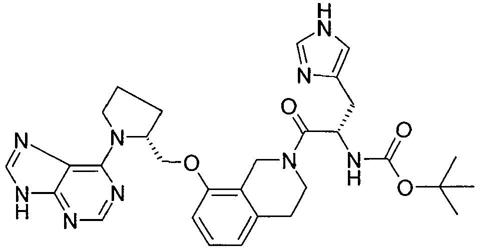 |
| | ((S)-1-(1H-Imidazol-4-ylmethyl)-2-oxo-2-{8-[(R)-1-(9H-purin-6-yl)-pyrrolidin-2-ylmethoxy]-3,4-dihydro-1H-isochinolin-2-yl}-ethyl)-carbaminsäure-tert.-butylester |
| "A190" | 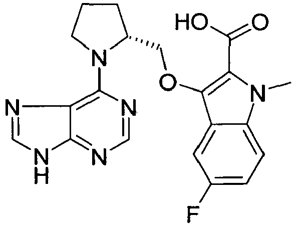 |
| | 5-Fluor-1-methyl-3-[(R)-1-(9H-purin-6-yl)-pyrrolidin-2-ylmethoxy]-1H-indol-2-carbonsäure |
| "A191" | 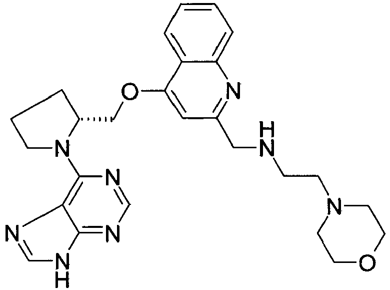 |
| | (2-Morpholin-4-yl-ethyl)-{4-[(R)-1-(9H-purin-6-yl)-pyrrolidin-2-ylmethoxy]-chinolin-2-ylmethyl}-amin |
| "A192" | 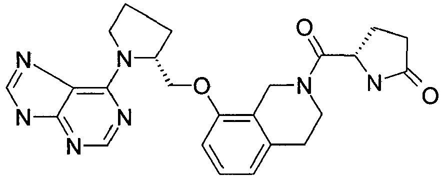 |
| | (S)-5-{8-[(R)-1-(9H-purin-6-yl)-pyrrolidin-2-ylmethoxy]-3,4-dihydro-1H-isochinolin-2-carbonyl}-pyrrolidin-2-on |
| "A193" | 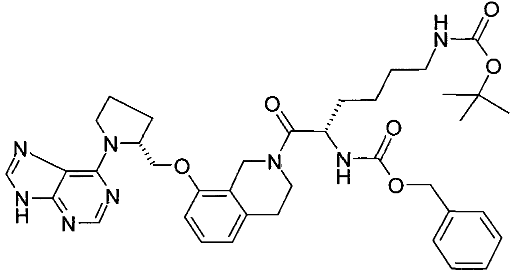 |
| | ((S)-5-Benzyloxycarbonylamino-6-oxo-6-{8-[(R)-1-(9H-purin-6-yl)-pyrrolidin-2-ylmethoxy]-3,4-dihydro-1H-isochinolin-2-yl}-hexyl)-carbaminsäure-tert.-butylester |
| "A194" | 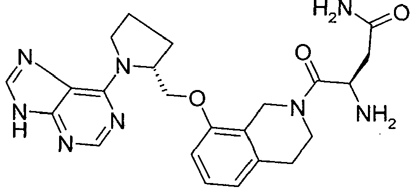 |
| | (R)-3-Amino-4-oxo-4-{8-[(R)-1-(9H-purin-6-yl)-pyrrolidin-2-ylmethoxy]-3,4-dihydro-1H-isochinolin-2-yl}-butyramid |
| "A195" | 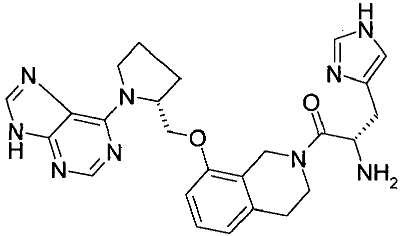 |
| | (S)-2-Amino-3-(1H-imidazol-4-yl)-1-{8-[(R)-1-(9H-purin-6-yl)-pyrrolidin-2-ylmethoxy]-3,4-dihydro-1H-isochinolin-2-yl}-propan-1-on |
| "A196" | 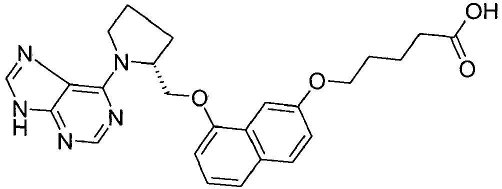 |
| | 5-{8-[(R)-1-(9H-Purin-6-yl)-pyrrolidin-2-ylmethoxy]-naphthalin-2-yloxy}-pentansäure |
| "A197" | 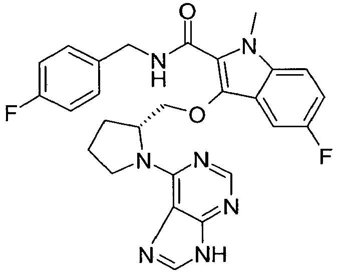 |
| | 5-Fluor-1-methyl-3-[(R)-1-(9H-purin-6-yl)-pyrrolidin-2-ylmethoxy]-1H-indol-2-carbonsäure-4-fluor-benzylamid |
| "A198" | 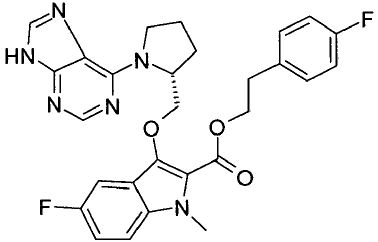 |
| | 5-Fluor-1-methyl-3-[(R)-1-(9H-purin-6-yl)-pyrrolidin-2-ylmethoxy]-1H-indol-2-carbonsäure-2-(4-fluor-phenyl)-ethylester |
| "A199" | 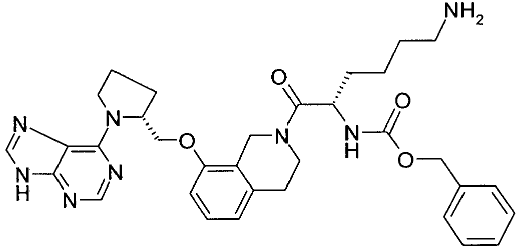 |
| | ((S)-5-Amino-1-{8-[(R)-1-(9H-purin-6-yl)-pyrrolidin-2-ylmethoxy]-3,4-dihydro-1H-isochinolin-2-carbonyl}-pentyl)-carbaminsäure-benzylester |
| "A200" | 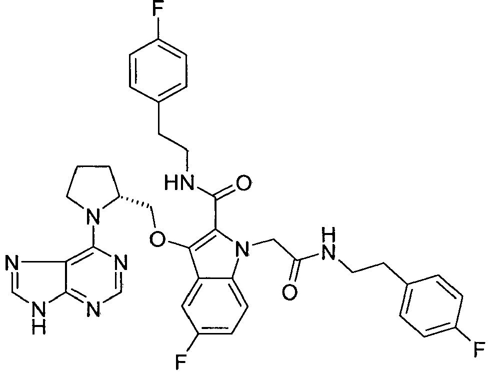 |
| | 5-Fluor-1-{[2-(4-fluor-phenyl)-ethylcarbamoyl]-methyl}-3-[(R)-1-(9H-purin-6-yl)-pyrrolidin-2-ylmethoxy]-1H-indol-2-carbonsäure-[2-(4-fluor-phenyl)-ethyl]-amid |
| "A201" | 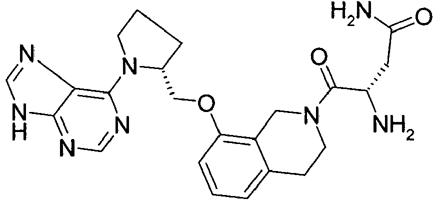 |
| | (S)-3-Amino-4-oxo-4-{8-[(R)-1-(9H-purin-6-yl)-pyrrolidin-2-ylmethoxy]-3,4-dihydro-1H-isochinolin-2-yl}-butyramid |
| "A202" | 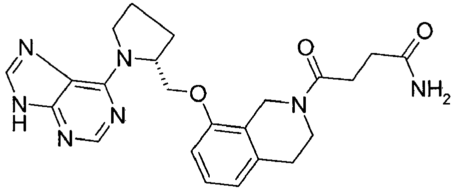 |
| | 4-Oxo-4-{8-[(R)-1-(9H-purin-6-yl)-pyrrolidin-2-ylmethoxy]-3,4-dihydro-1H-isochinolin-2-yl}-butyramid |
| "A203" | 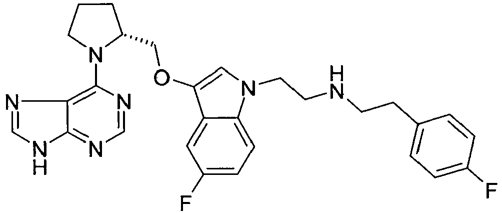 |
| | [2-(4-Fluor-phenyl)-ethyl]-(2-{5-fluor-3-[(R)-1-(9H-purin-6-yl)-pyrrolidin-2-ylmethoxy]-indol-1-yl}-ethyl)-amin |
sowie ihre pharmazeutisch verwendbaren Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

3. Verwendung von Verbindungen nach Anspruch 1
sowie ihrer pharmazeutisch verwendbaren Salze, Tautomeren und Stereoisomeren, einschließlich deren Mischungen in allen Verhältnissen, zur Herstellung eines Arzneimittels zur Behandlung von Tumoren, Tumormetastasen, proliferativer Erkrankungen der Mesangiumzellen, Hämangiom, proliferativer Retinopathie, rheumatoider Arthritis, atherosklerotischer Neovaskularisation, Psoriasis, okularer Neovaskularisation, Osteoporose, Diabetes und Fettleibigkeit, lymphoider Leukämie und Lymphom.

4. Verwendung nach Anspruch 3, wobei die Tumorerkrankung ausgewählt ist aus der Gruppe
des Plattenepithel, der Blase, des Magens, der Nieren, von Kopf und Hals, des Ösophagus, des Gebärmutterhalses, der Schilddrüse, des Darms, der Leber, des Gehirns, der Prostata, des Urogenitaltrakts, des lymphatischen Systems, des Kehlkopfs, der Lunge, der Haut, Monozytenleukämie, Lungenadenokarzinom, kleinzelliges Lungenkarzinom, Bauchspeicheldrüsenkrebs, Glioblastom, Brustkarzinom, akute myelotische Leukämie, chronische myelotische Leukämie, akute lymphatische Leukämie, chronische lymphatische Leukämie, Hodgkin-Lymphom, non-Hodgkin-Lymphom,

5. Verwendung von Verbindungen gemäß Anspruch 1 und/oder ihrer physiologisch unbedenklichen Salze zur Herstellung eines Arzneimittels zur Behandlung von Tumoren, wobei eine therapeutisch wirksame Menge einer Verbindung gemäß Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

6. Verwendung von Verbindungen gemäß Anspruch 1 und/oder ihrer physiologisch unbedenklichen Salze zur Herstellung eines Arzneimittels zur Behandlung von Tumoren wobei eine therapeutisch wirksame Menge einer Verbindung gemäß Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

1. Compounds selected from the group
| Compound No. | Name and/or structure |
|---|---|
| "A1" | 6-[(R)-2-(Naphthalen-1-yloxymethyl)pyrrolidin-1-yl]-9H-purine |
| "A2" | 4-[(R)-1-(9H-Purin-6-yl)pyrrolidin-2-ylmethoxy]naphthalene-1-carbaldehyde |
| "A3" | 6-[(R)-2-(4-Morpholin-4-ylmethylnaphthalen-1-yloxymethyl)-pyrrolidin-1-yl]-9H-purine |
| "A4" | 6-[(R)-2-(4-Butoxymethylnaphthalen-1-yloxymethyl)pyrrolidin-1-yl]-9H-purine |
| "A6" | Morpholin-4-yl(4-{2-[-1-(9H-purin-6-yl)pyrrolidin-2-yl]-ethyl}naphthalen-1-yl)methanone |
| "A8" | 6-[(R)-4,4-Difluoro-2-(naphthalen-1-yloxymethyl)pyrrolidin-1-yl]-9H-purine |
| "A12" | 6-[(R)-2-((E)-2-Naphthalen-1-ylvinyl)pyrrolidin-1-yl]-9H-purine |
| "A14" | N-Cyclopropyl-4-[(R)-1-(9H-purin-6-yl)pyrrolidin-2-yl-methoxy]quinoline-2-carboxamide |
| "A15" | 2-[1-(1H-Imidazol-4-yl)meth-(Z)-ylidene]-5-[(R)-1-(9H-purin-6-yl)pyrrolidin-2-ylmethoxy]-3,4-dihydro-2H-naphthalen-1-one |
| "A15.1" | 2-(1H-Imidazol-4-ylmethyl)-5-[(R)-1-(9H-purin-6-yl)pyrrolidin-2-ylmethoxy]-3,4-dihydro-2H-naphthalen-1-one |
| "A18" | 8-Bromo-6-[(R)-2-(naphthalen-1-yloxymethyl)pyrrolidin-1-yl]-9H-purine |
| "A19" | 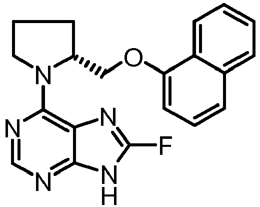 |
| "A20" | 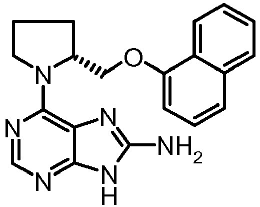 |
| "A23" | 4-[(R)-2-(Naphthalen-1-yloxymethyl)pyrrolidin-1-yl]pyrido[2,3-d]pyrimidine |
| "A25" | 6-[(R)-2-(Naphthalen-1-yloxymethyl)pyrrolidin-1-yl]purin-9-yl-amine |
| "A26" | 6-[(2R,4R)-4-Fluoro-2-(naphthalen-1-yloxymethyl)pyrrolidin-1-yl]-9H-purine |
| "A28" | 6-[(2R,4S)-4-Fluoro-2-(naphthalen-1-yloxymethyl)pyrrolidin-1-yl]-9H-purine |
| "A30" | (3R,5R)-5-(Naphthalen-1-yloxymethyl)-1-(9H-purin-6-yl)-pyrrol idin-3-ol |
| "A31" | 6-[(R)-2,2-Difluoro-5-(naphthalen-1-yloxymethyl)pyrrolidin-1-yl]-9H-purine |
| "A33" | N-[6-(3-{2-[1-(9H-Purin-6-yl)pyrrolidin-2-ylmethoxy]-5-trifluoromethylphenyl}ureidomethyl)-1H-benzimidazol-2-yl]-acetamide |
| "A34" | N-Methyl-4-[4-(3-{2-[1-(9H-purin-6-yl)pyrrolidin-2-ylmethoxy]-5-trifluoromethylphenyl}ureidomethyl)phenoxy]pyridine-2-carboxamide |
| "A35" | N-Methyl-4-[4-(3-{2-[1-(9H-purin-6-yl)pyrrolidin-2-ylmethoxy]-5-trifluoromethylphenyl}ureido)phenoxy]pyridine-2-carboxamide |
| "A36" | N-Methyl-4-[3-(3-{2-[1-(9H-purin-6-yl)pyrrolidin-2-ylmethoxy]-5-trifluoromethylphenyl}ureido)phenoxy]pyridine-2-carboxamide |
| "A37" | 6-[(R)-2-(Dibenzofuran-3-yloxymethyl)pyrrolidin-1-yl]-9H-purine |
| "A38" | 4-[(R)-1-(9H-Purin-6-yl)pyrrolidin-2-ylmethoxy]-9H-carbazole |
| "A39" | Ethyl 1-butyl-2-methyl-5-[(R)-1-(9H-purin-6-yl)pyrrolidin-2-yl-methoxy]-1H-indole-3-carboxylate |
| "A40" | 6-[(R)-2-(1H-lndol-4-yloxymethyl)pyrrolidin-1-yl]-9H-purine |
| "A41" | 1-{7-[(R)-1-(9H-Purin-6-yl)pyrrolidin-2-ylmethoxy]benzofuran-2-yl}ethanone |
| "A42" | 5-Piperidin-1-ylmethyl-8-[(R)-1-(9H-purin-6-yl)pyrrolidin-2-yl-methoxy]quinoline |
| "A43" | 8-[(R)-1-(9H-Purin-6-yl)pyrrolidin-2-ylmethoxy]quinoline |
| "A44" | 5-[(R)-1-(9H-Purin-6-yl)pyrrol id in-2-yl methoxy] isoqu inol ine |
| "A45" | 7-Benzyloxy-6-methoxy-4-[(R)-1-(9H-purin-6-yl)pyrrolidin-2-ylmethoxy]quinazoline |
| "A46" | 4-[(R)-1-(9H-Purin-6-yl)pyrrolidin-2-ylmethoxy]quinazoline |
| "A47" | 2-{2-[2-({4-[(R)-1-(9H-Purin-6-yl)pyrrolidin-2-yl-methoxy]naphthalen-1-ylmethyl}amino)ethoxy]ethoxy}ethyl-amine |
| "A48" | N-Methyl-4-[(R)-1-(9H-purin-6-yl)pyrrolidin-2-yl-methoxy]quinoline-2-carboxamide |
| "A49" | 2-Methyl-8-[(R)-1-(9H-purin-6-yl)pyrrolidin-2-yl-methoxy]quinoline |
| "A50" | N-Ethyl-4-[(R)-1-(9H-purin-6-yl)pyrrolidin-2-yl-methoxy]quinoline-2-carboxamide |
| "A51 " | 5-[(R)-1-(9H-Purin-6-yl)pyrrolidin-2-ylmethoxy]-3,4-dihydro-2H-naphthalen-1-one |
| "A52" | 2-Hydroxymethylene-5-[(R)-1-(9H-purin-6-yl)pyrrolidin-2-yl-methoxy]-3,4-dihydro-2H-naphthalen-1-one |
| "A53" | [1-Oxo-5-[(R)-1-(9H-purin-6-yl)pyrrolidin-2-ylmethoxy]-3,4-dihydro-1H-naphthalen-(2Z)-ylidene]acetic acid |
| "A54" | 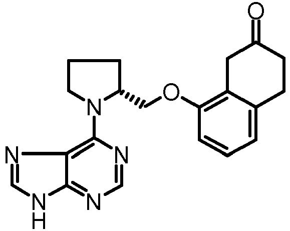 |
| | 8-[(R)-1-(9H-Purin-6-yl)pyrrolidin-2-ylmethoxy]-3,4-dihydro-1H-naphthalen-2-one |
| "A55" | 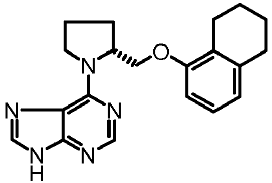 |
| | 6-[(R)-2-(5,6,7,8-Tetrahydronaphthalen-1-yloxymethyl)-pyrrolidin-1-yl]-9H-purine |
| "A56" | 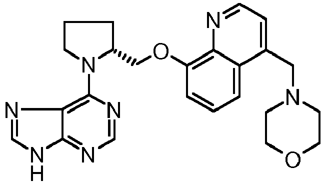 |
| | 4-Morpholin-4-ylmethyl-8-[(R)-1-(9H-purin-6-yl)pyrrolidin-2-ylmethoxy]quinoline |
| "A57" | 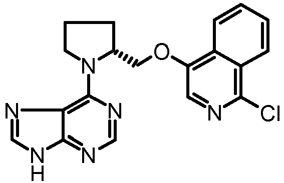 |
| | 1-Chloro-4-[(R)-1-(9H-purin-6-yl)pyrrolidin-2-yl-methoxy]isoquinoline |
| "A58" | 6-{(R)-2-[5-(2-Methoxyethoxy)naphthalen-1-yloxymethyl]-pyrrolidin-1-yl}-9H-purine |
| "A59" | 2-[(R)-1-(9H-Purin-6-yl)pyrrolidin-2-ylmethyl]-2H-isoquinolin-1-one |
| "A60" | (2-Morpholin-4-ylethyl)-{4-[(R)-1-(9H-purin-6-yl)pyrrolid in-2-yl-methoxy]naphthalen-1-ylmethyl}amine |
| "A61 " | 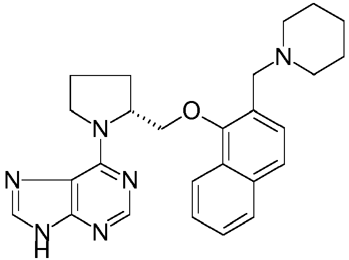 |
| | 6-[(R)-2-(2-Piperidin-1-ylmethylnaphthalen-1-yloxymethyl)-pyrrolidin-1-yl]-9H-purine |
| "A62" | 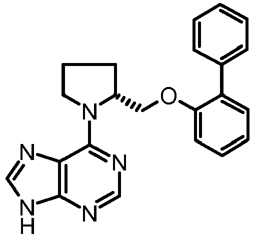 |
| | 6-[(R)-2-(Biphenyl-2-yloxymethyl)pyrrolidin-1-yl]-9H-purine |
| "A63" | 6-[(R)-2-(Biphenyl-3-yloxymethyl)pyrrolidin-1-yl]-9H-purine |
| "A64" | 6-[(R)-2-(Biphenyl-4-yloxymethyl)pyrrolidin-1-yl]-9H-purine |
| "A65" | Methyl 3-{5-[(R)-1-(9H-purin-6-yl)pyrrolidin-2-yl-methoxy]pyrimidin-2-yl}benzoate |
| "A66" | N-(2-Morpholin-4-ylethyl)-3-{5-[(R)-1-(9H-purin-6-yl)pyrrolidin-2-ylmethoxy]pyrimidin-2-yl}benzamide |
| "A67" | N-Methyl-2-[1-oxo-5-[(R)-1 -(9H-purin-6-yl)pyrrolidin-2-yl-methoxy]-3,4-dihydro-1H-naphthalen-(2Z)-ylidene]acetamide |
| "A68" | N-(2-Hydroxypropyl)-4-[(R)-1-(9H-purin-6-yl)pyrrolidin-2-yl-methoxy]quinoline-2-carboxamide |
| "A69" | 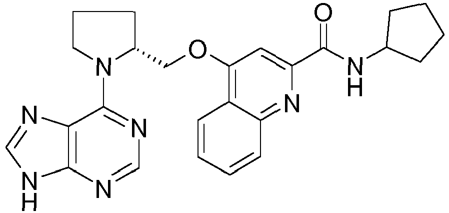 |
| | N-Cyclopentyl-4-[(R)-1-(9H-purin-6-yl )pyrrolidin-2-yl-methoxy]quinoline-2-carboxamide |
| "A70" | 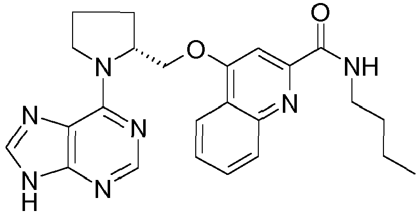 |
| | N-Butyl-4-[(R)-1-(9H-purin-6-yl)pyrrolidin-2-yl-methoxy]quinoline-2-carboxamide |
| "A71 " | 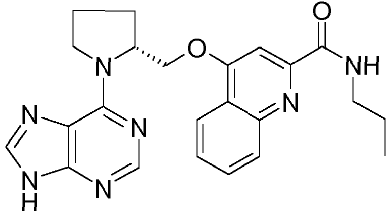 |
| | N-Propyl-4-[(R)-1-(9H-purin-6-yl)pyrrolidin-2-yl-methoxy]quinoline-2-carboxamide |
| "A72" | 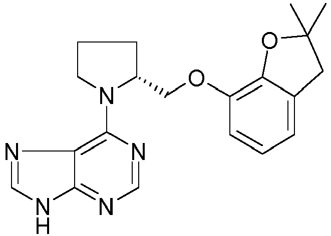 |
| | 6-[(R)-2-(2,2-Dimethyl-2,3-dihydrobenzofuran-7-yl-oxymethyl)pyrrolidin-1-yl]-9H-purine |
| "A73" | 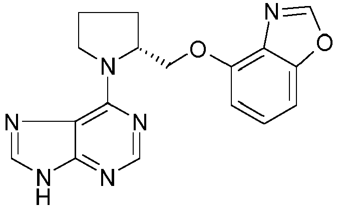 |
| | 6-[(R)-2-(Benzoxazol-4-yloxymethyl)pyrrolidin-1-yl]-9H-purine |
| "A75" | 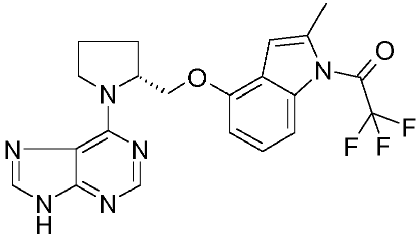 |
| | 2,2,2-Trifluoro-1-{2-methyl-4-[(R)-1-(9H-purin-6-yl)pyrrolidin-2-ylmethoxy]indol-1-yl}ethanone |
| "A77" | 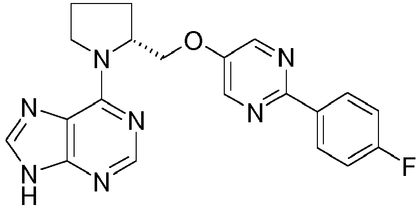 |
| | 6-{(R)-2-[2-(4-Fluorophenyl)pyrimidin-5-yloxymethyl]-pyrrolidin-1-yl}-9H-purine |
| "A80" | 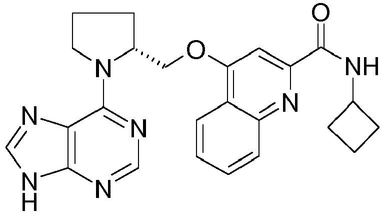 |
| | N-Cyclobutyl-4-[(R)-1-(9H-purin-6-yl )pyrrolidin-2-yl-methoxy]quinoline-2-carboxamide |
| "A81" | 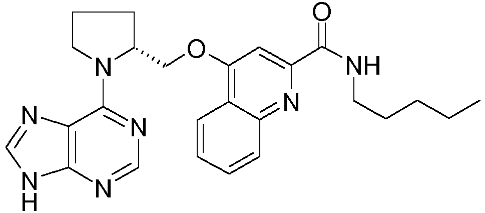 |
| | N-Pentyl-4-[(R)-1-(9H-purin-6-yl)pyrrolidin-2-yl-methoxy]qu inoline-2-carboxamide |
| "A82" | 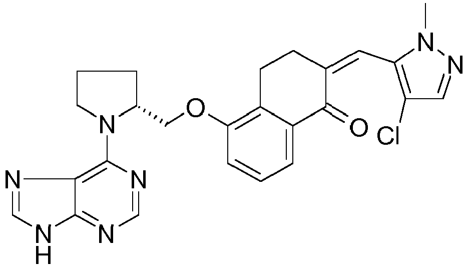 |
| | 2-[1-(4-Chloro-2-methyl-2H-pyrazol-3-yl)meth-(Z)-ylidene]-5-[(R)-1-(9H-purin-6-yl)pyrrolidin-2-ylmethoxy]-3,4-dihydro-2H-naphthalen-1-one |
| "A83" | 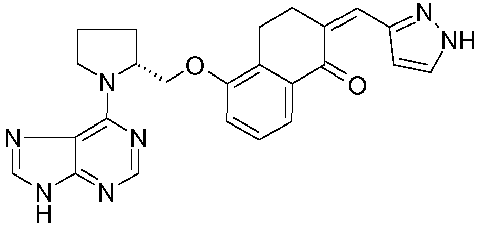 |
| | 5-[(R)-1-(9H-Purin-6-yl)pyrrolidin-2-ylmethoxy]-2-[1 -(1H-pyrazol-3-yl)meth-(Z)-ylidene]-3,4-dihydro-2H-naphthalen-1-one |
| "A84" | 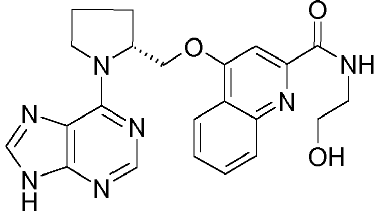 |
| | N-(2-Hydroxyethyl)-4-[(R)-1-(9H-purin-6-yl)pyrrolidin-2-yl-methoxy]quinoline-2-carboxamide |
| "A85" | 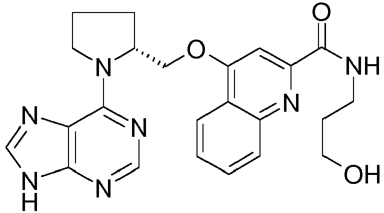 |
| | N-(3-Hydroxypropyl)-4-[(R)-1-(9H-purin-6-yl)pyrrolidin-2-yl-methoxy]quinoline-2-carboxamide |
| "A86" | 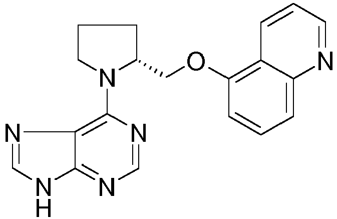 |
| | 5-[(R)-1-(9H-Purin-6-yl)pyrrolidin-2-ylmethoxy]quinoline |
| "A87" | 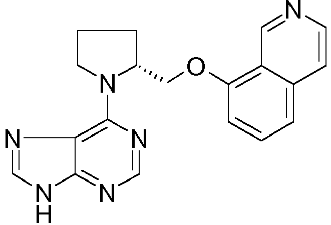 |
| | 8-[(R)-1-(9H-Purin-6-yl)pyrrolidin-2-ylmethoxy]isoquinoline |
| "A88" | 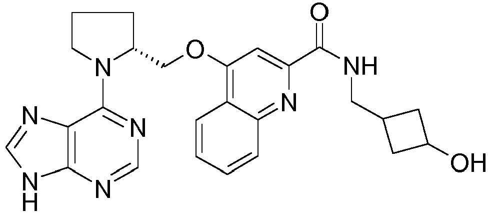 |
| | N-(3-Hydroxycyclobutylmethyl)-4-[(R)-1-(9H-purin-6-yl)-pyrrolidin-2-ylmethoxy]quinoline-2-carboxamide |
| "A89" | 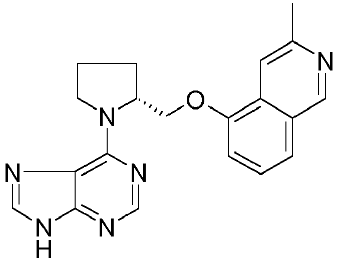 |
| | 3-Methyl-5-[(R)-1-(9H-purin-6-yl)pyrrolidin-2-yl-methoxy]isoquinoline |
| "A92" | N-(2-Morpholin-4-ylethyl)-N-{4-[(R)-1-(9H-purin-6-yl)-pyrrolidin-2-ylmethoxy]naphthalen-1-ylmethyl}acetamide |
| "A93" | 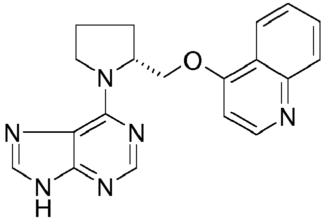 |
| | 4-[(R)-1-(9H-Purin-6-yl)pyrrolidin-2-ylmethoxy]quinoline |
| "A94" | 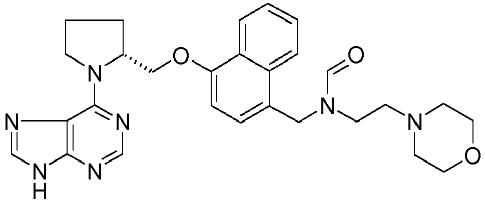 |
| | N-(2-Morpholin-4-ylethyl)-N-{4-[(R)-1-(9H-purin-6-yl)-pyrrolidin-2-ylmethoxy]naphthalen-1-ylmethyl}formamide |
| "A95" | 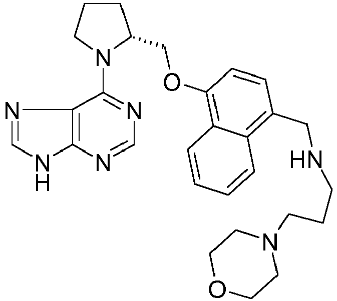 |
| | (3-Morpholin-4-ylpropyl)-{4-[(R)-1-(9H-purin-6-yl)pyrrolidin-2-ylmethoxy]naphthalen-1-ylmethyl}amine |
| "A96" | 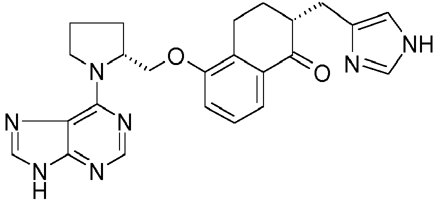 |
| | (R)-2-(1H-Imidazol-4-ylmethyl)-5-[(R)-1-(9H-purin-6-yl)-pyrrolidin-2-ylmethoxy]-3,4-dihydro-2H-naphthalen-1-one |
| "A97" | 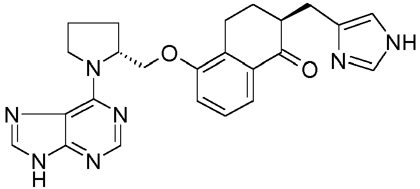 |
| | (S)-2-(1H-Imidazol-4-ylmethyl)-5-[(R)-1-(9H-purin-6-yl)-pyrrolidin-2-ylmethoxy]-3,4-dihydro-2H-naphthalen-1-one |
| "A98" | 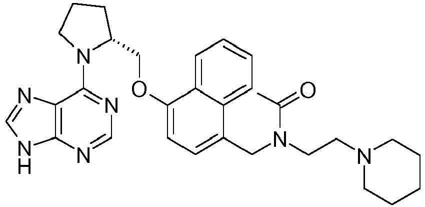 |
| | N-(2-Piperidin-1-ylethyl)-N-{4-[(R)-1-(9H-purin-6-yl)-pyrrolidin-2-ylmethoxy]naphthalen-1-ylmethyl}acetamide |
| "A99" | 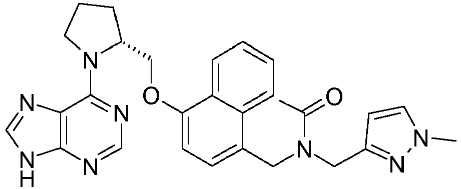 |
| | N-(1-Methyl-1H-pyrazol-3-ylmethyl)-N-{4-[(R)-1-(9H-purin-6-yl)pyrrolidin-2-ylmethoxy]naphthalen-1-ylmethyl}acetamide |
| "A100" | 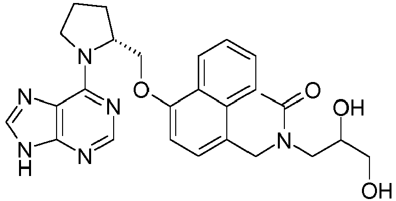 |
| | N-(2,3-Dihydroxypropyl)-N-{4-[(R)-1-(9H-purin-6-yl)-pyrrolidin-2-ylmethoxy]naphthalen-1-ylmethyl}acetamide |
| "A101" | 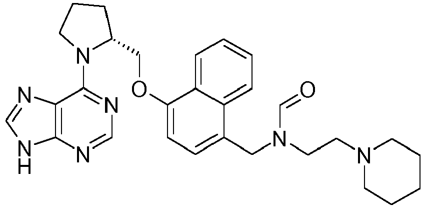 |
| | N-(2-Piperidin-1-ylethyl)-N-{4-[(R)-1-(9H-purin-6-yl)-pyrrolidin-2-ylmethoxy]naphthalen-1-ylmethyl}formamide |
| "A102" | 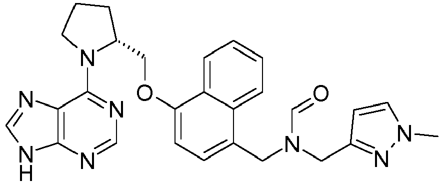 |
| | N-(1-Methyl-1H-pyrazol-3-ylmethyl)-N-{4-[(R)-1-(9H-purin-6-yl)pyrrolidin-2-ylmethoxy]naphthalen-1-ylmethyl}formamide |
| "A103" | 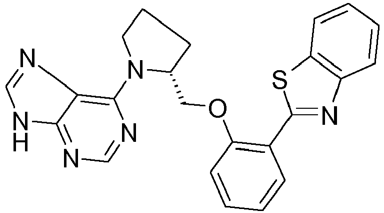 |
| | 6-[(R)-2-(2-Benzothiazol-2-ylo phenoxymethyl )pyrrolidin-1-yl]-9H-purine |
| "A104" | 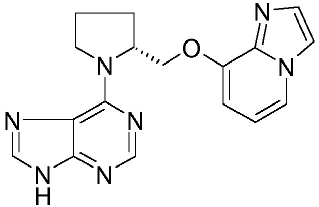 |
| | 6-[(R)-2-(Imidazo[1,2-a]pyridin-8-yloxymethyl)pyrrolidin-1-yl]-9H-purine |
| "A105" | 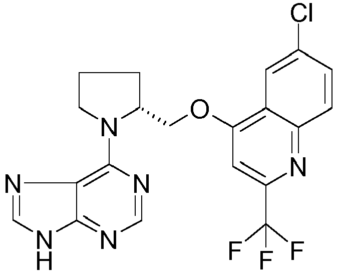 |
| | 6-Chloro-4-[(R)-1-(9H-purin-6-yl)pyrrolidin-2-ylmethoxy]-2-trifluoromethylquinoline |
| "A106" | 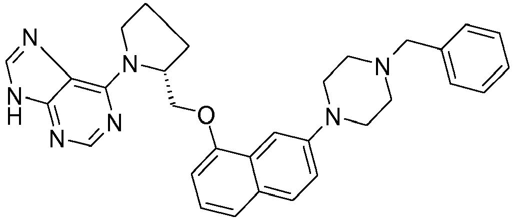 |
| | 6-{(R)-2-[7-(4-Benzylpiperazin-1-yl)naphthalen-1-yl-oxymethyl]pyrrolidin-1-yl}-9H-purine |
| "A107" | 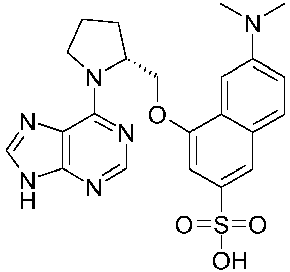 |
| | 6-Dimethylamino-4-[(R)-1-(9H-purin-6-yl)pyrrolidin-2-yl-methoxy]naphthalene-2-sulfonic acid |
| "A108" | 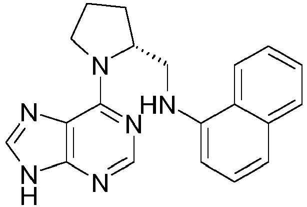 |
| | Naphthalen-1-yl [(R)-1-(9H-purin-6-yl)pyrrolidin-2-ylmethyl]-amine |
| "A109" | 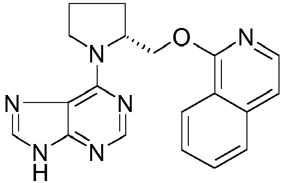 |
| | 1-[(R)-1-(9H-Purin-6-yl)pyrrolidin-2-ylmethoxy]isoquinoline |
| "A110" | 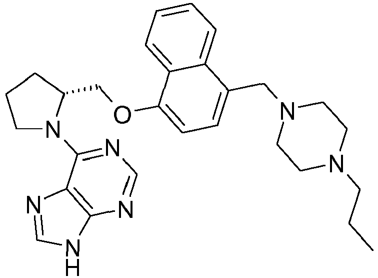 |
| | 6-{(R)-2-[4-(4-Propyl piperazin-1-yl methyl)naphthalen-1-yl-oxymethyl]pyrrolidin-1-yl}-9H-purine |
| "A111" | 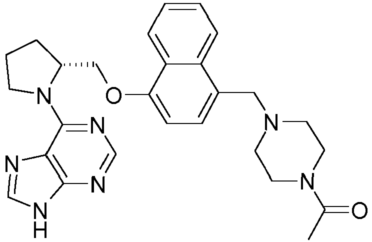 |
| | 1-(4-{4-[(R)-1-(9H-Purin-6-yl)pyrrolidin-2-yl-methoxy]naphthalen-1-ylmethyl}piperazin-1-yl)ethanone |
| "A112" | 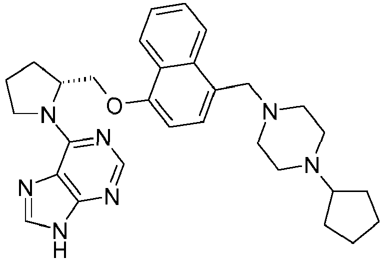 |
| | 6-{(R)-2-[4-(4-Cyclopentylpiperazin-1-ylmethyl)naphthalen-1-yloxymethyl]pyrrolidin-1-yl}-9H-purine |
| "A113" | 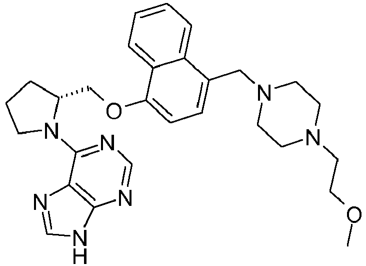 |
| | 6-((R)-2-{4-[4-(2-Methoxyethyl)piperazin-1-ylmethyl]-naphthalen-1-yloxymethyl}pyrrolidin-1-yl)-9H-purine |
| "A114" | 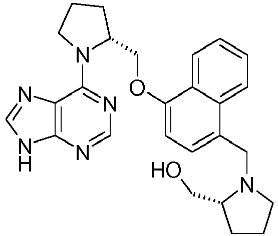 |
| | ((R)-1-{4-[(R)-1-(9H-Purin-6-yl)pyrrolidin-2-yl-methoxy]naphthalen-1-ylmethyl}pyrrolidin-2-yl)methanol |
| "A115" | 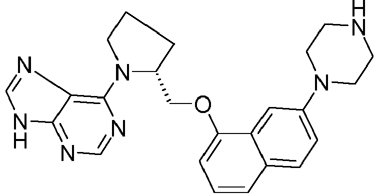 |
| | 6-[(R)-2-(7-Piperazin-1-ylnaphthalen-1-yloxymethyl)-pyrrolidin-1-yl]-9H-purine |
| "A116" | 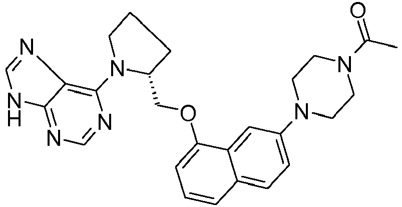 |
| | 1-(4-{8-[(R)-1-(9H-Purin-6-yl)pyrrolidin-2-yl-methoxy]naphthalen-2-yl}piperazin-1-yl)ethanone |
| "A117" | 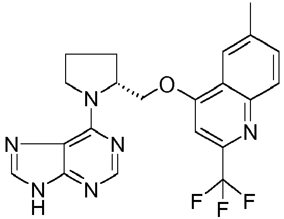 |
| | 6-Methyl-4-[(R)-1-(9H-purin-6-yl)pyrrolidin-2-ylmethoxy]-2-trifluoromethylquinoline |
| "A118" | 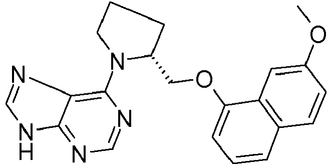 |
| | 6-[(R)-2-(7-Methoxynaphthalen-1-yloxymethyl)pyrrolidin-1-yl]-9H-purine |
| "A119" | 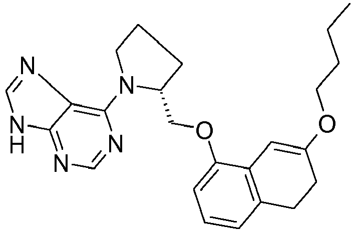 |
| | 6-[(R)-2-(7-Butoxy-5,6-dihydronaphthalen-1-yloxymethyl)-pyrrolidin-1-yl]-9H-purine |
| "A120" | 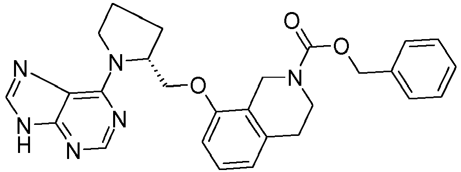 |
| | Benzyl 8-[(R)-1-(9H-purin-6-yl)pyrrolidin-2-ylmethoxy]-3,4-dihydro-1H-isoquinoline-2-carboxylate |
| "A121" | 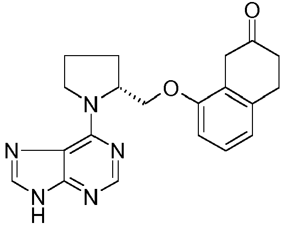 |
| | 8-[(R)-1-(9H-Purin-6-yl)pyrrolidin-2-ylmethoxy]-3,4-dihydro-1H-naphthalen-2-one |
| "A122" | 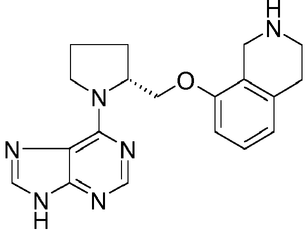 |
| | 8-[(R)-1-(9H-Purin-6-yl)pyrrolidin-2-ylmethoxy]-1,2,3,4-tetrahydroisoquinoline |
| "A123" | 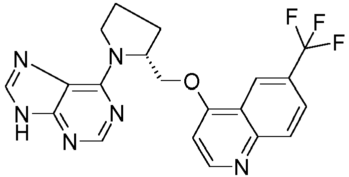 |
| | 4-[(R)-1-(9H-Purin-6-yl)pyrrolidin-2-ylmethoxy]-6-trifluoromethylquinoline |
| "A124" | 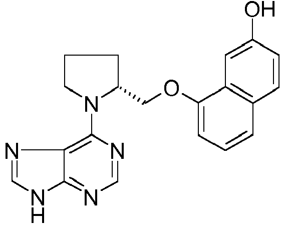 |
| | 8-[(R)-1-(9H-Purin-6-yl)pyrrolidin-2-ylmethoxy]naphthalen-2-ol |
| "A125" | 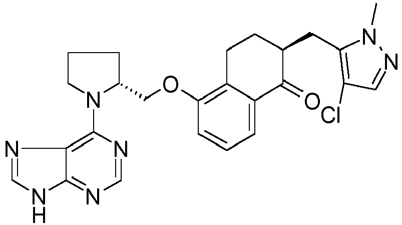 |
| | (S)-2-(4-Chloro-2-methyl-2H-pyrazol-3-ylmethyl)-5-[(R)-1-(9H-purin-6-yl)pyrrolidin-2-ylmethoxy]-3,4-dihydro-2H-naphthalen-1-one |
| "A126" | 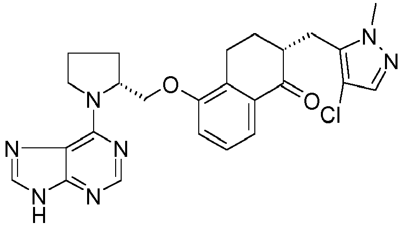 |
| | (R)-2-(4-Chloro-2-methyl-2H-pyrazol-3-ylmethyl)-5-[(R)-1-(9H-purin-6-yl)pyrrolidin-2-ylmethoxy]-3,4-dihydro-2H-naphthalen-1-one |
| "A127" | 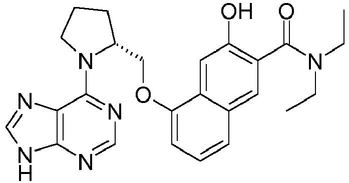 |
| | N,N-Diethyl-3-hydroxy-5-[(R)-1-(9H-purin-6-yl)pyrrolidin-2-yl-methoxy]naphthalene-2-carboxamide |
| "A128" | 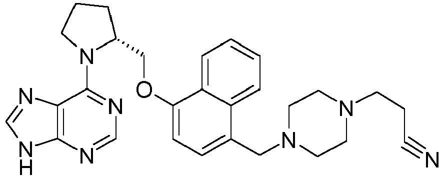 |
| | 3-(4-{4-[(R)-1-(9H-Purin-6-yl)pyrrolidin-2-yl-methoxy]naphthalen-1-ylmethyl}piperazin-1-yl)propionitrile |
| "A129" | 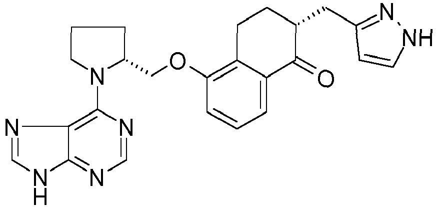 |
| | (R)-5-[(R)-1-(9H-Purin-6-yl)pyrrolidin-2-ylmethoxy]-2-(1H-pyrazol-3-ylmethyl)-3,4-dihydro-2H-naphthalen-1-one |
| "A130" | 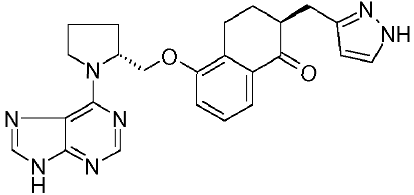 |
| | (S)-5-[(R)-1-(9H-Purin-6-yl)pyrrolidin-2-ylmethoxy]-2-(1H-pyrazol-3-ylmethyl)-3,4-dihydro-2H-naphthalen-1-one |
| "A131" | 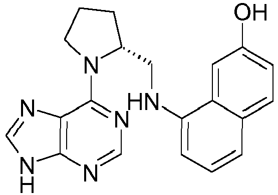 |
| | 8-{[(R)-1-(9H-Purin-6-yl)pyrrolidin-2-ylmethyl]-amino}naphthalen-2-ol |
| "A132" | 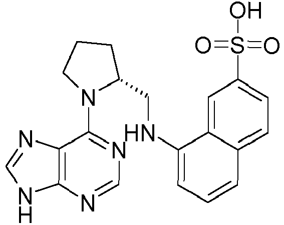 |
| | 8-{[(R)-1-(9H-Purin-6-yl)pyrrolidin-2-ylmethyl]-amino}naphthalene-2-sulfonic acid |
| "A133" | 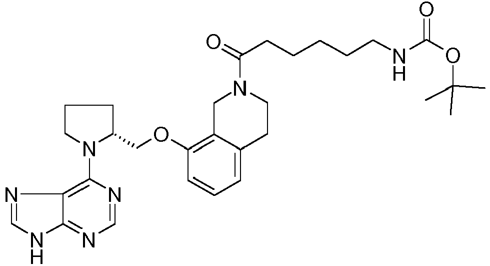 |
| | tert-Butyl (6-oxo-6-{8-[(R)-1-(9H-purin-6-yl)pyrrolidin-2-yl-methoxy]-3,4-dihydro-1H-isoquinolin-2-yl}hexyl)carbamate |
| "A134" | 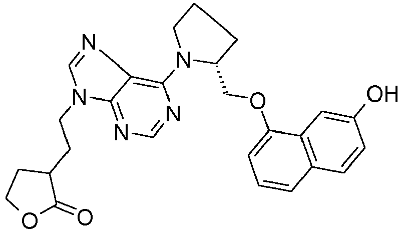 |
| | 3-(2-{6-[(R)-2-(7-Hydroxynaphthalen-1 -yloxymethyl)-pyrrolidin-1-yl]purin-9-yl}ethyl)dihydrofuran-2-one |
| "A135" | 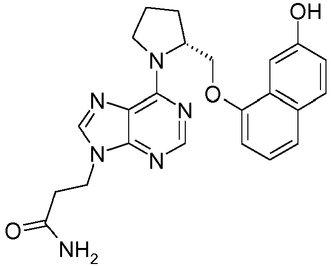 |
| | 3-{6-[(R)-2-(7-Hydroxynaphthalen-1-yloxymethyl)pyrrolidin-1-yl]purin-9-yl}propionamide |
| "A136" | 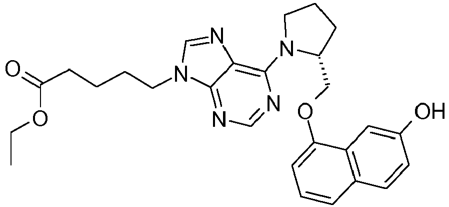 |
| | Ethyl 5-{6-[(R)-2-(7-hydroxynaphthalen-1-yloxymethyl)-pyrrolidin-1-yl]purin-9-yl}pentanoate |
| "A137" | 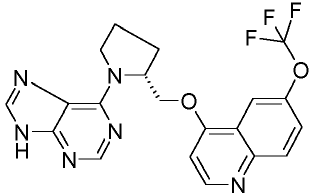 |
| | 4-[(R)-1-(9H-Purin-6-yl)pyrrolidin-2-ylmethoxy]-6-trifluoromethoxyquinoline |
| "A138" | 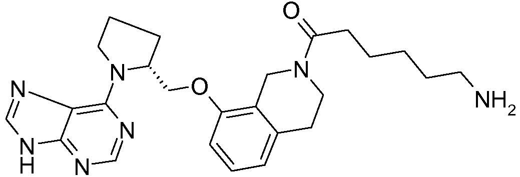 |
| | 6-Amino-1-{8-[(R)-1-(9H-purin-6-yl)pyrrolidin-2-ylmethoxy]-3,4-dihydro-1H-isoquinolin-2-yl}hexan-1-one |
| "A139" | 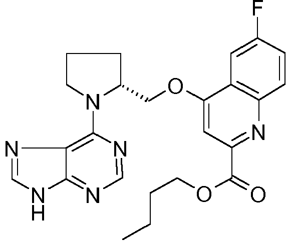 |
| | Butyl 6-fluoro-4-[(R)-1-(9H-purin-6-yl)pyrrolidin-2-yl-methoxy]quinoline-2-carboxylate |
| "A140" | 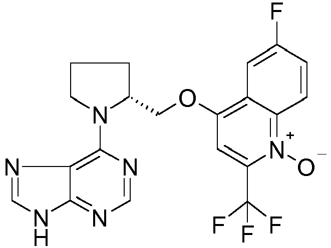 |
| | 6-Fluoro-4-[(R)-1-(9H-purin-6-yl)pyrrolidin-2-ylmethoxy]-2-trifluoromethylquinoline 1-oxide |
| "A141" | 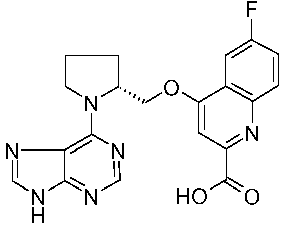 |
| | 6-Fluoro-4-[(R)-1-(9H-purin-6-yl)pyrrolidin-2-yl-methoxy]quinoline-2-carboxylic acid |
| "A142" | 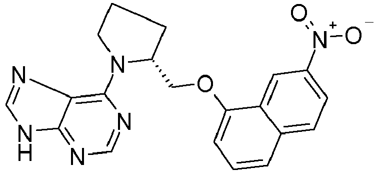 |
| | 6-[(R)-2-(7-Nitronaphthalen-1-yloxymethyl)pyrrolidin-1-yl]-9H-purine |
| "A144" | 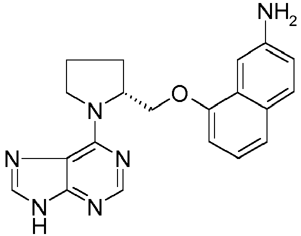 |
| | 8-[(R)-1-(9H-Purin-6-yl)pyrrolidin-2-ylmethoxy]naphthalen-2-ylamine |
| "A145" | 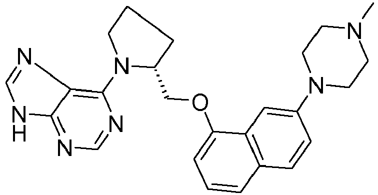 |
| | 6-{(R)-2-[7-(4-Methylpiperazin-1-yl)naphthalen-1-yl-oxymethyl]pyrrolidin-1-yl}-9H-purine |
| "A146" | 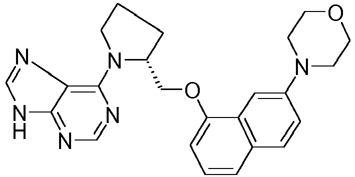 |
| | 6-[(R)-2-(7-Morpholin-4-ylnaphthalen-1-yloxymethyl)-pyrrolidin-1-yl]-9H-purine |
| "A147" | 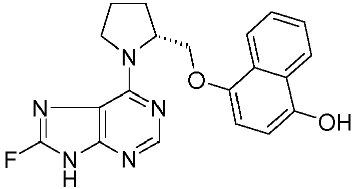 |
| | 4-[(R)-1-(8-Fluoro-9H-purin-6-yl)pyrrolidin-2-yl-methoxy]naphthalen-1-ol |
| "A148" | 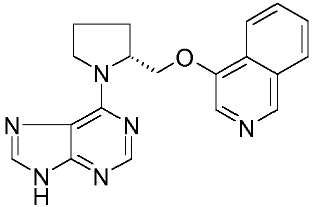 |
| | 4-[(R)-1-(9H-Purin-6-yl)pyrrolidin-2-ylmethoxy]isoquinoline |
| "A149" | 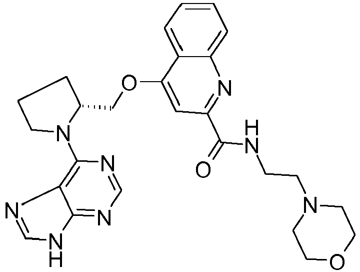 |
| | N-(2-Morpholin-4-ylethyl)-4-[(R)-1-(9H-purin-6-yl)pyrrolidin-2-ylmethoxy]quinoline-2-carboxamide |
| "A150" | 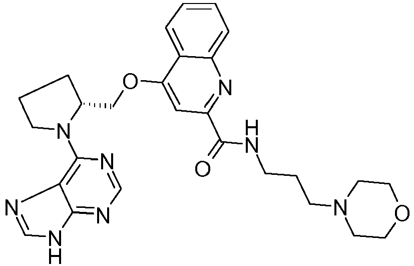 |
| | N-(3-Morpholin-4-ylpropyl)-4-[(R)-1-(9H-purin-6-yl)pyrrolidin-2-ylmethoxy]quinoline-2-carboxamide |
| "A151" | 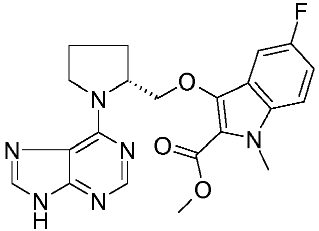 |
| | Methyl 5-fluoro-1-methyl-3-[(R)-1-(9H-purin-6-yl)pyrrolidin-2-ylmethoxy]-1H-indole-2-carboxylate |
| "A152" | 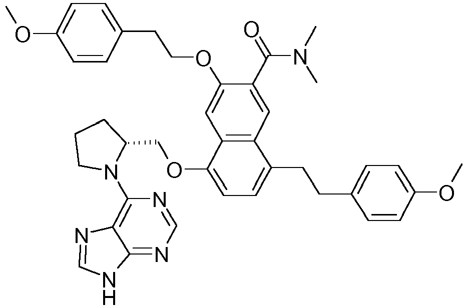 |
| | N,N-Dimethyl-3-[2-(4-methoxyphenyl)ethoxy]-8-[2-(4-methoxyphenyl)ethyl]-5-[(R)-1-(9H-purin-6-yl)pyrrolidin-2-yl-methoxy]naphthalene-2-carboxamide |
| "A153" | 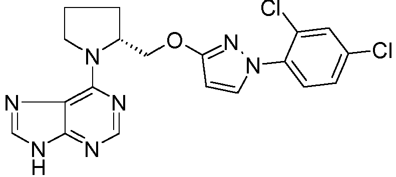 |
| | 6-{(R)-2-[1-(2,4-Dichlorophenyl)-1H-pyrazol-3-yloxymethyl]-pyrrolidin-1-yl}-9H-purine |
| "A154" | 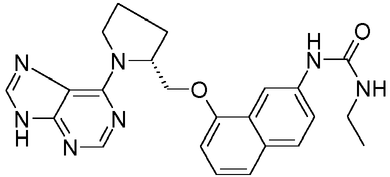 |
| | 1-Ethyl-3-{8-[(R)-1-(9H-purin-6-yl)pyrrolidin-2-yl-methoxy]naphthalen-2-yl}urea |
| "A155" | 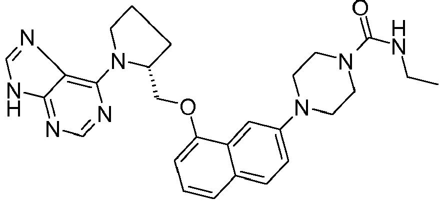 |
| | N-Ethyl-4-{8-[(R)-1-(9H-purin-6-yl )pyrrolidin-2-yl-methoxy]naphthalen-2-yl}piperazine-1-carboxamide |
| "A156" | 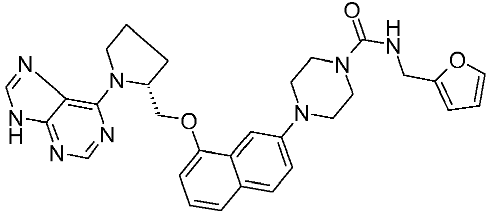 |
| | N-(Furan-2-ylmethyl)-4-{8-[(R)-1-(9H-purin-6-yl)pyrrolidin-2-ylmethoxy]naphthalen-2-yl}piperazine-1-carboxamide |
| "A157" | 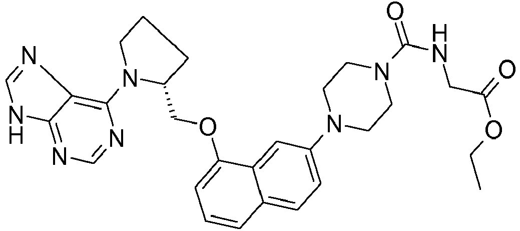 |
| | Ethyl [(4-{8-[(R)-1-(9H-purin-6-yl)pyrrolidin-2-yl-methoxy]naphthalen-2-yl}piperazine-1-carbonyl)-amino]acetate |
| "A158" | 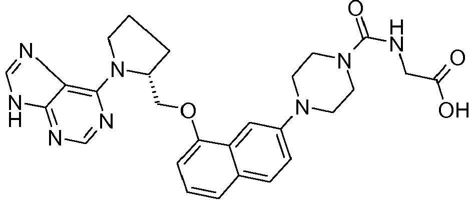 |
| | [(4-{8-[(R)-1-(9H-Purin-6-yl)pyrrolidin-2-yl-methoxy]naphthalen-2-yl}piperazine-1-carbonyl)amino]acetic acid |
| "A159" | 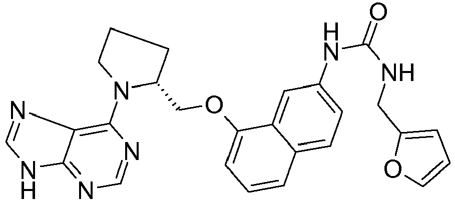 |
| | 1-Furan-2-ylmethyl-3-{8-[(R)-1-(9H-purin-6-yl)pyrrolidin-2-yl-methoxy]naphthalen-2-yl}urea |
| "A160" | 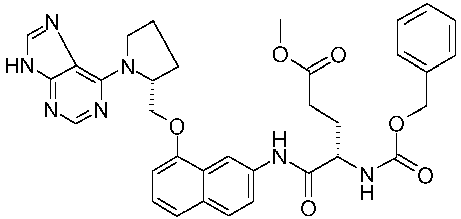 |
| | Methyl (S)-4-benzyloxycarbonylamino-4-{8-[(R)-1-(9H-purin-6-yl)pyrrolidin-2-ylmethoxy]naphthalen-2-yl-carbamoyl}butyrate |
| "A161" | 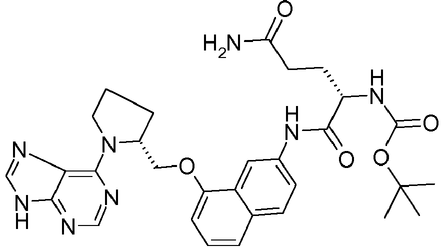 |
| | tert-Butyl ((S)-3-carbamoyl-1-{8-[(R)-1-(9H-purin-6-yl)-pyrrolidin-2-ylmethoxy]naphthalen-2-ylcarbamoyl}propyl)-carbamate |
| "A162" | 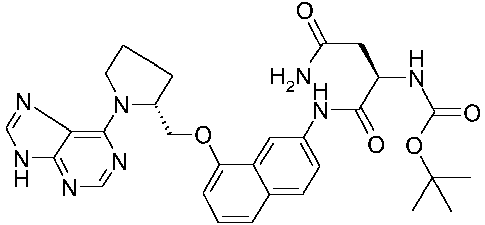 |
| | tert-Butyl ((R)-2-carbamoyl-1-{8-[(R)-1-(9H-purin-6-yl)-pyrrolidin-2-ylmethoxy]naphthalen-2-ylcarbamoyl}ethyl)-carbamate |
| "A163" | 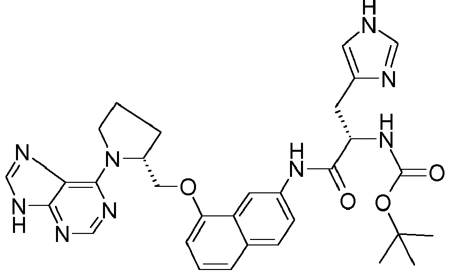 |
| | tert-Butyl ((S)-2-(1H-imidazol-4-yl-{8-[(R)-1-(9H-purin-6-yl)pyrrolidin-2-ylmethoxy]naphthalen-2-ylcarbamoyl}ethyl)-carbamate |
| "A164" | 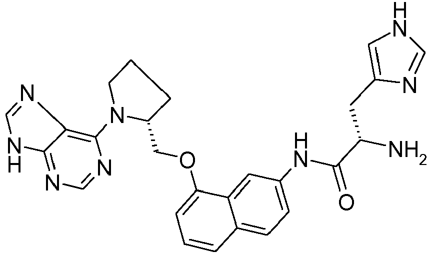 |
| | (S)-2-Amino-3-(1H-imidazol-4-yl)-N-{8-[(R)-1-(9H-purin-6-yl)-pyrrolidin-2-ylmethoxy]naphthalen-2-yl}propionamide |
| "A165" | 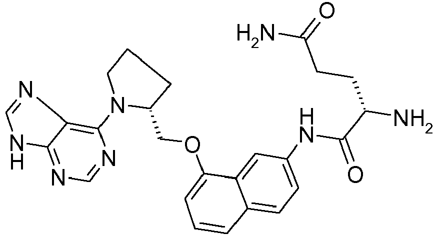 |
| | N-(1-({8-[(R)-1-(9H-Purin-6-yl)pyrrolidin-2-yl-methoxy]naphthalen-2-yl})-(S)-2-aminopentan-5-amide |
| "A166" | 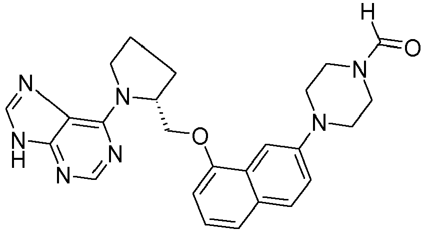 |
| | 4-{8-[(R)-1-(9H-Purin-6-yl)pyrrolidin-2-yl-methoxy]naphthalen-2-yl}piperazine-1-carbaldehyde |
| "A167" | 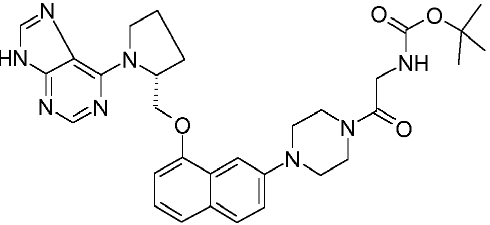 |
| | tert-Butyl [2-oxo-2-(4-{8-[(R)-1-(9H-purin-6-yl)pyrrolidin-2-yl-methoxy]naphthalen-2-yl}piperazin-1-yl)ethyl]carbamate |
| "A168" | 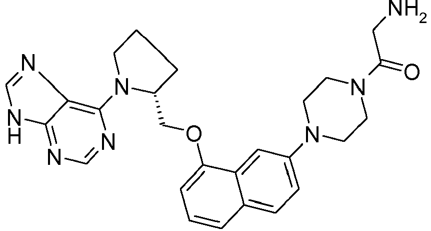 |
| | 2-Amino-1-(4-{8-[(R)-1-(9H-purin-6-yl)pyrrolidin-2-yl-methoxy]naphthalen-2-yl}piperazin-1-yl)ethanone |
| "A169" | 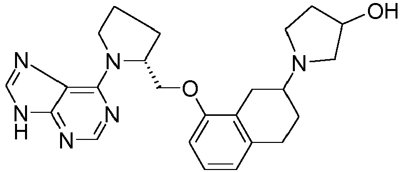 |
| | 1-{8-[(R)-1-(9H-Purin-6-yl)pyrrolidin-2-ylmethoxy]-1,2,3,4-tetrahydronaphthalen-2-yl}pyrrolidin-3-ol |
| "A170" | 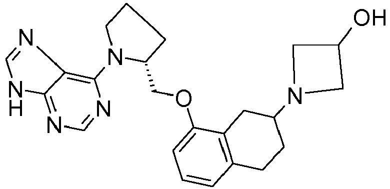 |
| | 1-{8-[(R)-1-(9H-Purin-6-yl)pyrrolidin-2-ylmethoxy]-1,2,3,4-tetrahydronaphthalen-2-yl}azetidin-3-ol |
| "A171" | 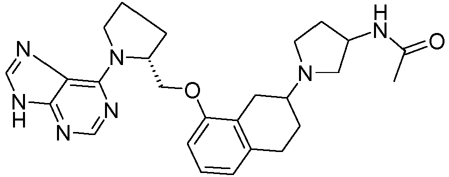 |
| | N-(1-{8-[(R)-1-(9H-Purin-6-yl)pyrrolidin-2-ylmethoxy]-1,2,3,4-tetrahydronaphthalen-2-yl}pyrrolidin-3-yl)acetamide |
| "A172" | 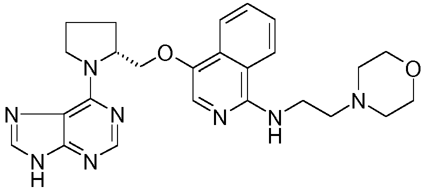 |
| | (2-Morpholin-4-ylethyl)-{4-[(R)-1-(9H-purin-6-yl)pyrrolidin-2-ylmethoxy]isoquinolin-1-yl}amine |
| "A173" | 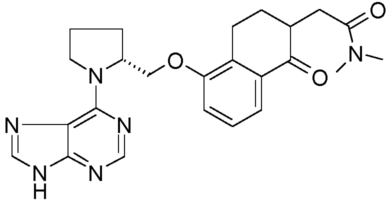 |
| | N,N-Dimethyl-2-{1-oxo-5-[(R)-1-(9H-purin-6-yl)pyrrolidin-2-ylmethoxy]-1,2,3,4-tetrahydronaphthalen-2-yl}acetamide |
| "A174" | 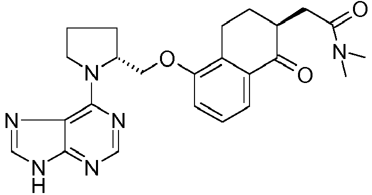 |
| | N, N-Dimethyl-2-{(S)-1-oxo-5-[(R)-1-(9H-purin-6-yl)pyrrolidin-2-ylmethoxy]-1,2,3,4-tetrahydronaphthalen-2-yl}acetamide |
| "A175" | 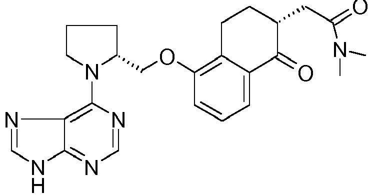 |
| | N,N-Dimethyl-2-{(R)-1-oxo-5-[(R)-1-(9H-purin-6-yl)pyrrolidin-2-ylmethoxy]-1,2,3,4-tetrahydronaphthalen-2-yl}acetamide |
| "A176" | 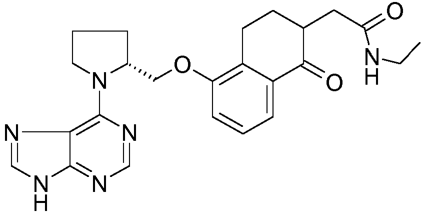 |
| | N-Ethyl-2-{1-oxo-5-[(R)-1-(9H-purin-6-yl)pyrrolidin-2-yl-methoxy]-1,2,3,4-tetrahydronaphthalen-2-yl}acetamide |
| "A177" | 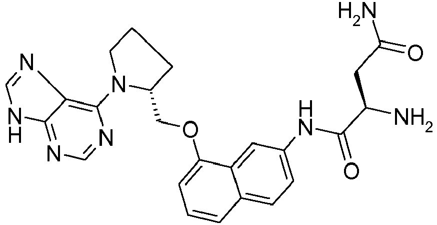 |
| | (R)-2-Amino-N1-{8-[(R)-1-(9H-purin-6-yl)pyrrolidin-2-yl-methoxy]naphthalen-2-yl}succinamide |
| "A178" | 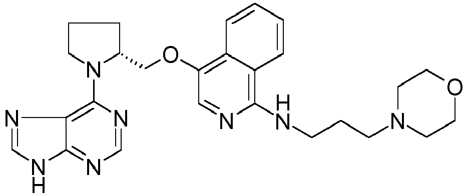 |
| | (3-Morpholin-4-ylpropyl)-{4-[(R)-1-(9H-purin-6-yl)pyrrolidin-2-ylmethoxy]isoquinolin-1-yl}amine |
| "A179" | 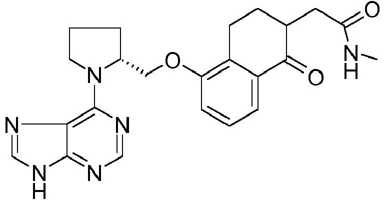 |
| | N-Methyl-2-{1-oxo-5-[(R)-1-(9H-purin-6-yl)pyrrolidin-2-yl-methoxy]-1,2,3,4-tetrahydronaphthalen-2-yl}acetamide |
| "A180" | 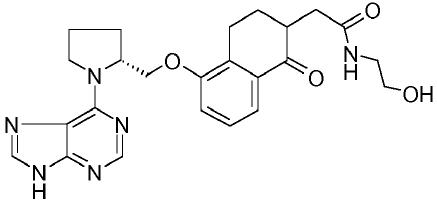 |
| | N-(2-Hydroxyethyl)-2-{1-oxo-5-[(R)-1-(9H-purin-6-yl)-pyrrol idin-2-ylmethoxy]-1,2,3,4-tetrahydronaphthalen-2-yl}acetamide |
| "A181" | 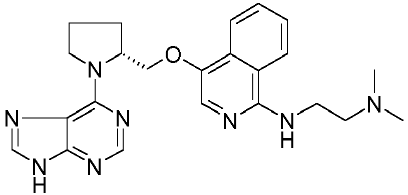 |
| | N,N-Dimethyl-N'-{4-[(R)-1-(9H-purin-6-yl)pyrrolidin-2-yl-methoxy]isoquinolin-1-yl}ethane-1,2-diamine |
| "A182" | 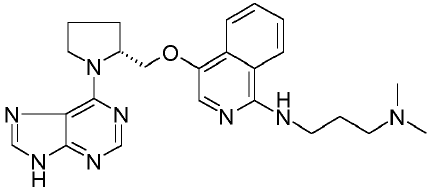 |
| | N,N-Dimethyl-N'-{4-[(R)-1-(9H-purin-6-yl)pyrrolidin-2-yl-methoxy]isoquinolin-1-yl}propane-1,3-diamine |
| "A183" | 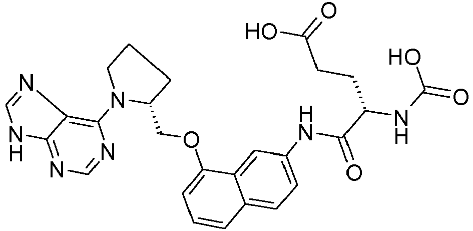 |
| | (S)-4-Carboxyamino-4-{8-[(R)-1-(9H-purin-6-yl)pyrrolidin-2-ylmethoxy]naphthalen-2-ylcarbamoyl}butyric acid |
| "A184" | 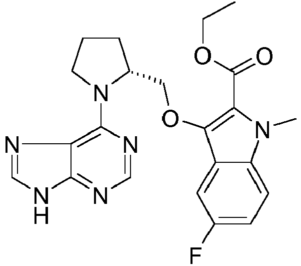 |
| | Ethyl 5-fluoro-1-methyl-3-[(R)-1-(9H-purin-6-yl)pyrrolidin-2-ylmethoxy]-1H-indole-2-carboxylate |
| "A185" | 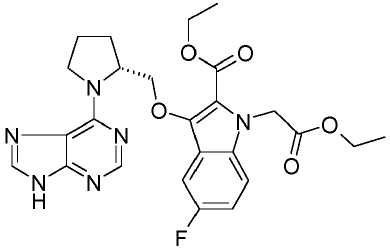 |
| | Ethyl 1-ethoxycarbonylmethyl-5-fluoro-3-[(R)-1-(9H-purin-6-yl)pyrrolidin-2-ylmethoxy]-1H-indole-2-carboxylate |
| "A186" | 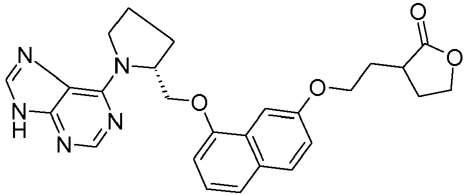 |
| | 3-(2-{8-[(R)-1-(9H-Purin-6-yl)pyrrolidin-2-yl-methoxy]naphthalen-2-yloxy}ethyl)dihydrofuran-2-one |
| "A187" | 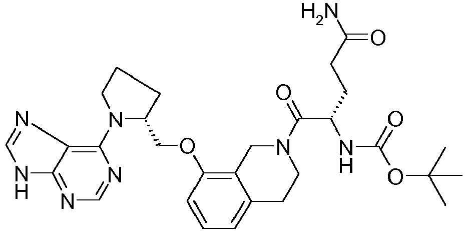 |
| | tert-Butyl ((S)-3-carbamoyl-1-{8-[(R)-1-(9H-purin-6-yl)-pyrrolidin-2-ylmethoxy]-3,4-dihydro-1H-isoquinoline-2-carbonyl}propyl)carbamate |
| "A188" | 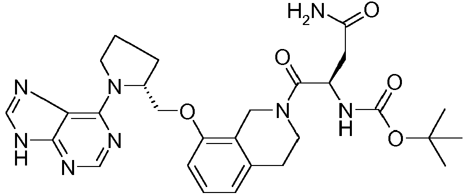 |
| | tert-Butyl ((R)-1-carbamoylmethyl-2-oxo-2-{8-[(R)-1-(9H-purin-6-yl)pyrrolidin-2-ylmethoxy]-3,4-dihydro-1H-isoquinolin-2-yl}ethyl)carbamate |
| "A189" | 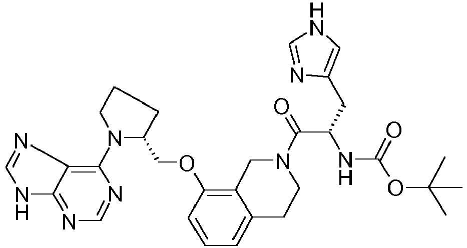 |
| | tert-Butyl ((S)-1-(1H-imidazol-4-ylmethyl)-2-oxo-2-{8-[(R)-1-(9H-purin-6-yl)pyrrolidin-2-ylmethoxy]-3,4-dihydro-1H-isoquinolin-2-yl}ethyl)carbamate |
| "A190" | 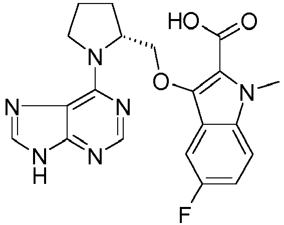 |
| | 5-Fluoro-1-methyl-3-[(R)-1-(9H-purin-6-yl)pyrrolidin-2-yl-methoxy]-1H-indole-2-carboxylic acid |
| "A191" | 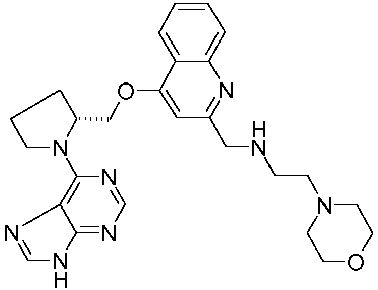 |
| | (2-Morpholin-4-ylethyl)-{4-[(R)-1-(9H-purin-6-yl)pyrrolidin-2-ylmethoxy]quinolin-2-ylmethyl}amine |
| "A192" | 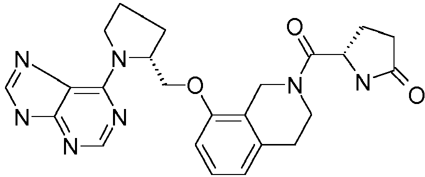 |
| | (S)-5-{8-[(R)-1-(9H-purin-6-yl)pyrrolidin-2-ylmethoxy]-3,4-dihydro-1H-isoquinoline-2-carbonyl}pyrrolidin-2-one |
| "A193" | 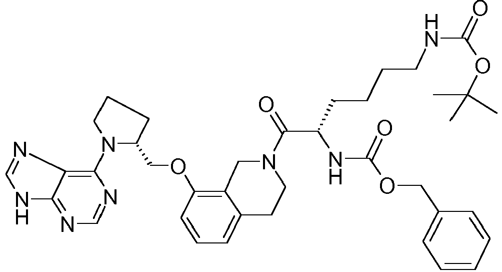 |
| | tert-Butyl ((S)-5-benzyloxycarbonylamino-6-oxo-6-{8-[(R)-1-(9H-purin-6-yl)pyrrolidin-2-ylmethoxy]-3,4-dihydro-1H-isoquinolin-2-yl}hexyl)carbamate |
| "A194" | 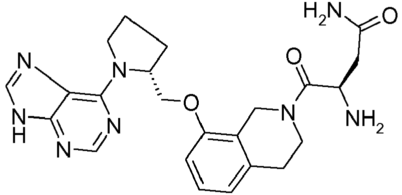 |
| | (R)-3-Amino-4-oxo-4-{8-[(R)-1-(9H-purin-6-yl)pyrrolidin-2-yl-methoxy]-3,4-dihydro-1H-isoquinolin-2-yl}butyramide |
| "A195" | 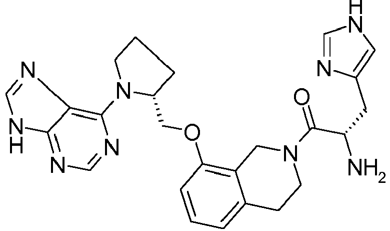 |
| | (S)-2-Amino-3-(1H-imidazol-4-yl)-1-{8-[(R)-1-(9H-purin-6-yl)-pyrrolidin-2-ylmethoxy]-3,4-dihydro-1H-isoquinolin-2-yl}propan-1-one |
| "A196" | 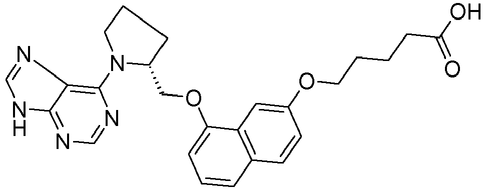 |
| | 5-{8-[(R)-1-(9H-Purin-6-yl)pyrrolidin-2-yl-methoxy]naphthalen-2-yloxy}pentanoic acid |
| "A197" | 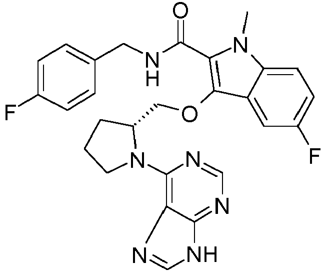 |
| | N-(4-Fluorobenzyl)-5-fluoro-1-methyl-3-[(R)-1-(9H-purin-6-yl)pyrrolidin-2-ylmethoxy]-1H-indole-2-carboxamide |
| "A198" | 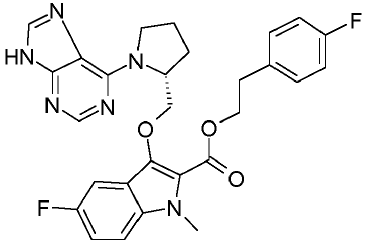 |
| | 2-(4-Fluorophenyl)ethyl 5-fluoro-1-methyl-3-[(R)-1-(9H-purin-6-yl)pyrrol id in-2-yl methoxy]-1H-indole-2-carboxylate |
| "A199" | 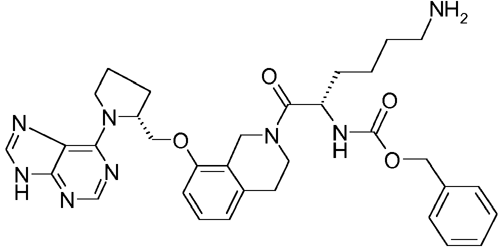 |
| | Benzyl ((S)-5-amino1-{8-[(R)-1-(9H-purin-6-yl)pyrrolidin-2-ylmethoxy]-3,4-dihydro-1H-isoquinoline-2-carbonyl}pentyl)-carbamate |
| "A200" | 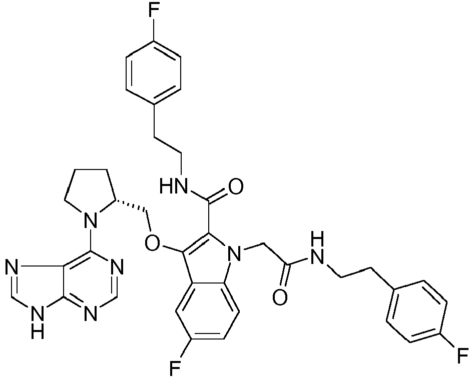 |
| | N-[2-(4-Fluorophenyl)ethyl]-5-fluoro-1-{[2-(4-fluorophenyl)-ethylcarbamoyl]methyl}-3-[(R)-1-(9H-purin-6-yl)pyrrolidin-2-ylmethoxy]-1H-indole-2-carboxamide |
| "A201" | 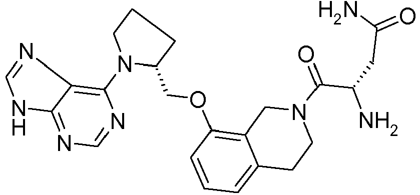 |
| | (S)-3-Amino-4-oxo-4-{8-[(R)-1-(9H-purin-6-yl)pyrrolidin-2-yl-methoxy]-3,4-dihydro-1H-isoquinolin-2-yl}butyramide |
| "A202" | 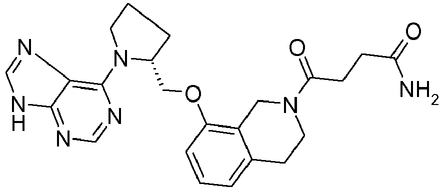 |
| | 4-Oxo-4-{8-[(R)-1-(9H-purin-6-yl)pyrrolidin-2-ylmethoxy]-3,4-dihydro-1H-isoquinolin-2-yl}butyramide |
| "A203" | 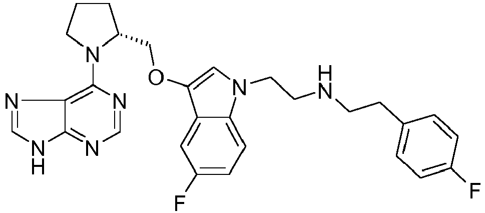 |
| | [2-(4-Fluorophenyl)ethyl]-(2-{5-fluoro-3-[(R)-1-(9H-purin-6-yl)pyrrolidin-2-ylmethoxy]indol-1-yl}ethyl)amine |
and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Use of compounds according to Claim 1
and pharmaceutically usable salts, tautomers and stereoisomers thereof, including mixtures thereof in all ratios, for the preparation of a medicament for the treatment of tumours, tumour metastases, proliferative diseases of the mesangial cells, haemangioma, proliferative retinopathy, rheumatoid arthritis, atherosclerotic neovascularisation, psoriasis, ocular neovascularisation, osteoporosis, diabetes and obesity, lymphoid leukaemia and lymphoma.

4. Use according to Claim 3, where the tumour disease is selected from the group of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx, of the lung, of the skin, monocytic leukaemia, lung adenocarcinoma, small-cell lung carcinoma, pancreatic cancer, glioblastoma, breast carcinoma, acute myeloid leukaemia, chronic myeloid leukaemia, acute lymphatic leukaemia, chronic lymphatic leukaemia, Hodgkin's lymphoma, non-Hodgkin's lymphoma.

5. Use of compounds according to Claim 1 and/or physiologically acceptable salts thereof for the preparation of a medicament for the treatment of tumours, where a therapeutically effective amount of a compound according to Claim 1 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

6. Use of compounds according to Claim 1 and/or physiologically acceptable salts thereof for the preparation of a medicament for the treatment of tumours where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés choisis dans le groupe constitué par
| Composé n° | Nom et/ou structure |
|---|---|
| « A1 » | 6-[(R)-2-(Naphtalén-1-yloxyméthyl)pyrrolidin-1-yl]-9H-purine |
| « A2 » | 4-[(R)-1-(9H-Purin-6-yl)pyrrolidin-2-ylméthoxy]naphtalène-1-carbaldéhyde |
| « A3 » | 6-[(R)-2-(4-Morpholin-4-ylméthylnaphtalén-1-yloxyméthyl)-pyrrolidin-1-yl]-9H-purine |
| « A4 » | 6-[(R)-2-(4-Butoxyméthylnaphtalén-1-yloxyméthyl)pyrrolidin-1-yl]-9H-purine |
| « A6 » | Morpholin-4-yl(4-{2-[-1-(9H-purin-6-yl)pyrrolidin-2-yl]éthyl}-naphtalén-1-yl)méthanone |
| « A8 » | 6-[(R)-4,4-Difluoro-2-(naphtalén-1-yloxyméthyl)pyrrolidin-1-yl]-9H-purine |
| « A12 » | 6-[(R)-2-((E)-2-Naphtalén-1-ylvinyl)pyrrolidin-1-yl]-9H-purine |
| « A14 » | N-Cyclopropyl-4-[(R)-1-(9H-purin-6-yl)pyrrolidin-2-yl-méthoxy]quinoléine-2-carboxamide |
| « A15 » | 2-[1-(1H-Imidazol-4-yl)méth-(Z)-ylidène]-5-[(R)-1-(9H-purin-6-yl)pyrrolidin-2-ylméthoxy]-3,4-dihydro-2H-naphtalén-1-one |
| « A15.1 » | 2-(1H-Imidazol-4-ylméthyl)-5-[(R)-1-(9H-purin-6-yl)pyrrolidin-2-ylméthoxy]-3,4-dihydro-2H-naphtalén-1-one |
| « A18 » | 8-Bromo-6-[(R)-2-(naphtalén-1-yloxyméthyl)pyrrolidin-1-yl]-9H-purine |
| « A19 » | 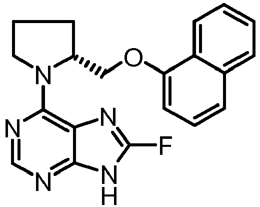 |
| « A20 » | 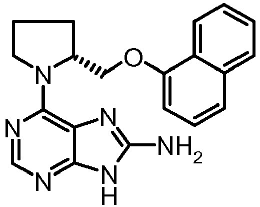 |
| « A23 » | 4-[(R)-2-(Naphtalén-1-yloxyméthyl)pyrrolidin-1-yl]pyrido[2,3-d]pyrimidine |
| « A25 » | 6-[(R)-2-(Naphtalén-1-yloxyméthyl)pyrrolidin-1-yl]purin-9-ylamine |
| « A26 » | 6-[(2R,4R)-4-Fluoro-2-(naphtalén-1-yloxyméthyl)pyrrolidin-1-yl]-9H-purine |
| « A28 » | 6-[(2R,4S)-4-Fluoro-2-(naphtalén-1-yloxyméthyl)pyrrolidin-1-yl]-9H-purine |
| « A30 » | (3R,5R)-5-(Naphtalén-1-yloxyméthyl)-1-(9H-purin-6-yl)-pyrrolidin-3-ol |
| « A31 » | 6-[(R)-2,2-Difluoro-5-(naphtalén-1-yloxyméthyl)pyrrolidin-1-yl]-9H-purine |
| « A33 » | N-[6-(3-{2-[1-(9H-Purin-6-yl)pyrrolidin-2-ylméthoxy]-5-trifluorométhylphényl}uréidométhyl)-1H-benzimidazol-2-yl]acétamide |
| « A34 » | N-Méthyl-4-[4-(3-{2-[1-(9H-purin-6-yl)pyrrolidin-2-ylméthoxy]-5-trifluorométhylphényl}uréidométhyl)phénoxy]pyridine-2-carboxamide |
| « A35 » | N-Méthyl-4-[4-(3-{2-[1-(9H-purin-6-yl)pyrrolidin-2-ylméthoxy]-5-trifluorométhylphényl}uréido)phénoxy]pyridine-2-carboxamide |
| « A36 » | N-Méthyl-4-[3-(3-{2-[1-(9H-purin-6-yl)pyrrolidin-2-ylméthoxy]-5-trifluorométhylphényl}uréido)phénoxy]pyridine-2-carboxamide |
| « A37 » | 6-[(R)-2-(Dibenzofuran-3-yloxyméthyl)pyrrolidin-1-yl]-9H-purine |
| « A38 » | 4-[(R)-1-(9H-Purin-6-yl)pyrrolidin-2-ylméthoxy]-9H-carbazole |
| « A39 » | 1-Butyl-2-méthyl-5-[(R)-1-(9H-purin-6-yl)pyrrolidin-2-ylméthoxy]-1H-indole-3-carboxylate d'éthyle |
| « A40 » | 6-[(R)-2-(1H-Indol-4-yloxyméthyl)pyrrolidin-1-yl]-9H-purine |
| « A41 » | 1-{7-[(R)-1-(9H-Purin-6-yl)pyrrolidin-2-ylméthoxy]benzofuran-2-yl}éthanone |
| « A42 » | 5-Pipéridin-1-ylméthyl-8-[(R)-1-(9H-purin-6-yl)pyrrolidin-2-ylméthoxy]quinoléine |
| « A43 » | 8-[(R)-1-(9H-Purin-6-yl)pyrrolidin-2-ylméthoxy]quinoléine |
| « A44 » | 5-[(R)-1-(9H-Purin-6-yl)pyrrolidin-2-ylméthoxy]isoquinoléine |
| « A45 » | 7-Benzyloxy-6-méthoxy-4-[(R)-1-(9H-purin-6-yl)pyrrolidin-2-ylméthoxy]quinazoline |
| « A46 » | 4-[(R)-1-(9H-Purin-6-yl)pyrrolidin-2-ylméthoxy]quinazoline |
| « A47 » | 2-{2-[2-({4-[(R)-1-(9H-Purin-6-yl)pyrrolidin-2-ylméthoxy]-naphtalén-1-ylméthyl}amino)éthoxy]éthoxy}éthylamine |
| « A48 » | N-Méthyl-4-[(R)-1-(9H-purin-6-yl)pyrrolidin-2-ylméthoxy]quinoléine-2-carboxamide |
| « A49 » | 2-Méthyl-8-[(R)-1-(9H-purin-6-yl)pyrrolidin-2-ylméthoxy]quinoléine |
| « A50 » | N-Éthyl-4-[(R)-1-(9H-purin-6-yl)pyrrolidin-2-ylméthoxy]quinoléine-2-carboxamide |
| « A51 » | 5-[(R)-1-(9H-Purin-6-yl)pyrrolidin-2-ylméthoxy]-3,4-dihydro-2H-naphtalén-1-one |
| « A52 » | 2-Hydroxyméthylène-5-[(R)-1-(9H-purin-6-yl)pyrrolidin-2-ylméthoxy]-3,4-dihydro-2H-naphtalén-1-one |
| « A53 » | Acide [1-oxo-5-[(R)-1-(9H-purin-6-yl)pyrrolidin-2-ylméthoxy]-3,4-dihydro-1H-naphtalén-(2Z)-ylidène]acétique |
| « A54 » | 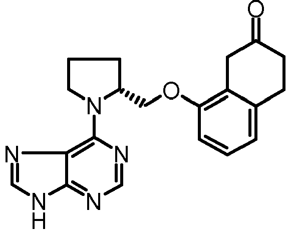 |
| | 8-[(R)-1-(9H-Purin-6-yl)pyrrolidin-2-ylméthoxy]-3,4-dihydro-1H-naphtalén-2-one |
| « A55 » | 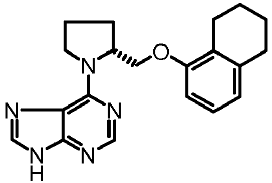 |
| | 6-[(R)-2-(5,6,7,8-Tétrahydronaphtalén-1-yloxyméthyl)-pyrrolidin-1-yl]-9H-purine |
| « A56 » | 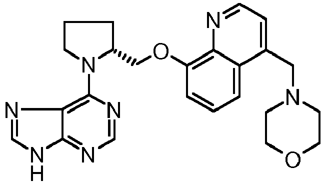 |
| | 4-Morpholin-4-ylméthyl-8-[(R)-1-(9H-purin-6-yl)pyrrolidin-2-ylméthoxy]quinoléine |
| « A57 » | 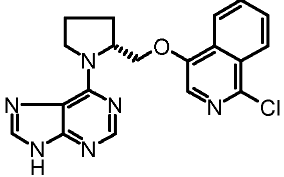 |
| | 1-Chloro-4-[(R)-1-(9H-purin-6-yl)pyrrolidin-2-ylméthoxy]isoquinoléine |
| « A58 » | 6-{(R)-2-[5-(2-Méthoxyéthoxy)naphtalén-1-yloxyméthyl]pyrrolidin-1-yl}-9H-purine |
| « A59 » | 2-[(R)-1-(9H-Purin-6-yl)pyrrolidin-2-ylméthyl]-2H-isoquinoléin-1-one |
| « A60 » | (2-Morpholin-4-yléthyl)-{4-[(R)-1-(9H-purin-6-yl)pyrrolidin-2-ylméthoxy]naphtalén-1-ylméthyl}amine |
| « A61 » | 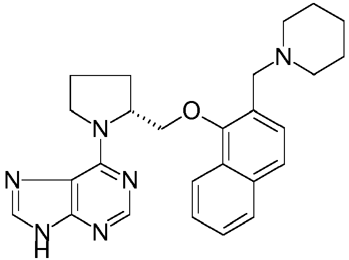 |
| | 6-[(R)-2-(2-Pipéridin-1-ylméthylnaphtalén-1-yloxyméthyl)pyrrolidin-1-yl]-9H-purine |
| « A62 » | 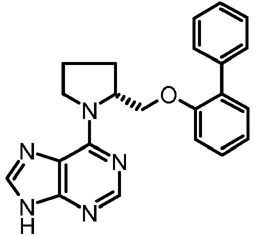 |
| | 6-[(R)-2-(Biphényl-2-yloxyméthyl)pyrrolidin-1-yl]-9H-purine |
| « A63 » | 6-[(R)-2-(Biphényl-3-yloxyméthyl)pyrrolidin-1-yl]-9H-purine |
| « A64 » | 6-[(R)-2-(Biphényl-4-yloxyméthyl)pyrrolidin-1-yl]-9H-purine |
| « A65 » | 3-{5-[(R)-1-(9H-Purin-6-yl)pyrrolidin-2-ylméthoxy]pyrimidin-2-yl}benzoate de méthyle |
| « A66 » | N-(2-Morpholin-4-yléthyl)-3-{5-[(R)-1-(9H-purin-6-yl)pyrrolidin-2-ylméthoxy] pyrim idin-2-yl}benzamide |
| « A67 » | N-Méthyl-2-[1-oxo-5-[(R)-1-(9H-purin-6-yl)pyrrolidin-2-ylméthoxy]-3,4-dihydro-1H-naphtalén-(2Z)-ylidène]acétamide |
| « A68 » | N-(2-Hydroxypropyl)-4-[(R)-1-(9H-purin-6-yl)pyrrolidin-2-ylméthoxy]quinoléine-2-carboxamide |
| « A69 » | 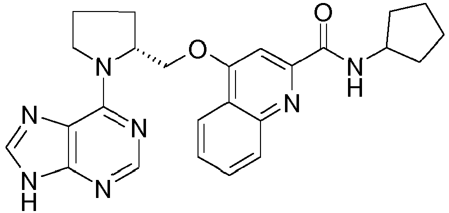 |
| | N-Cyclopentyl-4-[(R)-1-(9H-purin-6-yl )pyrrolidin-2-ylméthoxy]quinoléine-2-carboxamide |
| « A70 » | 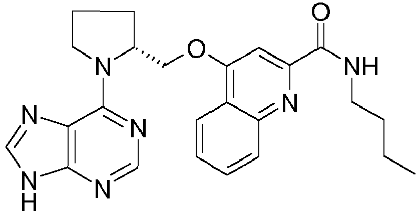 |
| | N-Butyl-4-[(R)-1-(9H-purin-6-yl)pyrrolidin-2-ylméthoxy]quinoléine-2-carboxamide |
| « A71 » | 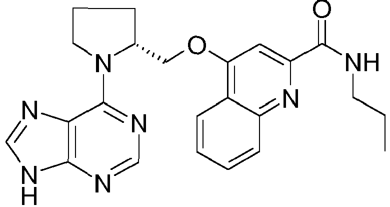 |
| | N-Propyl-4-[(R)-1-(9H-purin-6-yl)pyrrolidin-2-ylméthoxy]quinoléine-2-carboxamide |
| « A72 » | 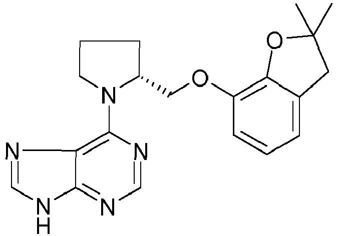 |
| | 6-[(R)-2-(2,2-Diméthyl-2,3-dihydrobenzofuran-7-yloxyméthyl)pyrrolidin-1-yl]-9H-purine |
| « A73 » | 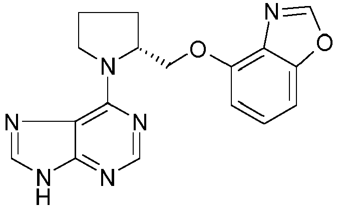 |
| | 6-[(R)-2-(Benzoxazol-4-yloxyméthyl)pyrrolidin-1-yl]-9H-purine |
| « A75 » | 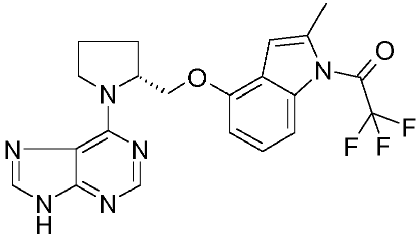 |
| | 2,2,2-Trifluoro-1-{2-méthyl-4-[(R)-1-(9H-purin-6-yl)pyrrolidin-2-ylméthoxy]indol-1-yl}éthanone |
| « A77 » | 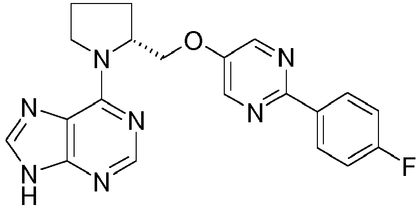 |
| | 6-{(R)-2-[2-(4-Fluorophényl)pyrimidin-5-yloxyméthyl]-pyrrolidin-1-yl}-9H-purine |
| « A80 » | 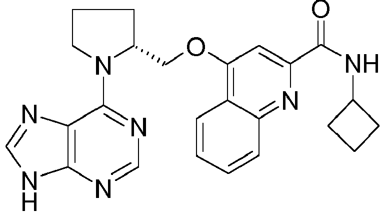 |
| | N-Cyclobutyl-4-[(R)-1-(9H-purin-6-yl )pyrrolidin-2-ylméthoxy]quinoléine-2-carboxamide |
| « A81 » | 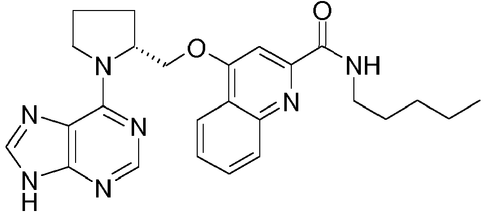 |
| | N-Pentyl-4-[(R)-1-(9H-purin-6-yl)pyrrolidin-2-ylméthoxy]quinoléine-2-carboxamide |
| « A82 » | 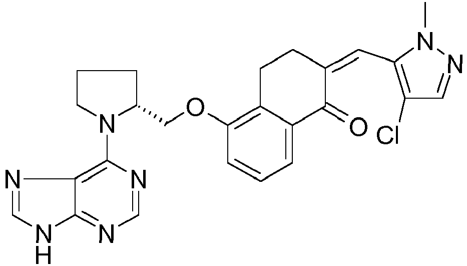 |
| | 2-[1-(4-Chloro-2-méthyl-2H-pyrazol-3-yl)méth-(Z)-ylidène]-5-[(R)-1-(9H-purin-6-yl)pyrrolidin-2-ylméthoxy]-3,4-dihydro-2H-naphtalén-1-one |
| « A83 » | 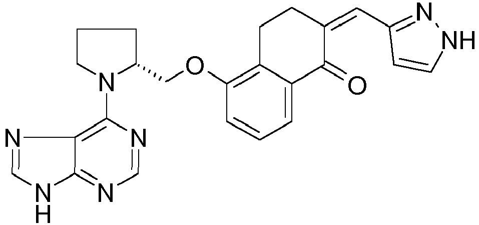 |
| | 5-[(R)-1-(9H-Purin-6-yl)pyrrolidin-2-ylméthoxy]-2-[1-(1H-pyrazol-3-yl)méth-(Z)-ylidène]-3,4-dihydro-2H-naphtalén-1-one |
| « A84 » | 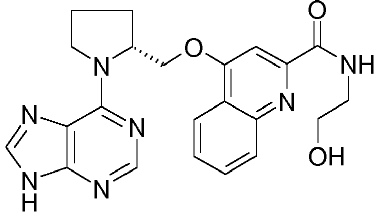 |
| | N-(2-Hydroxyéthyl)-4-[(R)-1-(9H-purin-6-yl)pyrrolidin-2-ylméthoxy]quinoléine-2-carboxamide |
| « A85 » | 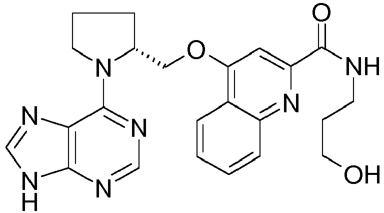 |
| | N-(3-Hydroxypropyl)-4-[(R)-1-(9H-purin-6-yl)pyrrolidin-2-ylméthoxy]quinoléine-2-carboxamide |
| « A86 » | 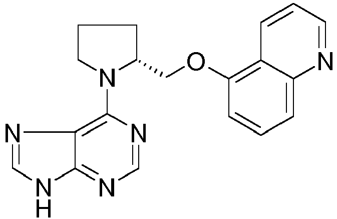 |
| | 5-[(R)-1-(9H-Purin-6-yl)pyrrolidin-2-ylméthoxy]quinoléine |
| « A87 » | 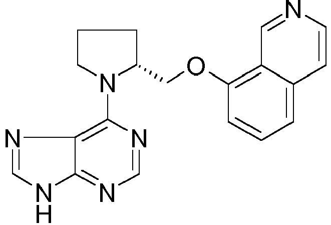 |
| | 8-[(R)-1-(9H-Purin-6-yl)pyrrolidin-2-ylméthoxy]isoquinoléine |
| « A88 » | 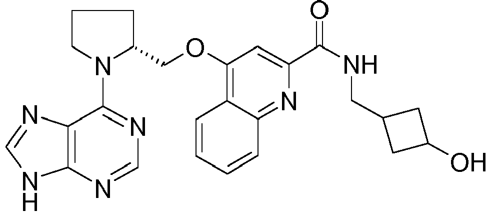 |
| | N-(3-Hydroxycyclobutylméthyl)-4-[(R)-1-(9H-purin-6-yl)-pyrrol idin-2-ylméthoxy]quinoléine-2-carboxamide |
| « A89 » | 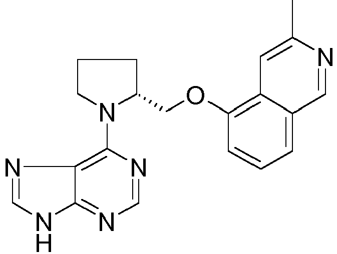 |
| | 3-Méthyl-5-[(R)-1-(9H-purin-6-yl)pyrrolidin-2-ylméthoxy]isoquinoléine |
| « A92 » | N-(2-Morpholin-4-yléthyl)-N-{4-[(R)-1-(9H-purin-6-yl)pyrrolidin-2-ylméthoxy]naphtalén-1-ylméthyl}acétannide |
| « A93 » | 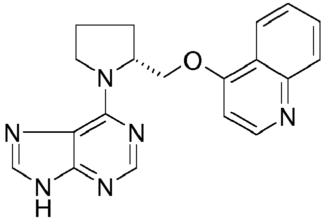 |
| | 4-[(R)-1-(9H-Purin-6-yl)pyrrolidin-2-ylméthoxy]quinoléine |
| « A94 » | 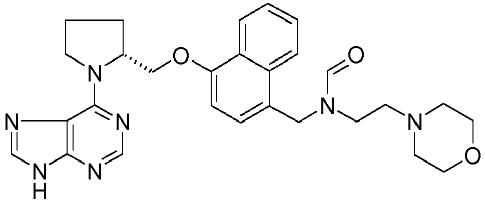 |
| | N-(2-Morpholin-4-yléthyl)-N-{4-[(R)-1-(9H-purin-6-yl)pyrrolidin-2-ylméthoxy]naphtalén-1-ylméthyl}formannide |
| « A95 » | 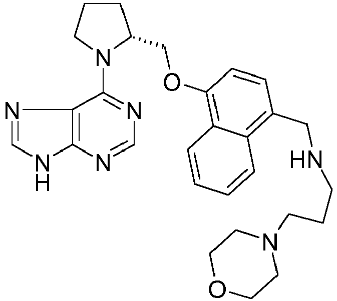 |
| | (3-Morpholin-4-ylpropyl)-{4-[(R)-1-(9H-purin-6-yl)pyrrolidin-2-ylméthoxy]naphtalén-1-ylméthyl}amine |
| « A96 » | 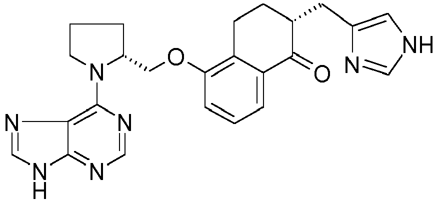 |
| | (R)-2-(1H-Imidazol-4-ylméthyl)-5-[(R)-1-(9H-purin-6-yl)pyrrolidin-2-ylméthoxy]-3,4-dihydro-2H-naphtalén-1-one |
| « A97 » | 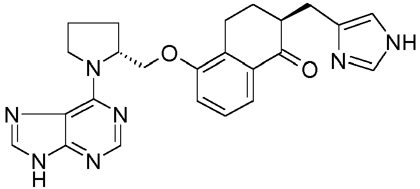 |
| | (S)-2-(1H-Imidazol-4-ylméthyl)-5-[(R)-1-(9H-purin-6-yl)-pyrrol id in-2-ylméthoxy]-3,4-d ihydro-2H-naphtalén-1-one |
| « A98 » | 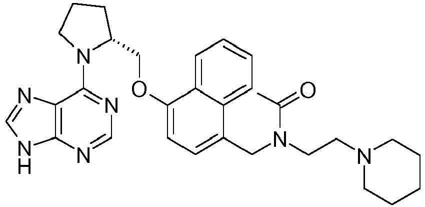 |
| | N-(2-Pipéridin-1-yléthyl)-N-{4-[(R)-1-(9H-purin-6-yl)pyrrolidin-2-ylméthoxy]naphtalén-1-ylméthyl}acétannide |
| « A99 » | 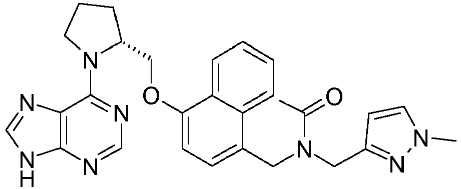 |
| | N-(1-Méthyl-1H-pyrazol-3-ylméthyl)-N-{4-[(R)-1-(9H-purin-6-yl)pyrrolidin-2-ylméthoxy]naphtalén-1-ylméthyl}acétannide |
| « A100 » | 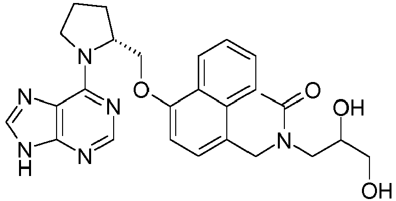 |
| | N-(2,3-Dihydroxypropyl)-N-{4-[(R)-1-(9H-purin-6-yl)-pyrrolidin-2-ylméthoxy]naphtalén-1-ylméthyl}acétamide |
| « A101 » | 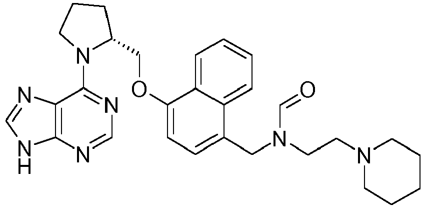 |
| | N-(2-Pipéridin-1-yléthyl)-N-{4-[(R)-1-(9H-purin-6-yl)pyrrolidin-2-ylméthoxy]naphtalén-1-ylméthyl}formannide |
| « A102 » | 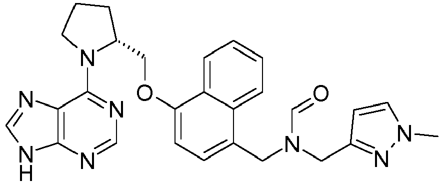 |
| | N-(1-Méthyl-1H-pyrazol-3-ylméthyl)-N-{4-[(R)-1-(9H-purin-6-yl)pyrrolidin-2-ylméthoxy]naphtalén-1-ylméthyl}formannide |
| « A103 » | 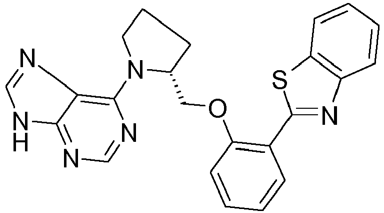 |
| | 6-[(R)-2-(2-Benzothiazol-2-yl phénoxyméthyl )pyrrolidin-1-yl]-9H-purine |
| « A104 » | 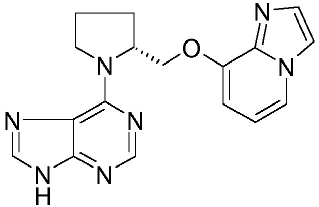 |
| | 6-[(R)-2-(Imidazo[1,2-a]pyridin-8-yloxyméthyl)pyrrolidin-1-yl]-9H-purine |
| « A105 » | 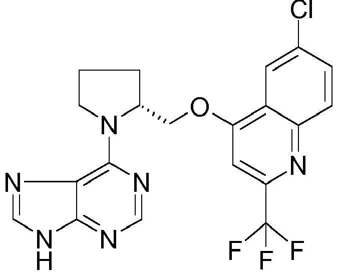 |
| | 6-Chloro-4-[(R)-1-(9H-purin-6-yl)pyrrolidin-2-ylméthoxy]-2-trifluorométhylquinoléine |
| « A106 » | 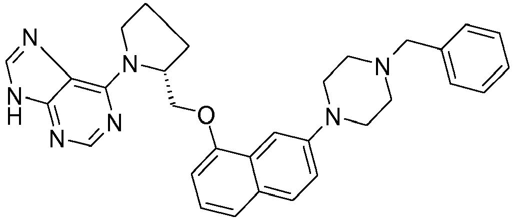 |
| | 6-{(R)-2-[7-(4-Benzylpipérazin-1-yl)naphtalén-1-yloxyméthyl]pyrrolidin-1-yl}-9H-purine |
| « A107 » | 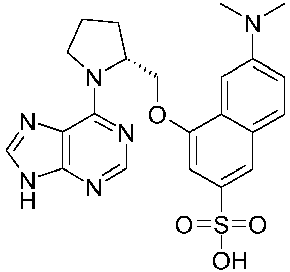 |
| | Acide 6-diméthylamino-4-[(R)-1-(9H-purin-6-yl)pyrrolidin-2-ylméthoxy]naphtalène-2-sulfonique |
| « A108 » | 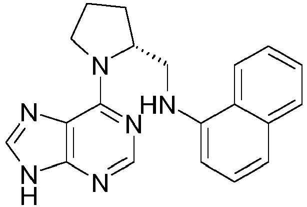 |
| | Naphtalén-1-yl [(R)-1-(9H-purin-6-yl)pyrrolidin-2-ylméthyl]amine |
| « A109 » | 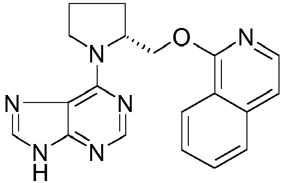 |
| | 1-[(R)-1-(9H-Purin-6-yl)pyrrolidin-2-ylméthoxy]isoquinoléine |
| « A110 » | 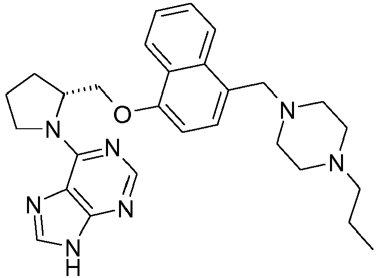 |
| | 6-{(R)-2-[4-(4-Propylpipérazin-1 -ylméthyl)naphtalén-1-yloxyméthyl]pyrrolidin-1-yl}-9H-purine |
| « A111 » | 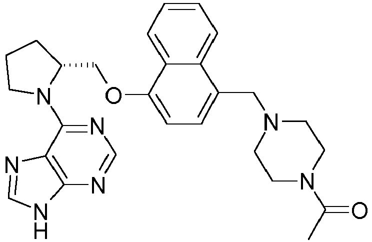 |
| | 1-(4-{4-[(R)-1-(9H-Purin-6-yl)pyrrolidin-2-ylméthoxy]-naphtalén-1-ylméthyl}pipérazin-1-yl)éthanone |
| « A112 » | 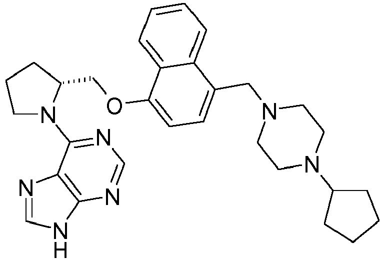 |
| | 6-{(R)-2-[4-(4-Cyclopentyl pipérazin-1-yl méthyl)naphtalén-1-yloxyméthyl]pyrrolidin-1-yl}-9H-purine |
| « A113 » | 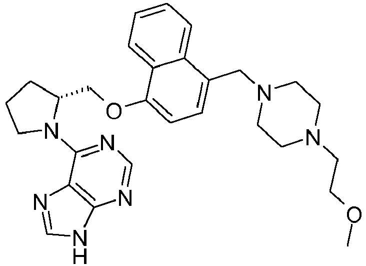 |
| | 6-((R)-2-{4-[4-(2-Méthoxyéthyl)pipérazin-1-ylméthyl]-naphtalén-1-yloxyméthyl}pyrrolidin-1-yl)-9H-purine |
| « A114 » | 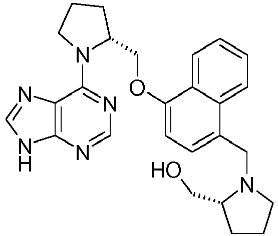 |
| | ((R)-1-{4-[(R)-1-(9H-Purin-6-yl)pyrrolidin-2-ylméthoxy]-naphtalén-1-ylméthyl}pyrrolidin-2-yl)méthanol |
| « A115 » | 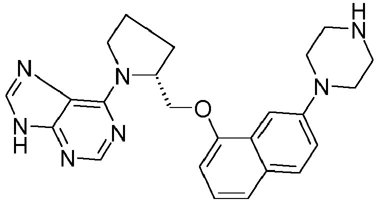 |
| | 6-[(R)-2-(7-Pipérazin-1-ylnaphtalén-1-yloxyméthyl)pyrrolidin-1-yl]-9H-purine |
| « A116 » | 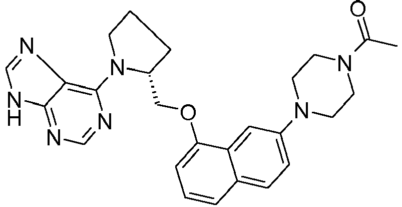 |
| | 1-(4-{8-[(R)-1 -(9H-Purin-6-yl)pyrrolidin-2-ylméthoxy]-naphtalén-2-yl}pipérazin-1-yl)éthanone |
| « A117 » | 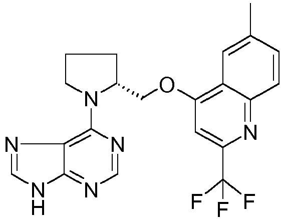 |
| | 6-Méthyl-4-[(R)-1-(9H-purin-6-yl)pyrrolidin-2-ylméthoxy]-2-trifluorométhylquinoléine |
| « A118 » | 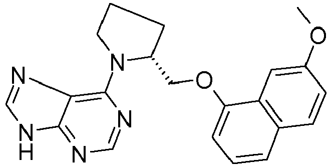 |
| | 6-[(R)-2-(7-Méthoxynaphtalén-1-yloxyméthyl)pyrrolidin-1-yl]-9H-purine |
| « A119 » | 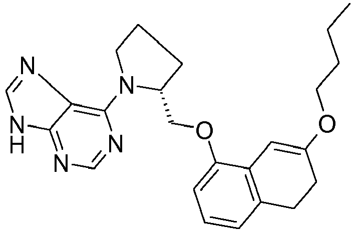 |
| | 6-[(R)-2-(7-Butoxy-5,6-dihydronaphtalén-1-yloxyméthyl)-pyrrolidin-1-yl]-9H-purine |
| « A120 » | 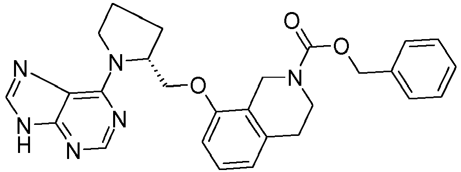 |
| | 8-[(R)-1-(9H-Purin-6-yl)pyrrolidin-2-ylméthoxy]-3,4-dihydro-1H-isoquinoléine-2-carboxylate de benzyle |
| « A121 » | 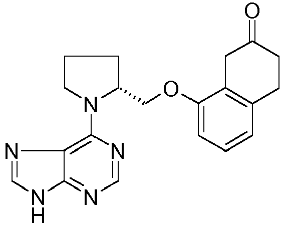 |
| | 8-[(R)-1-(9H-Purïn-6-yl)pyrrolidin-2-ylméthoxy]-3,4-dihydro-1H-naphtalén-2-one |
| « A122 » | 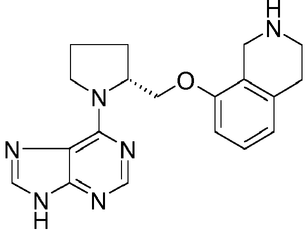 |
| | 8-[(R)-1-(9H-Purin-6-yl)pyrrolidin-2-ylméthoxy]-1,2,3,4-tétrahydroisoquinoléine |
| « A123 » | 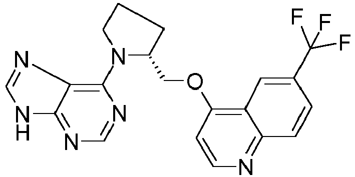 |
| | 4-[(R)-1-(9H-Purin-6-yl)pyrrolidin-2-ylméthoxy]-6-trifluorométhylquinoléine |
| « A124 » | 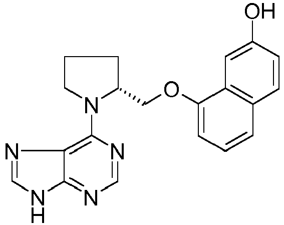 |
| | 8-[(R)-1-(9H-Purin-6-yl)pyrrolidin-2-ylméthoxy]naphtalén-2-ol |
| « A125 » | 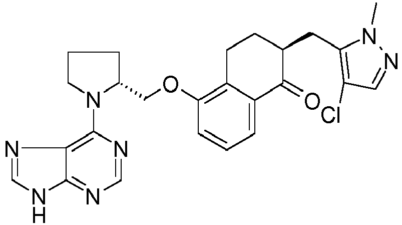 |
| | (S)-2-(4-Chloro-2-méthyl-2H-pyrazol-3-ylméthyl)-5-[(R)-1-(9H-purin-6-yl)pyrrolidin-2-ylméthoxy]-3,4-dihydro-2H-naphtalén-1-one |
| « A126 » | 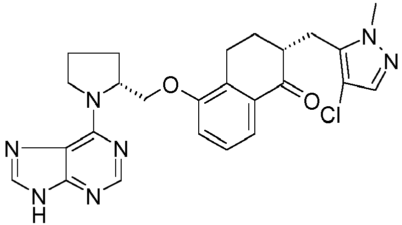 |
| | (R)-2-(4-Chloro-2-méthyl-2H-pyrazol-3-ylméthyl)-5-[(R)-1-(9H-purin-6-yl)pyrrolidin-2-ylméthoxy]-3,4-dihydro-2H-naphtalén-1-one |
| « A127 » | 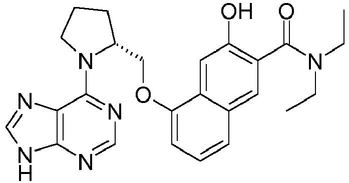 |
| | N,N-Diéthyl-3-hydroxy-5-[(R)-1-(9H-purin-6-yl)pyrrolidin-2-ylméthoxy]naphtalène-2-carboxamide |
| « A128 » | 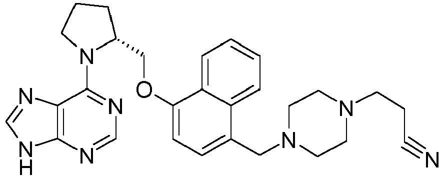 |
| | 3-(4-{4-[(R)-1-(9H-Purin-6-yl)pyrrolidin-2-ylméthoxy]-naphtalén-1-ylméthyl}pipérazin-1-yl)propionitrile |
| « A129 » | 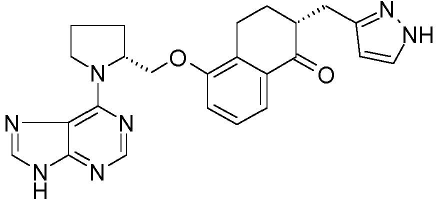 |
| | (R)-5-[(R)-1-(9H-Purin-6-yl)pyrrolidin-2-ylméthoxy]-2-(1H-pyrazol-3-ylméthyl)-3,4-dihydro-2H-naphtalén-1-one |
| « A130 » | 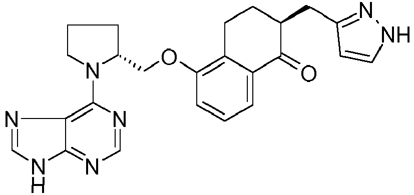 |
| | (S)-5-[(R)-1-(9H-Purin-6-yl)pyrrolidin-2-ylméthoxy]-2-(1H-pyrazol-3-ylméthyl)-3,4-dihydro-2H-naphtalén-1-one |
| « A131 » | 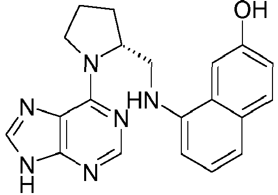 |
| | 8-{[(R)-1-(9H-Purin-6-yl)pyrrolidin-2-ylméthyl]-amino}naphtalén-2-ol |
| « A132 » | 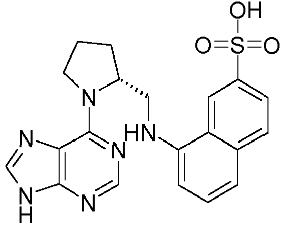 |
| | Acide 8-{[(R)-1-(9H-purin-6-yl)pyrrolidin-2-ylméthyl]amino}naphtalène-2-sulfonique |
| « A133 » | 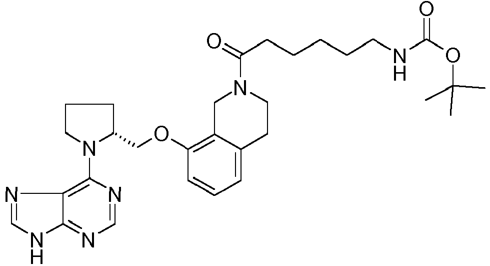 |
| | (6-oxo-6-{8-[(R)-1-(9H-Purin-6-yl)pyrrolidin-2-ylméthoxy]-3,4-dihydro-1H-isoquinoléin-2-yl}hexyl)carbamate de tertio-butyle |
| « A134 » | 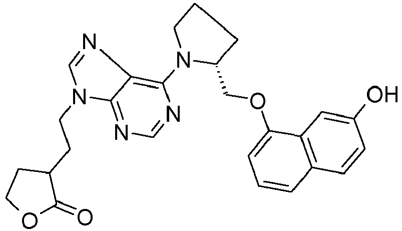 |
| | 3-(2-{6-[(R)-2-(7-Hydroxynaphtalén-1-yloxyméthyl)pyrrolidin-1-yl]purin-9-yl}éthyl)dihydrofuran-2-one |
| « A135 » | 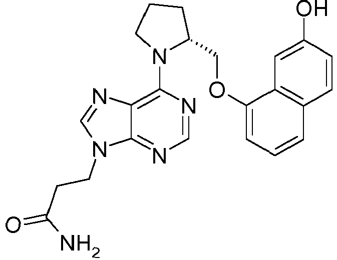 |
| | 3-{6-[(R)-2-(7-Hydroxynaphtalén-1-yloxyméthyl)pyrrolidin-1-yl]purin-9-yl}propionamide |
| « A136 » | 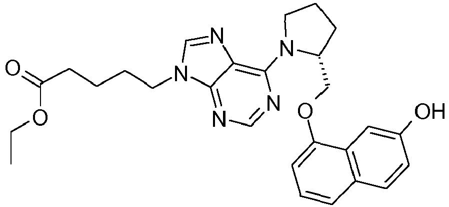 |
| | 5-{6-[(R)-2-(7-Hydroxynaphtalén-1-yloxyméthyl)pyrrolidin-1-yl]purin-9-yl}pentanoate d'éthyle |
| « A137 » | 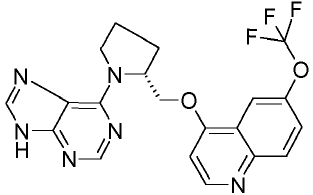 |
| | 4-[(R)-1-(9H-Purin-6-yl)pyrrolidin-2-ylméthoxy]-6-trifluorométhoxyquinoléine |
| « A138 » | 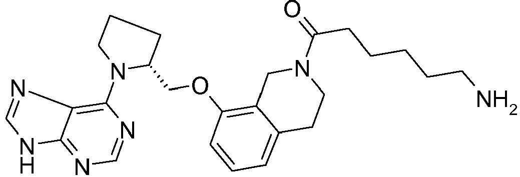 |
| | 6-Amino-1-{8-[(R)-1-(9H-purin-6-yl)pyrrolidin-2-ylméthoxy]-3,4-dihydro-1H-isoquinoléin-2-yl}hexan-1-one |
| « A139 » | 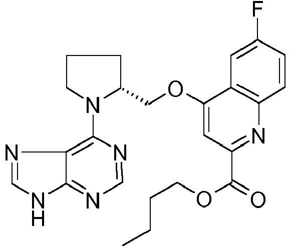 |
| | 6-Fluoro-4-[(R)-1-(9H-purin-6-yl)pyrrolidin-2-ylméthoxy]quinoléine-2-carboxylate de butyle |
| « A140 » | 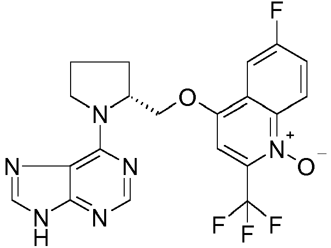 |
| | 1-Oxyde de 6-fluoro-4-[(R)-1-(9H-purin-6-yl)pyrrolidin-2-ylméthoxy]-2-trifluorométhylquinoléine |
| « A141 » | 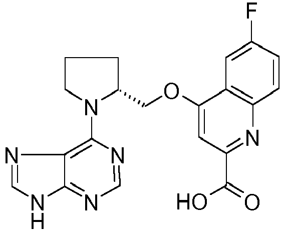 |
| | Acide 6-fluoro-4-[(R)-1-(9H-purin-6-yl)pyrrolidin-2-ylméthoxy]quinoléine-2-carboxylique |
| « A142 » | 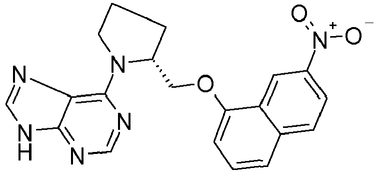 |
| | 6-[(R)-2-(7-Nitronaphtalén-1-yloxyméthyl)pyrrolidin-1-yl]-9H-purine |
| « A144 » | 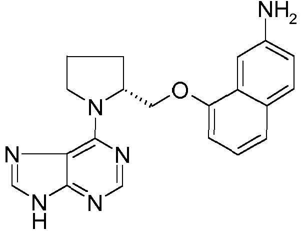 |
| | 8-[(R)-1-(9H-Purin-6-yl)pyrrolidin-2-ylméthoxy]naphtalén-2-ylamine |
| « A145 » | 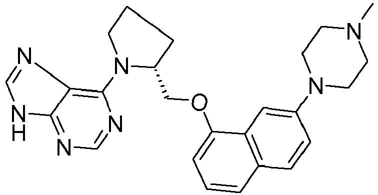 |
| | 6-{(R)-2-[7-(4-Méthylpipérazin-1-yl)naphtalén-1-yloxyméthyl]pyrrolidin-1-yl}-9H-purine |
| « A146 » | 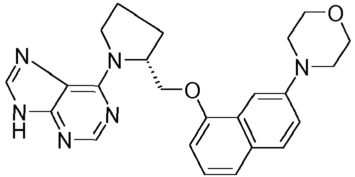 |
| | 6-[(R)-2-(7-Morpholin-4-ylnaphtalén-1-yloxyméthyl)pyrrolidin-1-yl]-9H-purine |
| « A147 » | 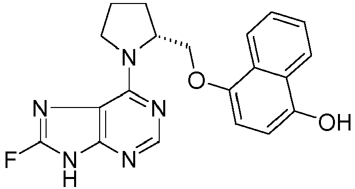 |
| | 4-[(R)-1-(8-Fluoro-9H-purin-6-yl)pyrrolidin-2-ylméthoxy]naphtalén-1-ol |
| « A148 » | 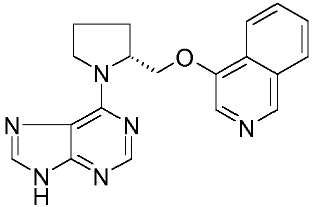 |
| | 4-[(R)-1-(9H-Purin-6-yl)pyrrolidin-2-ylméthoxy]isoquinoléine |
| « A149 » | 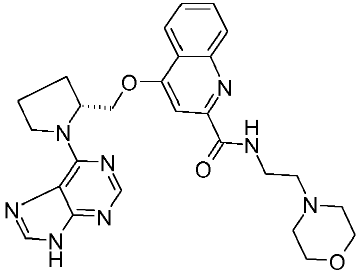 |
| | N-(2-Morpholin-4-yléthyl)-4-[(R)-1-(9H-purin-6-yl)pyrrolidin-2-ylméthoxy]quinoléine-2-carboxamide |
| « A150 » | 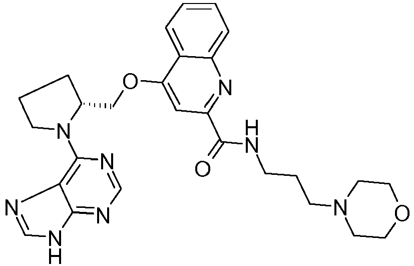 |
| | N-(3-Morpholin-4-ylpropyl)-4-[(R)-1-(9H-purin-6-yl)pyrrolidin-2-ylméthoxy]quinoléine-2-carboxamide |
| « A151 » | 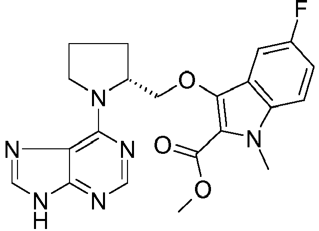 |
| | 5-Fluoro-1-méthyl-3-[(R)-1-(9H-purin-6-yl)pyrrolidin-2-ylméthoxy]-1H-indole-2-carboxylate de méthyle |
| « A152 » | 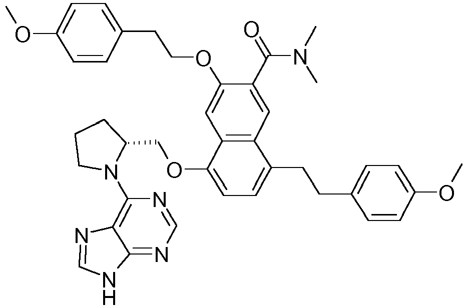 |
| | N,N-Diméthyl-3-[2-(4-méthoxyphényl)éthoxy]-8-[2-(4-méthoxyphényl)éthyl]-5-[(R)-1-(9H-purin-6-yl)pyrrolidin-2-ylméthoxy]naphtalène-2-carboxamide |
| « A153 » | 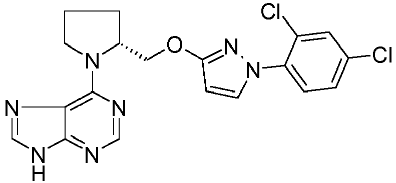 |
| | 6-{(R)-2-[1-(2,4-Dichlorophényl)-1H-pyrazol-3-yloxyméthyl]pyrrolidin-1-yl}-9H-purine |
| « A154 » | 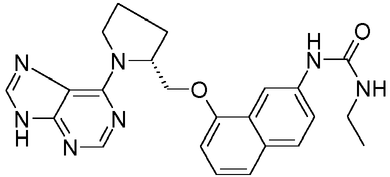 |
| | 1-Éthyl-3-{8-[(R)-1-(9H-purin-6-yl)pyrrolidin-2-ylméthoxy]naphtalén-2-yl}urée |
| « A155 » | 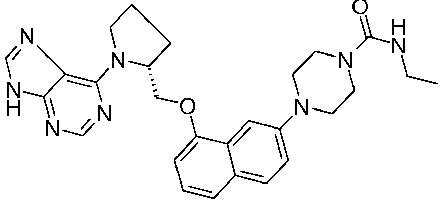 |
| | N-Éthyl-4-{8-[(R)-1-(9H-purin-6-yl )pyrrolidin-2-ylméthoxy]naphtalén-2-yl}pipérazine-1-carboxamide |
| « A156 » | 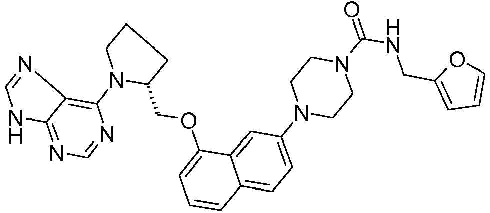 |
| | N-(Furan-2-ylméthyl)-4-{8-[(R)-1-(9H-purin-6-yl)pyrrolidin-2-ylméthoxy]naphtalén-2-yl}pipérazine-1-carboxamide |
| « A157 » | 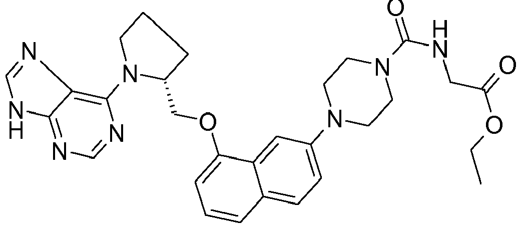 |
| | [(4-{8-[(R)-1-(9H-Purin-6-yl)pyrrolidin-2-ylméthoxy]naphtalén-2-yl}pipérazine-1-carbonyl)amino]acétate d'éthyle |
| « A158 » | 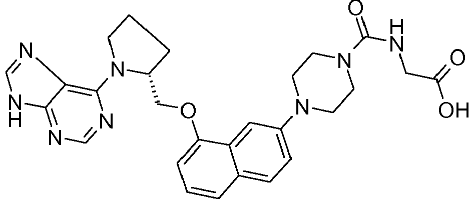 |
| | Acide [(4-{8-[(R)-1-(9H-purin-6-yl)pyrrolidin-2-ylméthoxy]-naphtalén-2-yl}pipérazine-1-carbonyl)amino]acétique |
| « A159 » | 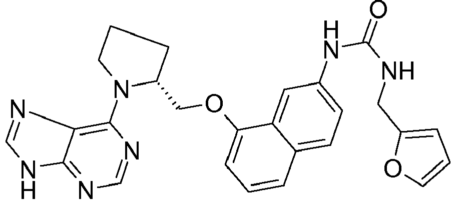 |
| | 1-Furan-2-ylméthyl-3-{8-[(R)-1-(9H-purin-6-yl)pyrrolidin-2-ylméthoxy]naphtalén-2-yl}urée |
| « A160 » | 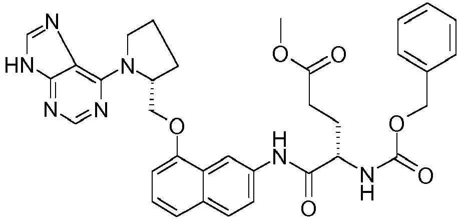 |
| | (S)-4-Benzyloxycarbonylamino-4-{8-[(R)-1-(9H-purin-6-yl)-pyrrolidin-2-ylméthoxy]naphtalén-2-ylcarbamoyl}butyrate de méthyle |
| « A161 » | 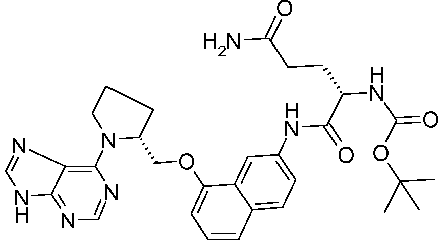 |
| | ((S)-3-Carbamoyl-1-{8-[(R)-1-(9H-purin-6-yl)pyrrolidin-2-ylméthoxy]naphtalén-2-ylcarbamoyl}propyl)carbamate de tertio-butyle |
| « A162 » | 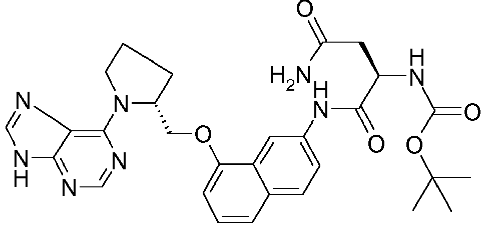 |
| | ((R)-2-Carbamoyl-1-{8-[(R)-1-(9H-purin-6-yl)pyrrolidin-2-ylméthoxy]naphtalén-2-ylcarbamoyl}éthyl)carbamate de tertio-butyle |
| « A163 » | 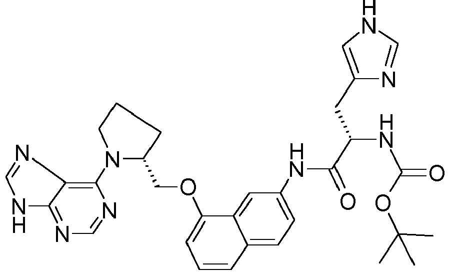 |
| | ((S)-2-(1H-Imidazol-4-yl)-1-{8-[(R)-1-(9H-purin-6-yl)-pyrrolidin-2-ylméthoxy]naphtalén-2-ylcarbamoyl}éthyl)carbamate de tertio-butyle |
| « A164 » | 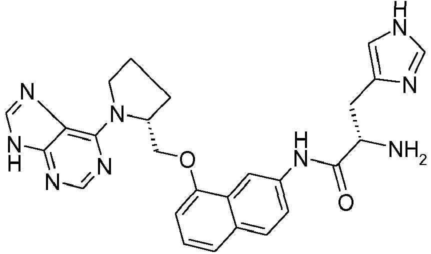 |
| | (S)-2-Amino-3-(1H-imidazol-4-yl)-N-{8-[(R)-1-(9H-purin-6-yl)pyrrolidin-2-ylméthoxy]naphtalén-2-yl}propionamide |
| « A165 » | 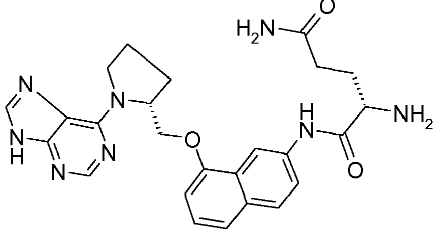 |
| | N-(1-({8-[(R)-1-(9H-Purin-6-yl)pyrrolidin-2-ylméthoxy]naphtalén-2-yl})-(S)-2-aminopentan-5-amide |
| « A166 » | 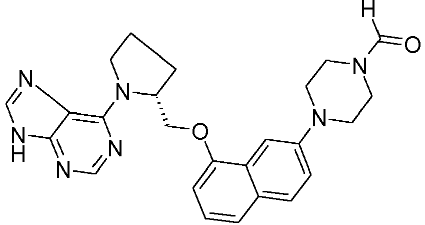 |
| | 4-{8-[(R)-1-(9H-Purin-6-yl)pyrrolidin-2-ylméthoxy]naphtalén-2-yl}pipérazine-1-carbaldéhyde |
| « A167 » | 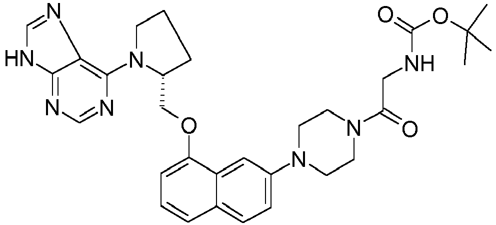 |
| | [2-Oxo-2-(4-{8-[(R)-1-(9H-purin-6-yl)pyrrolidin-2-yl-méthoxy]naphtalén-2-yl}pipérazin-1-yl)éthyl]carbamate de tertio-butyle |
| « A168 » | 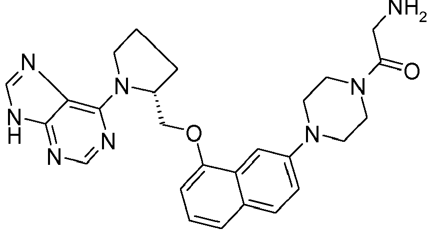 |
| | 2-Amino-1-(4-{8-[(R)-1-(9H-purin-6-yl)pyrrolidin-2-ylméthoxy]naphtalén-2-yl}pipérazin-1-yl)éthanone |
| « A169 » | 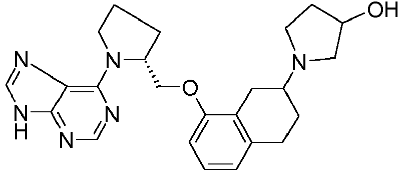 |
| | 1-{8-[(R)-1-(9H-Purin-6-yl)pyrrolidin-2-ylméthoxy]-1,2,3,4-tétrahydronaphtalén-2-yl}pyrrolidin-3-ol |
| « A170 » | 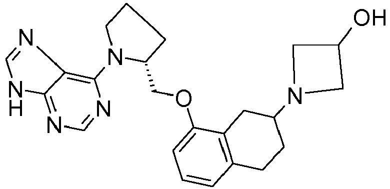 |
| | 1-{8-[(R)-1-(9H-Purin-6-yl)pyrrolidin-2-ylméthoxy]-1,2,3,4-tétrahydronaphtalén-2-yl}azétidin-3-ol |
| « A171 » | 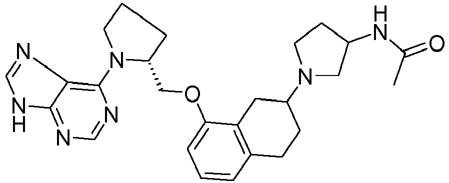 |
| | N-(1-{8-[(R)-1-(9H-Purin-6-yl)pyrrolidin-2-ylméthoxy]-1,2,3,4-tétrahydronaphtalén-2-yl}pyrrolidin-3-yl)acétamide |
| « A172 » | 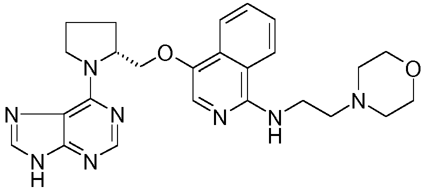 |
| | (2-Morpholin-4-yléthyl)-{4-[(R)-1-(9H-purin-6-yl)pyrrolidin-2-ylméthoxy]isoquinoléin-1-yl}amine |
| « A173 » | 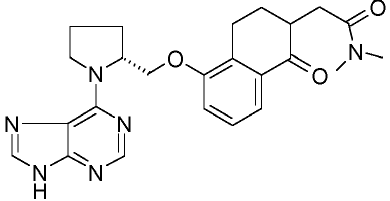 |
| | N,N-Diméthyl-2-{1-oxo-5-[(R)-1-(9H-purin-6-yl)pyrrolidin-2-ylméthoxy]-1,2,3,4-tétrahydronaphtalén-2-yl}acétamide |
| « A174 » | 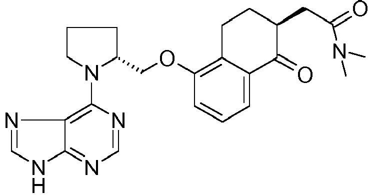 |
| | N, N-Diméthyl-2-{(S)-1-oxo-5-[(R)-1-(9H-purin-6-yl)pyrrolidin-2-ylméthoxy]-1,2,3,4-tétrahydronaphtalén-2-yl}acétamide |
| « A175 » | 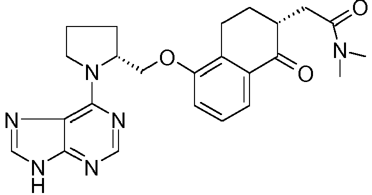 |
| | N,N-Diméthyl-2-{(R)-1-oxo-5-[(R)-1-(9H-purin-6-yl)pyrrolidin-2-ylméthoxy]-1,2,3,4-tétrahydronaphtalén-2-yl}acétamide |
| « A176 » | 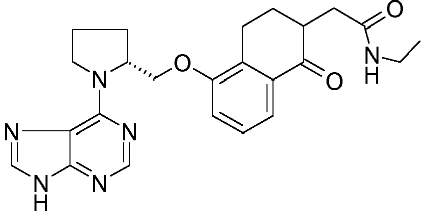 |
| | N-Éthyl-2-{1-oxo-5-[(R)-1-(9H-purin-6-yl)pyrrolidin-2-ylméthoxy]-1,2,3,4-tétrahydronaphtalén-2-yl}acétamide |
| « A177 » | 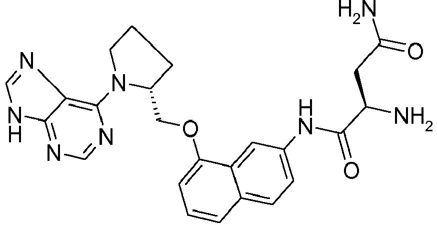 |
| | (R)-2-Amino-N1-{8-[(R)-1-(9H-purin-6-yl)pyrrolidin-2-ylméthoxy]naphtalén-2-yl}succinamide |
| « A178 » | 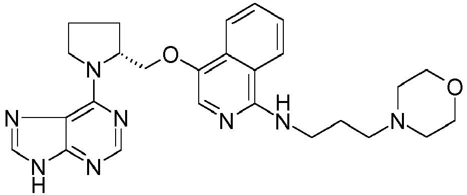 |
| | (3-Morpholin-4-ylpropyl)-{4-[(R)-1-(9H-purin-6-yl)pyrrolidin-2-ylméthoxy]isoquinoléin-1-yl}amine |
| « A179 » | 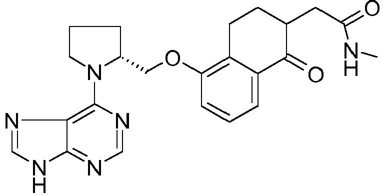 |
| | N-Méthyl-2-{1-oxo-5-[(R)-1-(9H-purin-6-yl)pyrrolidin-2-ylméthoxy]-1,2,3,4-tétrahydronaphtalén-2-yl}acétamide |
| « A180 » | 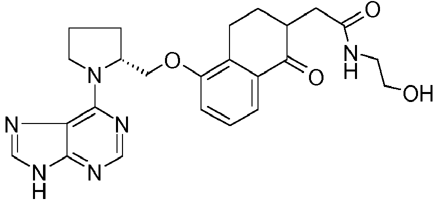 |
| | N-(2-Hydroxyéthyl)-2-{1-oxo-5-[(R)-1-(9H-purin-6-yl)-pyrrolidin-2-ylméthoxy]-1,2,3,4-tétrahydronaphtalén-2-yl}acétamide |
| « A181 » | 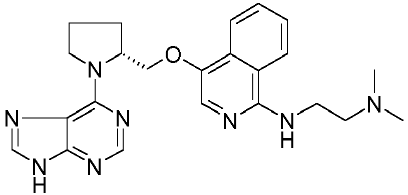 |
| | N,N-Diméthyl-N'-{4-[(R)-1-(9H-purin-6-yl)pyrrolidin-2-ylméthoxy]isoquinoléin-1-yl}éthane-1,2-diamine |
| « A182 » | 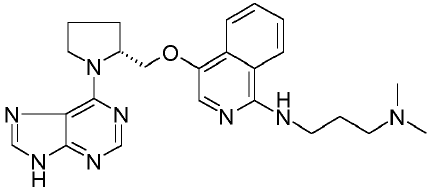 |
| | N,N-Diméthyl-N'-{4-[(R)-1-(9H-purin-6-yl)pyrrolidin-2-ylméthoxy]isoquinoléin-1-yl}propane-1,3-diamine |
| « A183 » | 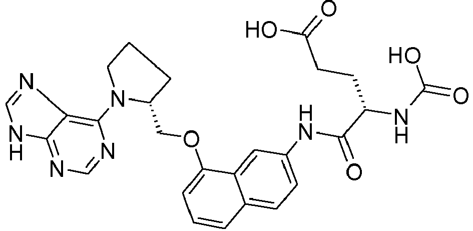 |
| | Acide (S)-4-carboxyamino-4-{8-[(R)-1-(9H-purin-6-yl)-pyrrolidin-2-ylméthoxy]naphtalén-2-ylcarbamoyl}butyrique |
| « A184 » | 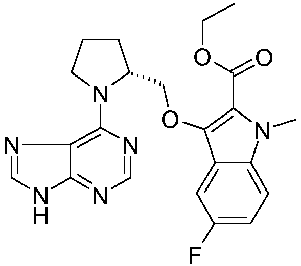 |
| | 5-Fluoro-1-méthyl-3-[(R)-1-(9H-purin-6-yl)pyrrolidin-2-ylméthoxy]-1H-indole-2-carboxylate d'éthyle |
| « A185 » | 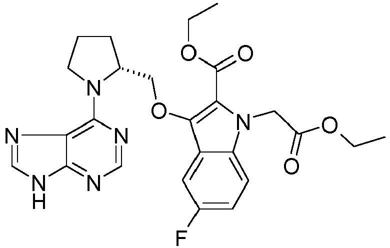 |
| | 1-Ethoxycarbonylméthyl-5-fluoro-3-[(R)-1-(9H-purin-6-yl)pyrrolidin-2-ylméthoxy]-1H-indole-2-carboxylate d'éthyle |
| « A186 » | 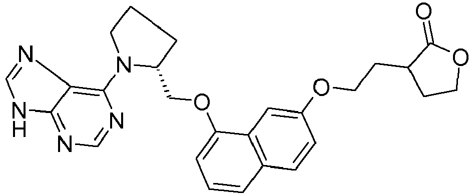 |
| | 3-(2-{8-[(R)-1-(9H-Purin-6-yl)pyrrolidin-2-yl-méthoxy]naphtalén-2-yloxy}éthyl)dihydrofuran-2-one |
| « A187 » | 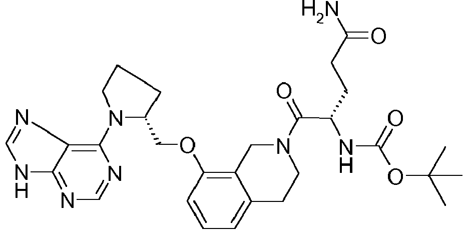 |
| | ((S)-3-Carbamoyl-1-{8-[(R)-1-(9H-purin-6-yl)pyrrolidin-2-ylméthoxy]-3,4-dihydro-1H-isoquinoléine-2-carbonyl}propyl)carbamate de tertio-butyle |
| « A188 » | 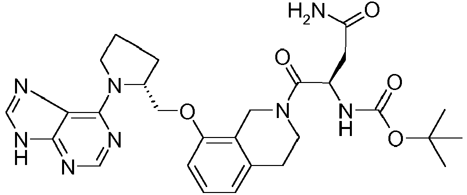 |
| | ((R)-1-Carbamoylméthyl-2-oxo-2-{8-[(R)-1-(9H-purin-6-yl)pyrrolidin-2-ylméthoxy]-3,4-dihydro-1H-isoquinoléin-2-yl}éthyl)carbamate de tertio-butyle |
| « A189 » | 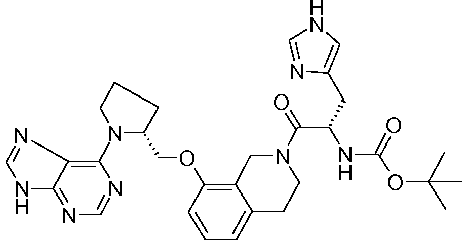 |
| | ((S)-1-(1H-Imidazol-4-ylméthyl)-2-oxo-2-{8-[(R)-1-(9H-purin-6-yl)pyrrolidin-2-ylméthoxy]-3,4-dihydro-1H-isoqu inoléin-2-yl}éthyl)carbamate de tertio-butyle |
| « A190 » | 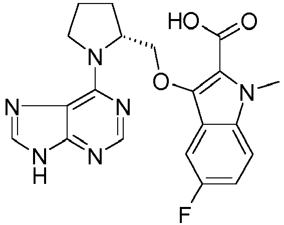 |
| | Acide 5-fluoro-1-méthyl-3-[(R)-1-(9H-purin-6-yl)pyrrolidin-2-ylméthoxy]-1H-indole-2-carboxylique |
| « A191 » | 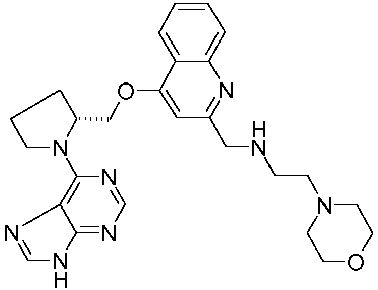 |
| | (2-Morpholin-4-yléthyl)-{4-[(R)-1-(9H-purin-6-yl)pyrrolidin-2-ylméthoxy]quinoléin-2-ylméthyl}amine |
| « A192 » | 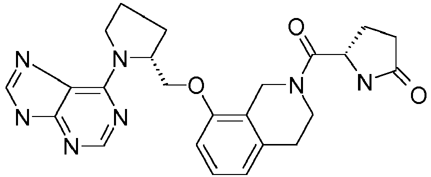 |
| | (S)-5-{8-[(R)-1-(9H-purin-6-yl)pyrrolidin-2-ylméthoxy]-3,4-dihydro-1H-isoquinoléine-2-carbonyl}pyrrolidin-2-one |
| « A193 » | 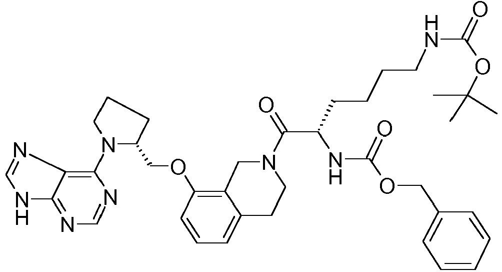 |
| | ((S)-5-Benzyloxycarbonylamino-6-oxo-6-{8-[(R)-1-(9H-purin-6-yl)pyrrolidin-2-ylméthoxy]-3,4-dihydro-1H-isoquinoléin-2-yl}hexyl)carbamate de tertio-butyle |
| « A194 » | 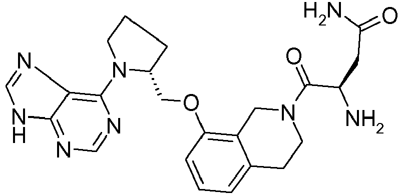 |
| | (R)-3-Amino-4-oxo-4-{8-[(R)-1-(9H-purin-6-yl)pyrrolidin-2-ylméthoxy]-3,4-dihydro-1H-isoquinoléin-2-yl}butyramide |
| « A195 » | 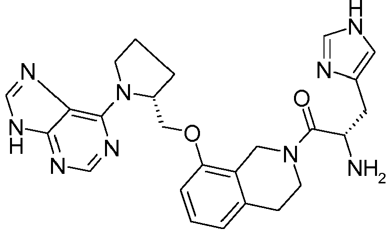 |
| | (S)-2-Amino-3-(1H-imidazol-4-yl)-1-{8-[(R)-1-(9H-purin-6-yl)pyrrolidin-2-ylméthoxy]-3,4-dihydro-1H-isoquinoléin-2-yl}propan-1-one |
| « A196 » | 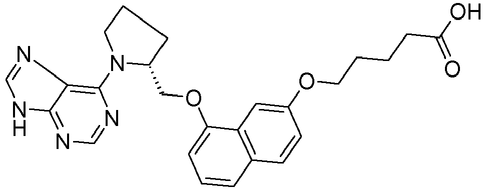 |
| | Acide 5-{8-[(R)-1-(9H-purin-6-yl)pyrrolidin-2-ylméthoxy]naphtalén-2-yloxy}pentanoïque |
| « A197 » | 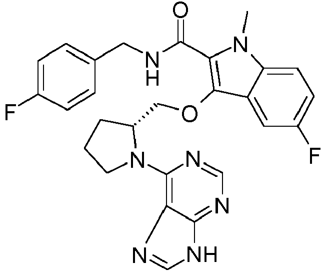 |
| | N-(4-Fluorobenzyl)-5-fluoro-1-méthyl-3-[(R)-1-(9H-purin-6-yl)pyrrolidin-2-ylméthoxy]-1H-indole-2-carboxamide |
| « A198 » | 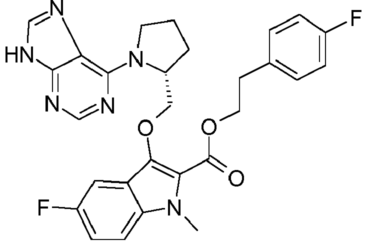 |
| | 5-Fluoro-1-méthyl-3-[(R)-1-(9H-purin-6-yl)pyrrolidin-2-ylméthoxy]-1H-indole-2-carboxylate de 2-(4-fluorophényl)éthyle |
| « A199 » | 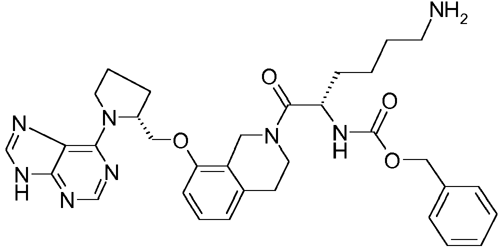 |
| | ((S)-5-Amino-1-{8-[(R)-1-(9H-purin-6-yl)pyrrolidin-2-ylméthoxy]-3,4-dihydro-1H-isoquinoléine-2-carbonyl}pentyl)carbamate de benzyle |
| « A200 » | 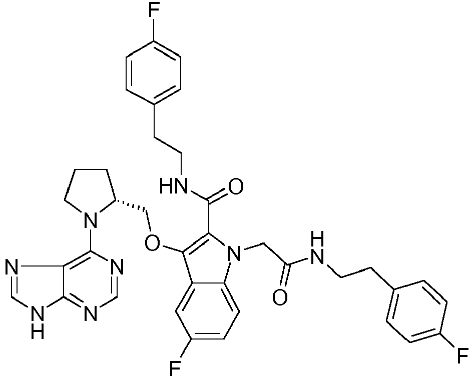 |
| | N-[2-(4-Fluorophényl)éthyl]-5-fluoro-1-{[2-(4-fluorophényl)éthylcarbamoyl]méthyl}-3-[(R)-1-(9H-purin-6-yl)pyrrolidin-2-ylméthoxy]-1H-indole-2-carboxamide |
| « A201 » | 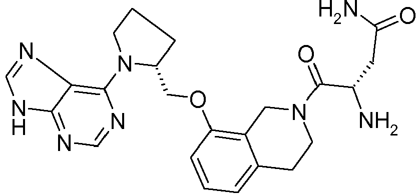 |
| | (S)-3-Amino-4-oxo-4-{8-[(R)-1-(9H-purin-6-yl)pyrrolidin-2-ylméthoxy]-3,4-dihydro-1H-isoquinoléin-2-yl}butyramide |
| « A202 » | 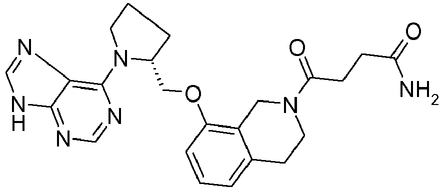 |
| | 4-Oxo-4-{8-[(R)-1-(9H-purin-6-yl)pyrrolidin-2-ylméthoxy]-3,4-dihydro-1H-isoquinoléin-2-yl}butyramide |
| « A203 » | 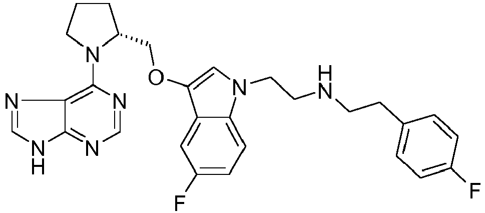 |
| | [2-(4-Fluorophényl)éthyl]-(2-{5-fluoro-3-[(R)-1-(9H-purin-6-yl)pyrrolidin-2-ylméthoxy]indol-1-yl}éthyl)amine |
ainsi que les sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1 ainsi que de sels, tautomères et stéréoisomères pharmaceutiquement utilisables de ceux-ci, y compris des mélanges de ceux-ci en toutes proportions, pour la préparation d'un médicament destiné au traitement de tumeurs, de métastases tumorales, de maladies prolifératives des cellules mésangiales, de l'hémangiome, de la rétinopathie proliférative, de la polyarthrite rhumatoïde, de la néovascularisation athérosclérotique, du psoriasis, de la néovascularisation oculaire, de l'ostéoporose, du diabète et de l'obésité, de la leucémie lymphoïde et du lymphome.

4. Utilisation selon la revendication 3, **caractérisée en ce que** la maladie tumorale est choisie dans le groupe de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, de la peau, la leucémie monocytique, l'adénocarcinome du poumon, le carcinome du poumon à petites cellules, le cancer du pancréas, le glioblastome, le carcinome du sein, la leucémie myéloïde aiguë, la leucémie myéloïde chronique, la leucémie lymphoïde aiguë, la leucémie lymphoïde chronique, le lymphome hodgkinien, le lymphome non hodgkinien.

5. Utilisation de composés selon la revendication 1 et/ou de sels physiologiquement acceptables de ceux-ci, pour la préparation d'un médicament destiné au traitement de tumeurs, où une quantité thérapeutiquement efficace d'un composé selon la revendication 1 est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

6. Utilisation de composés selon la revendication 1 et/ou de sels physiologiquement acceptables de ceux-ci, pour la préparation d'un médicament destiné au traitement de tumeurs, où une quantité thérapeutiquement efficace d'un composé selon la revendication 1 est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.
